(19) Europäisches Patentamt / European Patent Office / Office européen des brevets

(11) **EP 4 233 709 B1**

(12) **EUROPEAN PATENT SPECIFICATION**

(45) Date of publication and mention
of the grant of the patent:
**09.04.2025 Bulletin 2025/15**

(21) Application number: **23180318.0**

(22) Date of filing: **03.12.2018**

(51) International Patent Classification (IPC):
**G01S 5/02** *(2010.01)*     **G01S 5/22** *(2006.01)*
**A61B 5/08** *(2006.01)*     **H04L 25/02** *(2006.01)*
**G01S 5/06** *(2006.01)*     **A61B 5/113** *(2006.01)*
**A61B 5/00** *(2006.01)*

(52) Cooperative Patent Classification (CPC):
**G01S 5/0273; A61B 5/113; A61B 5/7253;
A61B 5/7264; G01S 5/02; G01S 5/0218;
G01S 5/22; H04L 25/0222;** A61B 5/7257;
A61B 5/7267; G01S 5/06; G16H 50/20;
H04L 25/0224

(54) **METHOD, APPARATUS, AND SYSTEM FOR PERIODIC MOTION DETECTION AND MONITORING**

VERFAHREN, VORRICHTUNG UND SYSTEM ZUR DETEKTION UND ÜBERWACHUNG PERIODISCHER BEWEGUNGEN

PROCÉDÉ, APPAREIL ET SYSTÈME DE DÉTECTION ET DE SURVEILLANCE DE MOUVEMENTS PÉRIODIQUES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 01.12.2017 US 201762593826 P
03.01.2018 US 201815861422
17.01.2018 US 201815873806
30.05.2018 US 201862678207 P
11.08.2018 US 201816101444
20.09.2018 US 201862734224 P
10.10.2018 US 201862744093 P
30.10.2018 US 201862753017 P
26.11.2018 US 201816200608
26.11.2018 US 201816200616
28.11.2018 US 201816203299
28.11.2018 US 201816203317

(43) Date of publication of application:
**30.08.2023 Bulletin 2023/35**

(62) Document number(s) of the earlier application(s) in
accordance with Art. 76 EPC:
**18209932.5 / 3 492 945**

(73) Proprietor: **ORIGIN RESEARCH WIRELESS, INC.**
**Rockville, MD 20852 (US)**

(72) Inventors:
• **CHEN, Chen**
**Rockville, 20852 (US)**
• **ZHANG, Feng**
**Rockville, 20852 (US)**
• **XU, Qinyi**
**Rockville, 20852 (US)**
• **WANG, Beibei**
**Clarksville, 21029 (US)**
• **WU, Chenshu**
**Hong Kong (HK)**
• **ZHANG, Hangfang**
**Rockville, 20852 (US)**
• **WONG, Chau-Wai**
**Raleigh, 27607 (US)**
• **CLAFFEY, David N.**
**Somerville, 02143 (US)**
• **CHEN, Chun-I**
**Brookeville, 20833 (US)**
• **LAI, Hung-Quoc Duc**
**Parkville, 21234 (US)**
• **WU, Zhung-Han**
**Rockville, 20852 (US)**
• **WU, Min**
**Clarksville, 21029 (US)**
• **HAN, Yi**
**Ellicott City, 21042 (US)**
• **AU, Oscar Chi-Lim**
**San Jose, 95136 (US)**

**(Cont. next page)**

- **LIU, K.J. Ray**
  **Potomac, 20854 (US)**

(74) Representative: **Boult Wade Tennant LLP**
  **Salisbury Square House**
  **8 Salisbury Square**
  **London EC4Y 8AP (GB)**

(56) References cited:
  **WO-A1-2017/180698**

**Description**

**CROSS-REFERENCE TO RELATED APPLICATIONS**

**TECHNICAL FIELD**

The present teaching generally relates to periodic motion detection and estimation. More specifically, the present teaching relates to vital signs (e.g. breathing) detection and monitoring based on time-reversal technology in a rich-scattering environment.

**BACKGROUND**

Regarding periodic motion detection, many important human vital signs such as breathing are periodic motions. Vital signs are important indicators of a person's health and well-being as well as predictors of acute medical conditions and chronic disease states for a person. Breathing rate is one of the most important vital signs, which can be measured by the number of exhalation and inhalation a person takes per minute. In addition, the breathing pattern may be highly correlated to psychological conditions of a human being, such as stress and anxiety.

Most traditional approaches for breathing monitoring are invasive in that they need physical contact of the human bodies. For instance, in hospitals, the patients are required to wear oxygen masks, Nasal cannulas, chest straps, or wearable sensors such as thermistors and pressure sensors. Another example is Polysomnography (PSG) used in sleep medicine, which typically requires a minimum of 22 wire attachments to the patient. These dedicated devices are often costly and bulky, create discomfort to the human bodies, and are limited only to clinical settings. Although these wired medical systems can measure breathing using wearables attached onto human body, such systems are clumsy and uncomfortable. To make it worse, the systems themselves would itself distort the very breathing that need to be monitored - as many patients become anxious or annoyed with all the attached wearables and wires.

Currently existing non-invasive (contact-free) breathing monitoring solutions can be categorized as below. (1) Radar-based breathing monitoring: Doppler radars are often used in breathing monitoring. They are operated by transmitting a signal and receiving a signal with a Doppler shift due to a periodic motion of objects. The breathing rates can be extracted from the Doppler shift. As a drawback, these systems use high transmission power, rely on sophisticated and expensive hardware, and use extremely large bandwidths. A vital sign monitoring system was disclosed utilizing frequency modulated continuous radar (FMCW). It used Universal Software Radio Peripheral (USRP) as the RF front-end to transmit a frequency-sweeping signal. But the additional cost and complexity of the dedicated hardware limited a large-scale deployment of FMCW radar. (2) Wireless-sensor based breathing monitoring: The received signal strength (RSS) measurements from 802.15.4 compliant sensors on multiple 802.15.4 channels were also used for breathing detection and breathing rate estimation. Dense deployment of wireless sensors is required in these methods as additional wireless infrastructures. In addition, the specific design of frequency-hopping mechanism is required to support multiple channel measurements. (3) Wi-Fi-based breathing monitoring: RSS is commonly used in the Wi-Fi-based breathing monitoring due to its availability on most commercial Wi-Fi network interface controllers (NICs). Measurements were also used with Wi-Fi devices for breathing estimation. But this method is accurate only when the users hold the Wi-Fi-enabled devices in close proximity to their chests.

In addition to the drawbacks mentioned above, methods (1) and (2) require design and manufacturing of special devices such as specialized radar devices or sensor network nodes, while method (3) has very low accuracy and sensitivity. A wireless breathing monitoring system was proposed to monitor breathing rate and heart rate based on UWB (ultra-wide band) signal. But the UWB-based system has many limitations such as: it has expensive, untested, uncommon, dedicated, limited edition experimental hardware components; it has a small range due to severe absorption of UWB signals by walls; it works only in a line-of-sight (LOS) condition; and it needs very tedious and laborious deployment of many sensors in practical situations to cover a reasonable area, making installation, maintenance and repair very expensive and labor intensive due to the restrictive LOS operation. Therefore, there is a need for methods and apparatus for vital sign detection and monitoring to solve the above-mentioned problems and to avoid the above-mentioned drawbacks.

Object motion detection becomes more and more important nowadays. For example, for security and/or management purposes, a user may want to detect any object motion in the user's house; a manager of a supermarket may want to detect any object motion in the supermarket; and a nurse in a hospital may want to detect any motion of a patient in the hospital.

Existing systems and methods for detecting object motions cannot provide enough accuracy and often lead to false alarms. Existing approaches include those based on passive infrared (PIR), active infrared (AIR) and Ultrasonic. PIR sensors are the most widely used motion sensor in home security systems, which detect human motions by sensing the difference between background heat and the heat emitted by moving people. However, solutions based on PIR sensors are prone to false alarms due to its sensitivity to environmental changes, like hot/cold air flow and sunlight. They are easily defeated by blocking the body heat emission (wearing a heat-insulating full-body suit). Also, their range is limited and need line-of-sight (LOS), and thus multiple devices are needed. In AIR based approaches, an IR emitter sends a beam of IR which will be received by an IR receiver. When the beam is interrupted, a motion is detected. However, this kind of approaches can be easily seen using a regular camera or any IR detection mechanism and also has limited range and thus need LOS. Ultrasonic sensors detect human motion by sending out ultrasonic sound waves into a space and measuring the speed at which they return, and motion can be detected if there exist frequency changes. However, this kind of approaches can be defeated by wearing an anechoic suit. Also, ultrasound cannot penetrate solid objects such as furniture or boxes and cause gaps in detection field. Slow movements by a burglar may not trigger an alarm, too. Thus, existing systems and methods for detecting object motions are not entirely satisfactory.

WO 2017/180698 recites, in accordance with its abstract, "The present teaching relates to object tracking based on time-reversal technology in a rich-scattering environment. In one example, a method for tracking a movement of an object in real-time is disclosed. The method may be implemented on a machine including at least a processor and a memory communicatively coupled with the processor. The method may comprise: obtaining an initial position of the object prior to a movement of the object; obtaining at least one wireless signal from a multipath channel that is impacted by the movement of the object; extracting a time series of channel state information (CSI) for the multipath channel from the at least one wireless signal; determining a distance of the movement of the object based on the time series of CSI; estimating a direction of the movement of the object; and determining a new position of the object after the movement based on the distance, the direction, and the initial position."

## SUMMARY

In accordance with a first aspect, there is provided a system for monitoring object motion in a venue in accordance with claim 1. In accordance with a second aspect, there is provided a method in accordance with claim 13. In accordance with a third aspect, there is provided a receiver in accordance with claim 14.

In one example useful for understanding the invention, a method/apparatus/device/server/client/system of a periodic motion monitoring system includes obtaining at least one TSCI of a wireless multipath channel using a processor, a memory communicatively coupled with the processor and a set of instructions stored in the memory. The at least one TSCI is extracted from a wireless signal transmitted between a Type1 heterogeneous wireless device at a first position in a venue and a Type2 heterogeneous wireless device at a second position in the venue through the wireless multipath channel. It is determined that at least one portion of the at least one TSCI in a current sliding time window is associated with a current pseudo-periodic motion of an object in the venue. The wireless multipath channel is impacted in the current sliding time window by the current pseudo-periodic motion of the object.

In one example useful for understanding the invention, a system for monitoring object motion in a venue is disclosed. The system comprises a transmitter, a receiver, and a vital sign estimator. The transmitter is located at a first position in the venue and configured for transmitting a wireless signal through a wireless multipath channel impacted by a pseudo-periodic motion of an object in the venue. The receiver is located at a second position in the venue and configured for: receiving the wireless signal through the wireless multipath channel impacted by the pseudo-periodic motion of the object in the venue, and obtaining at least one TSCI of the wireless multipath channel based on the wireless signal. The vital sign estimator is configured for: determining that at least one portion of the at least one TSCI (TSCI) in a current sliding time window is associated with the pseudo-periodic motion of the object in the venue, and computing a current characteristics related to the pseudo-periodic motion of the object in the current sliding time window based on at least one of: the at least one portion of the at least one TSCI in the current sliding time window, at least one portion of the at least one TSCI in a past sliding time window, and a past characteristics related to the pseudo-periodic motion of the object in the past sliding time window.

In another example useful for understanding the invention, a method for monitoring object motion in a venue is disclosed. The method comprises: receiving a wireless signal through a wireless multipath channel impacted by a pseudo-periodic motion of an object in the venue; obtaining at least one TSCI of the wireless multipath channel based on the wireless signal; determining that at least one portion of the at least one TSCI (TSCI) in a current sliding time window is associated with the pseudo-periodic motion of the object in the venue; and computing a current characteristics related to the pseudo-periodic motion of the object in the current sliding time window based on at least one of: the at least one portion of the at least one TSCI in the current sliding time window, at least one portion of the at least one TSCI in a past sliding time window, and a past characteristics related to the pseudo-periodic motion of the object in the past sliding time window.

In yet another example useful for understanding the invention, a receiver of a motion monitoring system is disclosed. The motion monitoring system comprises: a transmitter, the receiver, and a vital sign estimator. The receiver comprises: a wireless circuitry, a processor communicatively coupled with the wireless circuitry, a memory communicatively coupled with the processor, and a set of instructions stored in the memory. The wireless circuitry is configured to receive a wireless signal through a wireless multipath channel impacted by a pseudo-periodic motion of an object in a venue, wherein the wireless signal is transmitted asynchronously by the transmitter. The set of instructions, when executed, causes the processor to obtain at least one TSCI of the wireless multipath channel based on the wireless signal. At least one portion of the at least one TSCI (TSCI) in a current sliding time window is associated with the pseudo-periodic motion of the object in the venue. The at least one portion of the at least one TSCI in the current sliding time window is to be used by the vital sign estimator to compute a current characteristics related to the pseudo-periodic motion of the object in the current sliding time window based on at least one of: the at least one portion of the at least one TSCI in the current sliding time window, at least one portion of the at least one TSCI in a past sliding time window, and a past characteristics related to the pseudo-periodic motion of the object in the past sliding time window.

In still another example useful for understanding the invention, an estimator of a motion monitoring system is disclosed. The motion monitoring system comprises: a transmitter, a receiver, and the estimator. The estimator comprises: a processor, a memory communicatively coupled with the processor, and a set of instructions stored in the memory. The set of instructions, when executed, causes the processor to perform: obtaining at least one TSCI (TSCI) of a wireless multipath channel from a receiver of the motion monitoring system, wherein the receiver extracts the at least one TSCI from a wireless signal received from a transmitter of the motion monitoring system through the wireless multipath channel impacted by a pseudo-periodic motion of an object in a venue, determining that at least one portion of the at least one TSCI in a current sliding time window is associated with the pseudo-periodic motion of the object in the venue, and computing a current characteristics related to the pseudo-periodic motion of the object in the current sliding time window based on at least one of: the at least one portion of the at least one TSCI in the current sliding time window, at least one portion of the at least one TSCI in a past sliding time window, and a past characteristics related to the pseudo-periodic motion of the object in the past sliding time window.

In a different example useful for understanding the invention, a system for monitoring a repeating motion in a venue is disclosed. The system comprises a transmitter, a receiver, and a repeating motion monitor. The transmitter is located at a first position in the venue and configured for transmitting a wireless signal through a wireless multipath channel impacted by the repeating motion of an object in the venue. The receiver is located at a second position in the venue and configured for: receiving the wireless signal through the wireless multipath channel impacted by the repeating motion of the object in the venue, and obtaining a TSCI of the wireless multipath channel based on the wireless signal. The repeating motion monitor is configured for: monitoring a periodic characteristics of the repeating motion of the object based on the TSCI.

In another example useful for understanding the invention, a method for monitoring a repeating motion in a venue is disclosed. The method comprises: receiving a wireless signal through a wireless multipath channel impacted by the repeating motion of an object in the venue; obtaining a TSCI of the wireless multipath channel based on the wireless signal; and monitoring a periodic characteristics of the repeating motion of the object based on the TSCI.

In yet another example useful for understanding the invention, a receiver of a motion monitoring system is disclosed. The motion monitoring system comprises: a transmitter, the receiver, and a repeating motion monitor. The receiver comprises: a wireless circuitry, a processor communicatively coupled with the wireless circuitry, a memory communicatively coupled with the processor, and a set of instructions stored in the memory. The wireless circuitry is configured to receive a wireless signal through a wireless multipath channel impacted by a repeating motion of an object in a venue. The wireless signal is transmitted by the transmitter. The set of instructions, when executed, causes the processor to obtain a TSCI of the wireless multipath channel based on the wireless signal. The TSCI is to be used by a repeating motion monitor of the motion monitoring system to monitor periodic characteristics of the repeating motion.

In still another example useful for understanding the invention, a monitor of a motion monitoring system is disclosed. The motion monitoring system comprises: a transmitter, a receiver, and the monitor. The monitor comprises: a processor, a memory communicatively coupled with the processor, and a set of instructions stored in the memory. The set of instructions, when executed, causes the processor to perform: obtaining a TSCI of a wireless multipath channel from a receiver of the motion monitoring system, wherein the receiver extracts the TSCI from a wireless signal received from a transmitter of the motion monitoring system through the wireless multipath channel impacted by a repeating motion of an object in a venue, and monitoring a periodic characteristics of the repeating motion of the object based on the TSCI.

In one example useful for understanding the invention, a system having at least a processor and a memory with a set of instructions stored therein for detecting object motion in a venue is disclosed. The system comprises: a first wireless device configured for transmitting a wireless signal through a wireless multipath channel impacted by a motion of an object in the venue; and a second wireless device that has a different type from that of the first wireless device and is configured for: receiving the wireless signal through the wireless multipath channel impacted by the motion of the object in the venue, and obtaining a TSCI of the wireless multipath channel based on the wireless signal; and a motion detector configured for detecting the motion of the object in the venue based on motion information related to the motion of the object, wherein the motion information associated with the first and second wireless devices is computed based on the TSCI by at least one of: the motion detector and the second wireless device.

In another example useful for understanding the invention, a method of a wireless monitoring system is disclosed. The wireless monitoring system comprises at least one asynchronous heterogeneous Type1 wireless device and at least one asynchronous heterogeneous Type2 wireless device. The wireless monitoring system comprises at least one pair of Type1 and Type2 devices. Each pair comprises: one of the at least one asynchronous heterogeneous Type1 wireless device, and one of the at least one asynchronous heterogeneous Type2 wireless device. Each of the at least one Type2 wireless device is in at least one respective particular pair of Type1 and Type2 devices. Each Type2 wireless device is associated with at least one respective particular Type1 wireless device through the at least one respective particular pair of Type1 and Type2 devices. For each Type2 wireless device, and for each of the at least one respective particular pair of Type1 and Type2 devices comprising the Type2 wireless device, the method comprises obtaining asynchronously at least one respective TSCI of a wireless multipath channel impacted by a motion of an object in a venue, wherein the at least one respective TSCI (TSCI) associated with the particular pair of Type1 and Type2 wireless devices is extracted asynchronously from a respective asynchronous heterogeneous wireless signal, wherein the respective asynchronous heterogeneous wireless signal is transmitted asynchronously from a respective one of the at least one respective particular Type1 wireless device using a respective first heterogeneous processor, a respective first heterogeneous memory and a respective first heterogeneous set of instructions of the Type1 wireless device to at least one asynchronous heterogeneous Type2 wireless device through the wireless multipath channel. The method further comprises at least one of: monitoring the motion of the object in the venue individually and asynchronously, using a third processor, a third memory and a third set of instructions of a Type 3 device, based on TSCI associated with a pair of Type1 and Type2 devices comprising a particular Type2 device, monitoring the motion of the object jointly and asynchronously based on TSCI associated with any of the at least one pair of Type1 and Type2 devices associated with the particular Type2 wireless device, monitoring the motion of the object jointly and asynchronously based on TSCI associated with any of the at least one pair of Type1 and Type2 devices associated with one of the at least one respective particular Type1 wireless device, and monitoring the motion of the object globally and asynchronously based on TSCI associated with any of the at least one pair of Type1 and Type2 devices.

In yet another example useful for understanding the invention, a particular asynchronous heterogeneous Type2 device of a radio monitoring system is disclosed. The radio monitoring system comprises at least one asynchronous heterogeneous Type1 device and at least one asynchronous heterogeneous Type2 device. The radio monitoring system comprises at least one pairing of Type1 and Type2 devices, each pairing comprising one of the at least one Type1 device, and one of the at least one Type2 device. The particular asynchronous heterogeneous Type2 device is in at least one particular pairing of Type1 and Type2 devices of the radio monitoring system. The particular Type2 device is grouped with at least one particular Type1 device through the at least one particular pairing of Type1 and Type2 devices. The particular asynchronous heterogeneous Type2 device comprises: a wireless circuitry to receive asynchronously at least one asynchronous heterogeneous radio signal, wherein each asynchronous heterogeneous radio signal is transmitted asynchronously by one of the at least one particular Type1 device using a respective first heterogeneous processor, a respective first heterogeneous memory and a respective first heterogeneous set of instructions of the Type1 device to at least one asynchronous heterogeneous Type2 device through a wireless multipath channel influenced by a movement of a mass in a site; a second heterogeneous processor communicatively coupled with the wireless circuitry; a second heterogeneous memory communicative coupled with the second heterogeneous processor; a second heterogeneous set of instructions stored in the second heterogeneous memory which, when executed, cause the second heterogeneous processor to, for each of the at least one particular pairing of Type1 and Type2 devices, secure asynchronously at least one respective series of CI (TSCI) of the wireless multipath channel, wherein the at least one respective TSCI associated with the particular pairing of Type1 and Type2 devices is being derived asynchronously from the respective asynchronous heterogeneous radio signal received asynchronously by the wireless circuitry; wherein at least one of the following is included: the movement of the mass in the site is monitored individually and asynchronously, using a third processor, a third memory and a third set of instructions of a Type 3 device, based on TSCI associated with a pairing of Type1 and Type2 devices comprising the particular Type2 device, the movement of the mass is monitored jointly and asynchronously based on TSCI associated with any pairings of Type1 and Type2 devices comprising the particular Type2 device, the movement of the mass is monitored jointly and asynchronously based on TSCI associated with any pairings of Type1 and Type2 devices comprising one of the at least one particular Type1 device, and the movement of the mass is monitored globally and asynchronously based on TSCI associated with any pairings of Type1 and Type2 devices.

In still another example useful for understanding the invention, a particular asynchronous heterogeneous Type 3 device of a radio monitoring system is disclosed. The radio monitoring system comprises at least one asynchronous heterogeneous Type1 device and at least one asynchronous heterogeneous Type2 device. The radio monitoring system comprises at least one doublet of Type1 and Type2 devices, each doublet comprising one of the at least one asynchronous heterogeneous Type1 device, and one of the at least one asynchronous heterogeneous Type2 device. The particular asynchronous heterogeneous Type 3 comprises: a third processor communicatively coupled with at least one of: at least one asynchronous heterogeneous Type1 device, and at least one asynchronous heterogeneous Type2 device; a third memory communicative coupled with the third processor; and a third set of instructions stored in the third memory. The third set of instructions, when executed, causes the third processor to: for each Type2 device, and for each of the at least one doublet of Type1 and Type2 devices comprising the Type2 device: receive asynchronously at least one respective TSCI (TSCI) of a wireless multipath channel influenced by a motion of an item in a place received asynchronously by the Type2 device using a respective second processor, a respective second memory and a respective second set of instructions of the Type2 device, wherein the at least one respective TSCI associated with the respective doublet of Type1 and Type2 devices is obtained asynchronously from a respective asynchronous heterogeneous radio signal transmitted from a respective Type1 device of the respective doublet using a respective first processor, a respective first memory and a respective first set of instructions of the respective Type1 device to at least one asynchronous heterogeneous Type2 device through the wireless multipath channel; and at least one of: track the motion of the item in the place individually and asynchronously, based on TSCI associated with a particular doublet of Type1 and Type2 devices comprising a particular Type2 device and a particular Type1 device, track the motion of the item jointly and asynchronously based on TSCI associated with any of the at least one doublet of Type1 and Type2 devices associated with the particular Type2 device, track the motion of the item jointly and asynchronously based on TSCI associated with any of the at least one doublet of Type1 and Type2 devices associated with the particular Type1 device, track the motion of the item globally and asynchronously based on TSCI associated with any of the at least one doublet of Type1 and Type2 devices.

## BRIEF DESCRIPTION OF DRAWINGS

FIG. 1 illustrates a floorplan of experiments (a) single-person, NLOS (b) single-person, NLOS, with a fan running (c) two-person, NLOS (d) three-person, NLOS, according to one embodiment of the present teaching.

FIG. 2 illustrates a state transition of the FSM scheme with operating fan, according to one embodiment of the present teaching.

FIG. 3 illustrates a breathing estimation with subject standing up for five seconds, according to one embodiment of the present teaching.

FIG. 4 illustrates an exemplary flow of obtaining vital sign monitoring (breathing rate estimation) using time-reversal technology, according to one embodiment of the present teaching.

FIG. 5 illustrates a FFT-based breathing tracking algorithm the proposed architecture consists of the following steps, according to one embodiment of the present teaching.

FIG. 6 illustrates filter coefficients for the passband 0.133~0.7 Hz with 10 Hz CFR sampling rate, 81 taps, according to one embodiment of the present teaching.

FIG. 7 illustrates a CIR variation over time on one channel tap (a) before bandpass filtering (b) after bandpass filtering, according to one embodiment of the present teaching.

FIG. 8 illustrates a 2-D Spectrum of the breathing signal over all CIR taps on one antenna link, according to one embodiment of the present teaching.

FIG. 9 illustrates a spectrum after performing link average, according to one embodiment of the present teaching.

FIG. 10 illustrates a procedure for FSMs associated with different people selecting peaks, according to one embodiment of the present teaching

FIG. 11 illustrates a FSM for single-person breathing tracking, according to one embodiment of the present teaching.

FIG. 12 illustrates results of breathing tracking for single-person NLOS scenario (a) with FSM (b) without FSM, according to one embodiment of the present teaching.

FIG. 13 illustrates a comparison between the schemes with / without FSM with a fan running, according to one embodiment of the present teaching.

FIG. 14 illustrates a CDF performance with operating fan under single-person NLOS scenario with 18 BPM breathing rate, according to one embodiment of the present teaching.

FIG. 15 illustrates a CDF performance with operating fan under single-person NLOS scenario with 20 BPM breathing rate, according to one embodiment of the present teaching.

FIG. 16 illustrates a two-person breathing rate estimation with FSM under (a) $C_{window}$ = 300 (b) $C_{window}$ = 450 (c) $C_{window}$ = 600, according to one embodiment of the present teaching.

FIG. 17 illustrates a two-person breathing rate estimation without FSM under (a) $C_{window}$ = 300 (b) $C_{window}$ = 450 (c) $C_{window}$ = 600, according to one embodiment of the present teaching.

FIG. 18 illustrates an exemplary method for object tracking, according to one embodiment of the present teaching.

FIG. 19 illustrates examples of spatial-temporal information, according to one embodiment of the present teaching.

FIG. 20 illustrates examples of tasks performed based on spatial-temporal information, according to one embodiment of the present teaching.

FIG. 21 illustrates another exemplary method for object tracking, according to one embodiment of the present teaching.

FIG. 22 illustrates an exemplary presentation of the spatial-temporal information in a venue, according to one embodiment of the present teaching. FIG. 22 also illustrates an exemplary scenario where object motion is detected based on spatial-temporal information in a venue, according to one embodiment of the present teaching.

FIG. 23 illustrates an exemplary illustration of antenna matching based speed estimation, according to one embodiment of the present teaching.

FIG. 24 illustrates a flowchart illustrating a method for antenna matching based speed estimation, according to one embodiment of the present teaching.

FIG. 25 illustrates a flowchart of an exemplary method for motion detection, according to one embodiment of the present teaching.

FIG. 26 illustrates an exemplary block diagram of a first wireless device, according to one embodiment of the present teaching.

FIG. 27 illustrates a flowchart of an exemplary method performed by a first wireless device, according to one embodiment of the present teaching.

FIG. 28 illustrates an exemplary block diagram of a second wireless device, according to one embodiment of the present teaching.

FIG. 29 illustrates a flowchart of an exemplary method performed by a second wireless device, according to one embodiment of the present teaching.

FIG. 30 illustrates an exemplary motion detector, according to one embodiment of the present teaching.

FIG. 31 illustrates an exemplary algorithm of motion detection, according to one embodiment of the present teaching.

FIG. 32 illustrates an exemplary method for object motion detection, according to one embodiment of the present teaching.

FIG. 33 illustrates an exemplary flowchart for a method of object motion localization, according to one embodiment of the present teaching.

FIG. 34 illustrates a diagram showing an exemplary flow of detecting indoor event using time-reversal technology, according to one embodiment of the present teaching.

FIG. 35 illustrates an exemplary network environment for vital sign detection and monitoring in a venue, according to one embodiment of the present teaching.

FIG. 36 illustrates an exemplary block diagram of a system for vital sign detection and monitoring, according to one embodiment of the present teaching.

FIG. 37 illustrates an exemplary block diagram of a vital sign estimator for vital sign detection and monitoring, according to one embodiment of the present teaching.

FIG. 38 illustrates a flowchart of an exemplary method for vital sign detection and monitoring, according to one embodiment of the present teaching.

FIG. 39 illustrates an exemplary finite state machine (FSM) for single-person breathing monitoring, according to one embodiment of the present teaching.

FIG. 40 illustrates an exemplary procedure for FSMs associated with different people selecting peaks, according to one embodiment of the present teaching.

FIG. 41 illustrates a flowchart of an exemplary method for vital sign estimation, according to one embodiment of the present teaching.

FIG. 42 illustrates an exemplary system state controller which is a FSM for single-person breathing monitoring, according to one embodiment of the present teaching.

FIG. 43 illustrates an exemplary state transition of FSM for single-person breathing monitoring, according to one embodiment of the present teaching.

FIG. 44 illustrates an exemplary experiment result on comparison between schemes with and without FSM with a running fan, according to one embodiment of the present teaching.

FIG. 45 illustrates an exemplary state transition of the FSM scheme with an operating fan, according to one embodiment of the present teaching.

FIG. 46 illustrates an exemplary breathing estimation with subject standing up for five seconds, according to one embodiment of the present teaching.

FIG. 47 illustrates an exemplary method for vital sign detection and monitoring, according to one embodiment of the present teaching.

FIG. 48 illustrates an exemplary block diagram of a repeating motion monitor, according to one embodiment of the present teaching.

FIG. 49 illustrates a flowchart of an exemplary method for estimating and monitoring repetition rate (e.g. breathing rate), according to one embodiment of the present teaching.

FIG. 50 illustrates exemplary autocorrelation functions of the received signals under different scenarios, according to one embodiment of the present teaching.

FIG. 51 illustrates exemplary features extracted from the derived autocorrelation functions for breathing detection and estimation, according to one embodiment of the present teaching.

FIG. 52 illustrates an exemplary scheme for breathing signal extraction and maximization, according to one embodiment of the present teaching.

FIG. 53 illustrates an exemplary procedure of multi-person repetition rate (e.g. breathing rate) estimation, according to one embodiment of the present teaching.

FIG. 54 illustrates an exemplary diagram of a device in a motion monitoring system, according to one embodiment of the present teaching.

FIG. 55 illustrates an exemplary breathing signal based on real-world measurements, according to one embodiment of the present teaching.

FIG. 56 demonstrates gains of a disclosed scheme for breathing signal extraction and maximization, according to one embodiment of the present teaching.

FIG. 57A and FIG. 57B illustrate comparison results between a wake state and a sleep state, according to one embodiment of the present teaching.

FIG. 58A and FIG. 58B illustrate breathing rate performances of different sleep stages, according to one embodiment of the present teaching.

FIG. 59 illustrates an exemplary network environment for sleep monitoring, according to one embodiment of the present teaching.

FIG. 60 illustrates an exemplary algorithm design for sleep monitoring, according to one embodiment of the present teaching.

FIG. 61 illustrates an exemplary interior bird-view of a car for seat occupancy detection and people counting, according to one embodiment of the present teaching.

FIGs. 62A-62D illustrate changes of CI according to various seat occupancy situations in a car, according to one embodiment of the present teaching.

## DETAILED DESCRIPTION

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

*Periodic motion (e.g. breathing) detection and estimation*

The present teaching generally relates to periodic motion (e.g. human breathing) detection. More specifically, the present teaching relates to periodic motion detection and monitoring based on time-reversal technology in a rich-scattering wireless environment, such as an indoor environment or urban metropolitan area, enclosed environment, underground environment, open-air venue with barriers such as parking lot, storage, yard, square, forest, cavern, valley, etc.

In one example, a method/apparatus/device/server/client/system of a periodic motion monitoring system includes obtaining at least one TSCI of a wireless multipath channel using a processor, a memory communicatively coupled with the processor and a set of instructions stored in the memory. The at least one TSCI is extracted from a wireless signal transmitted between a Type1 heterogeneous wireless device at a first position in a venue and a Type2 heterogeneous wireless device at a second position in the venue through the wireless multipath channel. It is determined that at least one portion of the at least one TSCI in a current sliding time window is associated with a current pseudo-periodic motion of an object in the venue. The wireless multipath channel is impacted in the current sliding time window by the current pseudo-periodic motion of the object.

A current characteristic of the object in the current sliding time window is computed based on at least one of: the at least one portion of the at least one TSCI in the current sliding time window, another at least one portion of the at least one TSCI in a past sliding time window and a past characteristic of the object in the past sliding time window. The at least one TSCI is preprocessed. Computational workload associated with the method is shared among the processor, the Type 1 heterogeneous wireless device, the Type 2 heterogeneous wireless device and another processor. Time domain processing and frequency domain processing of the at least one TSCI may be performed in the current sliding time window. At least one peak in a frequency domain may be detected. The current characteristics of the object may be computed based on the at least one peak in the frequency domain.

For each original CI of the particular portion of the particular TSCI in the current sliding time window, N1 frequency domain components of the original CI may be converted using an inverse frequency transform to N2 time domain coefficients of the same CI. Each CI may be associated with an original timestamp. The N2 may not be smaller than N1. Each of the N2 coefficients may be associated with a time delay. Original times tamps of all original CI of the particular portion of the particular TSCI may be corrected in the current sliding time window so that corrected timestamps of time-corrected CI may be uniformly spaced in time.

For each of the N2 time domain coefficients of the time-corrected CI to be computed at a particular corrected timestamp, the time domain coefficient of the time-corrected CI at the particular corrected timestamp may be computed based on the time domain coefficient of the original CI at the original timestamps. A particular portion of a particular TSCI in the current sliding time window may be determined to be associated with the current pseudo-periodic motion of the object in the venue. The particular portion may comprise N0 CI. For each of the N0 CI, N1 frequency domain components may be obtained. A matrix G1 of size N1 by N0 may be determined, wherein each of the N0 columns may contain the respective N1 frequency elements of the respective CI. An original timestamp associated with each of the CI may be determined. Original timestamps of all CI of the particular portion of the particular TSCI in the current sliding time window may be corrected so that corrected timestamps of time-corrected CI may be uniformly spaced in time. Spectral analysis of the output of the operation may be performed in a frequency domain. For each original CI of the particular portion of the particular TSCI in the current sliding time window, N2 time domain coefficients of the same CI may be converted using a frequency transform to N2 frequency domain coefficients.

The Type1 device may have at least one antenna. The Type2 device may also have at least one antenna. Each of the at least one TSCI may be associated with one of the at least one antenna of the Type1 device and one of the at least one antenna of the Type2 device. The averaging over antenna links is averaging over the at least one TSCI. At least one local minimum and at least one local minimum in a frequency domain may be identified. At least one local signal-to-noise-ratio-like (SNR-like) parameter may be computed for each pair of adjacent local maximum and local minimum. In some examples, different parts of the frequency range may be treated differently. Past peaks (including "reserved" peaks) in past sliding time windows may be analyzed to give "predicted" peaks in the current sliding time window. Likely local regions or "predicted regions" (e.g. immediate local region around a past peak or a "predicted peak") in the frequency range may be identified. Instead of searching the whole frequency range for peaks, fast local search in the likely local regions may be performed in the predicted regions. The width of the predicted regions may be adaptively changed over time. As prediction becomes increasing accurate, the predicted region may be shrunk in size to save computation with maintaining accuracy. Fast global search (perhaps of lower accuracy) may also be performed in search of new peaks (not appearing in the past peaks).

The "predicted" peaks may be obtained by zero-order prediction, i.e. the "predicted" peaks may simply be a past peak. The "predicted" peaks may also be obtained by first order prediction, second order prediction, higher order prediction, and/or another prediction. Multiple predictions may be made and combined to form a hybrid prediction. For example, a human may initially be at rest and may be breathing at 18 breath per min (bpm). The breathing rate may naturally drift around 18 bpm over time. The person may get up and start moving (e.g. walking, swimming, running, doing push-up, doing exercise, etc.) such that the breathing rate may increase from 18bpm to 30bpm within in short time. The slow-drifting breathing rate (around 18bpm) may be tracked by the "predicted" peaks. However, the 30bpm may be a new peak that cannot be predicted by past peaks. The global search is used to discover the new peaks. Past peaks (including significant but not selected ones, and/or reserved ones) may be analyzed and peaks in different time instance may be associated to form "trace" of an evolving periodic motion. If a peak at a certain time t is close enough to a past peak at t-1, an immediately past time (with frequency difference being less than a threshold), the two peaks may be "associated" or "connected" into the same trace. In the previous example, if a new peak appears at 30bpm all of a sudden and the past peak of 18bpm disappears all of a sudden, the new peak at 30bpm may be connected or associated with the 18bpm trace. However, if the 18bpm remains when the 30bpm suddenly appear, the 30bpm may be considered a brand new trace. Preference may be given to local regions around long-trace, persistent trace, strong trace, close traces, traces crossing each other, intermittent trace with a long history, etc.

While all antennas links may be combined or averaged for the analysis, individual antenna and/or subsets of the antennas may be analyzed separately and strong/persistent peaks may be identified. They may be considered together with other significant peaks. Finite state machine (FSM) may be applied to all combinations (individual, subset and/or whole set of antennas). The FSM may be different for different combination. The states used in the FSM may be different for different combinations. The FSM may be exactly the same for all the different combinations. Different predicted peaks may be used in different combinations. Different combinations may have different peaks, different trace, etc. Different combinations may be different in any and/or all steps. For example, if a finite state machine (FSM) is used, it may select always the strongest peaks in its frequency in any state of the FSM.

The current characteristics of the object may be made available (e.g. to a software, a software module, a hardware module, a processor, another processor, another device, a remote device, a cloud device) in real time with/without a time delay, and/or non-real time. The current sliding time window may be moved by a shift-size as time progresses. A remaining spectral energy may be computed by subtracting the spectral energy associated with the set of selected significant local peaks from the total spectral energy. An event associated with the current characteristics of the object may be detected based on at least one of: the remaining spectral energy, an adaptive threshold, and a finite state machine.

*Finite State Machine for periodic motion detection/estimation*

Decision thresholds (i.e. thresholds for making decisions) may be computed adaptively based on a finite state machine (FSM). The thresholds, T1 and T2, may be computed adaptively based on the finite state machine. The finite state machine may comprise an INIT state, a Verification state, a PeakFound state, and/or Motion state.

The INIT state of the finite state machine (FSM) may be entered in at least one predetermined way. The thresholds may be computed adaptively in a first way in the INIT state. An event may be detected based on the thresholds. The INIT state may transition to another state based on at least one transition criterion. The thresholds may be computed adaptively in another way in the another state. In the INIT state, the thresholds may be computed adaptively in the first way. The event may be detected (i.e. a detection operation is applied in an attempt to detect the event, if happen) based on the thresholds and the set of selected significant local peaks and/or related characteristics. Excessive background interfering motion may be detected (i.e. detection operation is applied to detect any excessive background interfering motion, if any) based on the thresholds and the remaining spectral energy. The INIT state may remain unchanged when the event is concluded to be "not detected" and the excessive background interfering motion is concluded to be "not detected." The INIT state may transition to the Verification state when the event is concluded to be "detected" preliminarily and the preliminarily detected event needs to be verified. The INIT state may transition to the Motion state when the excessive background interfering motion is concluded to be "detected."

In the Verification state, the thresholds may be computed adaptively in a second way. At least one statistics (e.g. a count of successive "detected", or a percentage of "detected" in N successive time instance) may be accumulated and computed based on sets of selected significant local peaks and related characteristics in at least one adjacent sliding time window. The at least one statistics may be for verification of the preliminarily detected event. For example, the detection may be applied to each of the at least one adjacent sliding time window. A statistics may be a count of the amount of times the preliminarily detected event continued to be concluded as "detected." The Verification state may remain unchanged while the at least one statistics (e.g. a count of successive "detected", or a percentage of "detected" in N successive time instance) may be accumulated until sufficient statistics may be collected for the verification. The preliminarily detected event may be verified (i.e. an operation may be applied to verify the preliminarily detected event) based on the thresholds and the at least one statistics. The Verification state may transition to the PeakFound state when the preliminarily detected event may be concluded as "verified." The Verification state may transition to the INIT state when verification may be concluded as "not verified." The Verification state may remain unchanged when verification is not concluded. For example, "verified" may be concluded if a total of N successive are concluded as "detected", or percentage of "detected" in N successive time instance is greater than some threshold.

In the PeakFound state, the thresholds may be adaptively in a third way. The verified event (i.e. the event being verified immediately before entering the PeakFound state) may be detected based on the thresholds and the set of selected significant local peaks and/or related characteristics. The excessive background interfering motion may be detected based on the thresholds and the remaining spectral energy. The PeakFound state may remain unchanged when the verified event may be concluded as "detected." The PeakFound state may transition to the INIT state when the verified event may be concluded as "not detected" for a number of time instances. (e.g. 3 times). The PeakFound state may transition to the Motion state when the excessive background interfering motion may be concluded as "detected" for a number of time instances.

In the Motion state, the thresholds may be computed adaptively in a fourth way. The excessive background interfering motion may be detected based on the thresholds and the remaining spectral energy. The Motion state may remain unchanged when the excessive background interfering motion may be concluded as "detected." The Motion state may transition to the INIT state when the excessive background interfering motion may be concluded as "not detected" for a number of time instances. The Motion state may also transition to a previous state when the excessive background interfering motion is concluded as "not detected" for a number of time instances. The previous state is the state from which the current Motion state is entered.

When multiple periodic motions with multiple respective traces are being monitored, each trace may have a different FSM. In other words, one peak associated with one of the trace may be in verification state, while another peak associated with another trace may be in PeakFound state.

Additional states may be defined. For example, a "JustLost" state (for a trace that is just lost), or a "TemporarilyLost" state (for an intermittent trace that is temporarily lost) may be added between the transition from PeakFound state to INIT state. The Motion state may be split into two states: "Motion from PeakFound" for a peak (or a trace) that enter Motion state from a PeakFound state, "Motion from Init" for a peak (or a trace) that enter Motion state from INIT state, "Motion from Verification" for a peak (or trace) that enter Motion state from Verification state, "Motion from non-INIT" for a peak (or a trace) that enter Motion state from a state that is not the INIT state, etc. There may be an "Init after PeakFound" state for a peak (or a trace) that enter INIT state from PeakFound state. There may be a "Motion from Verification" state to which the Verification state may transition. There may also be "HugeMotion" state for large motion and "SmallMotion" state for moderate motion.

There may be multiple motion states (e.g. the Motion state) with different characteristics of the interfering background motion. There may be multiple verification states (e.g. the Verification state) with different characteristics of the detected peak, There may be multiple initial/standby states (e.g. the INIT state) with different initial and/or default characteristics. There may be some low power states with reduced computation to save power. There may be some low date-rate state that generates less data traffic for the network and/or storage. There may be some collaborative states in which multiple Type1 and/or multiple Type2 devices collaborate to find trustworthy periodic motion. There may be some low-priority states, high-priority states and/or variable priority states in which a priority is assigned to the peak/trace, based on trust, trustworthiness, value, commercial value, user class, user characteristics, location of Type1 devices, location of Type2 devices, a classification of the trace, a motion characteristics associated with the trace, etc. Machine learn, training, discriminative training, deep learning, neural network, continuous time processing, distributed computing, distributed storage, GPU/DSP/ coprocessor/multicore/multiprocessing acceleration may be applied to any/every step of this disclosure. There may be compound states such as "Current INIT, past Motion" state, "Current INIT, past PeakFound" state, for various combinations of past states and current states.

Sometimes two periodic motions may have their trace cross, touch or stay very close each other. There may be a "cross" state for two traces cross, an "interference" state for two traces close enough to interfere with each other (e.g. the two traces may/may not cross, may cross intermittently, may cross regularly/in a predicted pattern/in a statistically stationary pattern/in a cyclo-stationary pattern), "multiple trace" state for intersection of multiple (e.g. 2 or 3 or more) traces, "lost" state for a long-term trace disappear all of a sudden, etc.

When two traces cross each other and then separates, the association of the two peaks with the two traces after the crossing may be based on a prediction of the peaks. For example, a trace in an increasing trend after the crossing may be associated with a past trace with increasing trend before the cross, and vice versa. A highly fluctuating trace (e.g. with high variance and/or high total variation) after the crossing may be associated with a past trace with similarly fluctuating characteristics. Immediately after the crossing, an initial association may be given. However, the association may be reviewed (one, or two, or more times) in the near future as more observations come in and a short-term/medium-term/long-term trace behavior can be monitored and used to adjust any incorrect initial associations.

Characteristics may be defined, computed, stored, displayed, and provided to user for each trace. For example, it may be duration, past history, frequency of occurrence, intermittent measures, current runlength, previous runlengths, starting time of runs, ending time of runs, confidence, trust, trustworthiness, peak power, interfering background motion power, variance, total variation, antenna ID, Type1 device ID, Type2 device ID, multi-Type1-device, multi-Type2-device, etc.

Pattern recognition, and/or machine learn may be applied to a trace to recognize short-term, medium-term, long-term trends. For example, the trend may be long term increasing, short term increasing, oscillating, Doppler effects, etc. An approaching object may have an increasing frequency. A leaving/receding object may have decreasing frequency. A fan sweeping left and right may be repeating increasing/decreasing patterns. A gait may have a "signature" in the time trend of the associated peaks. An object with multiple Type1 devices attached (e.g. a vehicle may be large and may have four Type1 devices installed at the four corners of the vehicle), may have corresponding state correlated to each other. If the vehicle is rotating, the trace (and/or tracked motion in indoor motion tracking) of the multiple Type1 devices may be correlated. So multiple traces of related Type1 devices may be analyzed jointly, to make smart decisions, more accurate/precise decisions, to avoid erratic decision due to impulsive disturbance.

When multiple periodic motions (e.g. the breathing of multiple people) are being monitored, different motions may be associated with different FSM. One FSM may be correlated to another. For example, a husband and wife may be sleeping on the same bed. A FSM may be set up for the husband. Another FSM may be for the wife. When the husband gets up (e.g. to drink water or using the rest room), the wife may be disturbed. While the state of each person may be related to movement of the person, it may depend on movement (and state) of the other person. Thus the operation and state transitions of the multiple FSM may be determined jointly. For example, when one person undergoes a state change, the other person may have an elevated chance of undergoing some change. Thus the state prediction/peak prediction may be less aggressive (i.e. assuming the prediction is very accurate/trustworthy). More conservative approach may be used e.g. using large search range in local search, or using more global search, or using more conservative thresholds, or using larger/smaller thresholds.

System/interconnection

In one embodiment, the present teaching discloses a method, apparatus, device, system, and/or software (method/apparatus/device/system/software) of a wireless monitoring system. A time series of channel information (CI) of a wireless multipath channel (channel) may be obtained using a processor, a memory communicatively coupled with the processor and a set of instructions stored in the memory. The time series of CI (TSCI) may be extracted from a wireless signal (signal) transmitted between a Type1 (Type1) heterogeneous wireless device and a Type2 (Type2) heterogeneous wireless device in a venue through the channel. The channel may be impacted by a motion of an object in the venue. A characteristics and/or a spatial-temporal information (e.g. motion information) of the object and/or of the motion of an object may be monitored based on the TSCI. A task may be performed based on the characteristics and/or the spatial-temporal information (e.g. motion information). A presentation associated with the task may be generated in a user-interface (UI) on a device of a user. The TSCI may be preprocessed.

The Type1 device may comprise at least one heterogeneous wireless transmitter. The Type2 device may comprise at least one heterogeneous wireless receiver. The Type1 device and the Type2 device may be the same device. Any device may have a processor, a memory communicatively coupled with the processor, and a set of instructions stored in the memory to be executed by the processor. Some processors, memories and sets of instructions may be coordinated. There may be multiple Type1 devices interacting with the same Type2 device (or multiple Type2 devices), and/or there may be multiple Type2 devices interacting with the same Type1 device. The multiple Type1 devices/Type2 devices may be synchronized and/or asynchronous, with same/different window width/size and/or time shift. Wireless signals sent by the multiple Type1 devices may be synchronous and/or contemporaneous. The multiple Type1 devices/Type2 devices may operate independently and/or collaboratively. A Type1 and/or Type2 device may have/comprise/be heterogeneous hardware circuitry (e.g. a heterogeneous chip or a heterogeneous IC capable of generating/receiving the wireless signal, extracting CI from received signal, or making the CI available). They may be communicatively coupled to same or different servers (e.g. cloud server, edge server, local server). Operation of one device may be based on operation, state, internal state, storage, processor, memory output, physical location, computing resources, network of another device. Difference devices may communicate directly, and/or via another device/server/cloud server. The devices may be associated with one or more users, with associated settings. The settings may be chosen once, pre-programmed, and/or changed over time. In the case of one or multiple Type1 devices interacting with one or multiple Type2 devices, any processing (e.g. time domain, frequency domain) may be different for different devices. The processing may be based on locations, orientation, direction, roles, user-related characteristics, settings, configurations, available resources, available bandwidth, network connection, hardware, software, processor, co-processor, memory, battery life, available power, antennas, antenna types, directional/unidirectional characteristics of the antenna, power setting, and/or other parameters/characteristics of the devices.

The wireless receiver (e.g. Type2 device) may receive the signal and/or another signal from the wireless transmitter (e.g. Type1 device). The wireless receiver may receive another signal from another wireless transmitter (e.g. a second Type1 device). The wireless transmitter may transmit the signal and/or another signal to another wireless receiver (e.g. a second Type2 device). The wireless transmitter, wireless receiver, another wireless receiver and/or another wireless transmitter may be moving with the object and/or another object. The another object may be tracked.

The Type1 and/or Type2 device may be capable of wirelessly coupling with at least two Type2 and/or Type1 devices. The Type1 device may be caused to switch/establish wireless coupling from the Type2 device to a second Type2 device at another location in the venue. Similarly, the Type2 device may be caused to switch/establish wireless coupling from the Type1 device to a second Type1 device at yet another location in the venue. The switching may be controlled by a server, the processor, the Type1 device, the Type2 device, and/or another device. The radio used before and after switching may be different. A second wireless signal (second signal) may be caused to be transmitted between the Type1 device and the second Type2 device (or between the Type2 device and the second Type1 device) through the channel. A second TSCI of the channel extracted from the second signal may be obtained. The second signal may be the first signal. The characteristics, spatial-temporal information and/or another quantity of the object may be monitored based on the second TSCI. The Type1 device and the Type2 device may be the same.

The signal and/or another signal may have data embedded. The signal may be a series of probe signals. A probe signal may have data embedded. A probe signal may be replaced by a data signal. The wireless receiver, wireless transmitter, another wireless receiver and/or another wireless transmitter may be associated with at least one processor, memory communicatively coupled with respective processor, and/or respective set of instructions stored in the memory which when executed cause the processor to perform any and/or all steps needed to determine the spatial-temporal information (e.g. motion information), initial spatial-temporal information, initial time, direction, instantaneous location, instantaneous angle, and/or speed, of the object. The processor, the memory and/or the set of instructions may be associated with the Type1 heterogeneous wireless transceiver, one of the at least one Type2 heterogeneous wireless transceiver, the object, a device associated with the object, another device associated with the venue, a cloud server, and/or another server.

The Type1 device may transmit the signal in a broadcasting manner to at least one Type2 device(s) through the channel in the venue. The signal is transmitted without the Type1 device establishing wireless connection (connection) with any Type2 device. The Type1 device may transmit to a particular media access control (MAC) address common for more than one Type2 devices. Each Type2 device may adjust its MAC address to the particular MAC address.

The particular MAC address may be associated with the venue. The association may be recorded in an association table of an Association Server. The venue may be identified by the Type1 device, a Type2 device and/or another device based on the particular MAC address, the series of probe signals, and/or the at least one TSCI extracted from the probe signals. For example, a Type2 device may be moved to a new location in the venue (e.g. from another venue). The Type1 device may be newly set up in the venue such that the Type1 and Type2 devices are not aware of each other. During set up, the Type1 device may be instructed/guided/ caused/ controlled (e.g. using a dummy receiver, using a hardware pin setting/connection, using a stored setting, using a local setting, using a remote setting, using a downloaded setting, or using a server) to send the series of probe signals to the particular MAC address. Upon power up, the Type2 device may scan for probe signals according to a table of MAC addresses (e.g. stored in a designated source, server, cloud server) that may be used for broadcasting at different locations (e.g. different MAC address used for different venue such as house, office, enclosure, floor, multi-storey building, store, airport, mall, stadium, hall, station, subway, lot, area, zone, region, district, city, country, continent). When the Type2 device detects the probe signals sent to the particular MAC address, the Type2 device can use the table to identify the venue based on the MAC address. A location of a Type2 device in the venue may be computed based on the particular MAC address, the series of probe signals, and/or the at least one TSCI obtained by the Type2 device from the probe signals. The computing may be performed by the Type2 device.

The particular MAC address may be changed over time. It may be changed according to a time table, rule, policy, mode, condition, situation and/or change. The particular MAC address may be selected based on availability of the MAC address, a pre-selected list, collision pattern, traffic pattern, data traffic between the Type1 device and another device, effective bandwidth, random selection, and/or a MAC address switching plan. The particular MAC address may be the MAC address of a second wireless device (e.g. a dummy receiver, or a receiver that serves as a dummy receiver). The Type1 device may transmit the probe signals in a channel selected from a set of channels. At least one CI of the selected channel may be obtained by a respective Type2 device from the probe signal transmitted in the selected channel. The selected channel may be changed over time. The change may be according to a time table, rule, policy, mode, condition, situation, and/or change. The selected channel may be selected based on availability of channels, random selection, a pre-selected list, co-channel interference, inter-channel interference, channel traffic pattern, data traffic between the Type1 device and another device, effective bandwidth associated with channels, security criterion, channel switching plan, a criterion, and/or consideration. The particular MAC address and/or an information of the selected channel may be communicated between the Type1 device and a server through a network. The particular MAC address and/or the information of the selected channel may also be communicated between a Type2 device and a server through another network. The Type2 device may communicate the particular MAC address and/or the information of the selected channel to another Type2 device (e.g. via mesh network, Bluetooth, WiFi, etc.). The particular MAC address and/or selected channel may be chosen by a server. The particular MAC address and/or selected channel may be signaled in an announcement channel by the Type1 device, the Type2 device and/or a server. Before being communicated, any information may be pre-processed.

Wireless connection between the Type1 device and another wireless device may be established (e.g. using a signal handshake). The Type1 device may send a first handshake signal (e.g. sounding frame, probe signal, request-to-send RTS) to the another device. The another device may reply by sending a second handshake signal (e.g. a command, or a clear-to-send CTS) to the Type1 device, triggering the Type1 device to transmit the signal (e.g. series of probe signals) in the broadcasting manner to multiple Type2 devices without establishing connection with any Type2 device. The second handshake signals may be a response or an acknowledge (e.g. ACK) to the first handshake signal. The second handshake signal may contain a data with information of the venue, and/or the Type1 device.

The another device may be a dummy device with a purpose (e.g. primary purpose, secondary purpose) to establish the wireless connection with the Type1 device, to receive the first signal, and/or to send the second signal. The another device may be physically attached to the Type1 device. In another example, the another device may send a third handshake signal to the Type1 device triggering the Type1 device to broadcast the signal (e.g. series of probe signals) to multiple Type2 devices without establishing connection with any Type2 device. The Type1 device may reply to the third special signal by transmitting a fourth handshake signal to the another device. The another device may be used to trigger more than one Type1 devices to broadcast. The triggering may be sequential, partially sequential, partially parallel, or fully parallel. The another device may have more than one wireless circuitries to trigger multiple transmitters in parallel. Parallel trigger may also be achieved using at least one yet another device to perform the triggering (similar to what as the another device does) in parallel to the another device. The another device may not communicate (or suspend communication) with the Type1 device after establishing connection with the Type1 device. Suspended communication may be resumed. The another device may enter an inactive mode, hibernation mode, sleep mode, stand-by mode, low-power mode, OFF mode and/or power-down mode, after establishing the connection with the Type1 device. The another device may have the particular MAC address so that the Type1 device sends the signal to the particular MAC address. The Type1 device and/or the another device may be controlled and/or coordinated by a first processor associated with the Type1 device, a second processor associated with the another device, a third processor associated with a designated source and/or a fourth processor associated with another device. The first and second processors may coordinate with each other.

A first series of probe signals may be transmitted by a first antenna of the Type1 device to at least one first Type2 device through a first channel in a first venue. A second series of probe signals may be transmitted by a second antenna of the Type1 device to at least one second Type2 device through a second channel in a second venue. The first series and the second series may/may not be different. The at least one first Type2 device may/may not be different from the at least one second Type2 device. The first and/or second series of probe signals may be broadcasted without connection established between the Type1 device and any Type2 device. The first and second antennas may be same/different.

The two venues may have different sizes, shape, multipath characteristics. The first and second venues may overlap. The respective immediate areas around the first and second antennas may overlap. The first and second channels may be same/different. For example, the first one may be WiFi while the second may be LTE. Or, both may be WiFi, but the first one may be 2.4GHz WiFi and the second may be 5GHz WiFi. Or, both may be 2.4GHz WiFi, but have different channel numbers, SSID names, and/or WiFi settings. Each Type2 device may obtain at least one TSCI from the respective series of probe signals, the CI being of the respective channel between the Type2 device and the Type1 device. Some first Type2 device(s) and some second Type2 device(s) may be the same. The first and second series of probe signals may be synchronous/asynchronous. A probe signal may be transmitted with data or replaced by a data signal. The first and second antennas may be the same.

The first series of probe signals may be transmitted at a first rate (e.g. 30Hz). The second series of probe signals may be transmitted at a second rate (e.g. 200Hz). The first and second rates may be same/different. The first and/or second rate may be changed over time. The change may be according to a time table, rule, policy, mode, condition, situation, and/or change. Any rate may be changed over time. The first and/or second series of probe signals may be transmitted to a first MAC address and/or second MAC address respectively. The two MAC addresses may be same/different. The first series of probe signals may be transmitted in a first channel. The second series of probe signals may be transmitted in a second channel. The two channels may be same/different. The first or second MAC address, first or second channel may be changed over time. Any change may be according to a time table, rule, policy, mode, condition, situation, and/or change.

The Type1 device and another device may be controlled and/or coordinated, physically attached, or may be of/in/of a common device. They may be controlled by/connected to a common data processor, or may be connected to a common bus interconnect/ network/ LAN/ Bluetooth network/ BLE network/ wired network/ wireless network/ mesh network/ mobile network/ cloud. They may share a common memory, or be associated with a common user, user device, profile, account, identity (ID), household, house, physical address, location, geographic coordinate, IP subnet, SSID, home device, office device, and/or manufacturing device. Each Type1 device may be a signal source of a set of respective Type2 devices (i.e. it sends a respective signal (e.g. respective series of probe signals) to the set of respective Type2 devices). Each respective Type2 device chooses the Type1 device from among all Type1 devices as its signal source. Each Type2 device may choose asynchronously. At least one TSCI may be obtained by each respective Type2 device from the respective series of probe signals from the Type1 device, the CI being of the channel between the Type2 device and the Type1 device.

The respective Type2 device chooses the Type1 device from among all Type1 devices as its signal source based on identity (ID) of Type1/Type2 device, task to be performed, past signal source, history (e.g. of past signal source, Type1 device, another Type1 device, respective Type2 receiver, and/or another Type2 receiver), threshold for switching signal source, and/or information of a user, account, access info, parameter, characteristics, and/or signal strength (e.g. associated with the Type1 device and/or the respective Type2 receiver). Initially, the Type1 device may be signal source of a set of initial respective Type2 devices (i.e. the Type1 device sends a respective signal (series of probe signals) to the set of initial respective Type2 devices) at an initial time. Each initial respective Type2 device chooses the Type1 device from among all Type1 devices as its signal source.

The signal source (Type1 device) of a particular Type2 device may be changed when (1) time interval between two adjacent probe signals (e.g. between current probe signal and immediate past probe signal, or between next probe signal and current probe signal) received from current signal source of the Type2 device exceeds a first threshold; (2) signal strength associated with current signal source of the Type2 device is below a second threshold; (3) a processed signal strength associated with current signal source of the Type2 device is below a third threshold, the signal strength processed with low pass filter, band pass filter, median filter, moving average filter, weighted averaging filter, linear filter and/or non-linear filter; and/or (4) signal strength (or processed signal strength) associated with current signal source of the Type2 device is below a fourth threshold for a significant percentage of a recent time window (e.g. 70%, 80%, 90%, etc.). The percentage may exceed a fifth threshold. The first, second, third, fourth and/or fifth thresholds may be time varying. Condition (1) may occur when the Type1 device and the Type2 device become progressively far away from each other, such that some probe signal from the Type1 device becomes too weak and is not received by the Type2 device. Conditions (2)-(4) may occur when the two devices become far from each other such that the signal strength becomes very weak. The signal source of the Type2 device may not change if other Type1 devices have signal strength weaker than a factor (e.g. 1, 1.1, 1.2, or 1.5, etc.) of the current signal source. If the signal source is changed, the new signal source may take effect at a near future time (e.g. the respective next time). The new signal source may be the Type1 device with strongest signal strength, and/or processed signal strength. The current and new signal source may be same/different. A list of available Type1 devices may be initialized and maintained by each Type2 device. The list may be updated by examining signal strength and/or processed signal strength associated with the respective set of Type1 devices.

A Type2 device may choose between a first series of probe signals from a first Type1 device and a second series of probe signals from a second Type1 device based on: respective probe signal rate, MAC addresses, channels, characteristics/properties/ states, task to be performed by the Type2 device, signal strength of first and second series, and/or another consideration. The series of probe signals may be transmitted at a regular rate (e.g. 100 Hz). The series of probe signals may be scheduled at a regular interval (e.g. 0.01s for 100 Hz), but each probe signal may experience small time perturbation, perhaps due to timing requirement, timing control, network control, handshaking, message passing, collision avoidance, carrier sensing, congestion, availability of resources, and/or another consideration. The rate may be changed. The change may be according to a time table (e.g. changed once every hour), rule, policy, mode, condition and/or change (e.g. changed whenever some event occur). For example, the rate may normally be 100Hz, but changed to 1000Hz in demanding situations, and to 1Hz in low power/standby situation. The probe signals may be sent in burst.

The probe signal rate may change based on a task performed by the Type1 device or Type2 device (e.g. a task may need 100 Hz normally and 1000 Hz momentarily for 20 seconds). In one example, the transmitters (Type1 devices), receivers (Type2 device), and associated tasks may be associated adaptively to classes (e.g. classes that are: low-priority, high-priority, emergency, critical, regular, privileged, non-subscription, subscription, paying, and/or non-paying). A rate (of a transmitter) may be adjusted for the sake of some class (e.g. high priority class). When the need of that class changes, the rate may be changed. When a receiver has critically low power, the rate may be reduced to reduce power consumption of the receiver to respond to the probe signals. In one example, probe signals may be used to transfer power wirelessly to a receiver (Type2 device), and the rate may be adjusted to control the amount of power transferred to the receiver. The rate may be changed by (or based on): a server, the Type1 device and/or the Type2 device. Control signals may be communicated between them. The server may monitor, track, forecast and/or anticipate the needs of the Type2 device and/or the tasks performed by the Type2 device, and may control the Type1 device to change the rate. The server may make scheduled changes to the rate according to a time table. The server may detect an emergency situation and change the rate immediately. The server may detect a developing condition and adjust the rate gradually.

The characteristics and/or spatial-temporal information (e.g. motion information) may be monitored individually based on a TSCI associated with a particular Type1 device and a particular Type2 device, and/or monitored jointly based on any TSCI associated with the particular Type1 device and any Type2 device, and/or monitored jointly based on any TSCI associated with the particular Type2 device and any Type1 device, and/or monitored globally based on any TSCI associated with any Type1 device and any Type2 device. Any joint monitoring may be associated with: a user, user account, profile, household, map of venue, and/or user history, etc.

A first channel between a Type1 device and a Type2 device may be different from a second channel between another Type1 device and another Type2 device. The two channels may be associated with different frequency bands, bandwidth, carrier frequency, modulation, wireless standards, coding, encryption, payload characteristics, networks, network ID, SSID, network characteristics, network settings, and/or network parameters, etc. The two channels may be associated with different kinds of wireless system (e.g. two of the following: WiFi, LTE, LTE-A, 2.5G, 3G, 3.5G, 4G, beyond 4G, 5G, 6G, 7G, 802.11 system, 802.15 system, 802.16 system, mesh network, Zigbee, WiMax, Bluetooth, BLE, RFID, UWB, microwave system, radar like system, etc.). For example, one is WiFi and the other is LTE. The two channels may be associated with similar kinds of wireless system, but in different network. For example, the first channel may be associated with a WiFi network named "Pizza and Pizza" in the 2.4GHz band with a bandwidth of 20MHz while the second may be associated with a WiFi network with SSID of "StarBud hotspot" in the 5GHz band with a bandwidth of 40MHz. The two channels may be different channels in same network (e.g. the "StarBud hotspot" network).

In one embodiment, a wireless monitoring system may comprise training a classifier of multiple events in a venue based on training TSCI associated with the multiple events. For each of the multiple known events happening in the venue in a respective training time period associated with the known event, a respective training wireless signal (e.g. a respective series of training probe signals) may be transmitted by an antenna of a first Type1 heterogeneous wireless device using a processor, a memory and a set of instructions of the first Type1 device to at least one first Type2 heterogeneous wireless device through a wireless multipath channel in the venue in the respective training time period. At least one respective time series of training CI (training TSCI) may be obtained asynchronously by each of the at least one first Type2 device from the (respective) training signal. The CI may be CI of the channel between the first Type2 device and the first Type1 device in the training time period associated with the known event. The at least one training TSCI may be preprocessed.

For a current event happening in the venue in a current time period, a current wireless signal (e.g. a series of current probe signals) may be transmitted by an antenna of a second Type1 heterogeneous wireless device using a processor, a memory and a set of instructions of the second Type1 device to at least one second Type2 heterogeneous wireless device through the channel in the venue in the current time period associated with the current event. At least one time series of current CI (current TSCI) may be obtained asynchronously by each of the at least one second Type2 device from the current signal (e.g. the series of current probe signals). The CI may be CI of the channel between the second Type2 device and the second Type1 device in the current time period associated with the current event. The at least one current TSCI may be preprocessed.

The classifier may be applied to classify at least one current TSCI obtained from the series of current probe signals by the at least one second Type2 device, to classify at least one portion of a particular current TSCI, and/or to classify a combination of the at least one portion of the particular current TSCI and another portion of another TSCI. The classifier may also be applied to associate the current event with a known event, an unknown event and/or another event. Each TSCI may comprise at least one CI each associated with a respective timestamp. Two TSCI associated with two Type2 devices may be different with different: starting time, duration, stopping time, amount of CI, sampling frequency, sampling period. Their CI may have different features. The first and second Type1 devices may be at same location in the venue. They may be the same device. The at least one second Type2 device (or their locations) may be a permutation of the at least one first Type2 device (or their locations). A particular second Type2 device and a particular first Type2 device may be the same device.

A subset of the first Type2 device and a subset of the second Type2 device may be the same. The at least one second Type2 device and/or a subset of the at least one second Type2 device may be a subset of the at least one first Type2 device. The at least one first Type2 device and/or a subset of the at least one first Type2 device may be a permutation of a subset of the at least one second Type2 device. The at least one second Type2 device and/or a subset of the at least one second Type2 device may be a permutation of a subset of the at least one first Type2 device. The at least one second Type2 device and/or a subset of the at least one second Type2 device may be at same respective location as a subset of the at least one first Type2 device. The at least one first Type2 device and/or a subset of the at least one first Type2 device may be at same respective location as a subset of the at least one second Type2 device.

The antenna of the Type1 device and the antenna of the second Type1 device may be at same location in the venue. Antenna(s) of the at least one second Type2 device and/or antenna(s) of a subset of the at least one second Type2 device may be at same respective location as respective antenna(s) of a subset of the at least one first Type2 device. Antenna(s) of the at least one first Type2 device and/or antenna(s) of a subset of the at least one first Type2 device may be at same respective location(s) as respective antenna(s) of a subset of the at least one second Type2 device.

A first section of a first time duration of the first TSCI and a second section of a second time duration of the second section of the second TSCI may be aligned. A map between items of the first section and items of the second section may be computed. The first section may comprise a first segment of the first TSCI with a first starting /ending time, and/or another segment of a processed first TSCI. The processed first TSCI may be the first TSCI processed by a first operation. The second section may comprise a second segment of the second TSCI with a second starting time and a second ending time, and another segment of a processed second TSCI. The processed second TSCI may be the second TSCI processed by a second operation.

The first operation and/or the second operation may comprise: subsampling, re-sampling, interpolation, filtering, transformation, feature extraction, pre-processing, and/or another operation. A first item of the first section may be mapped to a second item of the second section. The first item of the first section may also be mapped to another item of the second section. Another item of the first section may also be mapped to the second item of the second section. The mapping may be one-to-one, one-to-many, many-to-one, many-to-many. At least one function of at least one of: the first item of the first section of the first TSCI, another item of the first TSCI, timestamp of the first item, time difference of the first item, time differential of the first item, neighboring timestamp of the first item, another timestamp associated with the first item, the second item of the second section of the second TSCI, another item of the second TSCI, timestamp of the second item, time difference of the second item, time differential of the second item, neighboring timestamp of the second item, and another timestamp associated with the second item, may satisfy at least one constraint.

One constraint may be that a difference between the timestamp of the first item and the timestamp of the second item may be upper-bounded by an adaptive upper threshold and lower-bounded by an adaptive lower threshold. The first section may be the entire first TSCI. The second section may be the entire second TSCI. The first time duration may be equal to the second time duration. A section of a time duration of a TSCI may be determined adaptively. A tentative section of the TSCI may be computed. A starting time and an ending time of a section (e.g. the tentative section, the section) may be determined. The section may be determined by removing a beginning portion and an ending portion of the tentative section. A beginning portion of a tentative section may be determined as follows. Iteratively, items of the tentative section with increasing timestamp may be considered as a current item, one item at a time. In each iteration, at least one activity measure may be computed and/or considered. The at least one activity measure may be associated with at least one of: the current item associated with a current timestamp, past items of the tentative section with timestamps not larger than the current timestamp, and/or future items of the tentative section with timestamps not smaller than the current timestamp. The current item may be added to the beginning portion of the tentative section if at least one criterion associated with the at least one activity measure is satisfied.

The at least one criterion associated with the activity measure may comprise at least one of: (a) the activity measure is smaller than an adaptive upper threshold, (b) the activity measure is larger than an adaptive lower threshold, (c) the activity measure is smaller than an adaptive upper threshold consecutively for at least a predetermined amount of consecutive timestamps, (d) the activity measure is larger than an adaptive lower threshold consecutively for at least another predetermined amount of consecutive timestamps, (e) the activity measure is smaller than an adaptive upper threshold consecutively for at least a predetermined percentage of the predetermined amount of consecutive timestamps, (f) the activity measure is larger than an adaptive lower threshold consecutively for at least another predetermined percentage of the another predetermined amount of consecutive timestamps, (g) another activity measure associated with another timestamp associated with the current timestamp is smaller than another adaptive upper threshold and larger than another adaptive lower threshold, (h) at least one activity measure associated with at least one respective timestamp associated with the current timestamp is smaller than respective upper threshold and larger than respective lower threshold, (i) percentage of timestamps with associated activity measure smaller than respective upper threshold and larger than respective lower threshold in a set of timestamps associated with the current timestamp exceeds a threshold, and (j) another criterion.

An activity measure associated with an item at time T1 may comprise at least one of: (1) a first function of the item at time T1 and an item at time T1-D1, wherein D1 is a pre-determined positive quantity (e.g. a constant time offset), (2) a second function of the item at time T1 and an item at time T1+D1, (3) a third function of the item at time T1 and an item at time T2, wherein T2 is a pre-determined quantity (e.g. a fixed initial reference time; T2 may be changed over time; T2 may be updated periodically; T2 may be the beginning of a time period and T1 may be a sliding time in the time period), and (4) a fourth function of the item at time T1 and another item.

At least one of: the first function, the second function, the third function, and/or the fourth function may be a function (e.g. $F(X, Y, ...)$) with at least two arguments: X and Y. The two arguments may be scalars. The function (e.g. F) may be a function of at least one of: X, Y, (X-Y), (Y-X), abs(X-Y), $X^a$, $Y^b$, abs($X^a$ - $Y^b$), $(X-Y)^a$, (X/Y), (X+a)/(Y+b), ($X^a/Y^b$), and ((X/Y)"a-b), wherein a and b are may be some predetermined quantities. For example, the function may simply be abs(X-Y), or $(X-Y)^2$, (X-Y)"4. The function may be a robust function. For example, the function may be $(X-Y)^2$ when abs (X-Y) is less than a threshold T, and (X-Y)+a when abs(X-Y) is larger than T. Alternatively, the function may be a constant when abs(X-Y) is larger than T. The function may also be bounded by a slowly increasing function when abs(X-y) is larger than T, so that outliers cannot severely affect the result. Another example of the function may be (abs(X/Y)-a), where *a=1*. In this way, if X=Y (i.e. no change or no activity), the function will give a value of 0. If X is larger than Y, (X/Y) will be larger than 1 (assuming X and Y are positive) and the function will be positive. And if X is less than Y, (X/Y) will be smaller than 1 and the function will be negative. In another example, both arguments X and Y may be n-tuples such that $X=(x_1, x_2, ..., x_n)$ and $Y=(y_1, y_2, ..., y_n)$. The function may be a function of at least one of: $x_i$, $y_i$, $(x_i - y_i)$, $(y_i - x_i)$, abs($x_i - y_i$), $x_i^a$, $y_i^b$, abs($x_i^a - y_i^b$), $(x_i - y_i)^a$, $(x_i/y_i)$, $(x_i+a)/(y_i +b)$, $(x_i^a/y_i^b)$, and $((x_i/y_i)^a-b)$, wherein i is a component index of the n-tuple X and Y, and 1<=i<=n. E.g. component index of $x_1$ is i=1, component index of $x_2$ is i=2. The function may comprise a component-by-component summation of another function of at least one of the following: $x_i$, $y_i$, $(x_i - y_i)$, $(y_i - x_i)$, abs($x_i - y_i$), $x_i^a$, $y_i^b$, abs($x_i^a - y_i^b$), $(x_i - y_i)^a$, $(x_i/y_i)$, $(x_i+a)/(y_i +b)$, $(x_i"a/y_i^b)$, and $((x_i/y_i)^a-b)$, wherein i is the component index of the n-tuple X and Y. For example, the function may be in a form of $sum_{i=1}^n (abs(x_i/y_i)-1)/n$, or $sum_{i=1}^n w_i*(abs(x_i/y_i)-1)$, where $w_i$ is some weight for component i.

The map may be computed using dynamic time warping (DTW). The DTW may comprise a constraint on at least one of: the map, the items of the first TSCI, the items of the second TSCI, the first time duration, the second time duration, the first section, and/or the second section. Suppose in the map, the i^{th} domain item is mapped to the j^{th} range item. The constraint may be on admissible combination of i and j (constraint on relationship between i and j). Mismatch cost between a first section of a first time duration of a first TSCI and a second section of a second time duration of a second TSCI may be computed. The first section and the second section may be aligned such that a map comprising more than one links may be established between first items of the first TSCI and second items of the second TSCI. With each link, one of the first items with a first timestamp may be associated with one of the second items with a second timestamp. A mismatch cost between the aligned first section and the aligned second section may be computed. The mismatch cost may comprise a function of: an item-wise cost between a first item and a second item associated by a particular link of the map, and a link-wise cost associated with the particular link of the map.

The aligned first section and the aligned second section may be represented respectively as a first vector and a second vector of same vector length. The mismatch cost may comprise at least one of: an inner product, inner-product-like quantity, quantity based on correlation, quantity based on covariance, discriminating score, distance, Euclidean distance, absolute distance, Lk distance (e.g. L1, L2, ... ), weighted distance, distance-like quantity and/or another similarity value, between the first vector and the second vector. The mismatch cost may be normalized by the respective vector length.

A parameter derived from the mismatch cost between the first section of the first time duration of the first TSCI and the second section of the second time duration of the second TSCI may be modeled with a statistical distribution. At least one of: a scale parameter, location parameter and/or another parameter, of the statistical distribution may be estimated. The first section of the first time duration of the first TSCI may be a sliding section of the first TSCI. The second section of the second time duration of the second TSCI may be a sliding section of the second TSCI. A first sliding window may be applied to the first TSCI and a corresponding second sliding window may be applied to the second TSCI. The first sliding window of the first TSCI and the corresponding second sliding window of the second TSCI may be aligned. Mismatch cost between the aligned first sliding window of the first TSCI and the corresponding aligned second sliding window of the second TSCI may be computed. The current event may be associated with at least one of: the known event, the unknown event and/or the another event, based on the mismatch cost.

The classifier may be applied to at least one of: each first section of the first time duration of the first TSCI, and/or each second section of the second time duration of the second TSCI, to obtain at least one tentative classification results. Each tentative classification result may be associated with a respective first section and a respective second section. The current event may be associated with at least one of: the known event, the unknown event and/or the another event, based on the mismatch cost. The current event may be associated with at least one of: the known event, the unknown event and/or the another event, based on a largest number of tentative classification results in more than one sections of the first TSCI and corresponding more than sections of the second TSCI. For example, the current event may be associated with a particular known event if the mismatch cost points to the particular known event for N consecutive times (e.g. N=10). In another example, the current event may be associated with a particular known event if the percentage of mismatch cost within the immediate past N consecutive N pointing to the particular known event exceeds a certain threshold (e.g. >80%).

In another example, the current event may be associated with a known event that achieve smallest mismatch cost for the most times within a time period. The current event may be associated with a known event that achieves smallest overall mismatch cost, which is a weighted average of at least one mismatch cost associated with the at least one first sections. The current event may be associated with a particular known event that achieves smallest of another overall cost. The current event may be associated with the "unknown event" if none of the known events achieve mismatch cost lower than a first threshold T1 in a sufficient percentage of the at least one first section. The current event may also be associated with the "unknown event" if none of the events achieve an overall mismatch cost lower than a second threshold T2. The current event may be associated with at least one of: the known event, the unknown event and/or the another event, based on the mismatch cost and additional mismatch cost associated with at least one additional section of the first TSCI and at least one additional section of the second TSCI. The known events may comprise at least one of: a door closed event, door open event, window closed event, window open event, multi-state event, on-state event, off-state event, intermediate state event, continuous state event, discrete state event, human-present event, human-absent event, sign-of-life-present event, and/or a sign-of-life-absent event. A projection for each CI may be trained using a dimension reduction method based on the training TSCI. The dimension reduction method may comprise at least one of: principal component analysis (PCA), PCA with different kernel, independent component analysis (ICA), Fisher linear discriminant, vector quantization, supervised learning, unsupervised learning, self-organizing maps, auto-encoder, neural network, deep neural network, and/or another method. The projection may be applied to at least one of: the training TSCI associated with the at least one events, and/or the current TSCI, for the classifier. The classifier of the at least one event may be trained based on the projection and the training TSCI associated with the at least one event. The at least one current TSCI may be classified based on the projection and the current TSCI. The projection may be re-trained using at least one of: the dimension reduction method, and another dimension reduction method, based on at least one of: the training TSCI, at least one current TSCI before retraining the projection, and/or additional training TSCI. The another dimension reduction method may comprise at least one of: principal component analysis (PCA), PCA with different kernels, independent component analysis (ICA), Fisher linear discriminant, vector quantization, supervised learning, unsupervised learning, self-organizing maps, auto-encoder, neural network, deep neural network, and/or yet another method. The classifier of the at least one event may be re-trained based on at least one of: the re-trained projection, the training TSCI associated with the at least one events, and/or at least one current TSCI. The at least one current TSCI may be classified based on: the re-trained projection, the re-trained classifier, and/or the current TSCI. Each CI may comprise a vector of complex values. Each complex value may be preprocessed to give the magnitude of the complex value. Each CI may be preprocessed to give a vector of non-negative real numbers comprising the magnitude of corresponding complex values. Each training TSCI may be weighted in the training of the projection. The projection may comprise more than one projected components. The projection may comprise at least one most significant projected component. The projection may comprise at least one projected component that may be beneficial for the classifier.

## Channel/channel information/venue/spatial-temporal info/motion/object

The channel information (CI) may be associated with/may comprise signal strength, signal amplitude, signal phase, received signal strength indicator (RSSI), channel state information (CSI), channel impulse response (CIR), channel frequency response (CFR), channel characteristics, channel filter response, timestamp, auxiliary information, data, meta data, user data, account data, access data, security data, session data, status data, supervisory data, household data, identity (ID), device data, network data, neighborhood data, environment data, real-time data, sensor data, stored data, encrypted data, compressed data, protected data, and/or another channel information. The CI may be associated with information associated with a frequency band, frequency signature, frequency phase, frequency amplitude, frequency trend, frequency characteristics, frequency-like characteristics, time domain element, frequency domain element, time-frequency domain element, orthogonal decomposition characteristics, and/or non-orthogonal decomposition characteristics of the signal through the channel.

The CI may also be associated with information associated with a time period, time signature, timestamp, time amplitude, time phase, time trend, and/or time characteristics of the signal. The CI may be associated with information associated with a time-frequency partition, signature, amplitude, phase, trend, and/or characteristics of the signal. The CI may be associated with a decomposition of the signal. The CI may be associated with information associated with a direction, angle of arrival (AoA), angle of a directional antenna, and/or a phase of the signal through the channel. The CI may be associated with attenuation patterns of the signal through the channel. Each CI may be associated with a Typel device and a Type2 device. Each CI may be associated with an antenna of the Typel device and an antenna of the Type2 device. The CI may be obtained from a communication hardware that is capable of providing the CI. The communication hardware may be a WiFi-capable chip/IC (integrated circuit), chip compliant with a 802.11 or 802.16 or another wireless/radio standard, next generation WiFi-capable chip, LTE-capable chip, 5G-capable chip, 6G/7G/8G-capable chip, Bluetooth-enabled chip, BLE (Bluetooth low power)-enabled chip, UWB chip, another communication chip (e.g. Zigbee, WiMax, mesh network), etc. The communication hardware computes the CI and stores the CI in a buffer memory and make the CI available for extraction. The CI may comprise data and/or at least one matrices related to channel state information (CSI). The at least one matrices may be used for channel equalization, and/or beam forming, etc.

The channel may be associated with a venue. The attenuation may be due to signal propagation in the venue, signal propagating/reflection/ refraction/ diffraction through/at/around air (e.g. air of venue), refraction medium/reflection surface such as wall, doors, furniture, obstacles and/or barriers, etc. The attenuation may be due to reflection at surfaces and obstacles (e.g. reflection surface, obstacle) such as floor, ceiling, furniture, fixtures, objects, people, pets, etc. Each CI may be associated with a timestamp. Each CI may comprise N1 components (e.g. N1 frequency domain components in CFR, N1 time domain components in CIR, or N1 decomposition components). Each component may be associated with a component index. Each component may be a real, imaginary, or complex quantity, magnitude, phase, flag, and/or set. Each CI may comprise a vector or matrix of complex numbers, a set of mixed quantities, and/or a multi-dimensional collection of at least one complex numbers.

Components of a TSCI associated with a particular component index may form a respective component time series associated with the respective index. A TSCI may be divided into N1 component time series. Each respective component time series is associated with a respective component index. The characteristics/spatial-temporal information of the motion of the object may be monitored based on the component time series.

A component-wise characteristics of a component-feature time series of a TSCI may be computed. The component-wise characteristics may be a scalar (e.g. energy) or a function with a domain and a range (e.g. an autocorrelation function, transform, inverse transform). The characteristics/spatial-temporal information of the motion of the object may be monitored based on the component-wise characteristics. A total characteristics of the TSCI may be computed based on the component-wise characteristics of each component time series of the TSCI. The total characteristics may be a weighted average of the component-wise characteristics. The characteristics/spatial-temporal information of the motion of the object may be monitored based on the total characteristics.

The Typel device and Type2 device may support WiFi, WiMax, 3G/beyond 3G, 4G/beyond 4G, LTE, 5G, 6G, 7G, Bluetooth, BLE, Zigbee, UWB, mesh network, proprietary wireless system, IEEE 802.11 standard, 802.15 standard, 802.16 standard, 3GPP standard, and/or another wireless system. A common wireless system and/or a common wireless channel may be shared by the Typel transceiver and/or the at least one Type2 transceiver. The at least one Type2 transceiver may transmit respective signal contemporaneously using the common wireless system and/or the common wireless channel. The Type1 transceiver may transmit a signal to the at least one Type2 transceiver using the common wireless system and/or the common wireless channel. A Type1 device may temporarily function as a Type2 device, and vice versa. A device may function as both a Type1 device (wireless transmitter) and a Type2 device (wireless receiver concurrently. Each Type1 device and Type2 device may have at least one transmitting/receiving antenna. Each CI may be associated with one of the transmitting antenna of the Type1 device and one of the receiving antenna of the Type2 device.

The at least one TSCI may correspond to various antenna pairs between the Type1 device and the Type2 device. The Type1 device may have at least one antenna. The Type2 device may also have at least one antenna. Each TSCI may be associated with an antenna of the Type1 device and an antenna of the Type2 device. Averaging or weighted averaging over antenna links may be performed. The averaging or weighted averaging may be over the at least one TSCI. The averaging may optionally be performed on a subset of the at least one TSCI corresponding to a subset of the antenna pairs. Timestamps of CI of a portion of a TSCI may be irregular and may be corrected so that corrected timestamps of time-corrected CI may be uniformly spaced in time. In the case of multiple Type1 devices and/or multiple Type2 devices, the corrected timestamp may be with respect to the same or different clock. An original timestamp associated with each of the CI may be determined. The original timestamp may not be uniformly spaced in time. Original timestamps of all CI of the particular portion of the particular TSCI in the current sliding time window may be corrected so that corrected timestamps of time-corrected CI may be uniformly spaced in time.

The characteristics and/or spatial-temporal information (e.g. motion information) may comprise: location, location coordinate, change in location, position (e.g. initial position, new position), position on map, height, horizontal location, vertical location, distance, displacement, speed, acceleration, rotational speed, rotational acceleration, angle of motion, azimuth, direction of motion, rotation, path, deformation, transformation, shrinking, expanding, gait, gait cycle, head motion, repeated motion, periodic motion, pseudo-periodic motion, impulsive motion, sudden motion, fall-down motion, transient motion, behavior, transient behavior, period of motion, frequency of motion, time trend, temporal profile, temporal characteristics, occurrence, change, change in frequency, change in timing, change of gait cycle, timing, starting time, ending time, duration, history of motion, motion type, motion classification, frequency, frequency spectrum, frequency characteristics, presence, absence, proximity, approaching, receding, identity of the object, composition of the object, head motion rate, head motion direction, mouth-related rate, eye-related rate, breathing rate, heart rate, hand motion rate, hand motion direction, leg motion, body motion, walking rate, hand motion rate, positional characteristics, characteristics associated with movement of the object, tool motion, machine motion, complex motion, and/or combination of multiple motions, event, motion statistics, motion magnitude, motion phase, similarity score, distance score, Euclidean distance, weighted distance, $L\_1$ norm, $L\_2$ norm, $L\_k$ norm for k>2, statistical distance, correlation, auto-correlation, covariance, auto-covariance, cross-covariance, inner product, outer product, motion signal transformation, motion feature, presence of motion, absence of motion, motion localization, motion identification, motion recognition, presence of object, absence of object, entrance of object, exit of object, a change of object, motion cycle, motion count, gait cycle, motion rhythm, deformation motion, gesture, handwriting, head motion, mouth motion, heart motion ,internal organ motion, motion trend, size, length, area, volume, capacity, shape, form, tag, starting location, ending location, starting quantity, ending quantity, event, fall-down event, security event, accident event, home event, office event, factory event, warehouse event, manufacturing event, assembly line event, maintenance event, car-related event, navigation event, tracking event, door event, door-open event, door-close event, window event, window-open event, window-close event, repeatable event, one-time event, consumed quantity, unconsumed quantity, state, physical state, health state, well-being state, emotional state, mental state, another event, and/or another information. The processor shares computational workload with the Type1 heterogeneous wireless device and Type2 heterogeneous wireless device.

The Type1 device and/or Type2 device may be a local device. The local device may be: a smart phone, smart device, TV, sound bar, set-top box, access point, router, repeater, remote control, speaker, fan, refrigerator, microwave, oven, coffee machine, hot water pot, utensil, table, chair, light, lamp, door lock, camera, microphone, motion sensor, security device, fire hydrant, garage door, switch, power adapter, computer, dongle, computer peripheral, electronic pad, sofa, tile, accessory, smart home device, smart vehicle device, smart office device, smart building device, smart manufacturing device, smart watch, smart glasses, smart clock, smart television, smart oven, smart refrigerator, smart air-conditioner, smart chair, smart table, smart accessory, smart utility, smart appliance, smart machine, smart vehicle, internet-of-thing (IoT) device, internet-enabled device, computer, portable computer, tablet, smart house, smart office, smart building, smart parking lot, smart system, and/or another device. Each Type1 device may be associated with a respective identify (ID). Each Type2 device may also be associated with a respective identify (ID). The ID may comprise: numeral, combination of text and numbers, name, password, account, account ID, web link, web address, index to some information, and/or another ID. The ID may be assigned. The ID may be assigned by hardware (e.g. hardwired, via dongle and/or other hardware), software and/or firmware. The ID may be stored (e.g. in database, in memory, in server, in the cloud, stored locally, stored remotely, stored permanently, stored temporarily) and may be retrieved. The ID may be associated with at least one record, account, user, household, address, phone number, social security number, customer number, another ID, timestamp, and/or collection of data. The ID and/or part of the ID of a Type1 device may be made available to a Type2 device. The ID may be used for registration, initialization, communication, identification, verification, detection, recognition, authentication, access control, cloud access, networking, social networking, logging, recording, cataloging, classification, tagging, association, pairing, transaction, electronic transaction, and/or intellectual property control, by the Type1 device and/or the Type2 device.

The object may be person, passenger, child, older person, baby, sleeping baby, baby in vehicle, patient, worker, high-value worker, expert, specialist, waiter, customer in mall, traveler in airport/ train station/ bus terminal/ shipping terminals, staff/worker/customer service personnel in factory/ mall/ supermarket/ office/ workplace, serviceman in sewage/air ventilation system/lift well, lifts in lift wells, elevator, inmate, people to be tracked/ monitored, animal, plant, living object, pet, dog, cat, smart phone, phone accessory, computer, tablet, portable computer, dongle, computing accessory, networked devices, WiFi devices, IoT devices, smart watch, smart glasses, smart devices, speaker, keys, smart key, wallet, purse, handbag, backpack, goods, cargo, luggage, equipment, motor, machine, air conditioner, fan, air conditioning equipment, light fixture, moveable light, television, camera, audio and/or video equipment, stationary, surveillance equipment, parts, signage, tool, cart, ticket, parking ticket, toll ticket, airplane ticket, credit card, plastic card, access card, food packaging, utensil, table, chair, cleaning equipment/tool, vehicle, car, cars in parking facilities, merchandise in warehouse/ store/ supermarket/ distribution center, boat, bicycle, airplane, drone, remote control car/-plane/ boat, robot, manufacturing device, assembly line, material/unfinished part/robot/wagon/ transports on factory floor, object to be tracked in airport/shopping mart/supermarket, non-object, absence of an object, presence of an object, object with form, object with changing form, object with no form, mass of fluid, mass of liquid, mass of gas/smoke, fire, flame, electromagnetic (EM) source, EM medium, and/or another object.

The object itself may be communicatively coupled with some network, such as WiFi, MiFi, 3G/ 4G/ LTE/ 5G, Bluetooth, BLE, WiMax, Zigbee, mesh network, adhoc network, and/or other network. The object itself may be bulky with AC power supply, but is moved during installation, cleaning, maintenance, renovation, etc. It may also be installed in moveable platform such as lift, pad, movable, platform, elevator, conveyor belt, robot, drone, forklift, car, boat, vehicle, etc.

The object may have multiple parts, each part with different movement. For example, the object may be a person walking forward. While walking, his left hand and right hand may move in different direction, with different instantaneous speed, acceleration, motion, etc.

The wireless transmitter (e.g. Type1 device), the wireless receiver (e.g. Type2 device), another wireless transmitter and/or another wireless receiver may move with the object and/or another object (e.g. in prior movement, current movement and/or future movement. They may be communicatively coupled to one or more nearby device. They may transmit TSCI and/or information associated with the TSCI to the nearby device, and/or each other. They may be with the nearby device. The wireless transmitter and/or the wireless receiver may be part of a small (e.g. coin-size, cigarette box size, or even smaller), light-weight portable device. The portable device may be wirelessly coupled with a nearby device.

The nearby device may be smart phone, iPhone, Android phone, smart device, smart appliance, smart vehicle, smart gadget, smart TV, smart refrigerator, smart speaker, smart watch, smart glasses, smart pad, iPad, computer, wearable computer, notebook computer, gateway. The nearby device may be connected to a cloud server, local server and/or other server via internet, wired internet connection and/or wireless internet connection. The nearby device may be portable. The portable device, the nearby device, a local server and/or a cloud server may share the computation and/or storage for a task (e.g. obtain TSCI, determine characteristics/spatial-temporal information of the object associated with the movement of the object, computation of time series of power information, determining/computing the particular function, searching for local extremum, classification, identifying particular value of time offset, de-noising, processing, simplification, cleaning, wireless smart sensing task, extract CI from signal, switching, segmentation, estimate trajectory, process the map, correction, corrective adjustment, adjustment, map-based correction, detecting error, checking for boundary hitting, thresholding, etc.) and information (e.g. TSCI).

The nearby device may/ may not move with the object. The nearby device may be portable/ not portable/ moveable/ non-moveable. The nearby device may use battery power, solar power, AC power and/or other power source. The nearby device may have replaceable/non-replaceable battery, and/or rechargeable/non-rechargeable battery. The nearby device may be similar to the object. The nearby device may have identical (and/or similar) hardware and/or software to the object. The nearby device may be a smart device, network enabled device, device with connection to WiFi/ 3G/ 4G/ 5G/ 6G/ Zigbee/ Bluetooth/ adhoc network/ other network, smart speaker, smart watch, smart clock, smart appliance, smart machine, smart equipment, smart tool, smart vehicle, internet-of-thing (IoT) device, internet-enabled device, computer, portable computer, tablet, and another device.

The nearby device and/or at least one processor associated with the wireless receiver, the wireless transmitter, the another wireless receiver, the another wireless transmitter and/or a cloud server (in the cloud) may determine the initial spatial-temporal information of the object. Two or more of them may determine the initial spatial-temporal info jointly. Two or more of them may share intermediate information in the determination of the initial spatial-temporal information (e.g. initial position). In one example, the wireless transmitter (e.g. Type1 device, or Tracker Bot) may move with the object. The wireless transmitter may send the signal to the wireless receiver (e.g. Type2 device, or Origin Register) or determining the initial spatial-temporal information (e.g. initial position) of the object. The wireless transmitter may also send the signal and/or another signal to another wireless receiver (e.g. another Type2 device, or another Origin Register) for the monitoring of the motion (spatial-temporal info) of the object. The wireless receiver may also receive the signal and/or another signal from the wireless transmitter and/or the another wireless transmitter for monitoring the motion of the object. The location of the wireless receiver and/or the another wireless receiver may be known. In another example, the wireless receiver (e.g. Type2 device, or Tracker Bot) may move with the object. The wireless receiver may receive the signal transmitted from the wireless transmitter (e.g. Type1 device, or Origin Register) for determining the initial spatial-temporal info (e.g. initial position) of the object. The wireless receiver may also receive the signal and/or another signal from another wireless transmitter (e.g. another Type1 device, or another Origin Register) for the monitoring of the current motion (e.g. spatial-temporal info) of the object. The wireless transmitter may also transmit the signal and/or another signal to the wireless receiver and/or the another wireless receiver (e.g. another Type2 device, or another Tracker Bot) for monitoring the motion of the object. The location of the wireless transmitter and/or the another wireless transmitter may be known.

The venue may be a space such as a room, house, office, workplace, hallway, walkway, lift, lift well, escalator, elevator, sewage system, air ventilations system, staircase, gathering area, duct, air duct, pipe, tube, enclosed structure, semi-enclosed structure, enclosed area, area with at least one wall, plant, machine, engine, structure with wood, structure with glass, structure with metal, structure with walls, structure with doors, structure with gaps, structure with reflection surface, structure with fluid, building, roof top, store, factory, assembly line, hotel room, museum, classroom, school, university, government building, warehouse, garage, mall, airport, train station, bus terminal, hub, transportation hub, shipping terminal, government facility, public facility, school, university, entertainment facility, recreational facility, hospital, seniors home, elderly care facility, community center, stadium, playground, park, field, sports facility, swimming facility, track and/or field, basketball court, tennis court, soccer stadium, baseball stadium, gymnasium, hall, garage, shopping mart, mall, supermarket, manufacturing facility, parking facility, construction site, mining facility, transportation facility, highway, road, valley, forest, wood, terrain, landscape, den, patio, land, path, amusement park, urban area, rural area, suburban area, metropolitan area, garden, square, plaza, music hall, downtown facility, over-air facility, semi-open facility, closed area, train platform, train station, distribution center, warehouse, store, distribution center, storage facility, underground facility, space (e.g. above ground, outer-space) facility, floating facility, cavern, tunnel facility, indoor facility, open-air facility, outdoor facility with some walls/ doors/ reflective barriers, open facility, semi-open facility, car, truck, bus, van, container, ship/boat, submersible, train, tram, airplane, vehicle, mobile home, cave, tunnel, pipe, channel, metropolitan area, downtown area with relatively tall buildings, valley, well, duct, pathway, gas line, oil line, water pipe, network of interconnecting pathways/alleys/roads/tubes/cavities/ caves/pipe-like structure/air space/fluid space, human body, animal body, body cavity, organ, bone, teeth, soft tissue, hard tissue, rigid tissue, non-rigid tissue, blood/body fluid vessel, windpipe, air duct, den, etc. The venue may be indoor space, outdoor space. The venue may include both the inside and outside of the space. For example, the venue may include both the inside of a building and the outside of the building. For example, the venue can be a building that has one floor or multiple floors, and a portion of the building can be underground. The shape of the building can be, e.g., round, square, rectangular, triangle, or irregular-shaped. These are merely examples. The disclosure can be used to detect events in other types of venue or spaces.

The wireless transmitter (e.g. Type1 device) and/or the wireless receiver (e.g. Type2 device) may be embedded in a portable device (e.g. a module, or a device with the module) that may move with the object (e.g. in prior movement and/or current movement). The portable device may be communicatively coupled with the object using a wired connection (e.g. through USB, microUSB, Firewire, HDMI, serial port, parallel port, and other connectors) and/or a connection (e.g. Bluetooth, Bluetooth Low Energy (BLE), WiFi, LTE, ZigBee, etc.). The portable device may be a lightweight device. The portable may be powered by battery, rechargeable battery and/or AC power. The portable device may be very small (e.g. at sub-millimeter scale and/or sub-centimeter scale), and/or small (e.g. coin-size, card-size, pocket-size, or larger). The portable device may be large, sizable, and/or bulky (e.g. heavy machinery to be installed). The portable device may be a WiFi hotspot, access point, mobile WiFi (MiFi), dongle with USB/micro USB/ Firewlire/ other connector, smartphone, portable computer, computer, tablet, smart device, internet-of-thing (IoT) device, WiFi-enabled device, LTE-enabled device, a smart watch, smart glass, smart mirror, smart antenna, smart battery, smart light, smart pen, smart ring, smart door, smart window, smart clock, small battery, smart wallet, smart belt, smart handbag, smart clothing/garment, smart ornament, smart packaging, smart paper/ book/ magazine/ poster/ printed matter/ signage/ display/ lighted system/ lighting system, smart key/ tool, smart bracelet/ chain/ necklace/ wearable/ accessory, smart pad/ cushion, smart tile/ block/ brick/ building material/ other material, smart garbage can/ waste container, smart food carriage/ storage, smart ball/ racket, smart chair/ sofa/ bed, smart shoe/ footwear/ carpet/ mat/ shoe rack, smart glove/ hand wear/ ring/ hand ware, smart hat/ headwear/ makeup/ sticker/ tattoo, smart mirror, smart toy, smart pill, smart utensil, smart bottle/food container, smart tool, smart device, IoT device, WiFi enabled device, network enabled device, 3G/4G/5G/6G enabled device, embeddable device, implantable device, air conditioner, refrigerator, heater, furnace, furniture, oven, cooking device, television/ set-top box (STB)/ DVD player/ audio player/ video player/ remote control, hi-fi, audio device, speaker, lamp/ light, wall, door, window, roof, roof tile/ shingle/ structure/ attic structure/ device/ feature/ installation/ fixtures, lawn mower/ garden tools/ yard tools/ mechanics tools/ garage tools/, garbage can/ container, 20-ft/40-ft container, storage container, factory/ manufacturing/ production device, repair tools, fluid container, machine, machinery to be installed, vehicle, cart, wagon, warehouse vehicle, car, bicycle, motorcycle, boat, vessel, airplane, basket/ box/ bag/ bucket/ container, smart plate/ cup/ bowl/ pot/ mat/ utensils/ kitchen tools/ kitchen devices/ kitchen accessories/ cabinets/ tables/ chairs/ tiles/ lights/ water pipes/ taps/ gas range/ oven/ dishwashing machine/ etc. The portable device may have a battery that may be replaceable, irreplaceable, rechargeable, and/or non-rechargeable. The portable device may be wirelessly charged. The portable device may be a smart payment card. The portable device may be a payment card used in parking lots, highways, entertainment parks, or other venues/facilities that need payment. The portable device may have an identity (ID) as described above.

An event may be monitored based on the TSCI. The event may be an object related event, such as fall-down of the object (e.g. an person and/or a sick person), rotation, hesitation, pause, impact (e.g. a person hitting a sandbag, door, window, bed, chair, table, desk, cabinet, box, another person, animal, bird, fly, table, chair, ball, bowling ball, tennis ball, football, soccer ball, baseball, basketball, volley ball, etc.), two-body action (e.g. a person letting go a balloon, catching a fish, molding a clay, writing a paper, person typing on a computer, etc.), car moving in a garage, person carrying a smart phone and walking around an airport/mall/government building/office/etc., autonomous moveable object/machine moving around (e.g. vacuum cleaner, utility vehicle, car, drone, self-driving car, etc.).

The task or the wireless smart sensing task may comprise: object detection, presence detection, object recognition, object verification, tool detection, tool recognition, tool verification, machine detection, machine recognition, machine verification, human detection, human recognition, human verification, baby detection, baby recognition, baby verification, human breathing detection, motion detection, motion estimation, motion verification, periodic motion detection, periodic motion estimation, periodic motion verification, stationary motion detection, stationary motion estimation, stationary motion verification, cyclo-stationary motion detection, cyclo-stationary motion estimation, cyclo-stationary motion verification, transient motion detection, transient motion estimation, transient motion verification, trend detection, trend estimation, trend verification, breathing detection, breathing estimation, breathing estimation, human biometrics detection, human biometrics estimation, human biometrics verification, environment informatics detection, environment informatics estimation, environment informatics verification, gait detection, gait estimation, gait verification, gesture detection, gesture estimation, gesture verification, machine learning, supervised learning, unsupervised learning, semi-supervised learning, clustering, feature extraction, featuring training, principal component analysis, eigen-decomposition, frequency decomposition, time decomposition, time-frequency decomposition, functional decomposition, other decomposition, training, discriminative training, supervised training, unsupervised training, semi-supervised training, neural network, sudden motion detection, fall-down detection, danger detection, life-threat detection, regular motion detection, stationary motion detection, cyclo-stationary motion detection, intrusion detection, suspicious motion detection, security, safety monitoring, navigation, guidance, map-based processing, map-based correction, irregularity detection, locationing, tracking, multiple object tracking, indoor tracking, indoor position, indoor navigation, power transfer, wireless power transfer, object counting, car tracking in parking garage, patient detection, patient monitoring, patient verification, wireless communication, data communication, signal broadcasting, networking, coordination, administration, encryption, protection, cloud computing, other processing and/or other task. The task may be performed by the Type1 device, the Type2 device, another Type1 device, another Type2 device, a nearby device, a local server, edge server, a cloud server, and/or another device.

A first part of the task may comprise at least one of: preprocessing, signal conditioning, signal processing, post-processing, denoising, feature extraction, coding, encryption, transformation, mapping, motion detection, motion estimation, motion change detection, motion pattern detection, motion pattern estimation, motion pattern recognition, vital sign detection, vital sign estimation, vital sign recognition, periodic motion detection, periodic motion estimation, breathing rate detection, breathing rate estimation, breathing pattern detection, breathing pattern estimation, breathing pattern recognition, heartbeat detection, heartbeat estimation, heart pattern detection, heart pattern estimation, heart pattern recognition, gesture detection, gesture estimation, gesture recognition, speed detection, speed estimation, object locationing, object tracking, navigation, acceleration estimation, acceleration detection, fall-down detection, change detection, intruder detection, baby detection, baby monitoring, patient monitoring, object recognition, wireless power transfer, and/or wireless charging.

A second part of the task may comprise at least one of: a smart home task, smart office task, smart building task, smart factory task (e.g. manufacturing using a machine or an assembly line), smart internet-of-thing (IoT) task, smart system task, smart home operation, smart office operation, smart building operation, smart manufacturing operation (e.g. moving supplies/parts/raw material to a machine/an assembly line), IoT operation, smart system operation, turning on a light, turning off the light, controlling the light in at least one of: a room, region, and/or the venue, playing a sound clip, playing the sound clip in at least one of: the room, the region, and/or the venue, playing the sound clip of at least one of: a welcome, greeting, farewell, first message, and/or a second message associated with the first part of the task, turning on an appliance, turning off the appliance, controlling the appliance in at least one of: the room, the region, and/or the venue, turning on an electrical system, turning off the electrical system, controlling the electrical system in at least one of: the room, the region, and/or the venue, turning on a security system, turning off the security system, controlling the security system in at least one of: the room, the region, and/or the venue, turning on a mechanical system, turning off a mechanical system, controlling the mechanical system in at least one of: the room, the region, and/or the venue, and/or controlling at least one of: an air conditioning system, heating system, ventilation system, lighting system, heating device, stove, entertainment system, door, fence, window, garage, computer system, networked device, networked system, home appliance, office equipment, lighting device, robot (e.g. robotic arm), smart vehicle, smart machine, assembly line, smart device, internet-of-thing (IoT) device, smart home device, and/or a smart office device.

The task may include: detect a user returning home, detect a user leaving home, detect a user moving from one room to another, detect/control/lock/unlock/open/close/partially open a window/door/garage door/blind/curtain/panel/-solar panel/sun shade, detect a pet, detect/monitor a user doing something (e.g. sleeping on sofa, sleeping in bedroom, running on treadmill, cooking, sitting on sofa, watching TV, eating in kitchen, eating in dining room, going upstairs/downstairs, going outside/coming back, in the rest room, etc.), monitor/detect location of a user/pet, do something automatically upon detection, do something for the user automatically upon detecting the user, turn on/off/dim a light, turn on/off music/radio/home entertainment system, turn on/off/adjust/control TV/HiFi/set-top-box (STB)/ home entertainment system/smart speaker/smart device, turn on/off/adjust air conditioning system, turn on/off/adjust ventilation system, turn on/off/adjust heating system, adjust/control curtains/light shades, turn on/off/wake a computer, turn on/off/pre-heat/control coffee machine/hot water pot, turn on/off/control/preheat cooker/oven/microwave oven/another cooking device, check/adjust temperature, check weather forecast, check telephone message box, check mail, do a system check, control/adjust a system, check/control/arm/disarm security system/baby monitor, check/control refrigerator, give a report (e.g. through a speaker such as Google home, Amazon Echo, on a display/screen, via a webpage/email/messaging system/notification system, etc.).

For example, when a user arrives home in his car, the task may be to, automatically, detect the user or his car approaching, open the garage door upon detection, turn on the driveway/garage light as the user approaches the garage, turn on air conditioner/heater/fan, etc. As the user enters the house, the task may be to, automatically, turn on the entrance light, turn off driveway/garage light, play a greeting message to welcome the user, turn on the music, turn on the radio and tuning to the user's favorite radio news channel, open the curtain/blind, monitor the user's mood, adjust the lighting and sound environment according to the user's mood or the current/imminent event (e.g. do romantic lighting and music because the user is scheduled to eat dinner with girlfriend in 1 hour) on the user's daily calendar, warm the food in microwave that the user prepared in the morning, do a diagnostic check of all systems in the house, check weather forecast for tomorrow's work, check news of interest to the user, check user's calendar and to-do list and play reminder, check telephone answer system/messaging system/email and give a verbal report using dialog system/speech synthesis, remind (e.g. using audible tool such as speakers/HiFi/speech synthesis/sound/voice/music/song/sound field/background sound field/ dialog system, using visual tool such as TV/entertainment system/computer/notebook/smart pad/display/-light/color/brightness/patterns/symbols, using haptic tool/virtual reality tool/gesture/ tool, using a smart device/appliance/material/furniture/fixture, using web tool/server/cloud server/fog server/edge server/home network/mesh network, using messaging tool/notification tool/communication tool/scheduling tool/email, using user interface/GUI, using scent/smell/ fragrance/taste, using neural tool/nervous system tool, using a combination, etc.) the user of his mother's birthday and to call her, prepare a report, and give the report (e.g. using a tool for reminding as discussed above). The task may turn on the air conditioner/heater/ventilation system in advance, or adjust temperature setting of smart thermostat in advance, etc. As the user moves from the entrance to the living room, the task may be to turn on the living room light, open the living room curtain, open the window, turn off the entrance light behind the user, turn on the TV and set-top box, set TV to the user's favorite channel, adjust an appliance according to the user's preference and conditions/states (e.g. adjust lighting and choose/play music to build a romantic atmosphere), etc.

Another example may be: When the user wakes up in the morning, the task may be to detect the user moving around in the bedroom, open the blind/curtain, open the window, turn off the alarm clock, adjust indoor temperature from nighttime temperature profile to day-time temperature profile, turn on the bedroom light, turn on the restroom light as the user approaches the restroom, check radio or streaming channel and play morning news, turn on the coffee machine and preheat the water, turn off security system, etc. When the user walks from bedroom to kitchen, the task may be to turn on the kitchen and hallway lights, turn off the bedroom and restroom lights, move the music/message/reminder from the bedroom to the kitchen, turn on the kitchen TV, change TV to morning news channel, lower the kitchen blind and open the kitchen window to bring in fresh air, unlock backdoor for the user to check the backyard, adjust temperature setting for the kitchen, etc. Another example may be: When the user leaves home for work, the task may be to detect the user leaving, play a farewell and/or have-a-good-day message, open/close garage door, turn on/off garage light and driveway light, turn off/dim lights to save energy (just in case the user forgets), close/lock all windows/doors (just in case the user forgets), turn off appliance (especially stove, oven, microwave oven), turn on/arm the home security system to guard the home against any intruder, adjust air conditioning/heating/ventilation systems to "away-from-home" profile to save energy, send alerts/reports/updates to the user's smart phone, etc.

A motion may comprise at least one of: a no-motion, resting motion, non-moving motion, deterministic motion, transient motion, fall-down motion, repeating motion, periodic motion, pseudo-periodic motion, periodic motion associated with breathing, periodic motion associated with heartbeat, periodic motion associated with living object, periodic motion associated with machine, periodic motion associated with man-made object, periodic motion associated with nature, complex motion with transient element and periodic element, repetitive motion, non-deterministic motion, probabilistic motion, chaotic motion, random motion, complex motion with non-deterministic element and deterministic element, stationary random motion, pseudo-stationary random motion, cyclo-stationary random motion, non-stationary random motion, stationary random motion with periodic autocorrelation function (ACF), random motion with periodic ACF for period of time, random motion that is pseudo-stationary for period of time, random motion of which an instantaneous ACF has pseudo-periodic element for period of time, machine motion, mechanical motion, vehicle motion, drone motion, air-related motion, wind-related motion, weather-related motion, water-related motion, fluid-related motion, ground-related motion, change in electro-magnetic characteristics, subsurface motion, seismic motion, plant motion, animal motion, human motion, normal motion, abnormal motion, dangerous motion, warning motion, suspicious motion, rain, fire, flood, tsunami, explosion, collision, imminent collision, human body motion, head motion, facial motion, eye motion, mouth motion, tongue motion, neck motion, finger motion, hand motion, arm motion, shoulder motion, body motion, chest motion, abdominal motion, hip motion, leg motion, foot motion, body joint motion, knee motion, elbow motion, upper body motion, lower body motion, skin motion, below-skin motion, subcutaneous tissue motion, blood vessel motion, intravenous motion, organ motion, heart motion, lung motion, stomach motion, intestine motion, bowel motion, eating motion, breathing motion, facial expression, eye expression, mouth expression, talking motion, singing motion, eating motion, gesture, hand gesture, arm gesture, keystroke, typing stroke, user-interface gesture, man-machine interaction, gait, dancing movement, coordinated movement, and/or coordinated body movement. The heterogeneous IC of the Typel device and/or any Type2 receiver may comprise low-noise amplifier (LNA), power amplifier, transmit-receive switch, media access controller, baseband radio, 2.4 GHz radio, 3.65 GHz radio, 4.9 GHz radio, 5 GHz radio, 5.9GHz radio, below 6GHz radio, below 60 GHz radio and/or another radio.

The heterogeneous IC may comprise a processor, a memory communicatively coupled with the processor, and a set of instructions stored in the memory to be executed by the processor. The IC and/or any processor may comprise at least one of: general purpose processor, special purpose processor, microprocessor, multi-processor, multi-core processor, parallel processor, CISC processor, RISC processor, microcontroller, central processing unit (CPU), graphical processor unit (GPU), digital signal processor (DSP), application specific integrated circuit (ASIC), field programmable gate array (FPGA), embedded processor (e.g. ARM), logic circuit, other programmable logic device, discrete logic, and/or a combination. The heterogeneous IC may support broadband network, wireless network, mobile network, mesh network, cellular network, wireless local area network (WLAN), wide area network (WAN), and metropolitan area network (MAN), WLAN standard, WiFi, LTE, 802.11 standard, 802.11a, 802.11b, 802.11g, 802.11n, 802.11ac, 802.11ad, 802.11af, 802,11ah, 802.11ax, 802.11ay, mesh network standard, 802.15 standard, 802.16 standard, cellular network standard, 3G, 3.5G, 4G, beyond 4G, 4.5G, 5G, 6G, 7G, 8G, 9G, Bluetooth, Bluetooth Low-Energy (BLE), Zigbee, WiMax, and/or another wireless network protocol. The processor may comprise general purpose processor, special purpose processor, microprocessor, microcontroller, embedded processor, digital signal processor, central processing unit (CPU), graphical processing unit (GPU), multi-processor, multi-core processor, and/or processor with graphics capability, and/or a combination. The memory may be volatile, non-volatile, random access memory (RAM), Read Only Memory (ROM), Electrically Programmable ROM (EPROM), Electrically Erasable Programmable ROM (EEPROM), hard disk, flash memory, CD-ROM, DVD-ROM, magnetic storage, optical storage, organic storage, storage system, storage network, network storage, cloud storage, edge storage, local storage, external storage, internal storage, or other form of non-transitory storage medium known in the art. The set of instructions (machine executable code) corresponding to the method steps may be embodied directly in hardware, in software, in firmware, or in combinations thereof. The set of instructions may be embedded, pre-loaded, loaded upon boot up, loaded on the fly, loaded on demand, pre-installed, installed, and/or downloaded. The presentation may be a presentation in an audio-visual way, graphical way (e.g. using GUI), textual way, symbolic way or mechanical way.

**Basic computation**

Computational workload associated with the method is shared among the processor, the Typel heterogeneous wireless device, the Type2 heterogeneous wireless device, a local server, a cloud server, and another processor. An operation, pre-processing, processing and/or postprocessing may be applied to data (e.g. TSCI, autocorrelation). An operation may be preprocessing, processing and/or postprocessing. The preprocessing, processing and/or postprocessing may be an operation. An operation may comprise preprocessing, processing, post-processing, computing a function of the operands, filtering, linear filtering, nonlinear filtering, folding, grouping, energy computation, lowpass filtering, bandpass filtering, highpass filtering, median filtering, rank filtering, quartile filtering, percentile filtering, mode filtering, finite impulse response (FIR) filtering, infinite impulse response (IIR) filtering, moving average (MA) filtering, autoregressive (AR) filtering, autoregressive moving averaging (ARMA) filtering, selective filtering, adaptive filtering, interpolation, decimation, subsampling, upsampling, resampling, time correction, time base correction, phase correction, magnitude correction, phase cleaning, magnitude cleaning, matched filtering, enhancement, restoration, denoising, smoothing, signal conditioning, enhancement, restoration, spectral analysis, linear transform, nonlinear transform, frequency transform, inverse frequency transform, Fourier transform, wavelet transform, Laplace transform, Hilbert transform, Hadamard transform, trigonometric transform, sine transform, cosine transform, power-of-2 transform, sparse transform, graph-based transform, graph signal processing, fast transform, a transform combined with zero padding, cyclic padding, padding, zero padding, feature extraction, decomposition, projection, orthogonal projection, non-orthogonal projection, over-complete projection, eigen-decomposition, singular value decomposition (SVD), principle component analysis (PCA), independent component analysis (ICA), grouping, sorting, thresholding, soft thresholding, hard thresholding, clipping, soft clipping, first derivative, second order derivative, high order derivative, convolution, multiplication, division, addition, subtraction, integration, maximization, minimization, local maximization, local minimization, optimization of a cost function, neural network, recognition, labeling, training, clustering, machine learning, supervised learning, unsupervised learning, semi-supervised learning, comparison with another TSCI, similarity score computation, quantization, vector quantization, matching pursuit, compression, encryption, coding, storing, transmitting, normalization, temporal normalization, frequency domain normalization, classification, clustering, labeling, tagging, learning, detection, estimation, learning network, mapping, remapping, expansion, storing, retrieving, transmitting, receiving, representing, merging, combining, splitting, tracking, monitoring, matched filtering, Kalman filtering, particle filter, intrapolation, extrapolation, importance sampling, Monte Carlo sampling, compressive sensing, representing, merging, combining, splitting, scrambling, error protection, forward error correction, doing nothing, time varying processing, conditioning averaging, weighted averaging, arithmetic mean, geometric mean, harmonic mean, averaging over selected frequency, averaging over antenna links, logical operation, permutation, combination, sorting, AND, OR, XOR, union, intersection, vector addition, vector subtraction, vector multiplication, vector division, inverse, norm, distance, and/or another operation. The operation may be the preprocessing, processing, and/or post-processing. Operations may be applied jointly on multiple time series or functions.

The function (e.g. function of operands) may comprise: scalar function, vector function, discrete function, continuous function, polynomial function, characteristics, feature, magnitude, phase, exponential function, logarithmic function, trigonometric function, transcendental function, logical function, linear function, algebraic function, nonlinear function, piecewise linear function, real function, complex function, vector-valued function, inverse function, derivative of function, integration of function, circular function, function of another function, one-to-one function, one-to-many function, many-to-one function, many-to-many function, zero crossing, absolute function, indicator function, mean, mode, median, range, statistics, variance, arithmetic mean, geometric mean, harmonic mean, trimmed mean, percentile, square, cube, root, power, sine, cosine, tangent, cotangent, secant, cosecant, elliptical function, parabolic function, hyperbolic function, game function, zeta function, absolute value, thresholding, limiting function, floor function, rounding function, sign function, quantization, piecewise constant function, composite function, function of function, time function processed with an operation (e.g. filtering), probabilistic function, stochastic function, random function, ergodic function, stationary function, deterministic function, periodic function, transformation, frequency transform, inverse frequency transform, discrete time transform, Laplace transform, Hilbert transform, sine transform, cosine transform, triangular transform, wavelet transform, integer transform, power-of-2 transform, sparse transform, projection, decomposition, principle component analysis (PCA), independent component analysis (ICA), neural network, feature extraction, moving function, function of moving window of neighboring items of time series, filtering function, convolution, mean function, variance function, statistical function, short-time transform, discrete transform, discrete Fourier transform, discrete cosine transform, discrete sine transform, Hadamard transform, eigen-decomposition, eigenvalue, singular value decomposition (SVD), singular value, orthogonal decomposition, matching pursuit, sparse transform, sparse approximation, any decomposition, graph-based processing, graph-based transform, graph signal processing, classification, labeling, learning, machine learning, detection, estimation, feature extraction, learning network, feature extraction, denoising, signal enhancement, coding, encryption, mapping, remapping, vector quantization, lowpass filtering, highpass filtering, bandpass filtering, matched filtering, Kalman filtering, preprocessing, postprocessing, particle filter, FIR filtering, IIR filtering, autoregressive (AR) filtering, adaptive filtering, first order derivative, high order derivative, integration, zero crossing, smoothing, median filtering, mode filtering, sampling, random sampling, resampling function, downsampling, upsampling, interpolation, extrapolation, importance sampling, Monte Carlo sampling, compressive sensing, statistics, short term statistics, long term statistics, mean, variance, autocorrelation function, cross correlation, moment generating function, time averaging, weighted averaging, special function, Bessel function, error function, complementary error function, Beta function, Gamma function, integral function, Gaussian function, Poisson function, etc.

Machine learning, training, discriminative training, deep learning, neural network, continuous time processing, distributed computing, distributed storage, acceleration using GPU/DSP/coprocessor/multicore/multiprocessing may be applied to a step (or each step) of this disclosure. A frequency transform may include Fourier transform, Laplace transform, Hadamard transform, Hilbert transform, sine transform, cosine transform, triangular transform, wavelet transform, integer transform, power-of-2 transform, combined zero padding and transform, Fourier transform with zero padding, and/or another transform. Fast versions and/or approximated versions of the transform may be performed. The transform may be performed using floating point, and/or fixed point arithmetic. An inverse frequency transform may include inverse Fourier transform, inverse Laplace transform, inverse Hadamard transform, inverse Hilbert transform, inverse sine transform, inverse cosine transform, inverse triangular transform, inverse wavelet transform, inverse integer transform, inverse power-of-2 transform, combined zero padding and transform, inverse Fourier transform with zero padding, and/or another transform. Fast versions and/or approximated versions of the transform may be performed. The transform may be performed using floating point, and/or fixed point arithmetic.

A quantity from a TSCI may be computed. The quantity may comprise statistic of at least one of: motion, location, map coordinate, height, speed, acceleration, movement angle, rotation, size, volume, time trend, time trend, time profile, periodic motion, frequency, transient, breathing, gait, action, event, suspicious event, dangerous event, alarming event, warning, belief, proximity, collision, power, signal, signal power, signal strength, received signal strength indicator (RSSI), signal amplitude, signal phase, signal frequency component, signal frequency band component, channel state information (CSI), map, time, frequency, time-frequency, decomposition, orthogonal decomposition, non-orthogonal decomposition, tracking, breathing, heartbeat, biometric, baby, patient, machine, device, temperature, vehicle, parking lot, venue, lift, elevator, spatial, road, fluid flow, home, room, office, house, building, warehouse, storage, system, ventilation, fan, pipe, duct, people, human, car, boat, truck, airplane, drone, downtown, crowd, impulsive event, cyclo-stationary, environment, vibration, material, surface, 3-dimensional, 2-dimensional, local, global, presence, and/or another.

### Sliding window/algorithm

Sliding time window may have time varying window width. It may be smaller at the beginning to enable fast acquisition and may increase over time to a steady-state size. The steady-state size may be related to the frequency, repeated motion, transient motion, and/or spatial-temporal information to be monitored. Even in steady state, the window size may be adaptively changed based on battery life, power consumption, available computing power, change in amount of targets, the nature of motion to be monitored, etc. The time shift between two sliding time windows at adjacent time instance may be constant/variable/locally adaptive over time. When shorter time shift is used, the update of any monitoring may be more frequent which may be used for fast changing situations, object motions, and/or objects. Longer time shift may be used for slower situations, object motions, and/or objects. The window width/size and/or time shift may be changed upon a user request/choice. The time shift may be changed automatically (e.g. as controlled by processor/ computer/-server/cloud server) and/or adaptively.

At least one characteristics of a function (e.g. auto-correlation function, auto-covariance function, cross-correlation function, cross-covariance function, power spectral density, time function, frequency domain function, frequency transform) may be determined (e.g. by an object tracking server, the processor, the Type1 heterogeneous device, the Type2 heterogeneous device, and/or another device). The at least one characteristics of the function may include: a local maximum, local minimum, local extremum, local extremum with positive time offset, first local extremum with positive time offset, $n$^th local extremum with positive time offset, local extremum with negative time offset, first local extremum with negative time offset, $n$^th local extremum with negative time offset, constrained (with argument within constraint) maximum, minimum, constrained maximum, constrained minimum, constrained extremum, slope, derivative, higher order derivative, maximum slope, minimum slope, local maximum slope, local maximum slope with positive time offset, local minimum slope, constrained maximum slope, constrained minimum slope, maximum higher order derivative, minimum higher order derivative, constrained higher order derivative, zero-crossing, zero crossing with positive time offset, $n$^th zero crossing with positive time offset, zero crossing with negative time offset, $n$^th zero crossing with negative time offset, constrained zero-crossing, zero-crossing of slope, zero-crossing of higher order derivative, and/or another characteristics. At least one argument of the function associated with the at least one characteristics of the function may be identified. Some quantity (e.g. spatial-temporal information of the object) may be determined based on the at least one argument of the function.

A characteristics (e.g. characteristics of motion of an object in the venue) may comprise at least one of: an instantaneous characteristics, short-term characteristics, repetitive characteristics, recurring characteristics, history, incremental characteristics, changing characteristics, deviational characteristics, phase, magnitude, time characteristics, frequency characteristics, time-frequency characteristics, decomposition characteristics, orthogonal decomposition characteristics, non-orthogonal decomposition characteristics, deterministic characteristics, probabilistic characteristics, stochastic characteristics, autocorrelation function (ACF), mean, variance, statistics, duration, timing, trend, periodic characteristics, long-term characteristics, historical characteristics, average characteristics, current characteristics, past characteristics, future characteristics, predicted characteristics, location, distance, height, speed, direction, velocity, acceleration, change of the acceleration, angle, angular speed, angular velocity, angular acceleration of the object, change of the angular acceleration, orientation of the object, angular of rotation, deformation of the object, shape of the object, change of shape of the object, change of size of the object, change of structure of the object, and/or change of characteristics of the object.

At least one local maximum and at least one local minimum of the function may be identified. At least one local signal-to-noise-ratio-like (SNR-like) parameter may be computed for each pair of adjacent local maximum and local minimum. The SNR-like parameter may be a function (e.g. linear, log, exponential function, monotonic function) of a fraction of a quantity (e.g. power, magnitude, etc.) of the local maximum over the same quantity of the local minimum. It may also be the function of a difference between the quantity of the local maximum and the same quantity of the local minimum.

Significant local peaks may be identified or selected. Each significant local peak may be a local maximum with SNR-like parameter greater than a threshold T1 and/or a local maximum with amplitude greater than a threshold T2. The at least one local minimum and the at least one local minimum in the frequency domain may be identified/computed using a persistence-based approach. A set of selected significant local peaks may be selected from the set of identified significant local peaks based on a selection criterion. The characteristics/spatial-temporal information of the object may be computed based on the set of selected significant local peaks and frequency values associated with the set of selected significant local peaks.

In one example, the selection criterion may always correspond to select the strongest peaks in a range. While the strongest peaks may be selected, the unselected peaks may still be significant (rather strong). Unselected significant peaks may be stored and/or monitored as "reserved" peaks for use in future selection in future sliding time windows. As an example, there may be a particular peak (at a particular frequency) appearing consistently over time. Initially, it may be significant but not selected (as other peaks may be stronger). But in later time, the peak may become stronger and more dominant and may be selected. When it became "selected", it may be back-traced in time and made "selected" in the earlier time when it was significant but not selected. In such case, the back-traced peak may replace a previously selected peak in an early time. The replaced peak may be the relatively weakest, or a peak that appear in isolation in time (i.e. appearing only briefly in time). In another example, the selection criterion may not correspond to select the strongest peaks in the range. Instead, it may consider not only the "strength" of the peak, but the "trace" of the peak - peaks that may have happened in the past, especially those peaks that have been identified for a long time. For example, if a finite state machine (FSM) is used, it may select the peak(s) based on the state of the FSM. Decision thresholds may be computed adaptively based on the state of the FSM.

A similarity score and/or component similarity score may be computed (e.g. by a server, the processor, the Type1 device, the Type2 device, a local server, a cloud server, and/or another device) based on a pair of temporally adjacent CI of a TSCI. The pair may come from the same sliding window or two different sliding windows. The similarity score may also be based on a pair of, temporally adjacent or not so adjacent, CI from two different TSCI. The similarity score and/or component similar score may be/comprise: time reversal resonating strength (TRRS), correlation, cross-correlation, auto-correlation, covariance, cross-covariance, auto-covariance, inner product of two vectors, distance score, norm, metric, statistical characteristics, discrimination score, metric, neural network, deep learning network, machine learning, training, discrimination, weighted averaging, preprocessing, denoising, signal conditioning, filtering, time correction, timing compensation, phase offset compensation, transformation, component-wise operation, feature extraction, finite state machine, and/or another score. The characteristics and/or spatial-temporal information may be determined/computed based on the similarity score. Any threshold may be pre-determined, adaptively determined and/or determined by a finite state machine. The adaptive determination may be based on time, space, location, antenna, path, link, state, battery life, remaining battery life, available power, available computational resources, available network bandwidth, etc. A threshold to be applied to a test statistics to differentiate two events (or two conditions, or two situations, or two states), A and B, may be determined. Data (e.g. CI, channel state information (CSI)) may be collected under A and/or under B in a training situation. The test statistics may be computed based on the data. Distributions of the test statistics under A may be compared with distributions of the test statistics under B, and the threshold may be chosen according to some criteria. The criteria may comprise: maximum likelihood (ML), maximum aposterior probability (MAP), discriminative training, minimum Type1 error for a given Type2 error, minimum Type2 error for a given Type1 error, and/or other criteria. The threshold may be adjusted to achieve different sensitivity to the A, B and/or another event!condition/situation/state. The threshold adjustment may be automatic, semi-automatic and/or manual. The threshold adjustment may be applied once, sometimes, often, periodically, occasionally, sporadically, and/or on demand. The threshold adjustment may be adaptive. The threshold adjustment may depend on the object, object movement/location/ direction/action, object characteristics/ spatial-temporal information/size/ property/trait/habit/behavior, the venue, feature/ fixture/ furniture/barrier/ material/ machine/living thing/thing/object/boundary/ surface/ medium that is in/at/of the venue, map, constraint of the map, the event/state/ situation/condition, time, timing, duration, current state, past history, user, and/or a personal preference, etc.

A stopping criterion of an iterative algorithm may be that change of a current parameter (e.g. offset value) in the updating in an iteration is less than a threshold. The threshold may be 0.5, 1, 1.5, 2, or another number. The threshold may be adaptive. It may change as the iteration progresses. For the offset value, the adaptive threshold may be determined based on the task, particular value of the first time, the current time offset value, the regression window, the regression analysis, the regression function, the regression error, the convexity of the regression function, and/or an iteration number. The local extremum may be determined as the corresponding extremum of the regression function in the regression window. The local extremum may be determined based on a set of time offset values in the regression window and a set of associated regression function values. Each of the set of associated regression function values associated with the set of time offset values may be within a range from the corresponding extremum of the regression function in the regression window.

The searching for a local extremum may comprise robust search, minimization, maximization, optimization, statistical optimization, dual optimization, constraint optimization, convex optimization, global optimization, local optimization an energy minimization, linear regression, quadratic regression, higher order regression, linear programming, nonlinear programming, stochastic programming, combinatorial optimization, constraint programming, constraint satisfaction, calculus of variations, optimal control, dynamic programming, mathematical programming, multi-objective optimization, multi-modal optimization, disjunctive programming, space mapping, infinite-dimensional optimization, heuristics, metaheuristics, convex programming, semidefinite programming, conic programming, cone programming, integer programming, quadratic programming, fractional programming, numerical analysis, simplex algorithm, iterative method, gradient descent, subgradient method, coordinate descent, conjugate gradient method, Newton's algorithm, sequential quadratic programming, interior point method, ellipsoid method, reduced gradient method, quasi-Newton method, simultaneous perturbation stochastic approximation, interpolation method, pattern search method, line search, non-differentiable optimization, genetic algorithm, evolutionary algorithm, dynamic relaxation, hill climbing, particle swarm optimization, gravitation search algorithm, simulated annealing, memetic algorithm, differential evolution, dynamic relaxation, stochastic tunneling, Tabu search, reactive search optimization, curve fitting, least square, simulation based optimization, variational calculus, and/or variant. The search for local extremum may be associated with an objective function, loss function, cost function, utility function, fitness function, energy function, and/or an energy function. Regression may be performed using regression function to fit sampled data (e.g. CI, feature of CI, component of CI) or another function (e.g. autocorrelation function) in a regression window. In at least one iteration, a length of the regression window and/or a location of the regression window may change. The regression function may be linear function, quadratic function, cubic function, polynomial function, and/or another function.

The regression analysis may minimize an absolute error, square error, higher order error (e.g. third order, fourth order, etc.), robust error (e.g. square error for smaller error magnitude and absolute error for larger error magnitude, or first kind of error for smaller error magnitude and second kind of error for larger error magnitude), another error, weighted sum of absolute error (e.g. for wireless transmitter with multiple antennas and wireless receiver with multiple antennas, each pair of transmitter antenna and receiver antenna form a link. Error associated with different links may have different weights. One possibility is that some links and/or some components with larger noise may have smaller or bigger weight.), weighted sum of square error, weighted sum of higher order error, weighted sum of robust error, weighted sum of the another error, absolute cost, square cost, higher order cost, robust cost, another cost, weighted sum of absolute cost, weighted sum of square cost, weighted sum of higher order cost, weighted sum of robust cost, and/or weighted sum of another cost.

The regression error determined may be an absolute error, square error, higher order error, robust error, yet another error, weighted sum of absolute error, weighted sum of square error, weighted sum of higher order error, weighted sum of robust error, and/or weighted sum of the yet another error. The time offset associated with maximum regression error (or minimum regression error) of the regression function with respect to the particular function in the regression window may become the updated current time offset in the iteration. A local extremum may be searched based on a quantity comprising a difference of two different errors (e.g. a difference between absolute error and square error). Each of the two different errors may comprise an absolute error, square error, higher order error, robust error, another error, weighted sum of absolute error, weighted sum of square error, weighted sum of higher order error, weighted sum of robust error, and/or weighted sum of the another error. The quantity may be compared with an F-distribution, central F-distribution, another statistical distribution, threshold, threshold associated with probability, threshold associated with probability of finding false peak, threshold associated with the F-distribution, threshold associated the central F-distribution, and/or threshold associated with the another statistical distribution.

The regression window may be determined based on at least one of: the movement of the object, quantity associated with the object, the at least one characteristics and/or spatial-temporal information of the object associated with the movement of the object, estimated location of the local extremum, noise characteristics, estimated noise characteristics, F-distribution, central F-distribution, another statistical distribution, threshold, preset threshold, threshold associated with probability, threshold associated with desired probability, threshold associated with probability of finding false peak, threshold associated with the F-distribution, threshold associated the central F-distribution, threshold associated with the another statistical distribution, condition that quantity at the window center is largest within the regression window, condition that the quantity at the window center is largest within the regression window, condition that there is only one of the local extremum of the particular function for the particular value of the first time in the regression window, another regression window, and/or another condition. The width of the regression window may be determined based on the particular local extremum to be searched. The local extremum may comprise first local maximum, second local maximum, higher order local maximum, first local maximum with positive time offset value, second local maximum with positive time offset value, higher local maximum with positive time offset value, first local maximum with negative time offset value, second local maximum with negative time offset value, higher local maximum with negative time offset value, first local minimum, second local minimum, higher local minimum, first local minimum with positive time offset value, second local minimum with positive time offset value, higher local minimum with positive time offset value, first local minimum with negative time offset value, second local minimum with negative time offset value, higher local minimum with negative time offset value, first local extremum, second local extremum, higher local extremum, first local extremum with positive time offset value, second local extremum with positive time offset value, higher local extremum with positive time offset value, first local extremum with negative time offset value, second local extremum with negative time offset value, and/or higher local extremum with negative time offset value.

A current parameter (e.g. time offset value) may be initialized based on a target value, target profile, trend, past trend, current trend, target speed, speed profile, target speed profile, past speed trend, the movement of the object, at least one characteristics and/or spatial-temporal information of the object associated with the movement of object, positional quantity of the object, initial speed of the object associated with the movement of the object, predefined value, initial width of the regression window, time duration, value based on carrier frequency of the signal, bandwidth of the signal, amount of antennas associated with the channel, noise characteristics, and/or an adaptive value. The current time offset may be at the center, on the left side, on the right side, and/or at another fixed relative location, of the regression window.

In the presentation, information may be displayed with a map of the venue. The information may comprise: location, zone, region, area, corrected location, approximate location, location with respect to (w.r.t.) a map of the venue, location w.r.t. a segmentation of the venue, direction, a path, a path w.r.t. the map and/or the segmentation, a trace (e.g. location within a time window such as the past 5 seconds, or past 10 seconds; the time window duration may be adjusted adaptive; the time window duration may be adaptively adjusted w.r.t. speed, acceleration, etc.), a history of a path, approximate regions/zones along a path, a history/ summary of past locations, a history of past locations of interest, frequently-visited areas, customer traffic, crowd distribution, crowd behavior, crowd control information, speed, acceleration, motion statistics, breathing rate, heart rate, presence/absence of motion, presence/absence of people, presence/absence of vital sign, gesture, gesture control (control of devices using gesture), location-based gesture control, information of a location-based operation, an identity (ID) of the respect object (e.g. a pet, a person, an self-guided machine/device, a vehicle, a drone, a car, a boat, a bicycle, a self-guided vehicle, a machine with a fan, an air-conditioner, a TV, a machine with a movable part), an identification of a user (e.g. a person), an information of the user, a location/-speed/acceleration/direction/motion/gesture/gesture control/ motion trace of the user, an ID of the user, an activity of the user, a state of the user, a sleeping/resting characteristics of the user, an emotional state of the user, a vital sign of the user, an environment information of the venue, a weather information of the venue, an earthquake, an explosion, a storm, a rain, a fire, a temperature, a collision, an impact, a vibration, a event, a door-open event, a door-close event, a window-open event, a window-close event, a fall-down event, a burning event, a freezing event, a water-related event, a wind-related event, an air-movement event, an accident event, a pseudo-periodic event (e.g. running on a treadmill, jumping up and down, skipping rope, somersault, etc), a crowd event, a vehicle event, a gesture of the user (e.g. a hand gesture, an arm gesture, a foot gesture, a leg gesture, a body gesture, a head gesture, a face gesture, a mouth gesture, an eye gesture, etc).

The location may be 2-dimensional (e.g. with 2D coordinates), 3-dimensional (e.g. with 3D coordinates). The location may be relative (e.g. w.r.t. a map) or relational (e.g. halfway between point A and point B, around a corner, up the stairs, on top of table, at the ceiling, on the floor, on a sofa, close to point A, a distance R from point A, within a radius of R from point A, etc). The location may be expressed in rectangular coordinate, polar coordinate, and/or another representation. The information (e.g. location) may be marked with at least one symbol. The symbol may be time varying. The symbol may be flashing and/or pulsating with or without changing color/intensity. The size may change over time. The orientation of the symbol may change over time. The symbol may be a number that reflects an instantaneous quantity (e.g. vital sign/ breathing rate/heart rate/gesture/state/status/action/motion of a user, temperature, network traffic, network connectivity, a status of a device/machine, remaining power of a device, a status of the device, etc). The rate of change, the size, the orientation, the color, the intensity and/or the symbol may reflect the respective motion. The information may be presented visually and/or described verbally (e.g. using pre-recorded voice, or voice synthesis). The information may be described in text. The information may also be presented in a mechanical way (e.g. an animated gadget, a movement of a movable part).

The user-interface (UI) device may be a smart phone (e.g. iPhone, Android phone), tablet (e.g. iPad), laptop (e.g. notebook computer), personal computer (PC), device with graphical user interface (GUI), smart speaker, device with voice/audio/speaker capability, virtual reality (VR) device, augmented reality (AR) device, smart car, display in the car, voice assistant, voice assistant in a car, etc. The map may be 2-dimensional, 3-dimensional and/or higher-dimensional. (e.g. a time varying 2D/3D map) Walls, windows, doors, entrances, exits, forbidden areas may be marked on the map. The map may comprise floor plan of a facility. The map may have one or more layers (overlays). The map may be a maintenance map comprising water pipes, gas pipes, wiring, cabling, air ducts, crawl-space, ceiling layout, and/or underground layout. The venue may be segmented into multiple zones such as bedroom, living room, storage room, walkway, kitchen, dining room, foyer, garage, first floor, second floor, rest room, offices, conference room, reception area, various office areas, various warehouse regions, various facility areas, etc. The segments/regions/areas may be presented in a map. Different regions may be color-coded. Different regions may be presented with a characteristic (e.g. color, brightness, color intensity, texture, animation, flashing, flashing rate, etc). Logical segmentation of the venue may be done using the at least one heterogeneous Type2 device, or a server, or a cloud server, etc.

Here is an example of the disclosed system, apparatus, and method. Stephen and his family want to install the disclosed wireless motion detection system to detect motion in their 2000 sqft two-storey town house in Seattle, Washington. Because his house has two storeys, Stephen decided to use one Type2 device (named A) and two Type1 devices (named B and C) in the ground floor. His ground floor has predominantly three rooms: kitchen, dining room and living room arranged in a straight line, with the dining room in the middle. The kitchen and the living rooms are on opposite end of the house. He put the Type2 device (A) in the dining room, and put one Type1 device (B) in the kitchen and the other Type1 device (C) in the living room. With this placement of the devices, he is practically partitioning the ground floor into 3 zones (dining room, living room and kitchen) using the motion detection system. When motion is detected by the AB pair and the AC pair, the system would analyze the motion information and associate the motion with one of the 3 zones.

When Stephen and his family go out on weekends (e.g. to go for a camp during a long weekend), Stephen would use a mobile phone app (e.g. Android phone app or iPhone app) to turn on the motion detection system. When the system detects motion, a warning signal is sent to Stephen (e.g. an SMS text message, an email, a push message to the mobile phone app, etc). If Stephen pays a monthly fee (e.g. $10/month), a service company (e.g. security company) will receive the warning signal through wired network (e.g. broadband) or wireless network (e.g. home WiFi, LTE, 3G, 2.5G, etc) and perform a security procedure for Stephen (e.g. call him to verify any problem, send someone to check on the house, contact the police on behalf of Stephen, etc). Stephen loves his aging mother and cares about her well-being when she is alone in the house. When the mother is alone in the house while the rest of the family is out (e.g. go to work, or shopping, or go on vacation), Stephen would turn on the motion detection system using his mobile app to ensure the mother is ok. He then uses the mobile app to monitor his mother's movement in the house. When Stephen uses the mobile app to see that the mother is moving around the house among the 3 regions, according to her daily routine, Stephen knows that his mother is doing ok. Stephen is thankful that the motion detection system can help him monitor his mother's well-being while he is away from the house.

On a typical day, the mother would wake up at around 7am. She would cook her breakfast in the kitchen for about 20 minutes. Then she would eat the breakfast in the dining room for about 30 minutes. Then she would do her daily exercise in the living room, before sitting down on the sofa in the living room to watch her favorite TV show. The motion detection system enables Stephen to see the timing of the movement in each of the 3 regions of the house. When the motion agrees with the daily routine, Stephen knows roughly that the mother should be doing fine. But when the motion pattern appears abnormal (e.g. there is no motion until 10am, or she stayed in the kitchen for too long, or she remains motionless for too long, etc), Stephen suspects something is wrong and would call the mother to check on her. Stephen may even get someone (e.g. a family member, a neighbor, a paid personnel, a friend, a social worker, a service provider) to check on his mother.

At some time, Stephen feels like repositioning the Type2 device. He simply unplugs the device from the original AC power plug and plug it into another AC power plug. He is happy that the wireless motion detection system is plug-and-play and the repositioning does not affect the operation of the system. Upon powering up, it works right away. Sometime later, Stephen is convinced that our wireless motion detection system can really detect motion with very high accuracy and very low alarm, and he really can use the mobile app to monitor the motion in the ground floor. He decides to install a similar setup (i.e. one Type2 device and two Type1 devices) in the second floor to monitor the bedrooms in the second floor. Once again, he finds that the system set up is extremely easy as he simply needs to plug the Type2 device and the Type1 devices into the AC power plug in the second floor. No special installation is needed. And he can use the same mobile app to monitor motion in the ground floor and the second floor. Each Type2 device in the ground floor/second floor can interact with all the Type1 devices in both the ground floor and the second floor. Stephen is happy to see that, as he doubles his investment in the Type1 and Type2 devices, he has more than double the capability of the combined systems.

According to various embodiments, each CI (CI) may comprise at least one of: a channel state information (CSI), a frequency domain CSI, a frequency domain CSI associated with at least one sub-band, a time domain CSI, a CSI in a domain, a channel impulse response (CIR), a channel frequency response (CFR), a channel characteristics, a channel filter response, a CSI of the wireless multipath channel, an information of the wireless multipath channel, a timestamp, an auxiliary information, a data, a meta data, a user data, an account data, an access data, a security data, a session data, a status data, a supervisory data, a household data, an identity (ID), a device data, a network data, a neighborhood data, an environment data, a real-time data, a sensor data, a stored data, an encrypted data, a compressed data, a protected data, and/or another CI. In one embodiment, the disclosed system has hardware components (e.g. wireless transmitter/receiver with antenna, analog circuitry, power supply, processor, memory, etc.) and corresponding software components. According to various embodiments of the present teaching, the disclosed system includes a Bot (referred to as a Type1 device) and an Origin (referred to as a Type2 device) for vital sign detection and monitoring. Each device comprises a transceiver, a processor and a memory.

The disclosed system can be applied in many cases. In one example, the Type1 device (transmitter) may be a small WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. In one example, the Type2 (receiver) may be a WiFi-enabled device resting on the table. It may also be a WiFi-enabled television (TV), set-top box (STB), a smart speaker (e.g. Amazon echo), a smart refrigerator, a smart microwave oven, a mesh network router, a mesh network satellite, a smart phone, a computer, a tablet, a smart plug, etc. The Type1 device and Type2 devices may be placed in/near a conference room to count people. The Type1 device and Type2 devices may be in a well-being monitoring system for older adults to monitor their daily activities and any sign of symptoms (e.g. dementia, Alzheimer's disease). The Type1 device and Type2 device may be used in baby monitors to monitor the vital signs (breathing) of a living baby. The Type1 device and Type2 devices may be placed in bedrooms to monitor quality of sleep and any sleep apnea. The Type1 device and Type2 devices may be placed in cars to monitor well-being of passengers and driver, detect any sleeping of driver and detect any babies left in a car. The Type1 device and Type2 devices may be used in logistics to prevent human trafficking by monitoring any human hidden in trucks and containers. The Type1 device and Type2 devices may be deployed by emergency service at disaster area to search for trapped victims in debris. The Type1 device and Type2 devices may be deployed in an area to detect breathing of any intruders. There are numerous applications of wireless breathing monitoring without wearables.

Hardware modules may be constructed to contain either the Type1 transceiver and the Type2 transceiver. The hardware modules may be sold to/used by variable brands to design, build and sell final commercial products. Products using the disclosed system and/or method may be home/office security products, sleep monitoring products, WiFi products, mesh products, TV, STB, entertainment system, HiFi, speaker, home appliance, lamps, stoves, oven, microwave oven, table, chair, bed, shelves, tools, utensils, torches, vacuum cleaner, smoke detector, sofa, piano, fan, door, window, door/window handle, locks, smoke detectors, car accessories, computing devices, office devices, air conditioner, heater, pipes, connectors, surveillance camera, access point, computing devices, mobile devices, LTE devices, 3G/4G/5G/6G devices, gaming devices, eyeglasses, glass panels, VR goggles, necklace, watch, waist band, belt, wallet, pen, hat, wearables, implantable device, tags, parking tickets, smart phones, etc.

The summary may comprise at least one of: an analytics, a selected time window, a subsampling, a transform, a projection, etc. The presenting may comprise presenting at least one of: a monthly view, a weekly view, a daily view, a simplified view, a detailed view, a cross-sectional view, a small form-factor view, a large form-factor view, a color-coded view, a comparative view, a summary view, an animation, a web view, a voice announcement, and another presentation related to the periodic characteristics of the repeating motion.

A Type1 device and/or a Type2 device may be an antenna, a device with antenna, device that has interface to attach/connect to/link antenna, device with wireless transceiver, device with wireless transmitter, device with wireless receiver, internet-of-thing (IoT) device, device with wireless network, device with both wired networking and wireless networking capability, device with wireless integrated circuit (IC), Wi-Fi device, device with Wi-Fi chip (e.g. 802.11a/b/g/n/ac/ax standard compliant, etc), Wi-Fi access point (AP), Wi-Fi client, Wi-Fi router, Wi-Fi repeater, Wi-Fi hub, Wi-Fi mesh network router/hub/AP, wireless mesh network router, adhoc network device, wireless mesh network device, mobile device (e.g. 2G/2.5G/3G/3.5G/4G/LTE/ 5G/6G/7G, etc.), cellular device, mobile network base station, mobile network hub, mobile network compatible device, LTE device, device with LTE module, mobile module (e.g. circuit board with mobile-enabling chip (IC) such as Wi-Fi chip, LTE chip, BLE chip, etc), Wi-Fi chip (IC), LTE chip, BLE chip, device with mobile module, smart phone, companion device (e.g. dongle, attachment, plugin) for smart phones, dedicated device, plug-in device, AC-powered device, battery-powered device, device with processor/memory/set of instructions, smart clock, smart stationary, smart pen, smart user-interface, smart paper, smart mat, smart camera, smart television (TV), set-top-box, smart microphone, smart speaker, smart refrigerator, smart oven, smart machine, smart phone, smart wallet, smart furniture, smart door, smart window, smart ceiling, smart floor, smart wall, smart table, smart chair, smart bed, smart night-stand, smart air-conditioner, smart heater, smart pipe, smart duct, smart cable, smart carpet, smart decoration, smart gadget, smart USB device, smart plug, smart dongle, smart lamp/light, smart tile, smart ornament, smart bottle, vehicle, smart car, smart AGV, drone, smart robot, laptop, tablet, computer, harddisk, network card, smart instrument, smart racket, smart ball, smart shoe, smart wearable, smart clothing, smart glasses, smart hat, smart necklace, smart food, smart pill, small device that moves in the body of creature (e.g. in blood vessels, in lymph fluid, digestive system, etc.). The Type1 device and/or Type2 device may be communicatively coupled with: the internet, another device with access to internet (e.g. smart phone), cloud server, edge server, local server, and/or storage.

An exemplary low-complexity breathing monitoring scheme that utilizes Fast Fourier Transform for spectral analysis using channel frequency response (CFRs) obtained from off-the-shelf WiFi devices is presented below. The scheme also leverages finite state machine (FSM) to counteract the motion of the subject or from other subjects nearby. The integration of FFT and FSM lead to a scalable, low-complexity, and robust scheme for breathing Monitoring.

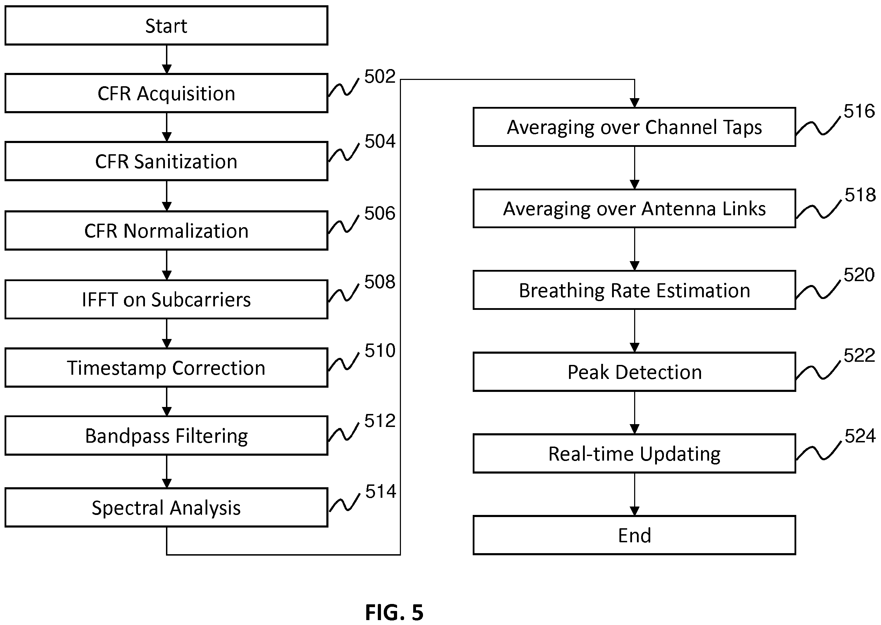

An exemplary flow chart of breathing estimation and detection is shown in FIG. 5, which consists of the following steps.

1. CFR Acquisition: The scheme acquires CFRs from off-the-shelf WiFi chips. For example, one can obtain 114 subcarriers in a CFR frame on a 5GHz WiFi channel with a maximum of 20 dBm transmission power.

2. CFR Sanitization: Due to the inevitable phase misalignment between the WiFi transmitter and receiver, there exist severe phase distortions in the CFRs. To remedy this issue, one can clean the CFR phase by the conventional linear regression method. One can write the cleaned CFR on subcarrier $k$ as $G[k]$.

3. CFR Normalization: The receiving power of the CFRs is time-varying. Unfortunately, in the absence of automatic-gain-control (AGC) values on the CFR chips adopted, the receiving power is unmeasurable. Therefore, one can normalize the CFRs across all subcarriers such that each CFR has unit power. Although this step incurs information loss, it still leads to much-improved performance. Here, one can write the CFRs captured on different subcarriers and different time instances as a CFR matrix G with dimension K $\times$ N where N is the number of CFR frames collected. The matrix is given as

$$\mathbf{G} = \begin{bmatrix} G_{s_0}[t_0] & G_{s_0}[t_1] & \cdots & G_{s_0}[t_{N-1}] \\ G_{s_1}[t_0] & G_{s_1}[t_1] & \cdots & G_{s_1}[t_{N-1}] \\ \vdots & \vdots & \vdots & \vdots \\ G_{s_{K-1}}[t_0] & G_{s_{K-1}}[t_1] & \cdots & G_{s_{K-1}}[t_{N-1}] \end{bmatrix} \tag{1}$$

where $t_i$ stands for the time instance where the i-th CFR is captured and $s_j$ stands for the subcarrier index of the j-th subcarrier.

4. Inverse Fourier Transform: In this step, one can convert the filtered CFRs into time-domain CIRs for de-noising using inverse fast Fourier transform (IFFT). The reason why IFFT could improve the performance lies in that: the useful signal contained in MPCs with a large time delay is less significant than the MPCs with a smaller time delay mainly due to the significant attenuations relevant to the propagation distance of EM waves. By using IFFT, one could focus on the first few channel taps and ignore channel taps with a large time delay. The CIR of time $t_i$ takes the form

$$\underline{G}_n[t_i] = \sum_{k=s_0}^{s_{K-1}-1} G_{s_k}[t_i]e^{j2\pi\frac{nk}{K'}}, n = 0,1,2,\cdots,K'-1 \tag{2}$$

where $K'$ is the size of IFFT given as $2^{\text{ceil}[\log_2(K)]}$, where ceil() means ceiling operation.

5. Timestamp Correction: Ideally speaking, the time instances $t_i$ can be written as $(i - 1)T_s$ where $T_s$ is the CFR sampling interval. Nevertheless, in practice, other wireless systems might co-exist with the breathing tracking system. Due to the Carrier-sense multiple access with collision avoidance (CSMA/CA) mechanism, the transmission of WiFi packets are not uniformly scheduled over the air.

More specifically, the WiFi devices would probe the medium before transmission. In case that some other WiFi devices are transmitting packets, the other WiFi networks in the same area must remain silent, back-off for a period of time, and probe the medium again to check if there exists on-going WiFi traffic. Therefore, one can no longer assume that $t_i = (i - 1)T_s$.

To resolve this problem, one can turn to the interpolation technique. One can define the exact time instances to be interpolated as $[t_0, t_{N-1}]$ with a unit step size of $T_s$. Then, one can perform a linear interpolation onto the exact time instances based on the actual timestamps $t_0, t_1, t_2, ..., t_{N-1}$. This leads to the temporal corrected CIRs which can be written as

$$\overline{\mathbf{G}} = \begin{bmatrix} \overline{G}_0[t_0] & \overline{G}_0[t_1] & \cdots & \overline{G}_0[t_{N-1}] \\ \overline{G}_1[t_0] & \overline{G}_1[t_1] & \cdots & \overline{G}_1[t_{N-1}] \\ \vdots & \vdots & \vdots & \vdots \\ \overline{G}_{K'-1}[t_0] & \overline{G}_{K'-1}[t_1] & \cdots & \overline{G}_{K'-1}[t_{N-1}] \end{bmatrix} \tag{3}$$

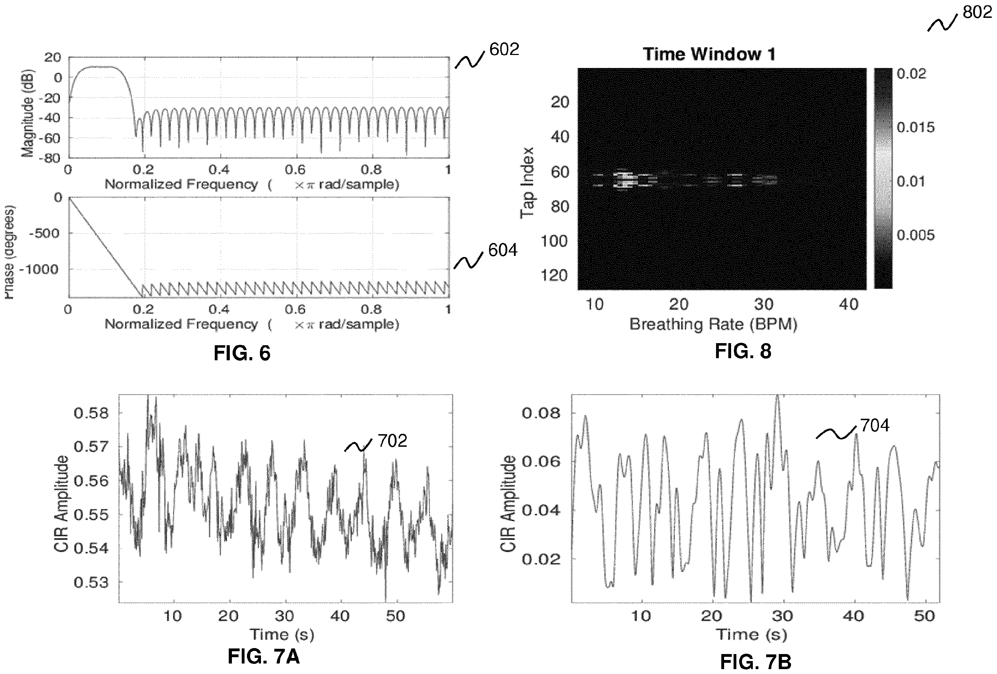

6. Bandpass Filtering: Now, the CFRs can be analyzed using standard spectral analysis tools. Notice that: the breathing rate of an average adult at rest ranges from 12 to 18 BPM. On the other hand, the breathing rate of an infant can be as high as 40 BPM, while the breathing rate of elderly people ranges from 10 to 30 BPM. Therefore, one can set the passband of the bandpass filter as 8 BPM to 42 BPM, which translates into 0.133 to 0.7 Hz. One can adopt a 81 taps finite-impulse-response (FIR) filter, with coefficients generated by MATLAB filter toolbox. The attenuation is -32 dB for first stopband ([0,8] BPM) and -40 dB for the right stopband ([42,300] BPM) (With a sounding rate of 10 Hz, the maximum resolvable frequency is 5 Hz which translates into 300 BPM). The amplitude and phase responses of the designed FIR filter are presented in FIG. 6. Denote the filter coefficients as $h_0, h_1, ..., h_{H-1}$, the filtered CFR matrix can be written as

$$\widetilde{\mathbf{G}} = \begin{bmatrix} \tilde{G}_0[t'_0] & \tilde{G}_0[t'_1] & \cdots & \tilde{G}_0[t'_{N'-1}] \\ \tilde{G}_1[t'_0] & \tilde{G}_1[t'_1] & \cdots & \tilde{G}_1[t'_{N'-1}] \\ \vdots & \vdots & \vdots & \vdots \\ \tilde{G}_{K'-1}[t'_0] & \tilde{G}_{K'-1}[t'_1] & \cdots & \tilde{G}_{K'-1}[t'_{N'-1}] \end{bmatrix} \tag{4}$$

where $t'_i = i \times T_s$, $N'$ is the length of one CFR frame on a given subcarrier expressed as $N - H + 1$, and $\tilde{G}_k[t'_j]$ represents the filtered CIR on the k-th tap, given as

$$\tilde{G}_k[t'_j] = \sum_{n=0}^{H-1} h[n]\tilde{G}_k[t_{j-n}], j \geq H - 1 \tag{5}$$

In FIG. 7a and FIG. 7b, it is shown that the temporal variations of CIR on a specific tap over time in the presence of breathing before and after performing the bandpass filtering. Clearly, bandpass filtering eliminates the high-frequency and low-frequency noise and makes the breathing signal much more stable over time. This lays the foundation of the spectral analysis via FFT as the next step.

7. Spectral Analysis via FFT: In this step, one can perform fast Fourier Transform (FFT) on the CIRs for each channel tap, which leads to K' spectrum estimations. The energy spectrum for the k-th tap on the u-th frequency bin is given as

$$F_k[u] = \left| \sum_{i=0}^{N''-1} \tilde{G}_n[t'_i]e^{-j2\pi\frac{iu}{N''}} \right|^2, u = -N''/2, -N''/2 + 1, \cdots, N''/2 - 1. \tag{6}$$

where $N''$ is the size of FFT given as $2^{\text{ceil}[\log_2(N')]}$, and the spectral resolution is thus $\Delta f = \frac{1}{N'' \times T_s}$. Given that the spectrum range-of-interest is $[f_1, f_2]$ (human breathing rates are confined in a small range), the range of frequency bins of interest can be written as $[u_1 = \text{ceil}[f_1 \times N'' \times T_s], u_2 = \text{floor}[f_2 \times N'' \times T_s]]$. Further taking the harmonics of breathing signal into consideration, one can extend the frequency bin range into $[-u_2, -u_1] \cup [u_1, u_2]$. Then, one can perform a spectrum folding which leads to the folded energy spectrum given as

$$\overline{F}_k[u] = \frac{1}{2}[F_k[u] + F_k[-u]], u \in \{u_1, u_1 + \Delta f, \cdots, u_2 - \Delta f, u_2\}. \tag{7}$$

Assembling the spectrum on all subcarriers together leads to the matrix F such that

$$\mathbf{F} = \begin{bmatrix} \overline{F}_{-K'/2}[u_1] & \overline{F}_{-K'/2}[u_1 + \Delta f] & \cdots & \overline{F}_{-K'/2}[u_2] \\ \overline{F}_{-K'/2+1}[u_1] & \overline{F}_{-K'/2+1}[u_1 + \Delta f] & \cdots & \overline{F}_{-K'/2+1}[u_2] \\ \cdots & \cdots & \cdots & \cdots \\ \overline{F}_{K'/2-1}[u_1] & \overline{F}_{K'/2-1}[u_1 + \Delta f] & \cdots & \overline{F}_{K'/2-1}[u_2] \\ \overline{F}_{K'/2}[u_1] & \overline{F}_{K'/2}[u_1 + \Delta f] & \cdots & \overline{F}_{K'/2}[u_2] \end{bmatrix} \tag{8}$$

FIG. 8 shows an example of the matrix F on a specific link in linear scale, with the ground-truth breathing rate as 12.5 BPM. One can observe strong spectral components around 12.5 BPM on multiple CIR taps, especially the CIR taps with index -5 to 5. This is intuitive since the energy of the breathing signal concentrates in the first few CIR taps.
8. Averaging over Channel Taps: One can take an average over channel taps with index falling in the range of $[0, S - 1]$, where S is termed as the stripe width. The averaged energy spectrum is then given as

$$\underline{F}[u] = \frac{1}{S}\sum_{k=0}^{S} \overline{F}_k[u], u \in \{u_1, u_1 + \Delta f, \cdots, u_2 - \Delta f, u_2\}. \tag{9}$$

9. Averaging over Antenna Links: In a MIMO system, there always exist multiple antennas on either or both ends of the transmitter and receiver which gives rise to diversity in wireless communication. In the proposed scheme, one can also incorporate multiple antenna links in MIMO systems to improve the overall performance of breathing tracking. For each antenna link $m$ out of a total of M links, one can calculate the folded energy spectrum as shown in (7) denoted as $\underline{F}_m[u]$, followed by computing the averaged energy spectrum over the available antenna links given as

$$\dot{F}[u] = \frac{1}{M}\sum_{m=1}^{M} \underline{F}_m[u], u \in \{u_1, u_2\}. \tag{10}$$

For the convenience of further processing, one can transform the linear scale energy spectrum into its dB scale counterpart as

$$\dot{F}_{dB}[u] = 10\log_{10}\dot{F}[u] \tag{11}$$

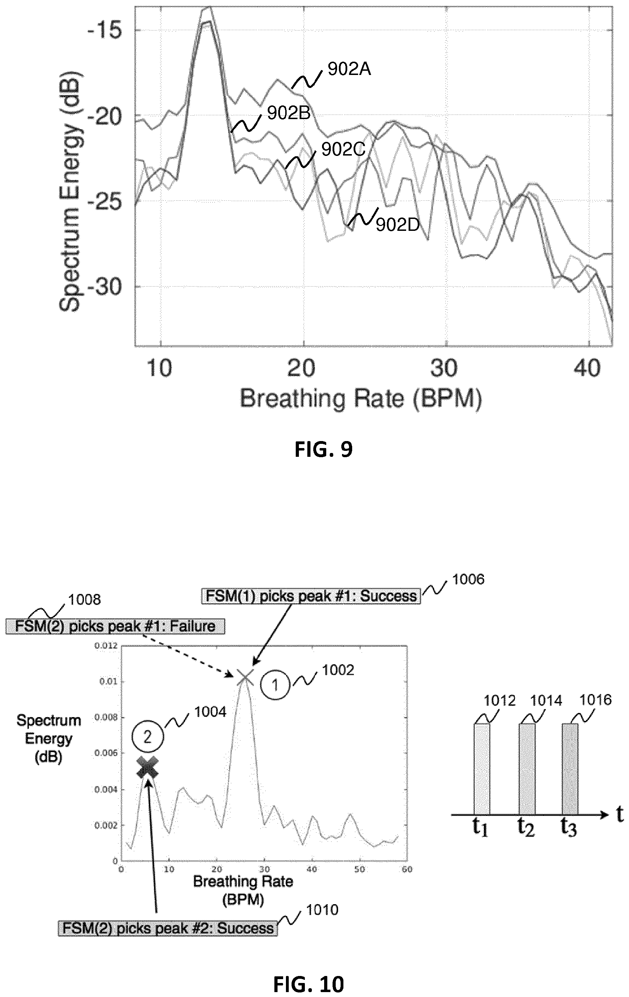

FIG. 9 shows several snapshots of the link averaged spectrum obtained from the same dataset that produces FIG. 8. Despite the temporal variations of the spectrum over time, one could always observe a strong spectral peak around the ground-truth breathing rate 12.5 BPM. 10. Peak Detection: The locations of the peaks in the energy spectrum $F_{dB}[u]$ indicate the estimated breathing rates. To extract these peaks, we leverage the persistence-based approach to obtain multiple pairs of local maximals and local minimals. For the i-th pair of local maximum $p_{max}[i]$ and minimum $p_{min}[i]$, we evaluate their difference as $v_i = p_{max}[i] - p_{min}[i]$, which can be regarded as the signal-to-noise ratio (SNR) for the i-th peak.

In the proposed scheme, assuming a total of U detected peaks in the energy spectrum, we use both the peak amplitude $\{p_{max}[i]\}_{i=1,2,\cdots,U}$ and the local peak SNR $\{v[i]\}_{i=1,2,\cdots,U}$ as features for further peak filtering. In particular, we keep those peaks with amplitudes and SNRs larger than two thresholds, namely, the amplitude threshold $p_{max}^0$ and SNR threshold $v^0$, respectively.

11. Breathing Rate Estimation: Assuming a total of $U'$ peaks remaining after the previous step and $P$ breathing rates to be detected, one can pick the top $P$ out of $U'$ peaks and use the corresponding peak locations $\{\hat{f}_i\}_{i=1,2,\cdots,U'}$ measured in Hz as the breathing rate estimations for $P$ people (When $P \geq U'$, then one can suffer from a miss detection rate of $\frac{P-U'}{P}$.

On the other hand, when $P < U'$, one can suffer from a false alarm rate of $\frac{U'-P}{U'}$). Meanwhile, one can record the spectrum energy without the peaks, denoted as F given as

$$\overline{F}_{dB} = 10\log_{10}\left(\sum_{u=u_1}^{u_2} 10^{\dot{F}_{dB}[u]} - \sum_{i=1}^{P} 10^{p_{\max}[i]}\right). \tag{12}$$

The metric $\overline{F}_{dB}$ is utilized to detect breathing in the finite state machine presented in the next section. The breathing rate estimations with BPM as the unit are then given as $\{\hat{b}_i\}_{i=1,2\cdots U}$, with $\hat{b}_i = 60\hat{f}_i$.

12. Real-time Updating: The proposed tracking system keeps updating the breathing rate estimations regularly. Here, one can define another two parameters: window size $C_{window}$ and shift size $C_{shift}$, both measured in the number of samples. The first set of breathing rate estimations are generated by performing spectral analysis on CFRs with sample index [0, $C_{window}$ - 1], and the second set of breathing rate estimations are generated by the same procedure on CFRs with sample index [$C_{shift}$, $C_{window}$ + $C_{shift}$ - 1]; the overlap between the two adjacent windows is then given as $C_{window}$ - $C_{shift}$. A smaller $C_{shift}$ leads to a more prompt real-time updating rate.

In a realistic environmental setting, there always exist motions from other people and/or objects, which introduces motion interference to the breathing tracking system. At the same time, motions of the subject under monitoring also inject interferences. Both types of interferences would significantly deteriorate the performance.

In light of this issue, one can supplement the proposed system with a finite-state-machine (FSM) composed of several states into the proposed breathing tracking system so that the proposed system could automatically tune its parameters such as peak detection thresholds under different states. In this work, there are four different states in the FSM which are illustrated below. For convenience, the transitions are shown under the single-person breathing tracking case. One can denote the peak search range as [$u_1$, $u_2$].

INIT: This is the default state when the system is powered on. In this state, the system uses the default peak detection thresholds ($p_{\max}^0$, $v^o$). If for a specific CFR time window w, it detects that $p_{\max}[0] \geq p^0$, $v[0] \geq v^0$, the system would tune the peak detection thresholds as ($p'^0 = p_{\max}^0 - \Delta p^0$, $v^0 = v^0 - \Delta v^0$). One can introduce $\Delta p^0$ and $\Delta v^0$ to leave margins for ensuing peak detections. Then, the FSM switches to state Verification. On the other hand, if $\overline{F}_{dB} \geq p_{motion}$ where $p_{motion}$ is the threshold for motion detection, the FSM switches to the state Motion to indicate that the current time window suffers from the major motion interferences. Otherwise, it stays in the current state. The output of the INIT state is a $P \times 1$ all-zero vector implying that no breathing rate estimations can be formulated. Also, in this state, the system restores the peak search range back to [$u_1$, $u_2$].

Verification: To avoid detection of fake peaks, one can use an internal counter to record how many times in consecutive that a peak can be detected. If a peak can be detected for a consecutive of y time windows, then the system switches its state into state PeakFound. In case that the peak becomes weaker, the FSM switches to state INIT. When $\overline{F}_{dB}$ exceeds $p_{motion}$, the FSM switches to the motion state. Otherwise, the system stays in the current state. The output of this state is the $P \times 1$ breathing rate estimations.

In this state, the system would zoom into a neighboring region of the estimated breathing rate. For instance, given an estimation of $\hat{b}$ with respect to a peak location at $\hat{f} = \hat{b}/60$, the system would select [$u'_1$, $u'_2$] where $u'_1 = \max[u_1, \hat{f} - \frac{\Delta S}{2} \times N''T_s]$ and $u'_2 = \min[u_2, \hat{f} + \frac{\Delta S}{2} \times N''T_s]$ are the starting and ending index of the updated search range. Here, $\Delta S$ stands for the size of the search range.

PeakFound: Now, the system determines that an authentic peak is detected and can be used as the breathing rate estimation. To prevent potential error propagation, the system no longer updates the two thresholds as shown in the Verification state. In case that the peak becomes weaker, the system switches back to the INIT state. Likewise, the system would switch to the motion state for sufficiently large $\overline{F}_{dB}$. The output of this state is the $P \times 1$ breathing rate estimations. Also, the search range is updated based on the breathing rate estimations.

Motion: In this state, the system decides that no credible breathing rate estimations can be produced due to the presence of strong motions. It can switches to the INIT state when $\overline{F}_{dB}$ falls below $p_{motion}$. The output of this state is the $P \times 1$ breathing rate estimations formulated in the latest Verification or PeakFound state.

The idea of FSM to overcome the motion interference issue under the single-person case can be extended to the multi-person case by running multiple FSMs in parallel, with each FSM captures the status of one specific person. The FSMs associated with different people interact with each other, i.e., they are not independent. To make the FSMs more predictable, one can define the following rules:

1. FSMs operating first would always select the strongest peak in its frequency range. For example, the i-th FSM, denoted as *FSM(i)*, selects the strongest peak in its search range [$u'_{1,i}$, $u'_{2,i}$].
2. To avoid duplication of breathing rate estimations, no two FSMs can pick the same peak in each time window. For example, if *FSM(i)* selects peak at location $\hat{f}_i$, then *FSM(j),j* > i would look for other peaks not located at $\hat{f}_i$.

3. To circumvent the situation of selecting peaks encapsulating the harmonics of breathing, *FSM(i)* would select the peak in its search range first denoted as $\hat{f}_i$. Then, *FSM(i)* checks the peaks selected by *FSM(j),j* = 1,2,···, i - 1 to see if there exists a selected peak $\hat{f}_j$ such that $|2\hat{f}_i - \hat{f}_j| < \varepsilon_{harmonic}$ or $|\hat{f}_i - 2\hat{f}_j| < \varepsilon_{harmonic}$ where $\varepsilon_{harmonic}$ is a predetermined threshold. If at least one such peak can be found, then *FSM(i)* continues to select the next significant peak in its search range. Otherwise, *FSM(i)* uses $\hat{f}_i$ as the detected peak, and the same procedure is repeated for the other FSMs sequentially.

Remark 1: Rule #2 cannot handle the following issue: two persons breathing with different breathing rates. The breathing rate of one person increases and the other decreases. Sooner or later, these two breathing rates would meet each other and the proposed FSM cannot get the breathing rate of the second person (the rendezvous problem).

FIG. 10 visualizes an example of two FSMs selecting two peaks sequentially. We assume that both peaks satisfy the criterion of thresholds. At time $t_1$, FSM 1 searches the energy spectrum and picks the strongest peak denoted as peak 1. Then, *FSM(2)* comes in and attempts to select peak 1. However, since the first peak is already taken by *FSM(1), FSM(2)* gives up its selection and moves on to peak 2.

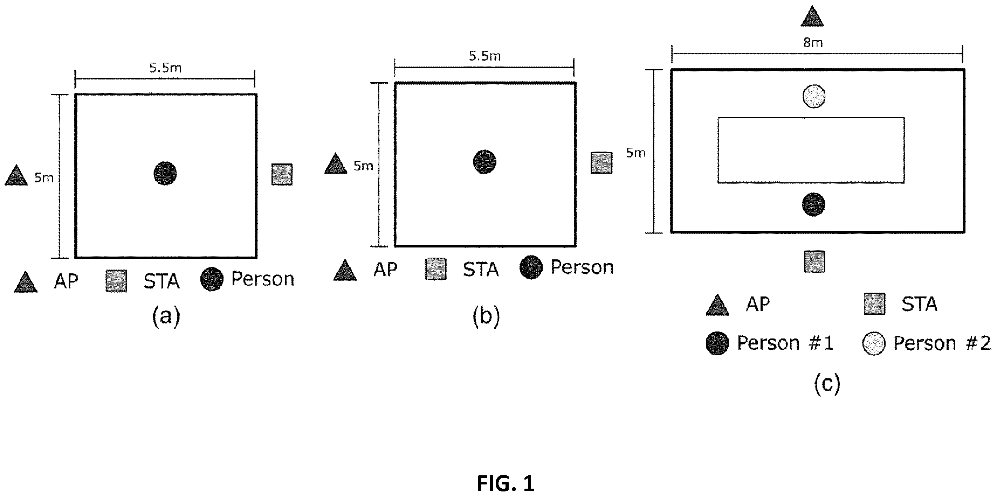

Experiments are shown below, which are done in an office environment. The experimental settings are demonstrated in FIG. 1. The adopted parameters are given in Table 1.

Table 1: Parameter settings for FFT-based breathing tracking with FSM

| Parameter | Notation | Value |
|---|---|---|
| CFR Sampling Interval | $T_s$ | 100ms |
| Usable subcarrier size | $K$ | 114 |
| IFFT size | $K'$ | 128 |
| FFT size | $N''$ | 512 |
| Bandpass filter length | $H$ | 81 |
| Spectrum range of interest | $[f_1, f_2]$ | [0.133Hz, 0.7Hz] |
| CFR window size | $C_{window}$ | 300 samples |
| CFR shift size | $C_{shift}$ | 50 samples |
| Peak detection threshold | $p^0_{max}, v^0$ | -30dB, 5dB |
| Search range size | $\Delta S$ | 1Hz |
| Peak detection updating margin | $\Delta p^0, \Delta v^0$ | 5dB, 5dB |
| Harmonic threshold | $\varepsilon_{harmonic}$ | 1Hz |

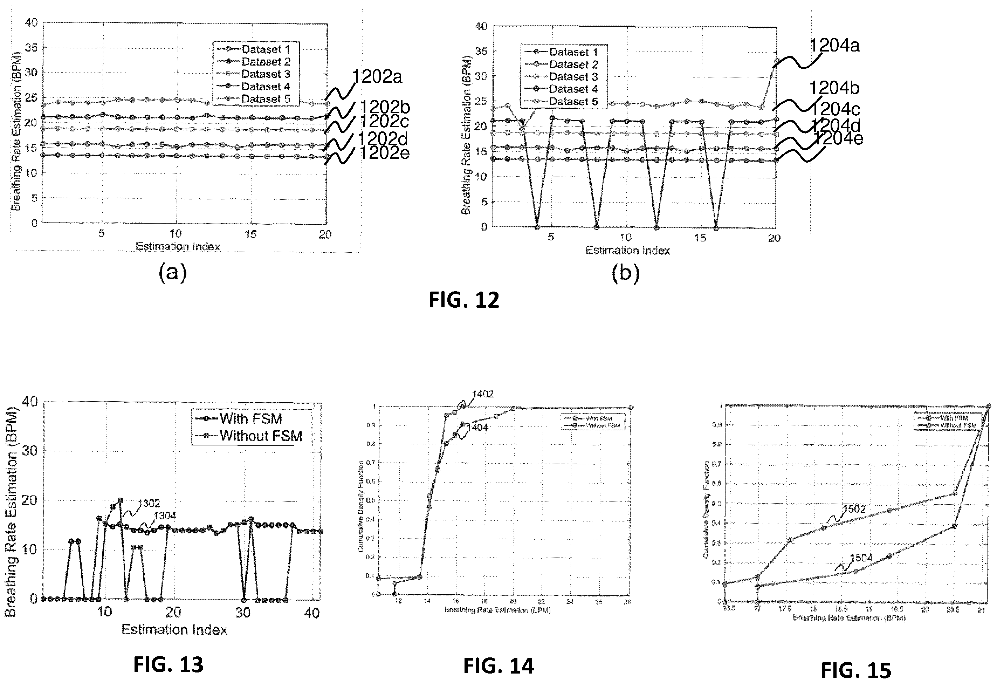

Single-Person NLOS: One can evaluate the performance of the proposed scheme for single-person NLOS breathing tracking. A total of 5 experiments have been conducted, with each experiment lasting for 2 minutes. The experimental setting is shown in FIG. 1(a). The ground-truths are given as [12.5,15,17.5,20,22.5] BPM with an increment of 2.5 BPM. The results are shown in FIG. 12. As one can see, when the ground-truths are [12.5,15,17.5] BPM, the differences between schemes with FSM and without FSM are negligible. However, when the breathing rates increase to 20 and 22.5 BPM, the FSM-based scheme outperforms the scheme without FSM. This is mainly due to the fact that when a person breathes faster, his/her breathing rate is not as stable as when he/she breathes slower, which introduces variations into the breathing signal strength and can be dealt with using FSM. The accuracy performances with FSM are given as [97.65%, 94.53%, 92.86%, 94.53%, 93.88%] respectively.

Single-Person NLOS with an Operating Fan: In this experiment, one can turn on an electronic fan inside the same room with the subject. Since the fan is an electronic device with metal cover, it would reflect some EM waves and thus introduce interference to the CFRs and degrade the tracking performance. The setting is shown in FIG. 1(b). FIG. 13 compares the scheme with and without FSM. A breathing rate estimation of 0 indicates that the system is unable to obtain a reliable breathing rate. Although using FSM does not fully recover the ground-truth breathing rate (15 BPM), the utilization of FSM still enhances the overall performance. The accuracy with FSM is 94.48%. In FIG. 14, one can compare the CDFs of the breathing rate as defined in (9). Again, it shows the advantages of using FSM, implying by that the CDF with FSM is more steep than the one without FSM.

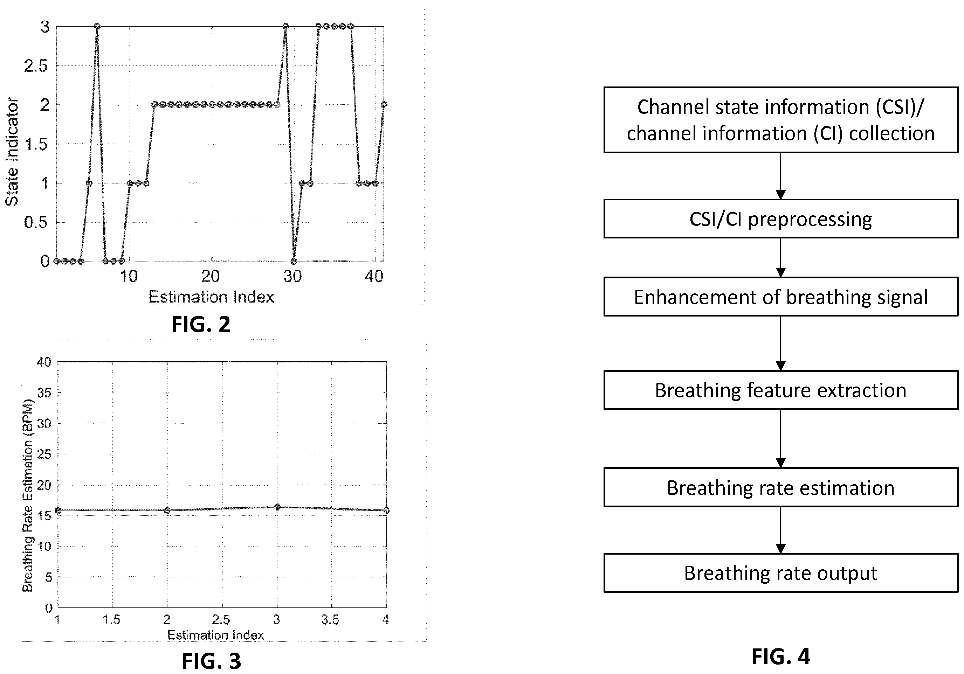

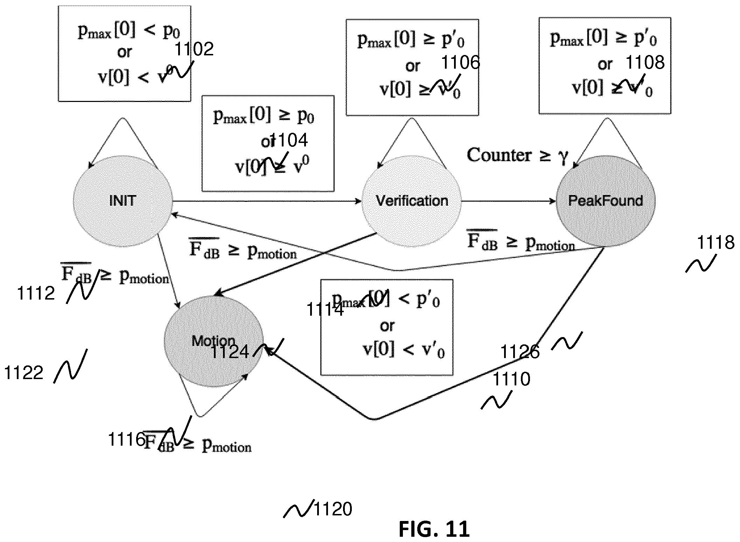

FIG. 2 demonstrates the state transition of the FSM-based scheme in this experiment. Different states shown in FIG. 11 are encoded as follows: Init → 0, Verification → 1, PeakFound → 2, Motion → 3. FIG. 2 shows that the FSM correctly reacts to the fan moving by switching to the Motion state from time to time.

One can conduct another experiment using the same setting except for a breathing rate of 20 BPM with results shown in FIG. 15. Again, using FSM enhances the performance indicated by the more concentrated breathing rate estimations. The accuracy is given as 94.53%. One can use the settings shown in FIG. 1(c) and FIG. 1b) for performance evaluation of multi-person breathing tracking.

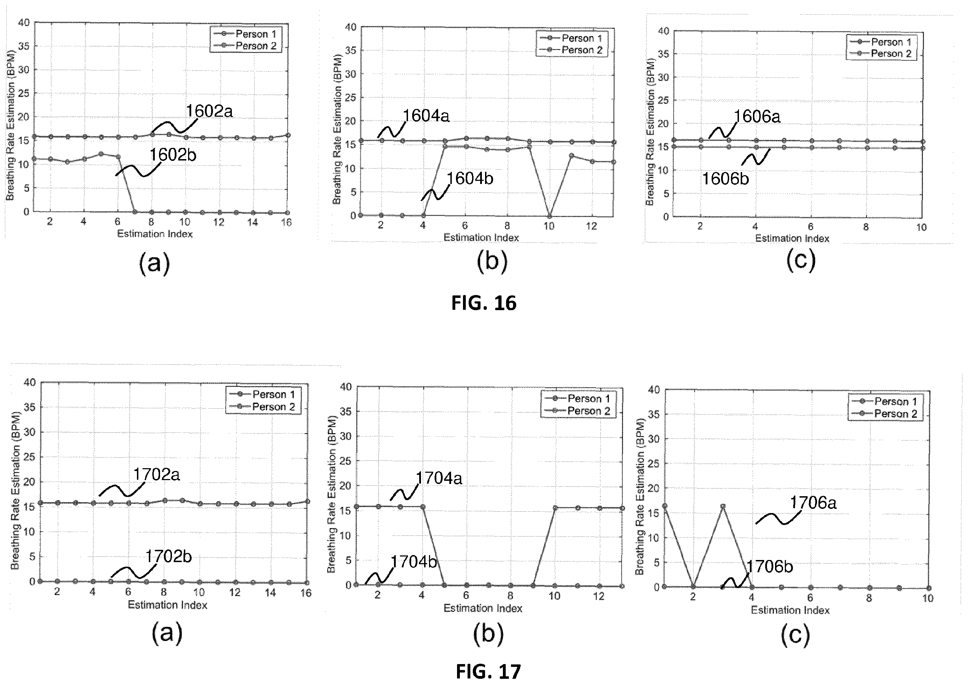

Firstly, one can study the performances of two-person breathing. The ground-truths are given as [15,16.5] BPM. The breathing rate estimations with $C_{window}$ = 300, $C_{window}$ = 450, and $C_{window}$ = 600 with FSM are shown in FIG. 16(a), FIG. 16(b), and FIG. 16(c), respectively. As can be seen from the figures, a time window size of 300 samples insuffices to resolve the two different breathing rates. The resolvability improves when the window size enlarges to 450 samples and 600 samples, with respect to a duration of 45 seconds and 60 seconds. The accuracies of breathing rate estimations under the three window sizes are [75.52%, 96.54%], [90.33%, 96.70%], and [99.61%, 99.43%], respectively. The breathing rate estimation results without FSM with $C_{window}$ = 300, $C_{window}$ = 450, and $C_{window}$ = 600 are demonstrated in FIG. 17(a), FIG. 17(b), and FIG. 17(c), respectively. In comparison with the results utilizing FSM, the performance degradation is very severe. In particular, when $C_{window}$ = 600, the breathing rate of the second person cannot be recovered.

FIG. 3 demonstrates the performance of the proposed breathing tracking scheme when the subject stands up for five seconds and sits down during the measurement. Clearly, the proposed breathing tracking scheme overcomes the impact of such large motions and produce stable breathing rate estimations over time. The present disclosure provided a robust vital sign tracking scheme that analyzes the tiny temporal variations in the CFRs. On each antenna link, the proposed scheme transforms CFRs into CIRs and performs spectral analysis on the CIRs via FFT. The spectrum of different antenna links are then fused into one averaged spectrum. Then, persistence-based peak detection is performed on the spectrum which finally leads to the breathing rate estimation. Furthermore, FSM is introduced into the presented system such that the vital sign tracking system would adopt different thresholds in different states, and it significantly improves the robustness of vital sign tracking. Extensive experimental results validate that the proposed scheme could perform well for single-person as well as multi-person breathing tracking.

An exemplary explanation of a speed estimation method is as below. Assume that the three receive antennas are aligned in a line parallel to the moving direction. Furthermore, one can assume that the speed of the receive antennas is changes slowly in time, i.e., within a short period, the speed can be approximated to be a constant value v. One can group the receive antennas Rx2 and Rx3 together and call them the leading antennas, and group the receive antennas Rx1 and Rx2 together and call them the following antennas. This is because the following antennas will go through the same trajectory as the leading antennas, but with an unknown delay $\tau$. The speed can be estimated by the relation: $\hat{v} = \frac{d}{\tau}$, where d is the separation between adjacent antennas (equal separation). Therefore, the speed estimation problem is transformed to the estimation of $\tau$. In the following, one can use the TRRS to estimate $\tau$. Let use $H_i(t)$ to denote the CSI measured at the i-th receive antenna at time t and use $\Delta t$ to stand for the duration between two adjacent CSI measurement. For each time $t_1$, one can form the reference CSI, which is measured by the leading antennas, as $\boldsymbol{H}_{lead}(t_1) = [H_2(t_1), H_3(t_1), H_2(t_1 + \Delta t), H_3(t_1 + \Delta t), ... , H_2(t_1 + (N - 1)\Delta t), H_3(t_1 + (N - 1)\Delta t)]$, and form the CSI for comparison, which is measured by the following antennas, as $\boldsymbol{H}_{follow}(t) = [H_1(t), H_2(t), H_1(t + \Delta t), H_2(t + \Delta t), ... , H_1(t + (N - 1)\Delta t), H_2(t + (N - 1)\Delta t)]$. The TRRS $\gamma(t_1, t)$ value between $\boldsymbol{H}_{lead}(t_1)$ and $\boldsymbol{H}_{follow}(t)$ is computed. When the following antennas reach the location of leading antennas corresponding to time $t_1$, $\gamma(t_1, t)$ will achieve its maximum over the variable t. In the example, one can have t = $t_2$ and thus one can have the estimation $\hat{\tau} = t_2 - t_1$.

============

*Coin-sized Bot*

This disclosure is also related to object tracking using a small (e.g. coin-size, cigarette box size, or even smaller, etc.) bot/origin. In our disclosure, the wireless transmitter and/or the wireless receiver may be part of a small light-weight first portable device. The first portable device may be wirelessly coupled with a second device. The second device may be smart phone, iPhone, Android phone, smart device, smart appliance, smart vehicle, smart gadget, smart TV, smart refrigerator, smart speaker, smart watch, smart glasses, smart pad, iPad, computer, wearable computer, notebook computer, gateway. The second device may be connected to a cloud server, a local server and/or other server via internet, wired internet connection and/or wireless internet connection. The second device may be portable. The first portable device, the second device, a local server and/or a cloud server may share the computation and/or storage for any of the tasks (e.g. obtain TSCI, determine characteristics of the object associated with the movement of the object, computation of time series of power information, determining/computing the particular function, searching for local extremum, classification, identifying particular value of time offset, de-noising, processing, simplification, cleaning, wireless smart sensing task, extract CI from wireless signal, switching, segmentation, estimate trajectory, process the map, correction, corrective adjustment, adjustment, map-based correction, detecting error, checking for boundary hitting, thresholding, etc.) and information (e.g. TSCI) associated with this disclosure.

*CSI ready Chip*

This disclosure is also related to communication hardware that is capable of providing the CI. The communication hardware may be a WiFi-capable chip (e.g. integrated circuit or IC), a next generation WiFi-capable chip, a LTE-capable chip, a 5G-capable chip, a 6G/7G/8G-capable chip, etc. The communication hardware computes the CI and stores the CI in a buffer memory and make the CI available for extraction. The CI may comprise data and/or at least one matrices related to channel state information (e.g. CSI). The at least one matrices may be used for channel equalization, and/or beam forming, etc.

*Passive Speed Estimation (gait cycle estimation, fall down detection)*

Another aspect of the disclosure is related to the estimation of one or more characteristics and/or spatial-temporal information of the object such as speed, acceleration, gait cycle, a gait, an object motion, and an event. More specifically, the present teaching is related to determining at least one characteristics and/or spatial-temporal information of the object based on time reversal technology in a rich scattering environment. The characteristics and/or spatial-temporal information may be related to a event. The event may be monitored based on the characteristics and/or spatial-temporal information.

In one example, a system, apparatus and/or method for determining at least one characteristics and/or spatial-temporal information of an object (e.g. object) associated with a movement of the object (e.g. current movement, and/or prior movement) comprises obtaining one or more TSCI (e.g. CI and/or) of a wireless multipath channel (e.g. wireless multipath channel) using a processor, a memory communicatively coupled with the processor and a set of instructions (e.g. set of instructions) stored in the memory. The at least one TSCI (e.g. CI, or channel state information) is obtained from a wireless signal (e.g. wireless signal) sent through the wireless multipath channel. The wireless multipath channel is impacted by the movement of the object. The system, apparats and/or method further comprises determining the one or more characteristics and/or spatial-temporal information of the object associated with the movement of the object based on the one or more TSCI.

One or more time series of power information may be computed based on the one or more TSCI of the wireless multipath channel. Each power information may be associated with a CI of the wireless multipath channel. Both the power information and the CI may be associated with a time. The vector of the CI and the vector of the power information may have the same number of elements. Each real element of the power information vector may be computed based on respective magnitude, phase, real part, imaginary part and/or another function of corresponding complex element of the CI. In particular, the real element of the power information vector may be based on square of magnitude of corresponding complex element of the channel info.

A particular function may be determined based on a first time, a time offset, a first window of power information and a second window of power information. Although power information is mentioned here, the particular function may be applied to CI and/or other information. The first window of power information may be a first subset of a particular time series of power information associated with the first time. The second window of power information may be a second subset of the particular time series of power information associated with a second time which is at the time offset from the first time. The first window and the second window may be of the same size. The second window is at the time offset from the first window. The first window and the second window may both be windows of the same particular time series of power information (and/or or CI, and/or other information). The cardinality of the first subset may be equal to the cardinality of the second subset. The particular function may be computed for at least one value of the first time and for at least one value of the time offset for each of the first time. The one or more characteristics and/or spatial-temporal information of the object associated with the movement of the object may be determined based on the articular function computed for the at least one value of time offset. A particular value of the first time may be associated with the movement of the object and the at least one characteristics and/or spatial-temporal information of the object. The particular function may be a second function of at least one of: a covariance function, a covariance-like function, an auto-covariance function, an auto-covariance-like function, a correlation function, a correlation-like function, an auto-correlation function, an auto-correlation-like function, an inner product, another function, a combination of any of these functions with a preprocessing function, a combination of any of these functions with a post-processing function, and a combination of any of these functions with at least one of: frequency decomposition, time decomposition, time-frequency decomposition, and another decomposition. The second function may be at least one of: de-noising, smoothing, conditioning, enhancement, restoration, feature extraction, weighted averaging, low-pass filtering, bandpass filtering, high-pass filtering, median filtering, ranked filtering, quartile filtering, percentile filtering, mode filtering, linear filtering, nonlinear filtering, finite impulse response (FIR) filtering, infinite impulse response (IIR) filtering, moving average (MA) filtering, auto-regressive (AR) filtering, auto-regressive moving average (ARMA) filtering, thresholding, soft thresholding, hard thresholding, soft clipping, local maximization, local minimization, optimization of a cost function, neural network, machine learning, supervised learning, unsupervised learning, semi-supervised learning, transform, Fourier transform, Laplace, Hadamard transform, transformation, decomposition, selective filtering, adaptive filtering, derivative, first order derivative, second order derivative, higher order derivative, integration, zero crossing, indicator function, absolute conversion, convolution, multiplication, division, preprocessing, post-processing, another transform, another processing, another filter, and a third function, and a third function. The particular function may be an estimate of a weighted sum of square of autocorrelation function (ACF) of electric field components of the received electromagnetic (EM) wave of the wireless signal.

The power information may include a frequency decomposition, time decomposition, time-frequency decomposition, and/or another decomposition. Another function may be determined based on a frequency of the frequency decomposition (or a variable/index of other decompositions), the first time, the time offset, the first window of power information and the second window of power information. The another function may be computed for at least one value of the frequency of the frequency decomposition. The particular function may be computed by averaging the another function computed for the at least one value of the frequency of the frequency decomposition.

In another example, the power information may include a frequency subband decomposition. Another function is determined based on a frequency subband of the frequency decomposition, the first time, the time offset, the first window of power information and the second window of power information. The another function may be computed for at least one instance of the frequency subband of the frequency decomposition. The particular function may be computed by averaging the another function computed for the at least one instance of the frequency subband of the frequency decomposition. In another example, the power information may comprise a time-frequency decomposition. Another function may be determined based on a time-frequency partition of the time-frequency decomposition, the first time, the time offset, the first window of power information and the second window of power information. The another function may be computed for at least one instance of the time-frequency partition of the time-frequency decomposition. The particular function may be computed by averaging the another function computed for the at least one instance of the time-frequency partition of the time-frequency decomposition.

The computing of the particular function may comprise determining a preliminary function based on the first time, a third time, the time offset, a third window of power information and a fourth window of power information. The third window of power information may be a third subset of the particular time series of power information associated with the third time. The fourth window of power information may be a fourth subset of the particular time series of power information associated with a fourth time at the time offset from the third time. The preliminary function may be computed for a particular value of the first time and a particular value of the time offset for at least one value of the third time close to the first time. The particular function for the particular value of the first time and the particular value of the time offset may be computed by averaging the preliminary function for the particular value of the first time and the particular value of the time offset computed for the at least one value of the third time close to the first time.

At least one special characteristics and/or special spatial-temporal info (e.g. local maximum, local minimum, zero crossing, etc.) may be determined/computed for a particular value of the first time. At least one particular value of the time offset each associated with a respective special characteristics and/or spatial-temporal information (e.g. local maximum, local minimum, zero crossing, etc.) of the particular function for the particular value of the first time may be identified. The at least one special characteristics and/or special spatial-temporal info of the object associated with the movement of the object may be determined based on the at least one particular value of the time offset.

There may be a search for a local extremum of the particular function for a particular value of the first time. The search may comprise the following steps. (1) A current time offset value may be initialized. This is followed by a recursion. (2) In each iteration of the recursion, a regression window around the current time offset value may be determined. (3) The particular function within the regression window around the current time offset value may be approximated with/by a regression function using regression analysis. (4) Regression error of the regression function with respect to the particular function may be determined. (5) The current time offset value may be updated based on convexity of the regression function, another characteristics and/or spatial-temporal information of the regression function, and/or regression error of the regression function with respect to the particular function in the regression window. (6) Performing the iteration of the recursion until at least one stopping criterion is satisfied.

The local extremum of the particular function may be determined for the particular value of the first time based on the corresponding extremum of the regression function in the regression window. A particular value of the time offset associated with the local extremum of the particular function may be identified in the regression window. At least one characteristics and/or spatial-temporal information of the object associated with the movement of the object may be computed based on the particular value of the time offset.

In one example, if the regression function is locally convex in the regression window, time offset associated with maximum regression error of the regression function with respect to the particular function in the regression window may become the updated current time offset. In another example, if the regression function is locally concave in the regression window, time offset associated with maximum regression error of the regression function with respect to the particular function in the regression window may become the updated current time offset.

If the regression function is locally convex in the regression window, at least one of time offsets associated with two ends of the regression window may become the updated current time offset. If the regression function is locally concave in the regression window, at least one of time offsets associated with two ends of the regression window may become the updated current time offset.

At least one of time offsets associated with two ends of the regression window may become the updated current time offset based on the local convexity of the regression function in the regression window. If the regression is locally convex in part of the window and locally concave in another part of the window, the window size may be reduced.

A time offset near time offset associated with maximum regression error of the regression function with respect to the particular function in the regression window may become the updated current time offset based on a condition.

One of the at least one characteristics and/or spatial-temporal information of the object associated with the movement of the object may be speed of the object. The speed of the object may be computed based on the particular value of time offset associated with the local extremum of the particular function for a particular value of the first time in the regression window. The speed of the object at a time may be computed based on the particular value of time offset associated with the local extremum of the particular function for a particular value of the first time in the regression window. The particular value of the first time may be equal to the time. An acceleration of the object at a particular time may be determined based on at least one value of the speed of the object each at a time close to the particular time. The acceleration of the object at the particular time may be one of the at least one characteristics and/or spatial-temporal information of the object associated with the movement of the object. An acceleration of the object at a particular time may be determined based on at least one value of the speed of the object at one or more time each close to the particular time. The determination of the acceleration of the object at the particular time may comprise: approximating the speed of the object at the one or more time by a time function, determining one or more slopes of the time function close to the particular time, and computing the acceleration based on the one or more slopes of the time function. The acceleration may be computed as a function of the one or more slopes of the time function. The function may be an average, a weighted average, a median, a mode, a quartile, a percentile, a robust function, a linear function, a nonlinear function, and/or another function.

One of the one or more slopes of the time function may be determined as a difference between two values of the speed of the object at two adjacent times divided by a difference of the two adjacent time values. The time function may be a piecewise linear function. The acceleration may be computed as a slope of the piecewise linear function at the particular time. The one or more time may be within a time window around the particular time. The one or more time may comprise a time window around the particular time. The acceleration of the object may be the acceleration of the object associated with the movement of the object at the particular time. An acceleration of the object at a particular time may be determined based on a speed of the object computed at multiple values of time close to the particular time. The speed of the object may be one of the at least one characteristics and/or spatial-temporal information of the object associated with the movement of the object determined based on the at least one TSCI.

The speed of the object may be one of the at least one characteristics and/or spatial-temporal information of the object associated with the movement of the object determined based on the particular function computed for the particular value of first time and the at least one value of time offset, with the particular value of the first time being one of the multiple values of time close to the particular time. The speed of the object may also be one of the at least one characteristics and/or spatial-temporal information of the object associated with the movement of the object determined based on the time offset associated with the local extremum of the particular function for the particular value of the first time in the regression window, the particular value of the first time being equal to the time. The speed of the object at the particular value of the first time may be computed as a quantity divided by the particular value of time offset associated with the local extremum of the particular function in the regression window.

In another example, one of the at least one characteristics and/or spatial-temporal information may be speed of the object. The speed of the object at the first time may be computed based on the particular value of time offset associated with the local extremum of the particular function in the regression window. The local extremum may be the first local maximum with positive time offset, and/or the first local maximum with negative time offset. The particular function may be a derivative of a characteristic auto-correlation function with respect to the time offset. The CI may be CSI associated with a frequency decomposition. Each CSI may be a vector of complex components each corresponding to a frequency of the frequency decomposition. Component-wise auto-correlation function may be computed for each frequency of the frequency decomposition based on the particular time series of CSI. The characteristic auto-correlation may be computed as average of the component-wise auto-correlation function over the frequency of the frequency decomposition.

The iteration may be applied at least one time to obtain at least one extremum. Each extremum may be associated with a respective current time offset value, a respective regression window, a respective regression function, and/or a respective particular time offset value in the respective iteration. The at least one characteristics and/or spatial-temporal information of the object associated with the movement of the object may be computed based on at least one of the respective particular time offset value of the particular function in the regression window. The iteration may be applied two times to obtain a first local extremum and a second local extremum. The first local extremum may be associated with a first current time offset value, a first regression window, a first regression function, and/or a first particular time offset value. The second local extremum may be associated with a second current time offset value, a second regression window, a second regression function, and/or a second particular time offset value. The at least one characteristics and/or spatial-temporal information of the object associated with the movement of the object may be computed based on the first particular time offset value and/or the second particular time offset value of the particular function in the regression window. The iteration may be applied two times to obtain a first local extremum which is first local maximum with positive time offset, and a second local extremum which is first local maximum with negative time offset. The first local extremum may be associated with a first current time offset value, a first regression window, a first regression function, and/or a first particular time offset value. The second local extremum may be associated with a second current time offset value, a second regression window, a second regression function, and/or a second particular time offset value. The at least one characteristics and/or spatial-temporal information of the object associated with the movement of the object may be computed based on the first particular time offset value and/or the second particular time offset value, of the particular function in the regression window.

A gait cycle of the object may be determined based on at least one value of the speed of the object, and/or at least one value of acceleration of the object. At least one gait cycle of the object may also be determined based on at least one value of the speed of the object and/or at least one value of the acceleration of the object. The at least one gait cycle may be determined by detecting a succession of a period of positive acceleration and a period of negative acceleration. The at least one gait cycle may be determined by detecting a succession of a period of increasing speed and a period of decreasing speed. The at least one gait cycle may be determined by detecting a succession of a period of positive speed and a period of negative speed. The at least one gait cycle may be determined by detecting an underlying periodic behavior of the speed of the object, a speed change of the object, and/or the acceleration of the object, with an associated time period. The at least one gait cycle may be determined by detecting an underlying periodic behavior of local maximum speed of the object, local minimum speed of the object, local maximum acceleration of the object, local minimum acceleration of the object, local maximum speed change of the object, and/or local minimum speed change of the object, with an associated time period. The at least one gait cycle may be determined to be associated with a time period. A function of the speed of the object and/or the acceleration of the object may exhibit a local maximum at a location associated with the time period. The at least one gait cycle may be determined to be associated with a time period. A function of the speed of the object and/or the acceleration of the object may be determined to exhibit peak localization with multiple pairs of local maximum and local minimum with associated multiple pairs of peak location associated with the time period.

The time period may be determined based on a distance between at least one of: two adjacent local maximum, two adjacent local minimum, a pair of adjacent local maximum and local minimum, two local maximums separated by a predetermined number of maximums, two local minimums separated by a predetermined number of minimums, and a pair of local maximum and local minimum separated by a predetermined number of extremum, of the function. The function may comprise an auto-correlation function, an auto-correlation like function, an auto-covariance function, an auto-covariance like function, a product of the speed and a shifted version of the acceleration, a product of the speed and a shifted version of a periodic function with known period, a product of the acceleration and the shifted version of the periodic function with known period, an optimization of a cost function, a constrained optimization of the cost function, a unconstrained optimization of the cost function, a Fourier transform, a Laplace transform, another transform, and/or another function.

At least one gait cycle of the object may be determined based on at least one of: at least one value of a speed of the object and at least one value of an acceleration of the object. The speed of the object may be one of the at least one characteristics and/or spatial-temporal information of the object associated with the movement of the object determined based on the at least one TSCI. The acceleration of the object may be determined based on the speed of the object. At least one gait cycle of the object may be determined based on at least one of: at least one value of a speed of the object and at least one value of an acceleration of the object. The speed of the object may be one of the at least one characteristics and/or spatial-temporal information of the object associated with the movement of the object determined based on the particular function computed for the particular value of first time and the at least one value of time offset, with the particular value of the first time being one of the multiple values of time close to the particular time. The acceleration of the object may be determined based on the speed of the object. At least one gait cycle of the object may be determined based on at least one of: at least one value of a speed of the object and at least one value of an acceleration of the object. The speed of the object may be one of the at least one characteristics and/or spatial-temporal information of the object associated with the movement of the object determined based on the time offset associated with the local extremum of the particular function for the particular value of the first time in the regression window, the particular value of the first time being equal to the time. The acceleration of the object may be determined based on the speed of the object. A gait of the object and/or an event may be detected based on at least one of: at least one segment of a TSCI, at least one segment of a time series of channel state information (CSI), at least one value of the particular function, at least one value of the speed of the object, and/or at least one value of an acceleration of the object each based on at least one value of the speed of the object. The gait of the object and/or the event may be detected based on a time trend of at least one of: the CI, the channel state information (CSI), the particular function, the speed of the object, and/or the acceleration of the object. The detection of the gait of the object and/or the event may comprise determining the gait of the object and/or the event, using an engine based on the time trend of the speed of the object. Input to the engine may comprise at least one of: at least one segment of a TSCI, at least one segment of a time series of channel state information (CSI), at least one value of the particular function, at least one value of the speed of the object, at least one value of an acceleration of the object. The engine may be a neural network and/or a classification engine.

The engine may be trained in a training phase with training input to generate at least one reference time trend associated with known gait of the object and known event. The training input of the engine may be associated with the known gait of the object and the known event. The training may be at least one of: discriminative training, decision feedback training, machine learning, supervised learning, unsupervised learning, shallow learn, deep learning, and/or another training. The determination of the gait of the object and/or the event using the engine may comprise: obtaining the time trend, comparing the time trend with each of the reference time trend, and aligning the time trend of a first duration with each of the at least one reference time trend of heterogeneous duration. It may further comprise computing at least one similarity score each between the time trend and respective aligned reference time trend, and/or determining the gait of the object and/or the event as one of the known gaits of the object and/or one of the known events, based on the at least one similarity score.

The speed of the object may be one of the at least one characteristics and/or spatial-temporal information of the object associated with the movement of the object determined based on the at least one TSCI. The speed of the object may be one of the at least one characteristics and/or spatial-temporal information of the object associated with the movement of the object determined based on the particular function computed for the particular value of first time and the at least one value of time offset, with the particular value of the first time being one of the multiple values of time close to the particular time.

At least one threshold may be determined. The at least one characteristics and/or spatial-temporal information of the object may be determined based on the at least one threshold. The at least one threshold may be determined based on at least one time series of training CI (CI) of the wireless multipath channel using a second processor, a second memory communicatively coupled with the second processor and a second set of instructions stored in the second memory . The second processor may be the first processor, part of the first processor, and/or connected with the first processor. Some resources (hardware, network, communication, user-interface, and/or software) of the second processor may be shared with the first processor. The second memory may be the first memory , part of the first memory , connected to the first memory , and/or shared with the first memory . The second set of instructions may be the first set of instructions, and/or part of the first set of instructions. The second set of instructions and the first set of instructions may share some software library, some software routine, some hardware resources, some communication resources and/or other resource. The second set of instructions and the first set of instructions may work independently, cooperatively, jointly, sequentially, in parallel, alternately, and/or interactively.

The at least one time series of training CI (CI) of the wireless multipath channel may be obtained from a second wireless signal (e.g. 140) sent through the wireless multipath channel in a training phase. The wireless multipath channel may be impacted by a training movement of a second object in the training phase. The training phase may be a training session, which may be carried out once, occasionally, regularly, and/or on demand.

At least one time series of first training CI of the wireless multipath channel associated with a target positive training movement of the second object in the training phase may be obtained. The positive training movement may be a target movement to be recognized, monitored, measured, studied, processed, detected, estimated, verified, and/or captured. At least one time series of second training CI of the wireless multipath channel associated with a target negative training movement of the second object in the training phase may be obtained. The negative training movement may be a target movement to be ignored, missed, not monitored, not detected, not estimated, not recognized, not verified, not captured, not measured, and/or not studied. The at least one threshold may be determined based on the at least one first quantity and/or the at least one second quantity. The at least one threshold may be determined such that a first percentage of the first quantity is larger than, equal to and/or less than a first threshold (not the at least one threshold). The at least one threshold may be determined such that a second percentage of the second quantity is larger than, equal to and/or less than a second threshold (not the at least one threshold). The first threshold may be greater than, equal to and/or less than the second threshold. The first threshold may be the second threshold. The first percentage may be greater than, equal to and/or less than the second percentage.

The at least one time series of first training CI of the wireless multipath channel may be associated with a training movement of the second object inside a monitored area in the training phase. The target positive training movement of the second object may be the training movement of the second object inside the monitored area. The at least one time series of second training CI of the wireless multipath channel may be associated with a training movement of the second object outside the monitored area in the training phase. The target negative training movement of the second object may be the training movement of the second object outside the monitored area.

The second object may be the first object. The second object may be an imitation, a replacement, a backup, and/or a replica of the first object. The second object may be another object similar to the first object. The second object may be similar to the first object in terms of structure, size, shape, functionality, periodicity, deformation characteristics, motion characteristics, speed, acceleration, gait, trend, habit, wireless properties, and other characteristics.

In the case of at least one Type1 devices interacting with at least one Type2 devices, the processing, preprocessing, post-processing, and/or other processing may be different for different devices. The processing/preprocessing/post-processing/other processing may be based on locations, orientation, direction, roles, user-related characteristics, settings, configurations, available resources, available bandwidth, network connection, hardware, software, processor, co-processor, memory, battery life, available power, antennas, antenna types, directional/unidirectional characteristics of the antenna, power setting, and/or other parameters/characteristics of the devices.

*Breathing based on ACF*

Another embodiment is a method/apparatus/device/system/software of a wireless monitoring system comprising transmitting a series of probe signals by an antenna of a first wireless device to at least one heterogeneous target wireless receiver through a wireless multipath channel in a venue.

The transmitting may be using a processor, a memory and a set of instructions. The first wireless device may have a heterogeneous integrated circuit (IC). Each of the at least one heterogeneous target wireless receiver may have a heterogeneous IC.

It further comprises obtaining asynchronously at least one TSCI (TSCI) from the series of probe signals by each of the at least one heterogeneous target wireless receiver. The CI (CI) is CI of the wireless multipath channel between the heterogeneous target wireless receiver and the first wireless device. It further comprises monitoring asynchronously a respective motion associated with the venue and/or a respective object in the venue, based on respective at least one TSCI (obtained from the series of probe signals by respective heterogeneous target wireless receiver). The respective motion may be associated with a respective event associated with at least one of: the venue and the respective object in the venue. More than one of the respective objects may be the same object such that more than one heterogeneous target wireless receivers may be monitoring the same object. More than one of the respective motions may be a same motion. More than one heterogeneous target wireless receivers may be monitoring the same motion. The method/apparatus/device/system/software of the wireless monitoring system may comprise monitoring a same motion jointly based on TSCI of more than one heterogeneous target wireless receivers. The respective motion monitored by each of the more than one heterogeneous target wireless receivers may be the same motion. The method/apparatus/device/system/software of the wireless monitoring system may comprise monitoring a same object jointly based on TSCI of more than one heterogeneous target wireless receivers. The respective object in the venue monitored by each of the more than one heterogeneous target wireless receivers may be the same object. The method/apparatus/device/system/software of the wireless monitoring system may comprise monitoring a respective characteristics of the respective motion of the respective object in the venue. The method/apparatus/device/system/software of the wireless monitoring system may comprise monitoring a periodic characteristics of the respective motion in the venue based on the respective at least one TSCI.

Each CI (CI) may be a channel frequency response with more than one components. Each respective component may be associated with a respective frequency. Respective components (of each TSCI) associated with a respective frequency may form a respective component time series. The component time series may be associated with the respective frequency. Each TSCI may be divided into more than one component time series. Each respective component time series may be associated with a respective frequency.

The method/apparatus/device/system/software of the wireless monitoring system may comprise monitoring the respective motion based on respective more than one component time series of each of respective at least one TSCI. Each component time series may be associated with respective frequency. The method/apparatus/device/system/software of the wireless monitoring system may comprise monitoring a periodic characteristics of the respective motion based on respective more than one component time series of each of the respective at least one TSCI. A periodic characteristics of the respective motion may be monitored based on the component-wise periodic characteristics of each of the more than one component time series of each of the respective at least one TSCI.

A component-wise periodic characteristics of a component-feature time series of each of the more than one component time series of each of the respective at least one TSCI may be computed. Each component-wise periodic characteristics may be associated with respective component index. The periodic characteristics of the respective motion may be monitored based on the component-wise periodic characteristics of each of the more than one component time series of each of the respective at least one TSCI.

A total periodic characteristics of each of the respective at least one TSCI may be computed based on the component-wise periodic characteristics of each component time series of the TSCI. The periodic characteristics of the respective motion may be monitored based on the total periodic characteristics of each of the respective at least one TSCI.

The total periodic characteristics of the TSCI may be a weighted sum of the component-wise periodic characteristics of the more than one component time series of the TSCI. In the total periodic characteristics, the weight for a component-wise periodic characteristics of a component time series may be adaptively determined based on the component time series.

A combined total periodic characteristics of all of the respective at least one TSCI may be computed based on the total periodic characteristics of each of the respective at least one TSCI. The periodic characteristics of the respective motion may be monitored based on the combined total periodic characteristics. The combined total periodic characteristics may be a weighted sum of the total periodic characteristics of each of the respective at least one TSCI. In the combined total periodic characteristics, the weight for a total periodic characteristics of a TSCI may be adaptively determined based on the TSCI.

At least one local extremum may be computed. The at least one local extremum may be local extremum of at least one of: a component-wise periodic characteristics of one of the more than one component time series of a particular one of the respective at least one TSCI, a total periodic characteristics of the particular one of the respective at least one TSCI, and/or a combined total periodic characteristics of all of the respective at least one TSCI. The periodic characteristics of the respective motion may be monitored based on the at least one local extremum. The local extremum may be a local maximum. The local extremum may be a local minimum. The local extremum may be positive. A time (or time shift or time lag) associated with the local extremum may be positive. Noise and/or unwanted disturbance of the respective periodic characteristics may be suppressed and/or removed before computing the at least one extremum of the respective periodic characteristics.

A period of the periodic characteristics of the respective motion may be monitored based on a selected one of the at least one extremum. The selected extremum may be the first positive significant local maximum of the respective periodic characteristics, after noise and/or undesirable disturbance of the respective periodic characteristics may be suppressed and/or removed. The period of the periodic characteristics of the respective motion may be associated with a time lag associated with the selected extremum.

Each item of the component-feature time series may be at least one of: a corresponding item of the component time series, a magnitude of the corresponding item, a phase of the corresponding item, a first function of the corresponding item, the magnitude of the corresponding item, the phase of the corresponding item, and/or another item, a second function of the component time series and/or another function of the component time series, a first function of the second function of the component time series, a second function of the first function of the component time series, and/or a combination of the first function and the second function. There may be an injective (one-to-one) mapping from the component-feature time series (domain) to the component time series (range). Each item of the component-feature time series (domain) may be mapped to an item of the component time series (range). Each item of the component time series (range) may be mapped to at most one item of the component-feature time series (domain).

The mapping may be bijective (one-to-one and onto). Each item of the component-feature time series (domain) may be mapped to an item of the component time series (range). Each item of the component time series (range) may be mapped to exactly one item of the component-feature time series (domain). The mapping may not be onto. At least one item of the component time series (range) may not be mapped to any item of the component-feature time series (domain). The component-feature time series may be a subsampled version of the component time series with an associated subsampling factor. The associated subsampling factor may be an integer. The items of the component time series may be periodically sampled with a period associated with the integer subsampling factor. Each sampled item of the component time series (range) may be mapped to exactly one item of the component-feature time series (domain). Items of the component time series (range) not being sampled may not be mapped to any item of the component-feature time series (domain). The component-feature time series may correspond to a re-sampling of the component time series with an associated re-sampling factor. The re-sampling factor may be an integer or a non-integer. Each item of the component-feature time series (domain) may be mapped to at least one of: an item of the component time series (range), an interpolated item obtained by applying an interpolation filter to the component time series (range) and/or another item.

The re-sampling of the component time series may comprise at least one of: anti-aliasing filtering for re-sampling, subsampling, local subsampling, subsampling with adaptive subsampling factor, subsampling with time varying subsampling factor, up-sampling, local up-sampling, up-sampling with adaptive up-sampling factor, up-sampling with time varying up-sampling factor, adaptive re-sampling, adaptive subsampling, adaptive up-sampling, combined subsampling and up-sampling, alternating subsampling and up-sampling, and/or another sampling. Different re-sampling may be applied to different component time series. Each item of the component-feature time series (domain) may be mapped to at least one of: an item of the component time series (range), an interpolated item obtained by applying an interpolation filter to the component time series (range), and/or a feature item obtained by applying a feature filter to the component time series (range).

An item of a first component-feature time series may be mapped to a first feature item obtained by applying a first feature filter to the first component time series. A corresponding item of a second component-feature time series may mapped to a second feature item obtained by applying a second feature filter to the second component time series. The first feature filter and the second feature filter may be different. The first function may comprise at least one of: a composite function, a function of function, a complex function, a complex conjugate, a modulus, a phase, a continuous function, a non-continuous function, a real function, a linear function with an offset, a polynomial, a robust function, a robust function with outlier suppression, a robust function being a relatively fast increasing function for smaller magnitude argument and a relatively slow increasing function for larger magnitude argument, a nonlinear function, a logarithm, an exponential function, a hyperbolic function, a power function, a periodic function, a trigonometric function, sine, cosine, tangent, co-tangent, secant, cosecant, an inverse function, an inverse-trigonometric function, arc-sine, arc-cosine, arc-tangent, a special function, Bessel function, error function, complementary error function, Gamma function, Beta function, integral function, log integral function, exponential integral, trigonometric integral, Gaussian, Poisson, an absolute value, a sign function, a limiting function, a floor function, a rounding function, a quantization function, an indicator function, a step function, a sawtooth wave, an approximation function, a Taylor series, an asymptotic series, an analytic function, and/or another function.

Respective autocorrelation function (ACF) of a respective feature of the respective component time series may be computed. The respective ACF may be associated with respective component index. The respective motion may be monitored based on the respective ACF. Respective combined autocorrelation function (ACF) of each of the respective at least one TSCI may be computed. The respective combined ACF may be a linear combination of the ACF of the more than one component time series of the TSCI. The respective motion may be monitored based on the respective combined ACF. Each of the more than one component time series of the TSCI may be weighted in the linear combination by a weight that is function of the component time series. Respective at least one TSCI obtained from the series of probe signals by respective heterogeneous target wireless receiver may be preprocessed.

Another embodiment is a method/apparatus/device/system/software of a wireless monitoring system, comprising transmitting a series of first probe signals by an antenna of a first wireless device to at least one heterogeneous first target wireless receiver through a first wireless multipath channel in a venue. The transmitting may be using a processor, a memory and a set of instructions. The first wireless device may comprise a heterogeneous integrated circuits (IC). Each of the at least one heterogeneous first target wireless receiver may comprise a heterogeneous IC.

The method/apparatus/device/system/software of the wireless monitoring system further comprises obtaining asynchronously by each of the at least one heterogeneous first target wireless receiver at least one time series of first CI (first TSCI) from the series of first probe signals. The first CI (CI) is CI of the first wireless multipath channel between the heterogeneous first target wireless receiver and the first wireless device. The method/apparatus/device/system/software of the wireless monitoring system further comprises monitoring asynchronously a respective first motion associated with at least one of: the venue and a respective first object in the venue based on respective at least one first TSCI obtained by respective heterogeneous first target wireless receiver from the series of first probe signal. The method/apparatus/device/system/software of the wireless monitoring system further comprises obtaining asynchronously by each of the at least one heterogeneous second target wireless receiver at least one time series of second CI (second TSCI) from the series of second probe signals. The second CI (CI) is CI of the second wireless multipath channel between the heterogeneous second target wireless receiver and the second wireless device. The method/apparatus/device/system/software of the wireless monitoring system further comprises monitoring asynchronously a respective second motion associated with at least one of: the venue and a respective second object in the venue, based on respective at least one second TSCI obtained from the series of second probe signals by respective heterogeneous second target wireless receiver.

A particular first motion associated with a particular heterogeneous first target wireless receiver and a particular second motion associated with a particular heterogeneous second target wireless receiver may be a same motion. The same motion may be monitored jointly based on respective at least one first TSCI obtained from the series of first probe signals by the particular heterogeneous first target wireless receiver, and respective at least one second TSCI obtained from the series of second probe signals by the particular heterogeneous second target wireless receiver. A particular first object associated with a particular heterogeneous first target wireless receiver and a particular second object associated with a particular heterogeneous second target wireless receiver may be a same object.

A particular classifier may be configured to compute, for a first section of a first time duration of the current TSCI and for each of the at least one known event, pairwise mismatch between the first section of the first time duration of the current TSCI and a second section of a second time duration of a trained representative TSCI associated with the known event. The particular classifier may associate the first section of the first time duration of the current TSCI to a known event whose second section has smallest pairwise mismatch with the first section. The particular classifier may associate the first section of the first time duration of the current TSCI to a known event whose second section has smallest pairwise mismatch with the first section, if a previous first section of another first time duration of the current TSCI has smallest pairwise mismatch with another second section of another second time duration of the trained TSCI associated with the known event.

The trained representative TSCI associated with the known event may be obtained based on the at least one training TSCI associated with the known event. The trained representative TSCI associated with the known event may be one of the at least one training TSCI associated with the known event. The trained representative TSCI associated with the known event may be a particular one of the at least one training TSCI associated with the known event such that it has smallest aggregate mismatch compared with the at least one training TSCI.

The aggregate mismatch may be a function of at least one pairwise mismatch between the particular one and each of the rest of the at least one training TSCI. The function may comprise at least one of: average, weighed average, mean, trimmed mean, median, mode, arithmetic mean, geometric mean, harmonic mean, truncated mean, generalized mean, power mean, f-mean, interquartile mean, and/or another mean. A particular trained representative TSCI associated with a particular known event may have a particular time duration. The particular trained representative TSCI of the particular time duration may be aligned with each of the at least one training TSCI associated with the known event. Respective pairwise mismatch may be computed. The particular trained representative TSCI may minimize an aggregate mismatch compared with the at least one training TSCI. The aggregate mismatch may be a function of at least one pairwise mismatch between the particular one and each of the at least one training TSCI. The particular time duration may minimize the aggregate mismatch among more than one candidate time durations of the particular trained representative TSCI. The function may comprise at least one of: average, weighed average, mean, trimmed mean, median, mode, arithmetic mean, geometric mean, harmonic mean, truncated mean, generalized mean, power mean, f-mean, interquartile mean, and/or another mean.

At least one of: the aggregate mismatch and/or each pairwise mismatch, may be normalized, so that the aggregate mismatch of each of the at least one training TSCI can be compared in search of the one with minimum aggregate mismatch. The particular time duration may minimize the aggregate mismatch among more than one candidate time durations of the particular trained representative TSCI. For each of at least one candidate time duration, the optimal trained representative TSCI with the candidate time duration may be computed. The particular time duration may be chosen as the candidate time duration that gives minimal normalized aggregate mismatch. The normalized aggregated mismatch associated with a candidate time duration may be the respective aggregate mismatch normalized by the candidate time duration. The particular time duration may be obtained by performing a search among the at least one candidate time durations with the cost function being at least one of: normalized aggregate mismatch, hybrid normalized aggregate mismatch, combination normalized aggregate match, a simplified mismatch, and/or another cost function. The normalized aggregated mismatch associated with a candidate time duration may be the respective aggregate mismatch normalized by the candidate time duration. The search may comprise at least one of: brute force exhaustive search, gradient descent, steepest descent, stochastic search, genetic search, predictive search, local search, multi-resolution search, hierarchical search, constrained search, unconstrained search, fast search, simplified search, and/or another search.

The particular time duration and the particular trained representative TSCI with the particular time duration may be computed iteratively. A current time duration may be initialized as one of the candidate time durations. For the current time duration, a current optimal trained representative TSCI with the current time duration may be computed. For the current optimal trained representative TSCI with the current time duration, the current time duration may be changed to give smaller normalized aggregate mismatch. The current time duration may be initialized with a value based on durations associated with the at least one training TSCI associated with the known event. The current time duration may be initialized with a value based on durations associated with the at least one training TSCI associated with the known event. At least one of: the first wireless device, and/or the first target wireless receiver, may comprise at least two antennas. Each pair of first wireless device antenna and first target wireless receiver antenna may form a link between the first wireless device and the first target wireless receiver. At least two links may be formed between the first wireless device and the first target wireless receiver. Each link may correspond to a current TSCI. As an example, the tracking of the object may be achieved by obtaining an initial location of the object at time T, and then computing an updated location of the object at time T+ T based on the at least one TSCI.

The initial location of the object at time T may be obtained by installing some directional antennas at an entrance such that when the object moves under the directional antenna, a wireless signal may be received indicating that the object is at the location associated with the directional antenna. The updated location of the object may be computed by computing a distance travelled by the object in the incremental time T and combining the distance with an object motion direction (e.g. obtained from a gyroscope associated with the object) to obtain a displacement vector. The displacement vector may be added to the location at time T to obtain the updated location of the object at time T+ T.

The Type1 heterogeneous wireless device is a heterogeneous wireless device of a first type (e.g. a wireless transmitter that sends a first probing signal (such as an impulse), a wireless receiver that receives a second probing signal, a wireless device functioning as a Bot, or a wireless device functioning as an Origin) and the Type2 heterogeneous wireless device is a heterogeneous wireless device of a second type (e.g. a wireless receiver that receives the first probing signal, a wireless transmitter that sends the second probing signal, or a wireless transceiver, or a wireless device functioning as an Origin, or a wireless device functioning as a Bot). The Type1 device may work in collaboration with a Type2 device such that one sends a probing signal and the other receives it. A Type1 device and/or a Type2 device may be a smart speaker, a smart phone, an attachment to a phone, a portable dongle to plug into another device, a USB dongle, a computer, a WiFi router, an LTE base station, a WiFi repeater, an LTE repeater, a TV, a DVD player, a HiFi, an audio equipment, a lighting equipment, a clock, a lamp, a refrigerator, a microwave oven, an appliance, a utensil, a torch, a security camera, a dedicated security box, an ornament, etc. According to various embodiments of the present teaching, an Origin may be a transceiver and a Bot may be another transceiver in the system for object tracking and navigation. Each of the Origin and the Bot may be a Type1 or Type2 heterogeneous wireless device.

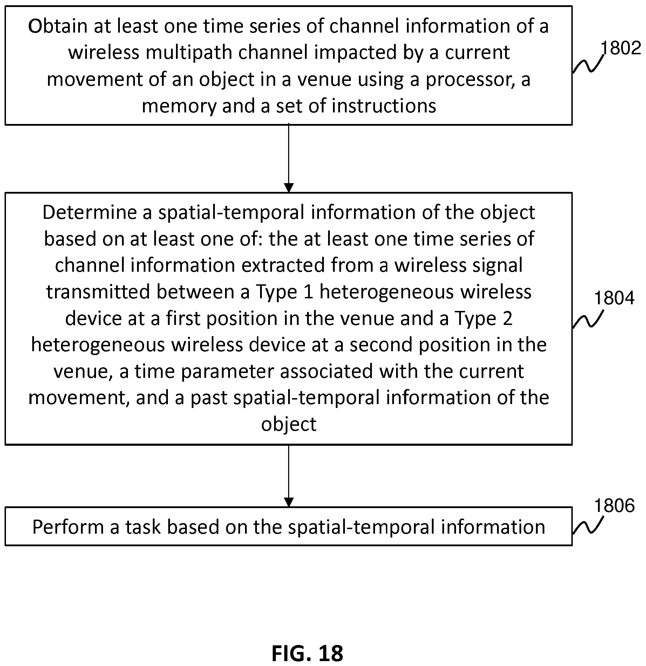

FIG. 18 illustrates an exemplary method for object tracking, according to one embodiment of the present teaching. At operation 1802, at least one TSCI f a wireless multipath channel is obtained, using a processor, a memory and a set of instructions stored in the memory. The at least one TSCI is extracted from a wireless signal transmitted between a Type1 heterogeneous wireless device at a first position in a venue and a Type2 heterogeneous wireless device at a second position in the venue through the wireless multipath channel. The wireless multipath channel is impacted by a current movement of an object in the venue. At operation 1804, a spatial-temporal information of the object is determined based on at least one of: the at least one TSCI, a time parameter associated with the current movement, and a past spatial-temporal information of the object. At operation 1906, a task is performed based on the spatial-temporal information.

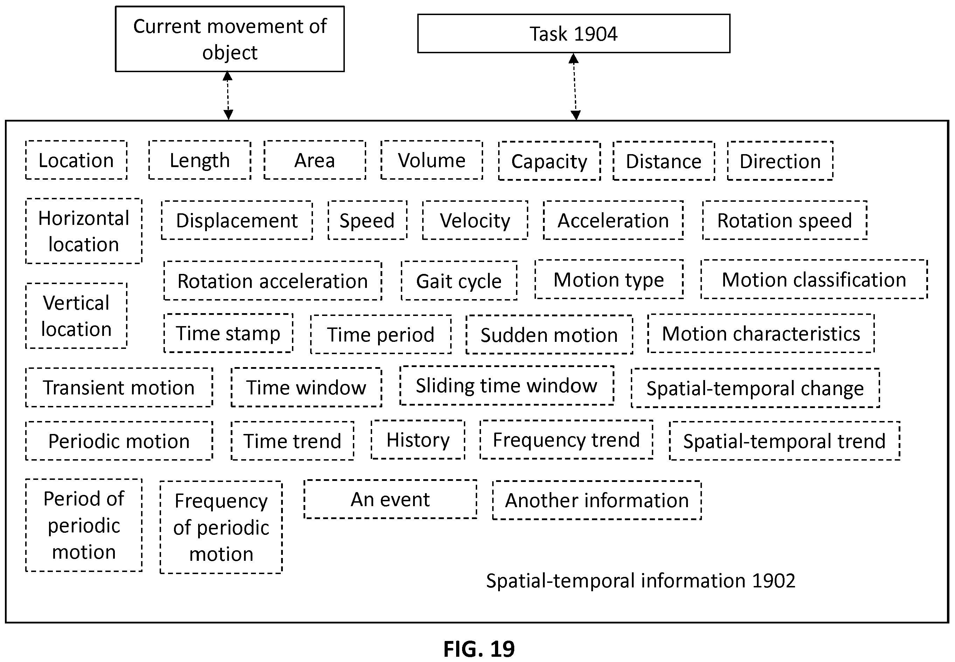

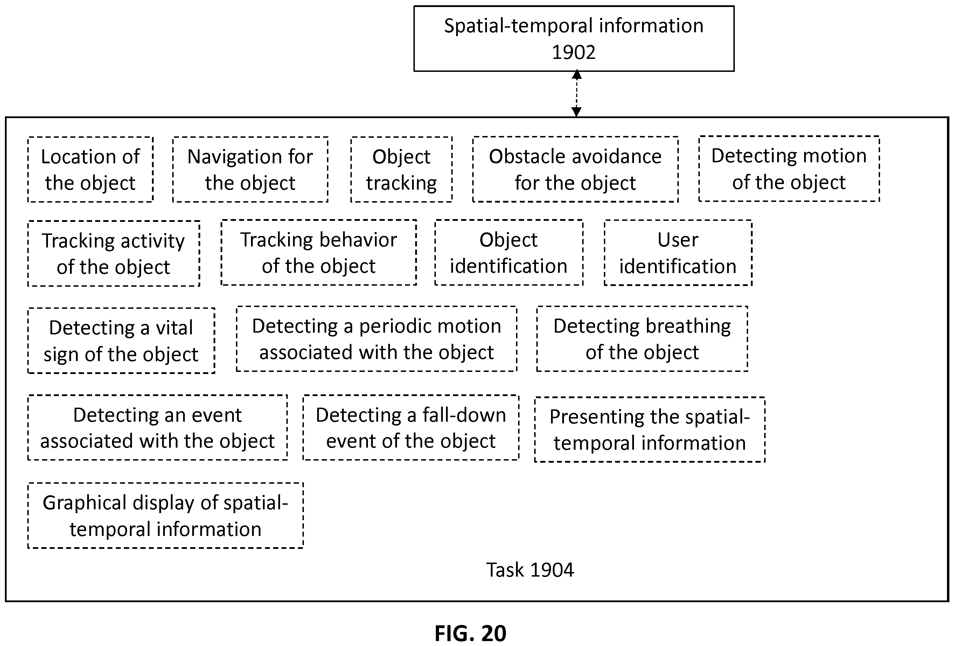

FIG. 19 illustrates examples of the spatial-temporal information 1902, according to one embodiment of the present teaching. Accordingly, FIG. 20 illustrates examples of task 1904 performed based on spatial-temporal information, according to one embodiment of the present teaching.

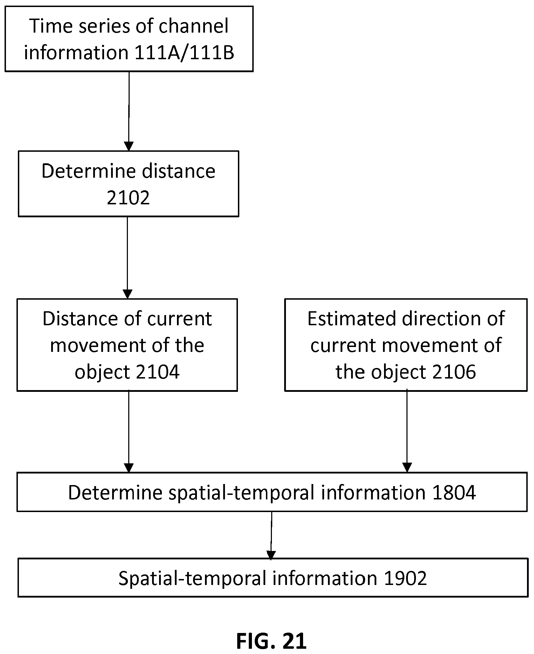

FIG. 21 illustrates another exemplary method for object tracking, according to one embodiment of the present teaching. As shown in FIG. 21, TSCI 111A/111B may be obtained to determined distance at operation 2102, such that distance information 2104 of the current movement of the object may be obtained. In parallel, some mechanism may be used to obtain an estimated direction 2106 of the current movement of the object. As such, the spatial-temporal information may be determined, similar to the operation 1804 as shown in FIG. 18, based on the distance information 2104 and the estimated direction 2106, to obtain the spatial-temporal information 1902 as illustrated in FIG. 19,

In general, a task may be performed based on the spatial-temporal information, as shown in operation 1806 of FIG. 18. In one embodiment, the task may comprise a presentation of the spatial-temporal information. It may be presented in an audio-visual way, a graphical way, a textual way, symbolic way or mechanical way. For example, the spatial-temporal information may be a detection of motion of the object in different rooms of a house. A graphical user interface (GUI) may be constructed to show that the where-about of the object in a house. For example, the object may be a person. The location or approximate location of the object may be shown/marked. And the GUI may partition a house into living-room area, family-room area, dining-room area, bedroom1-area, bedroom2-area, etc. Each area may be assigned a color and/or shaded with the color. Each area may be animated (e.g. size of the area, shape of the area, color of the area, intensity of the color of the area, text display, symbol display, etc.). Or, the GUI may have separate representation of each area with or without a map. The representation may be animated. The animation may be in real time, or at a later time. Predicted object (user) behavior/activity may be animated also. The presentation may also be in the form of vibration, mechanical feedback, physical feedback, haptic feedback, light, shade, shape, etc. to reflect the spatial-temporal information. The spatial-temporal information may include more than one analytics, e.g. number of people, existence of motion, motion intensity, motion duration, motion frequency, "abnormal" or "unexpected" motion, vital sign, alive/death, motionless, asleep, suspicious event, and/or fall-down, etc. For example, if motion is large, the color may be darker (more black/grey element) or more saturated or brighter. If motion is small, the color may be lighter or less saturated or dimmer. When the person enters a house, the GUI may show that he is at the front foyer area. living room, bedroom1, etc. The GUI may be a software for a computer/a tablet, an app on a smart phone (e.g. iPhone, Android phone, etc.), an app in a smart device (e.g. smart glass, smart watch, etc.).

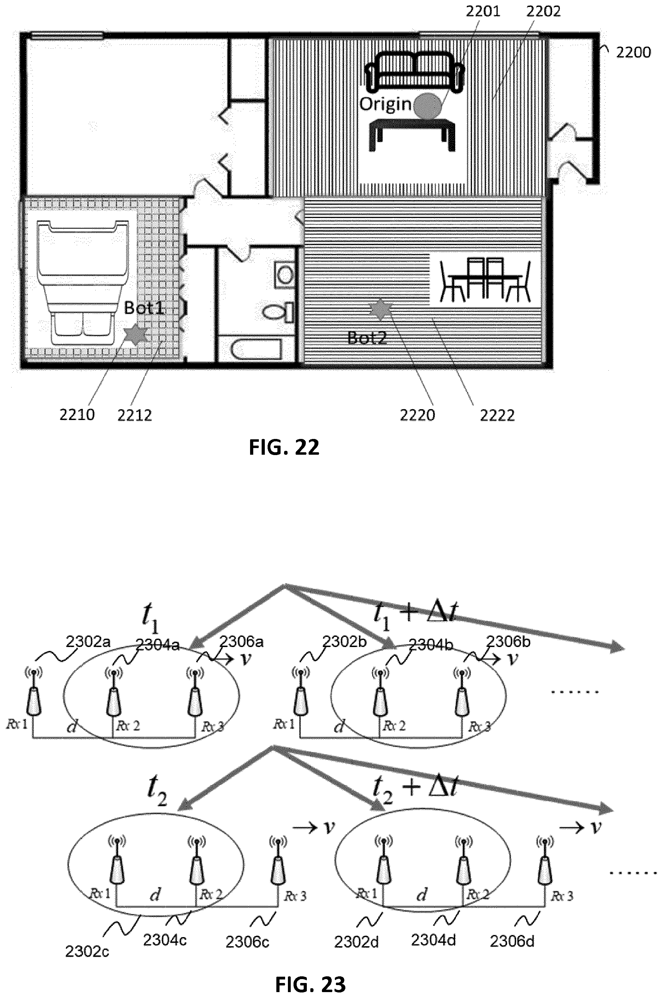

FIG. 22 illustrates an exemplary presentation of the spatial-temporal information in a venue, according to one embodiment of the present teaching. For example, as shown in FIG. 22, in a 2-bedroom apartment 2200 (or 900), Origin 2201 (or 901) may be placed in the living-room area 2202 (or 902), Bot 1 2210 (or 910) may be placed in a bedroom1-area 2212 (or 912), and Bot 2 2220 (or 920) may be placed in the dining-room area 2222 (or 922). If both Bot 1 2210 (or 910) and Bot 2 2220 (or 920) detect motion/activity, the activity/motion/person/user may be in the living-room area 2202 (or 902). If only Bot 1 2210 (or 910) detects motion/activity, the activity/motion/person/user may be in the bedroom-1 area 2212 (or 912). If only Bot 2 2220 (or 920) detects motion/activity, the activity/motion/person/user may be in the dining-room area 2222 (or 922). If neither of the two Bots detects motion/activity, then nobody may be in the apartment 2200 (or 900). The corresponding area where the activity/motion/person/user is detected may be marked with a predetermined pattern or color. A number of implementations of the disclosure have been described. Nevertheless, it will be understood that various modifications may be made without departing from the spirit and scope of the disclosure. For example, the wireless transmitter, the wireless receiver, the antenna, the directional antenna, the wireless transmission, the multipath channel, the wireless signal, the object, the object, the processor, the memory, the set of instructions, the CI (CI), the channel state information (CSI), the power information (PI), the format/features/components/contexts of CI/CSI/PI, the compression/-representation/storage/transmission/encryption/processing/preprocessing /post-processing of CI/CSI/PI, the time series format/order/sequencing, the extraction of CI from the wireless signal, the spatial-temporal information, the past information, the initial information, the time quantities, the spatial quantities, the spatial-temporal quantities, the estimated direction, the similarity score, the object, the distance, the movement of the object, the characteristics points, the local device, and/or the computational workload sharing may be different from those described above. The spatial-temporal information may be speed. An exemplary explanation of a speed estimation method is as below. The three receive antennas may be aligned in a line parallel to the moving direction as shown in FIG. 23. FIG. 23 is an exemplary illustration of antenna matching based speed estimation, according to one embodiment of the present teaching. Furthermore, the speed of the receive antennas may change slowly in time, i.e., within a short period, the speed may be approximated to be a constant value v. One may group the receive antennas Rx2 and Rx3 together and call them the leading antennas, and group the receive antennas Rx1 and Rx2 together and call them the following antennas or trailing antennas. This is because the following antennas (or trailing antennas) may go through the same trajectory as the leading antennas, but with an unknown delay $\tau$. The speed may be estimated by the relation: $\hat{v} = \frac{d}{\tau}$, where d is the separation between adjacent antennas (equal separation). Therefore, the speed estimation problem may be transformed to the estimation of $\tau$. In the following, the TRRS may be used to estimate $\tau$. Let $H_i(t)$ denote the CSI (or CI CI) measured at the i-th receive antenna at time t and let $\Delta t$ stand for the duration between two adjacent CSI measurement. For each time $t_1$, one may form the reference CSI, which is measured by the leading antennas, as $\boldsymbol{H}_{lead}(t_1) = [H_2(t_1), H_3(t_1), H_2(t_1 + \Delta t), H_3(t_1 + \Delta t), ... , H_2(t_1 + (N - 1)\Delta t), H_3(t_1 + (N - 1)\Delta t)]$, where N may be 1, 2, 3..., and form the CSI for comparison, which is measured by the following antennas (or trailing antennas), as $\boldsymbol{H}_{follow}(t) = [H_1(t), H_2(t), H_1(t + \Delta t), H_2(t + \Delta t), ... , H_1(t + (N - 1)\Delta t), H_2(t + (N - 1)\Delta t)]$. The TRRS $\gamma(t_1, t)$ value and/or some similarity measure and/or some distance measure between $\boldsymbol{H}_{lead}(t_1)$ and $\boldsymbol{H}_{follow}(t)$ may be computed. When the following antennas reach the location of the leading antennas corresponding to time $t_1$, $\gamma(t_1, t)$ may achieve its maximum over the variable t. In the example, one may have $t = t_2$ and thus one may have the estimation $\hat{\tau} = t_2 - t_1$.

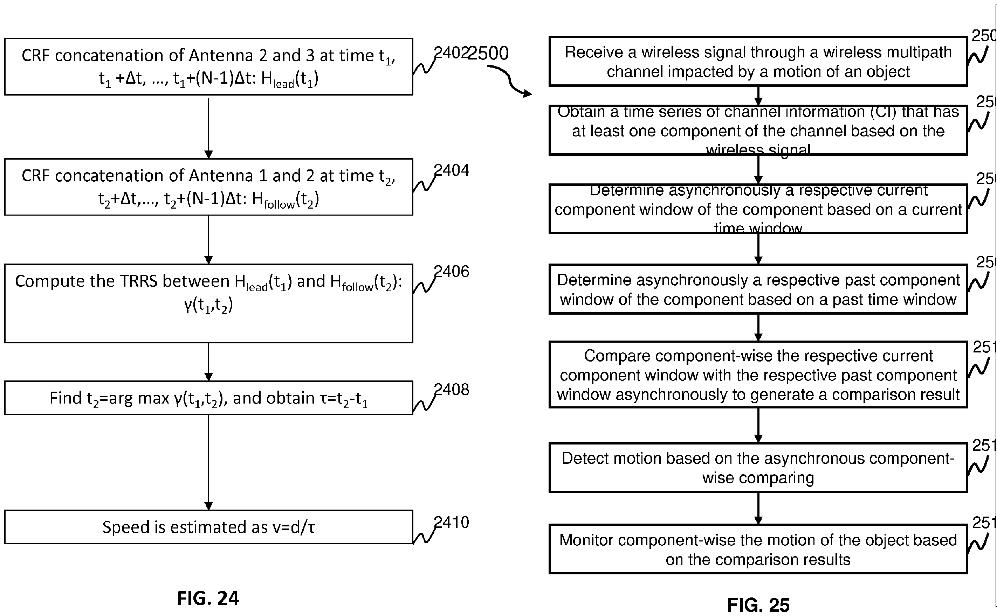

An exemplary flow chart of antenna matching based speed estimation is shown in FIG. 24. In operation 2402, the detailed formulation of the CFR concatenation for the leading antennas is $\boldsymbol{H}_{lead}(t_1) = [H_2(t_1), H_3(t_1), H_2(t_1 + \Delta t), H_3(t_1 + \Delta t), ..., H_2(t_1 + (N - 1)\Delta t), H_3(t_1 + (N - 1)\Delta t)]$. In operation 2404, the detailed formulation of the CFR concatenation for the following antennas is $\boldsymbol{H}_{follow}(t_2) = [H_1(t_2), H_2(t_2), H_1(t_2 + \Delta t), H_2(t_2 + \Delta t), ... , H_1(t_2 + (N - 1)\Delta t), H_2(t_2 + (N - 1)\Delta t)]$. In operation 2406, for each time slot $t_1$, the TRRS $\gamma(t_1, t_2)$ between the two CFR fingerprint $\boldsymbol{H}_{lead}(t_1)$ and $\boldsymbol{H}_{follow}(t_2)$ is computed for different $t_2$'s nearby $t_1$. In operation 2408, it may determine the $t_2$ that gives the maximum value of $\gamma(t_1, t_2)$ and then the delay $\tau$ between the group of leading antennas and the group of following antennas is thus $\tau = t_2 - t_1$. In operation 2410, the speed estimation is thus v=d/$\tau$, where d the distance between any two adjacent uniformly separated antennas. Accordingly, other embodiments are within the scope of the following claims.

*Motion detection*

In the following detailed description, numerous specific details are set forth by way of examples in order to provide a thorough understanding of the relevant teachings. However, it should be apparent to those skilled in the art that the present teachings may be practiced without such details. In other instances, well known methods, procedures, components, and/or circuitry have been described at a relatively high-level, without detail, in order to avoid unnecessarily obscuring aspects of the present teachings.

The present teaching discloses systems, devices, and methods for detecting object motion in a venue based on CI of a wireless multipath channel that is impacted by the object motion. In one embodiment, the motion of the object is detected based on motion information computed based on a TSCI of the wireless multipath channel. The motion information may include a heterogeneous similarity score between a current time window and a past time window of the TSCI.

In one embodiment, the disclosed system comprises a first wireless device configured for transmitting a wireless signal through a wireless multipath channel impacted by a motion of an object in the venue; and a second wireless device that has a different type from that of the first wireless device. The second wireless device is configured for: receiving the wireless signal through the wireless multipath channel impacted by the motion of the object in the venue, and obtaining a TSCI of the wireless multipath channel based on the wireless signal. The disclosed system further comprises a motion detector configured for detecting the motion of the object in the venue based on motion information related to the motion of the object. The motion information associated with the first and second wireless devices is computed based on the TSCI by at least one of: the motion detector and the second wireless device.

The motion detector may be coupled to at least one of: the first wireless device; the second wireless device; a third wireless device having a same type as that of the first wireless device; a fourth wireless device having a same type as that of the second wireless device; a cloud server; a fog server; a local server; and an edge server. In one embodiment, the first wireless device is a Type1 device having a Type1; the second wireless device is a Type2 device having a Type2; the system further comprises at least one of: at least one additional Type1 device, and at least one additional Type2 device; the Type1 devices and the Type2 devices form a plurality of pairs of Type1 and Type2 devices. For each pair of Type1 and Type2 devices comprising a Type1 device and a Type2 device: the respective Type1 device may be configured for transmitting a respective wireless signal through the wireless multipath channel impacted by the motion of the object in the venue, the respective Type2 device may be configured for receiving the respective wireless signal through the wireless multipath channel and obtaining a respective TSCI of the wireless multipath channel based on the respective wireless signal, and at least one of the motion detector and the respective Type2 device may be configured for asynchronously computing respective heterogeneous motion information related to the motion of the object based on the respective TSCI; and at least one of the motion detector and the Type2 devices is configured for performing at least one of: monitoring the motion of the object in the venue individually and asynchronously based on the asynchronously computed heterogeneous motion information associated with a pair of Type1 and Type2 devices, monitoring the motion of the object in the venue jointly and asynchronously based on the asynchronously computed heterogeneous motion information associated with any pair of Type1 and Type2 devices comprising a particular Type2 device, monitoring the motion of the object in the venue jointly and asynchronously based on the asynchronously computed heterogeneous motion information associated with any pair of Type1 and Type2 devices comprising a particular Type1 device, and monitoring the motion of the object in the venue globally and asynchronously based on the asynchronously computed heterogeneous motion information associated with any pair of Type1 and Type2 devices.

According to various embodiments of the present teaching, an Origin may be a transceiver and a Bot may be another transceiver in the disclosed system for object motion detection and monitoring. Each of the Bot and the Origin may be a Type1 or Type2 heterogeneous wireless device.

In one embodiment, each CI of the respective TSCI may have at least one respective component. For each Type2 device, and for each of the at least one respective particular pair of Type1 and Type2 devices comprising the Type2 device, and for each of the at least one respective component, the following may be done: (a) a respective current component window of the component of the respective TSCI may be determined asynchronously based on the respective current window; (b) a respective past component window of the component of the respective TSCI may be determined asynchronously based on the respective past window; (c) the respective current component window may be compared component-wise with the respective past component window asynchronously; and (d) the motion of the object may be monitored component-wise based on the component-wise comparing of the respective current component window and the respective past component window asynchronously. The motion of the object may be detected based on the asynchronous component-wise comparing.

In one embodiment, the disclosed system has hardware components (e.g. wireless transmitter/receiver with antenna, analog circuitry, RF/IF/baseband elements, low-noise amplifier, power supply, processor, memory, etc.) working in pairs (a Type1 device and a Type2 device) and corresponding software components. The disclosed system is capable of detecting object motions in a venue, with a very high detection rate and a very low false alarm rate. In particular, the disclosed system is much better than an existing passive infra-red (PIR) sensor which is a widely used motion sensor in security systems.

First, the disclosed system has a much larger motion detection range. In one embodiment, the disclosed wireless motion detection system uses wireless signals (such as Wi-Fi or LTE) to probe the environment (venue) and have a large inherent range of about 10,000 square feet due to Wi-Fi. PIR has a much smaller range of about 100-200 square feet by using an infra-red signal.

Second, the disclosed system can detect motion around corners. While a PIR sensor relies on line-of-sight (LOS) operation, it cannot detect motion behind a wall or around a corner or behind an obstacle (e.g. TV, sofa, window, partitions, etc.). In contrast, the disclosed system works in both line-of-sight (LOS) and non-line-of-sight (NLOS) conditions. The disclosed system is fully capable of detecting motion behind multiple walls, around multiple corners and behind obstacles. In security applications, being able to detect motion around corners and behind walls is extremely important because intruders may exactly be hiding there in the dark waiting to do bad things.

Third, the disclosed system has a much better false alarm rate. A false alarm means that a system detects a motion when there is actually no motion. Such false alarms are very annoying to the operators and police. The disclosed system has a very low false alarm rate of even less than 0.1% while the false alarm rate of a PIR based detector is in the range of 10% or more. While a PIR based detector is sensitive to temperature change due to e.g. a strong sunshine, the disclosed system is not sensitive to sunshine or temperature change.

Fourth, the disclosed system does not need special installation like a PIR based detector. PIR based detectors need special installation. Once installed, they cannot be moved around. As the PIR based detector has very limited range, many PIR devices are labor intensive, time consuming and expensive. In addition, replacement of faulty PIR devices requires additional effort. The disclosed system can use existing Wi-Fi or LTE to perform motion detection. In other words, no special installation is needed. The disclosed system may just use some existing Wi-Fi-compliant device such as Wi-Fi router, smart phone, smart speaker (e.g. Amazon Echo, Google Home, etc.), smart devices (e.g. smart thermometer, smart camera, smart TV, smart set-top box, smart refrigerator, etc.). Type1 devices and Type2 devices may be plug-and-play devices without a requirement of special installation. Due to the plug-and-play nature, the disclosed wireless motion detection system still works when the devices are moved around by consumers (e.g. from living room to family room, or from one plug to another plug).

Moreover, the disclosed system can exploit existing standard-compliant mass-market infrastructure such as Wi-Fi and LTE. Thus many components of the disclosed system are typically mass-produced and thus have a low cost, suitable for the consumer market as well as niche markets.

In one embodiment, a task related to object motion may be performed based on some spatial-temporal information (e.g. location, speed, velocity, acceleration, a periodic motion, a time trend, a transient motion, a period, a characteristic, etc.) of an object based on at least one TSCI, a time parameter associated with a current movement, and/or a past spatial-temporal information of the object. The one or more TSCI may be preprocessed. In one embodiment, the task may comprise a presentation of the spatial-temporal information. It may be presented in an audio-visual way, a graphical way, a textual way, symbolic way or mechanical way. For example, the spatial-temporal information may indicate a detection of motion of an object in different rooms of a house. A graphical user interface (GUI) may be constructed to show that the where-about of the object in a house. For example, the object may be a person. The location or approximate location of the object may be shown or marked.

The GUI may partition a house into living-room area, family-room area, dining-room area, bedroom1-area, bedroom2-area, etc. Each area may be assigned a color and/or shaded with the color. Each area may be animated (e.g. size of the area, shape of the area, color of the area, intensity of the color of the area, text display, symbol display, etc.). Or, the GUI may have separate representation of each area with or without a map. The representation may be animated. The animation may be in real time, or at a later time. Predicted object (user) behavior/activity may be animated as well. The presentation may also be in the form of vibration, mechanical feedback, physical feedback, haptic feedback, light, shade, shape, etc. to reflect the spatial-temporal information. The spatial-temporal information may include more than one analytics, e.g. number of people, existence of motion, motion intensity, motion duration, motion frequency, "abnormal" or "unexpected" motion, vital sign, alive/death, motionless, asleep, suspicious event, and/or fall-down, etc. For example, if motion is large, the color may be darker (more black/grey element) or more saturated or brighter. If motion is small, the color may be lighter or less saturated or dimmer. When the person enters a house, the GUI may show that he is at the front foyer area, living room, bedroom1, etc. The GUI may be implemented by software for a computer/a tablet, an app on a smart phone (e.g. iPhone, Android phone, etc.), an app in a smart device (e.g. smart glass, smart watch, etc.).

FIG. 22 illustrates an exemplary scenario where object motion is detected based on spatial-temporal information in a venue, according to one embodiment of the present teaching. FIG. 22 also illustrates an exemplary presentation of the spatial-temporal information in the venue. For example, as shown in FIG. 22, in a 2-bedroom apartment 2200, Origin 2201 (or 901) may be placed in the living-room area 2202 (or 902), Bot 1 2210 (or 910) may be placed in a bedroom1-area 2212 (or 912), and Bot 2 2220 (or 920) may be placed in the dining-room area 2222 (or 922). Each of Bot 1 2210 (or 910) and Bot 2 2220 (or 920) can transmit a wireless signal to the Origin 2201 (or 901), which can obtain CI of a wireless multipath channel based on the wireless signal. The Origin 2201 (or 901), by itself or through a third device like a motion detector, can compute motion information based on the CI and detect object motion/activity based on the motion information. That is, the Origin 2201 (or 901), by itself or through a third device like a motion detector, can detect object motion/activity based on wireless signals transmitted by Bot 1 2210 (or 910) and/or Bot 2 2220 (or 920).

If object motion/activity is detected based on wireless signals transmitted by both Bot 1 2210 (or 910) and Bot 2 2220 (or 920), the activity/motion or the object (e.g. person/user) may be in the living-room area 2202 (or 902). If object motion/activity is detected based only on wireless signals transmitted by Bot 1 2210 (or 910), the activity/motion or the object (e.g. person/user) may be in the bedroom-1 area 2212 (or 912). If object motion/activity is detected based only on wireless signals transmitted by Bot 2 2220 (or 920), the activity/motion or the object (e.g. person/user) may be in the dining-room area 122. If object motion/activity cannot be detected based on wireless signals transmitted by either Bot 1 2210 (or 910) or Bot 2 2220 (or 920), then it may be determined that nobody and no object is in the apartment 2200 (or 900). The corresponding area where the activity/motion/person/user is detected may be marked with a predetermined pattern or color.

FIG. 25 illustrates a flowchart of an exemplary method for motion detection performed by a motion detector based on wireless CI, according to one embodiment of the present teaching. At operation 2502, a wireless signal is received through a wireless multipath channel impacted by a motion of an object. At operation 2504, a TSCI that has at least one component of the channel is obtained based on the wireless signal. A respective current component window of the component is determined asynchronously, at operation 2506, based on a current time window. A respective past component window of the component is determined asynchronously, at operation 2508, based on a past time window. At operation 2510, the respective current component window is compared component-wise with the respective past component window asynchronously to generate a comparison result. At operation 2512, object motion is detected based on the asynchronous component-wise comparison result. Optionally at operation 2514, the motion of the object is monitored based on the continuously generated comparison results.

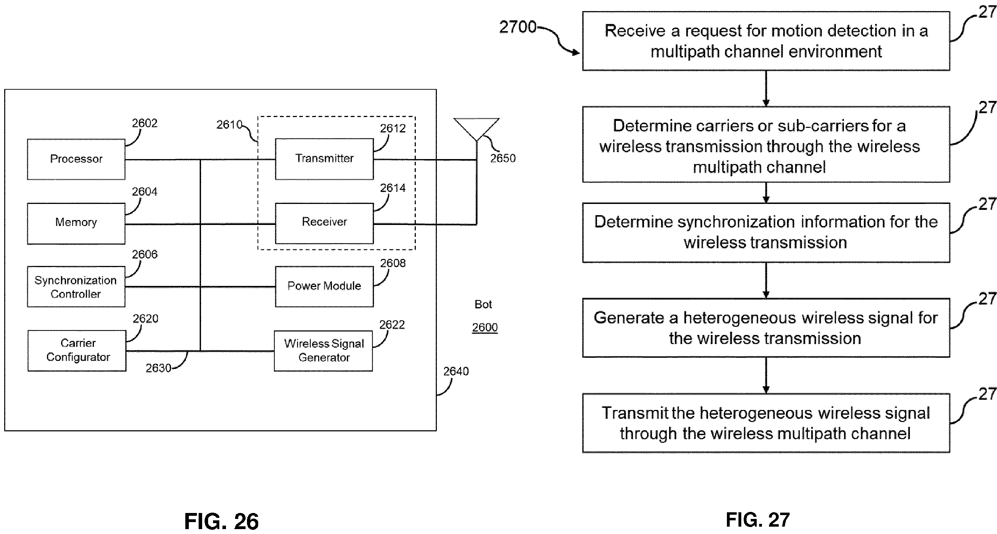

FIG. 26 illustrates an exemplary block diagram of a first wireless device, e.g. a Bot 2600, according to one embodiment of the present teaching. The Bot 2600 is an example of a device that can be configured to implement the various methods described herein. As shown in FIG. 26, the Bot 2600 includes a housing 2640 containing a processor 2602, a memory 2604, a transceiver 2610 comprising a transmitter 2612 and receiver 2614, a synchronization controller 2606, a power module 2608, a carrier configurator 2620 and a wireless signal generator 2622.

In this embodiment, the processor 2602 controls the general operation of the Bot 2600 and can include one or more processing circuits or modules such as a central processing unit (CPU) and/or any combination of general-purpose microprocessors, microcontrollers, digital signal processors (DSPs), field programmable gate array (FPGAs), programmable logic devices (PLDs), controllers, state machines, gated logic, discrete hardware components, dedicated hardware finite state machines, or any other suitable circuits, devices and/or structures that can perform calculations or other manipulations of data.

The memory 2604, which can include both read-only memory (ROM) and random access memory (RAM), can provide instructions and data to the processor 2602. A portion of the memory 2604 can also include non-volatile random access memory (NVRAM). The processor 2602 typically performs logical and arithmetic operations based on program instructions stored within the memory 2604. The instructions (a.k.a., software) stored in the memory 2604 can be executed by the processor 2602 to perform the methods described herein. The processor 2602 and the memory 2604 together form a processing system that stores and executes software. As used herein, "software" means any type of instructions, whether referred to as software, firmware, middleware, microcode, etc. which can configure a machine or device to perform one or more desired functions or processes. Instructions can include code (e.g., in source code format, binary code format, executable code format, or any other suitable format of code). The instructions, when executed by the one or more processors, cause the processing system to perform the various functions described herein.

The transceiver 2610, which includes the transmitter 2612 and receiver 2614, allows the Bot 2600 to transmit and receive data to and from a remote device (e.g., an Origin or another Bot). An antenna 2650 is typically attached to the housing 2640 and electrically coupled to the transceiver 2610. In various embodiments, the Bot 2600 includes (not shown) multiple transmitters, multiple receivers, and multiple transceivers. In one embodiment, the antenna 2650 is replaced with a multi-antenna array 2650 that can form a plurality of beams each of which points in a distinct direction. The transmitter 2612 can be configured to wirelessly transmit signals having different types or functions, such signals being generated by the processor 2602. Similarly, the receiver 2614 is configured to receive wireless signals having different types or functions, and the processor 2602 is configured to process signals of a plurality of different types.

The Bot 2600 in this example may serve as Bot 1 2210 (or 910) or Bot 2 2220 (or 920) in FIG. 22 for detecting object motion in a venue. For example, the wireless signal generator 2622 may generate and transmit, via the transmitter 2612, a wireless signal through a wireless multipath channel impacted by a motion of an object in the venue. The wireless signal carries information of the channel. Because the channel was impacted by the motion, the CI includes motion information that can represent the motion of the object. As such, the motion can be indicated and detected based on the wireless signal. The generation of the wireless signal at the wireless signal generator 2622 may be based on a request for motion detection from another device, e.g. an Origin, or based on a system pre-configuration. That is, the Bot 2600 may or may not know that the wireless signal transmitted will be used to detect motion.

In one embodiment, the Bot 2600 may be a particular asynchronous heterogeneous Type1 device of a radio monitoring system. The radio monitoring system may comprise at least one asynchronous heterogeneous Type1 device and at least one asynchronous heterogeneous Type2 device. The radio monitoring system may comprise at least one combination of Type1 and Type2 devices, each combination comprising one of the at least one Type1 device, and one of the at least one Type2 device. The particular asynchronous heterogeneous Type1 device is in at least one particular combination of Type1 and Type2 devices of the radio monitoring system. The particular Type1 device is paired with at least one particular Type2 device through the at least one particular combination of Type1 and Type2 devices. The particular asynchronous heterogeneous Type1 device comprises a wireless circuitry; a first processor communicatively coupled with the wireless circuitry; a first memory communicative coupled with the first processor; and a first set of instructions stored in the first memory which, when executed, cause the first processor to transmit asynchronously using the wireless circuitry an asynchronous heterogeneous wireless signal from the particular Type1 device to the at least one particular Type2 device through a wireless multipath channel impacted by a dynamics of a substance in a site. For each of the at least one particular Type2 device: at least one series of CI (TSCI) of the wireless multipath channel gleaned from the asynchronous heterogeneous wireless signal transmitted by the wireless circuitry is fetched by the Type2 device using a second processor, a second memory and a second set of instructions of the Type2 device. In one example, the dynamics of the substance in the site is tracked individually and asynchronously, using a third processor, a third memory and a third set of instructions of a Type 3 device, based on TSCI associated with a combination of Type1 and Type2 devices comprising the particular Type1 device. In another example, the dynamics of the substance is tracked jointly and asynchronously based on TSCI associated with any combinations of Type1 and Type2 devices comprising the particular Type1 device. In another example, the dynamics of the substance is tracked jointly and asynchronously based on TSCI associated with any combinations of Type1 and Type2 devices comprising one of the at least one particular Type2 device. In another example, the dynamics of the substance is tracked globally and asynchronously based on TSCI associated with any combinations of Type1 and Type2 devices.

In another embodiment, for each Type1 device, and for each of the at least one combination of Type1 and Type2 devices comprising the Type1 device, a respective heterogeneous similarity score associated with the respective combination of Type1 and Type2 devices is computed asynchronously based on comparing a respective current window and a respective past window of at least one respective TSCI associated with the respective combination of Type1 and Type2 devices. In one example, the dynamics of the substance in the site is tracked individually and asynchronously based on asynchronously computed heterogeneous similarity score associated with the combination of Type1 and Type2 devices comprising the particular Type1 device. In another example, the dynamics of the substance in the site is detected individually and asynchronously when a heterogeneous similarity score associated with the combination of Type1 and Type2 devices comprising the particular Type1 device is greater than and/or equal to an individual threshold. In another example, the dynamics of the substance in the site is tracked jointly and asynchronously based on asynchronously computed heterogeneous similarity score associated with any combination of Type1 and Type2 devices comprising the particular Type1 device. In another example, the dynamics of the substance in the site is detected jointly and asynchronously when a first joint score computed based on heterogeneous similarity score associated with any combination of Type1 and Type2 devices comprising the particular Type1 device is at least one of: greater than, and equal to, a first joint threshold. In another example, the dynamics of the substance in the site is tracked jointly and asynchronously based on asynchronously computed heterogeneous similarity score associated with any combination of Type1 and Type2 devices associated with one of the at least one particular Type2 device. In another example, the dynamics of the substance in the site is detected jointly and asynchronously when a second joint score computed based on heterogeneous similarity score associated with any combination of Type1 and Type2 devices associated with one of the at least one particular Type2 device is at least one of: greater than, and equal to, a second joint threshold. In another example, the dynamics of the substance in the site is tracked globally and asynchronously based on asynchronously computed heterogeneous similarity score associated with any combination of Type1 and Type2 devices. In another example, the dynamics of the substance in the site is detected globally and asynchronously when a third joint score computed based on heterogeneous similarity score associated with any combination of Type1 and Type2 devices is greater than and/or equal to a third joint threshold.

The synchronization controller 2606 in this example may be configured to control the operations of the Bot 2600 to be synchronized or un-synchronized with another device, e.g. an Origin or another Bot. In one embodiment, the synchronization controller 2606 may control the Bot 2600 to be synchronized with an Origin that receives the wireless signal transmitted by the Bot 2600. In another embodiment, the synchronization controller 2606 may control the Bot 2600 to transmit the wireless signal asynchronously with other Bots. In another embodiment, each of the Bot 2600 and other Bots may transmit the wireless signals individually and asynchronously. The carrier configurator 2620 in this example may configure transmission resources, e.g. time and carrier, for transmitting the wireless signal generated by the wireless signal generator 2622. In one embodiment, each CI of the TSCI has one or more components each corresponding to a carrier or sub-carrier of the transmission of the wireless signal. The detection of the motion may be based on motion detections on any one or any combination of the components. The power module 2608 can include a power source such as one or more batteries, and a power regulator, to provide regulated power to each of the above-described modules in FIG. 26. In some embodiments, if the Bot 2600 is coupled to a dedicated external power source (e.g., a wall electrical outlet), the power module 2608 can include a transformer and a power regulator. The various modules discussed above are coupled together by a bus system 2630. The bus system 2630 can include a data bus and, for example, a power bus, a control signal bus, and/or a status signal bus in addition to the data bus. It is understood that the modules of the Bot 2600 can be operatively coupled to one another using any suitable techniques and mediums.

Although a number of separate modules or components are illustrated in FIG. 26, persons of ordinary skill in the art will understand that one or more of the modules can be combined or commonly implemented. For example, the processor 2602 can implement not only the functionality described above with respect to the processor 2602, but also implement the functionality described above with respect to the wireless signal generator 2622. Conversely, each of the modules illustrated in FIG. 26 can be implemented using a plurality of separate components or elements.

FIG. 27 illustrates a flowchart of an exemplary method 2700 performed by a first wireless device, e.g. the Bot 2600 in FIG. 26, in accordance with some embodiments of the present disclosure. Optionally at operation 2702, the Bot receives a request for motion detection in a multipath channel environment. At operation 2704, the Bot determines carriers or sub-carriers for a wireless transmission through the wireless multipath channel. At operation 2706, the Bot may determine synchronization information for the wireless transmission. At operation 2708, the Bot may generate a heterogeneous wireless signal for the wireless transmission. At operation 2710, the Bot transmits the heterogeneous wireless signal through the wireless multipath channel.

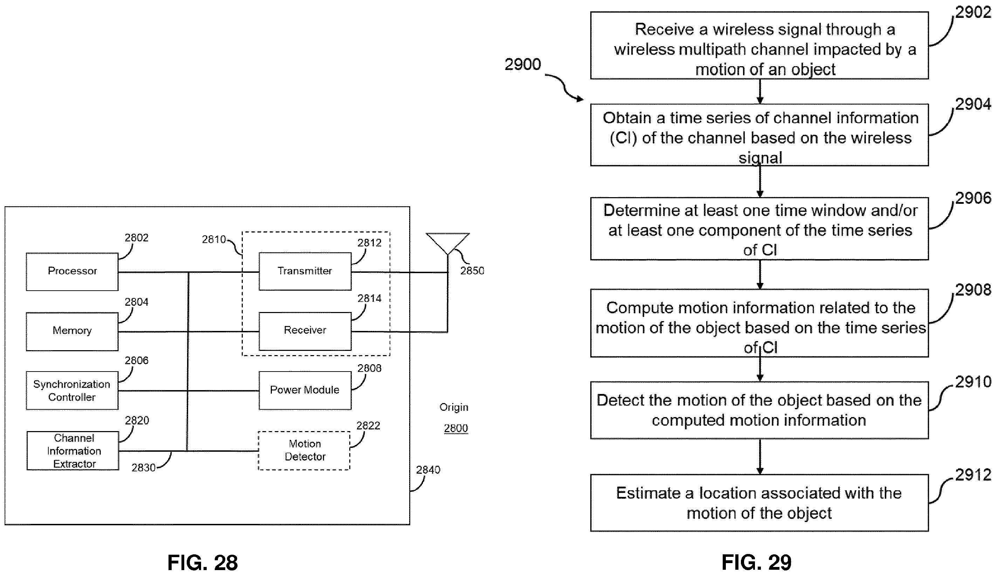

FIG. 28 illustrates an exemplary block diagram of a second wireless device, e.g. an Origin 2800, according to one embodiment of the present teaching. The Origin 2800 is an example of a device that can be configured to implement the various methods described herein. As shown in FIG. 28, the Origin 2800 includes a housing 2840 containing a processor 2802, a memory 2804, a transceiver 2810 comprising a transmitter 2812 and a receiver 2814, a power module 2808, a synchronization controller 2806, a CI extractor 2820, and an optional motion detector 2822.

In this embodiment, the processor 2802, the memory 2804, the transceiver 2810 and the power module 2808 work similarly to the processor 2602, the memory 2604, the transceiver 2610 and the power module 2608 in the Bot 2600. An antenna 2850 or a multi-antenna array 2850 is typically attached to the housing 2840 and electrically coupled to the transceiver 2810.

The Origin 2800 may be a second wireless device that has a different type from that of the first wireless device (the Bot 2600). In particular, the CI extractor 2820 in the Origin 2800 is configured for receiving the wireless signal through the wireless multipath channel impacted by the motion of the object in the venue, and obtaining a TSCI of the wireless multipath channel based on the wireless signal. The CI extractor 2820 may send the extracted CI to the optional motion detector 2822 or to a motion detector outside the Origin 2800 for detecting object motion in the venue.

The motion detector 2822 is an optional component in the Origin 2800. In one embodiment, it is within the Origin 2800 as shown in FIG. 28. In another embodiment, it is outside the Origin 2800 and in another device, which may be a Bot, another Origin, a cloud server, a fog server, a local server, and an edge server. The optional motion detector 2822 may be configured for detecting the motion of the object in the venue based on motion information related to the motion of the object. The motion information associated with the first and second wireless devices is computed based on the TSCI by the motion detector 2822 or another motion detector outside the Origin 2800.

In one embodiment, the Origin 2800 is a particular asynchronous heterogeneous Type2 device of a radio monitoring system. The radio monitoring system may comprise at least one asynchronous heterogeneous Type1 device and at least one asynchronous heterogeneous Type2 device. The radio monitoring system may comprise at least one pairing of Type1 and Type2 devices, each pairing comprising one of the at least one Type1 device, and one of the at least one Type2 device. The particular asynchronous heterogeneous Type2 device is in at least one particular pairing of Type1 and Type2 devices of the radio monitoring system. The particular Type2 device is grouped with at least one particular Type1 device through the at least one particular pairing of Type1 and Type2 devices. In one example, the particular asynchronous heterogeneous Type2 device comprises: a wireless circuitry to receive asynchronously at least one asynchronous heterogeneous radio signal, a second heterogeneous processor communicatively coupled with the wireless circuitry, a second heterogeneous memory communicative coupled with the second heterogeneous processor, and a second heterogeneous set of instructions stored in the second heterogeneous memory. Each asynchronous heterogeneous radio signal is transmitted asynchronously by one of the at least one particular Type1 device using a respective first heterogeneous processor, a respective first heterogeneous memory and a respective first heterogeneous set of instructions of the Type1 device to at least one asynchronous heterogeneous Type2 device through a wireless multipath channel influenced by a movement of a mass in a site. The second heterogeneous set of instructions, when executed, cause the second heterogeneous processor to secure asynchronously at least one respective series of CI (TSCI) of the wireless multipath channel, for each of the at least one particular pairing of Type1 and Type2 devices.

In one embodiment, the at least one respective TSCI associated with the particular pairing of Type1 and Type2 devices is being derived asynchronously from the respective asynchronous heterogeneous radio signal received asynchronously by the wireless circuitry. In one example, the movement of the mass in the site is monitored individually and asynchronously, using a third processor, a third memory and a third set of instructions of a Type 3 device, based on TSCI associated with a pairing of Type1 and Type2 devices comprising the particular Type2 device. In another example, the movement of the mass is monitored jointly and asynchronously based on TSCI associated with any pairings of Type1 and Type2 devices comprising the particular Type2 device. In another example, the movement of the mass is monitored jointly and asynchronously based on TSCI associated with any pairings of Type1 and Type2 devices comprising one of the at least one particular Type1 device. In another example, the movement of the mass is monitored globally and asynchronously based on TSCI associated with any pairings of Type1 and Type2 devices.

In one embodiment, for each Type2 device, and for each of the at least one pairing of Type1 and Type2 devices comprising the Type2 device, a respective heterogeneous similarity score associated with the respective pairing of Type1 and Type2 devices is computed asynchronously based on comparing a respective current window and a respective past window of at least one respective TSCI associated with the pairing of Type1 and Type2 devices. In one example, the movement of the mass in the site is monitored individually and asynchronously based on asynchronously computed heterogeneous similarity score associated with the pairing of Type1 and Type2 devices comprising the particular Type2 device. In another example, the movement of the mass in the site is detected individually and asynchronously when a heterogeneous similarity score associated with the pair of Type1 and Type2 devices comprising the particular Type2 device is at least one of: greater than, and equal to, an individual threshold. In another example, the movement of the mass in the site is monitored jointly and asynchronously based on asynchronously computed heterogeneous similarity score associated with any pairing of Type1 and Type2 devices comprising the particular Type2 device. In another example, the movement of the mass in the site is detected jointly and asynchronously when a first joint score computed based on heterogeneous similarity score associated with any pairing of Type1 and Type2 devices comprising the particular Type2 device is at least one of: greater than, and equal to, a first joint threshold. In another example, the movement of the mass in the site is monitored jointly and asynchronously based on asynchronously computed heterogeneous similarity score associated with any pairing of Type1 and Type2 devices associated with one of the at least one particular Type1 device. In another example, the movement of the mass in the site is detected jointly and asynchronously when a second joint score computed based on heterogeneous similarity score associated with any pairing of Type1 and Type2 devices associated with one of the at least one particular Type1 device. In another example, the movement of the mass in the site is monitored globally and asynchronously based on asynchronously computed heterogeneous similarity score associated with any pairing of Type1 and Type2 devices. In another example, the movement of the mass in the site is detected globally and asynchronously when a third joint score computed based on heterogeneous similarity score associated with any pairing of Type1 and Type2 devices is at least one of: greater than, and equal to, a third joint threshold.

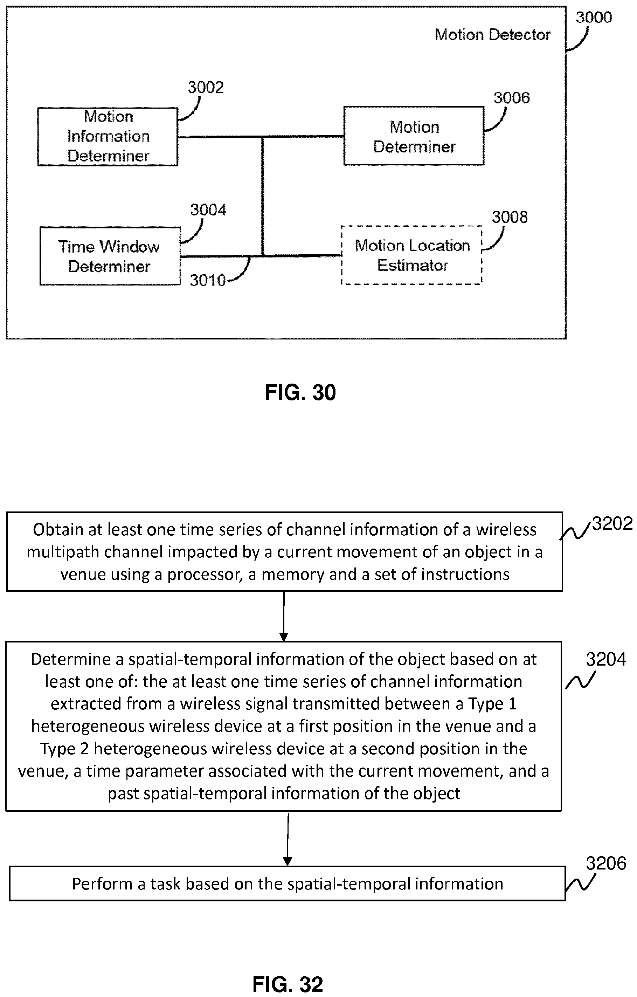

The synchronization controller 2806 in this example may be configured to control the operations of the Origin 2800 to be synchronized or un-synchronized with another device, e.g. a Bot, another Origin, or an independent motion detector. In one embodiment, the synchronization controller 2806 may control the Origin 2800 to be synchronized with a Bot that transmits a wireless signal. In another embodiment, the synchronization controller 2806 may control the Origin 2800 to receives the wireless signal asynchronously with other Origins. In another embodiment, each of the Origin 2800 and other Origins may receive the wireless signals individually and asynchronously. In one embodiment, the optional motion detector 2822 or a motion detector outside the Origin 2800 is configured for asynchronously computing respective heterogeneous motion information related to the motion of the object based on the respective TSCI. In one example, the optional motion detector 2822 or a motion detector outside the Origin 2800 is configured for monitoring and/or detecting the motion of the object in the venue individually and asynchronously based on the asynchronously computed heterogeneous motion information associated with a pair of Type1 and Type2 devices. In another example, the optional motion detector 2822 or a motion detector outside the Origin 2800 is configured for monitoring and/or detecting the motion of the object in the venue jointly and asynchronously based on the asynchronously computed heterogeneous motion information associated with any pair of Type1 and Type2 devices comprising a particular Type2 device. In another example, the optional motion detector 2822 or a motion detector outside the Origin 2800 is configured for monitoring and/or detecting the motion of the object in the venue jointly and asynchronously based on the asynchronously computed heterogeneous motion information associated with any pair of Type1 and Type2 devices comprising a particular Type1 device. In another example, the optional motion detector 2822 or a motion detector outside the Origin 2800 is configured for monitoring and/or detecting the motion of the object in the venue globally and asynchronously based on the asynchronously computed heterogeneous motion information associated with any pair of Type1 and Type2 devices. A detailed structure of the optional motion detector 2822 or a motion detector outside the Origin 2800 will be described with respect to FIG. 30.

The various modules discussed above are coupled together by a bus system 2830. The bus system 2830 can include a data bus and, for example, a power bus, a control signal bus, and/or a status signal bus in addition to the data bus. It is understood that the modules of the Origin 2800 can be operatively coupled to one another using any suitable techniques and mediums. Although a number of separate modules or components are illustrated in FIG. 28, persons of ordinary skill in the art will understand that one or more of the modules can be combined or commonly implemented. For example, the processor 2802 can implement not only the functionality described above with respect to the processor 2802, but also implement the functionality described above with respect to the CI extractor 2820. Conversely, each of the modules illustrated in FIG. 28 can be implemented using a plurality of separate components or elements. In one embodiment, in addition to the Bot 2600 and the Origin 2800, the system may also comprise: a third wireless device, e.g. another Bot, configured for transmitting an additional heterogeneous wireless signal through an additional wireless multipath channel impacted by the motion of the object in the venue, and a fourth wireless device, e.g. another Origin, that has a different type from that of the third wireless device. The fourth wireless device may be configured for: receiving the additional heterogeneous wireless signal through the additional wireless multipath channel impacted by the motion of the object in the venue, and obtaining a time series of additional CI (CI) of the additional wireless multipath channel based on the additional heterogeneous wireless signal. The additional CI of the additional wireless multipath channel is associated with a different protocol or configuration from that associated with the CI of the wireless multipath channel. For example, the wireless multipath channel is associated with LTE, while the additional wireless multipath channel is associated with Wi-Fi. In this case, the optional motion detector 2822 or a motion detector outside the Origin 2800 is configured for detecting the motion of the object in the venue based on both the motion information associated with the first and second wireless devices and additional motion information associated with the third and fourth wireless devices computed by at least one of: an additional motion detector and the fourth wireless device based on the time series of additional CI.

FIG. 29 illustrates a flowchart of an exemplary method performed by a second wireless device, e.g. the Origin 2800 in FIG. 28, in accordance with some embodiments of the present disclosure. At operation 2902, the Origin receives and analyzes a wireless signal through a wireless multipath channel impacted by a motion of an object. At operation 2904, the Origin obtains a TSCI of the channel based on the wireless signal. Optionally at operation 2906, the Origin determines at least one time window and/or at least one component of the TSCI. Optionally at operation 2908, the Origin computes motion information related to the motion of the object based on the TSCI. Optionally at operation 2910, the Origin detects the motion of the object based on the computed motion information. Optionally at operation 2912, the Origin estimates a location associated with the motion of the object.

FIG. 30 illustrates an exemplary motion detector 3000, according to one embodiment of the present teaching. The motion detector 3000 may serve as a motion detector (e.g. the optional motion detector 2822) in an Origin, or an independent motion detector outside any Origin. As shown in FIG. 30, the motion detector 3000 in this example includes a motion information determiner 3002, a motion determiner 3006, a time window determiner 3004, and an optional motion location estimator 3008. According to various embodiments, the motion detector 3000 may be coupled to at least one of: the first wireless device; the second wireless device; a third wireless device having a same type as that of the first wireless device; a fourth wireless device having a same type as that of the second wireless device; a cloud server; a fog server; a local server; and an edge server, for detecting and monitoring object motions.

In one embodiment, the motion detector 3000 may receive the TSCI from the CI extractor 2820, and compute motion information based on the TSCI. For example, the motion information determiner 3002 may receive the respective TSCI from an Origin, and asynchronously compute respective heterogeneous motion information related to the motion of the object based on the respective TSCI. In one example, the motion information determiner 3002 may compute a heterogeneous similarity score between a current time window and a past time window of the TSCI, based on at least one of: a distance score, an absolute distance, a Euclidean distance, a norm, a metric, a statistical characteristic, a time reversal resonating strength (TRRS), a cross-correlation, an auto-correlation, a covariance, an auto-covariance, an inner product of two vectors, a preprocessing, a signal conditioning, a denoising, a phase correction, a timing correction, a timing compensation, a phase offset compensation, a transformation, a projection, a filtering, a feature extraction, a finite state machine, a history of past similarity score, another past window of the at least one TSCI, a component-wise operation, machine learning, a neural network, a deep learning, a training, a discrimination, a weighted averaging, and another operation, such that the motion of the object in the venue is detected based on the computed heterogeneous similarity score. The motion information determiner 3002 may send the computed motion information to the motion determiner 3006 for detecting and monitoring object motions.

In one embodiment, the time window determiner 3004 can determine a current window and a past window of the TSCI, and send the current time window and the past time window to the motion information determiner 3002. Then the motion information determiner 3002 can compute asynchronously a heterogeneous similarity score between the current window and the past window of the TSCI, such that the motion of the object in the venue is detected based on the motion information and the heterogeneous similarity score.

The motion determiner 3006 in this example may receive the computed motion information from the motion information determiner 3002, and detect the motion of the object in the venue based on motion information related to the motion of the object. In one embodiment, for each of the at least one component, the time window determiner 3004 can determine asynchronously a respective current component window of the component based on a current time window, determine asynchronously a respective past component window of the component based on a past time window, and send the information about the respective current component window and the respective past component window to the motion determiner 3006. The motion determiner 3006 may compare component-wise the respective current component window with the respective past component window asynchronously to generate a comparison result, monitor component-wise the motion of the object based on the comparison result, and detect the motion of the object in the venue based on the asynchronous component-wise comparing.

In one embodiment, for each of the at least one component, the motion information determiner 3002 can compute asynchronously a component similarity score associated with the respective current component window and the respective past component window, based on at least one of: a distance score, a norm, a metric, a statistical characteristic, a time reversal resonating strength (TRRS), a cross-correlation, an auto-correlation, a covariance, an auto-covariance, an inner product of two vectors, a preprocessing, a signal conditioning, a denoising, a phase correction, a timing correction, a timing compensation, a phase offset compensation, a transformation, a projection, a filtering, a feature extraction, a finite state machine, a history of past similarity score, another past window of the at least one TSCI, a component-wise operation, machine learning, a neural network, a deep learning, a training, a discrimination, a weighted averaging, and another operation. The motion information determiner 3002 may then compute asynchronously a heterogeneous similarity score based on a heterogeneous function of the at least one component similarity score. The heterogeneous function comprises at least one of: a representative value, a typical value, a weighted average, a percentile, a maximum, a minimum, a 40% quartile, a 50% quartile, a 60% quartile, a mean, a median, a mode, a sum, a product, a root, an arithmetic mean, a geometric mean, a harmonic mean, a generalized mean, an ordered statistic, a trimmed mean, a statistical function, an expected value, a variance, a selected one, and another function. The motion determiner 3006 may detect the motion of the object in the venue when the heterogeneous similarity score is greater than or equal to a first threshold.

In another embodiment, for each of the at least one component, the motion information determiner 3002 may compute asynchronously a component similarity score associated with the respective current component window and the respective past component window, based on at least one of: a distance score, a norm, a metric, a statistical characteristic, a time reversal resonating strength (TRRS), a cross-correlation, an auto-correlation, a covariance, an auto-covariance, an inner product of two vectors, a preprocessing, a signal conditioning, a denoising, a phase correction, a timing correction, a timing compensation, a phase offset compensation, a transformation, a projection, a filtering, a feature extraction, a finite state machine, a history of past similarity score, another past window of the at least one TSCI, a component-wise operation, machine learning, a neural network, a deep learning, a training, a discrimination, a weighted averaging, and another operation. Then for each of the at least one component, the motion determiner 3006 may detect component-wise the motion of the object asynchronously when the respective component similarity score is greater than or equal to a respective component threshold, and detect the motion of the object in the venue when a percentage of component-wise motion detection in a selected group of components is greater than or equal to a second threshold.

In one embodiment, the first wireless device is a Type1 device having a Type1; the second wireless device is a Type2 device having a Type2. In one example, the motion determiner 3006 may monitor and/or detect the motion of the object in the venue individually and asynchronously based on the asynchronously computed heterogeneous motion information associated with a pair of Type1 and Type2 devices. In another example, the motion determiner 3006 may monitor and/or detect the motion of the object in the venue jointly and asynchronously based on the asynchronously computed heterogeneous motion information associated with any pair of Type1 and Type2 devices comprising a particular Type2 device. In another example, the motion determiner 3006 may monitor and/or detect the motion of the object in the venue jointly and asynchronously based on the asynchronously computed heterogeneous motion information associated with any pair of Type1 and Type2 devices comprising a particular Type1 device. In another example, the motion determiner 3006 may monitor and/or detect the motion of the object in the venue globally and asynchronously based on the asynchronously computed heterogeneous motion information associated with any pair of Type1 and Type2 devices.

In one embodiment, for each pair of Type1 and Type2 devices comprising a Type1 device and a Type2 device, the respective Type2 device or the motion information determiner 3002 in the motion detector 3000 is configured for computing asynchronously a respective heterogeneous similarity score between a respective current window and a respective past window of the respective TSCI. Then the motion determiner 3006 or the Type2 device may detect the motion of the object in the venue individually and asynchronously when a first function of the respective heterogeneous similarity score associated with a pair of Type1 and Type2 devices is greater than or equal to a respective individual threshold, detect the motion of the object in the venue jointly and asynchronously when a second function of the heterogeneous similarity scores associated with any pair of Type1 and Type2 devices comprising a particular Type2 device is greater than or equal to a respective joint threshold, detect the motion of the object in the venue jointly and asynchronously when a third function of the heterogeneous similarity scores associated with any pair of Type1 and Type2 devices comprising a particular Type1 device is greater than or equal to another respective joint threshold, and/or detect the motion of the object in the venue globally and asynchronously when a fourth function of the heterogeneous similarity scores associated with any pair of Type1 and Type2 devices is greater than or equal to a respective global threshold.

The optional motion location estimator 3008 in this example is an optional component in the motion detector 3000. In one embodiment, the optional motion location estimator 3008 may be outside the motion detector 3000 and serve as a supervisory device, that is configured for: partitioning a map of the venue into a plurality of regions; and/or associating asynchronously the motion of the object with at least one of the plurality of regions of the map of the venue based on heterogeneous motion information received from the Type2 wireless devices. In one embodiment, at least one of the motion detector 3000 and the Type2 devices is configured for performing at least one of: partitioning a respective feature space of each of the heterogeneous motion information into a plurality of respective feature segments; constructing a respective mapping that associates each respective feature segment with at least one of the plurality of regions of the map of the venue; associating asynchronously the motion of the object individually with at least one of the plurality of regions of the map of the venue based on the respective mapping; partitioning a joint feature space of heterogeneous motion information associated with a plurality of pairs of Type1 and Type2 devices into a plurality of joint feature segments; constructing a joint mapping that associates each joint feature segment with at least one of the plurality of regions of the map of the venue; and associating asynchronously the motion of the object jointly with a particular one of the plurality of regions of the map of the venue based on the joint mapping.

In one embodiment, the motion detector 3000 may serve as a Type 3 device of a radio monitoring system. The radio monitoring system may comprise at least one asynchronous heterogeneous Type1 device and at least one asynchronous heterogeneous Type2 device. The radio monitoring system may comprise at least one doublet of Type1 and Type2 devices, each doublet comprising one of the at least one asynchronous heterogeneous Type1 device, and one of the at least one asynchronous heterogeneous Type2 device. The Type 3 device may comprise a third processor communicatively coupled with at least one of: at least one asynchronous heterogeneous Type1 device, and at least one asynchronous heterogeneous Type2 device; a third memory communicative coupled with the third processor; and a third set of instructions stored in the third memory. The third set of instructions, when executed, cause the third processor to: for each Type2 device, and for each of the at least one doublet of Type1 and Type2 devices comprising the Type2 device, receive asynchronously at least one respective TSCI (TSCI) of a wireless multipath channel influenced by a motion of an item in a place received asynchronously by the Type2 device using a respective second processor, a respective second memory and a respective second set of instructions of the Type2 device. The at least one respective TSCI associated with the respective doublet of Type1 and Type2 devices is obtained asynchronously from a respective asynchronous heterogeneous radio signal transmitted from a respective Type1 device of the respective doublet using a respective first processor, a respective first memory and a respective first set of instructions of the respective Type1 device to at least one asynchronous heterogeneous Type2 device through the wireless multipath channel. In addition, the third set of instructions, when executed, cause the third processor to perform at least one of the following: tracking the motion of the item in the place individually and asynchronously, based on TSCI associated with a particular doublet of Type1 and Type2 devices comprising a particular Type2 device and a particular Type1 device, tracking the motion of the item jointly and asynchronously based on TSCI associated with any of the at least one doublet of Type1 and Type2 devices associated with the particular Type2 device, tracking the motion of the item jointly and asynchronously based on TSCI associated with any of the at least one doublet of Type1 and Type2 devices associated with the particular Type1 device, and tracking the motion of the item globally and asynchronously based on TSCI associated with any of the at least one doublet of Type1 and Type2 devices.

In one embodiment, the system may comprise a monitoring device configured for signaling asynchronously information associated with at least one of: the map of the venue, the plurality of partitioned regions of the map, regions associated with any detected motion of the object, heterogeneous motion information associated with at least one pair of Type1 and Type2 devices, the component-wise similarity score, the heterogeneous similarity score, past motion information, past similarity score, another past information, Type1 devices, and Type2 devices. In one example, the monitoring device may be a mobile phone running an app or GUI to display the detection or monitoring result. In other examples, the monitoring device may be a desktop, a laptop, a tablet, an IoT device, or a smart speaker.

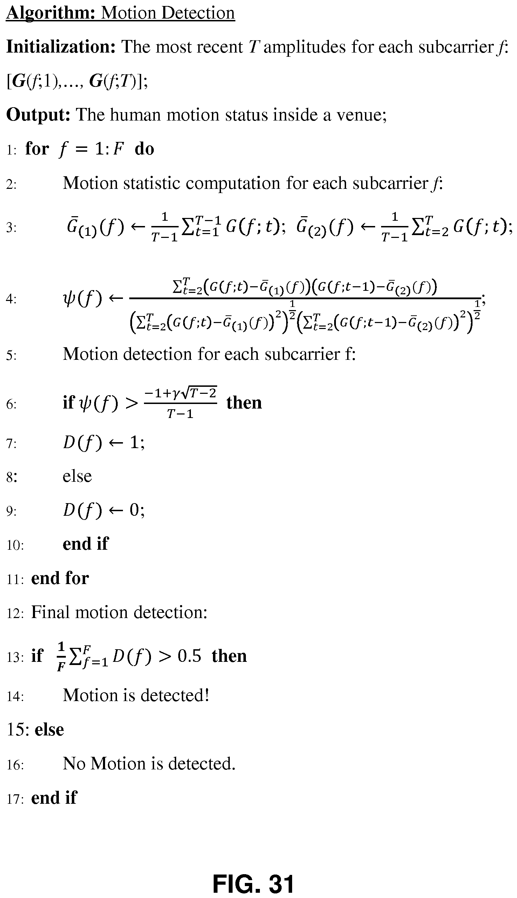

FIG. 31 illustrates an exemplary algorithm of motion detection, according to one embodiment of the present teaching. In FIG. 31, $G(f; t)$ may denote the CSI (or CI) amplitude of subcarrier $f$ at time slot $t$. For a system with $M$ by $N$ antenna configuration, the total number of subcarriers is $F=MNL$, where $L$ is the number subcarrier for each antenna pair. For each subcarrier $f$, one may compute a *motion statistic as the first order of the sample auto-correlation coefficient,* where $T$ is the length of the time window to compute the motion statistic. The physical meaning of the motion statistic is that the higher the motion statistic, the stronger the motion is. On each subcarrier, there is a motion statistic calculated for detecting a motion, e.g., when its sample auto-correlation coefficient is larger than a predefined threshold. A majority vote may be applied to fuse all the decisions from F subcarriers of the system. When more than half of the total number of the available subcarriers detect the human motion inside the elevator, then the system detects the motion; otherwise, no motion is detected. In another embodiment, $G(f; t)$ may be defined as another function of the CI on subcarrier $f$ at time slot $t$, for example, (CSI amplitude)^2, (CSI amplitude)^4, real/imaginary part of the CSI after phase offset cleaning. The motion static may also be defined as the sample auto-correlation coefficient with another order, if the order is less than a quarter of the time window length T. Other decision fusion rule such as weighted combining of the individual decision may also be adopted.

FIG. 32 illustrates an exemplary method for object motion detection, according to one embodiment of the present teaching. At operation 3202, at least one TSCI of a wireless multipath channel is obtained, using a processor, a memory and a set of instructions stored in the memory. The at least one TSCI is extracted from a wireless signal transmitted between a Type1 heterogeneous wireless device at a first position in a venue and a Type2 heterogeneous wireless device at a second position in the venue through the wireless multipath channel. The wireless multipath channel is impacted by a motion of an object in the venue. At operation 3204, a spatial-temporal information of the object is determined based on at least one of: the at least one TSCI, a time parameter associated with the motion, and a past spatial-temporal information of the object. At operation 3206, a task is performed based on the spatial-temporal information. The spatial-temporal information may be a form of motion information, and motion information may be a form of spatial-temporal information. A Type1 device may be the First Device, the Type2 device may be the Second Device. The "task" mentioned above may be to "monitor motion" and/or to "detect motion".

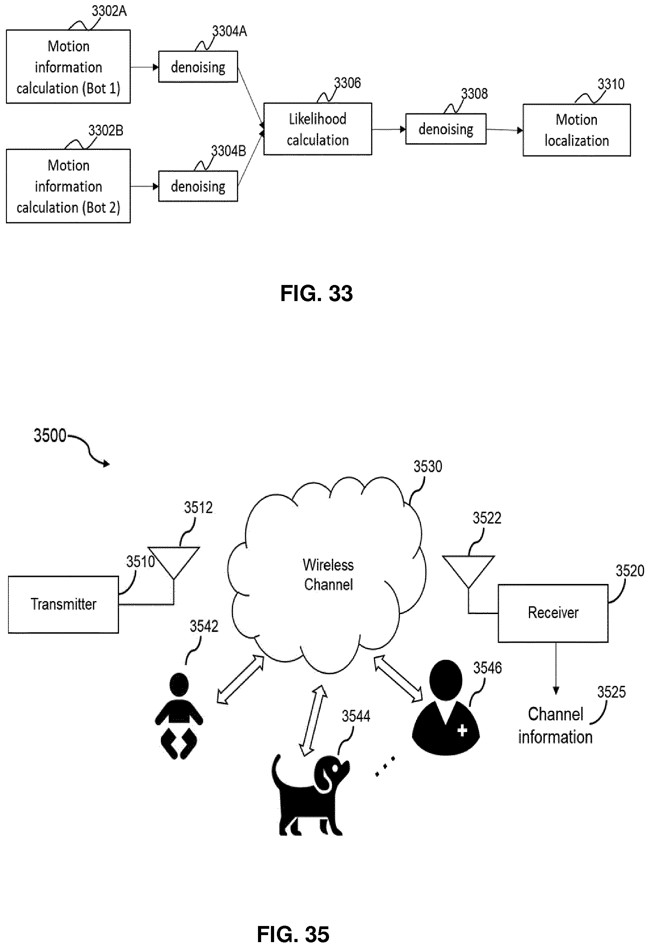

FIG. 33 illustrates an exemplary flowchart for a method of object motion localization, according to one embodiment of the present teaching. The object motion localization in FIG. 33 is based on 2 Bots (Type1 Devices), paired with a particular Origin (Type2 Device). First, for each Bot, the motion information/statistics associated with each Bot-Origin pair (3302A and 3302B) is calculated. Then each of the calculated motion information is passed through a denoising processor (e.g. a low pass filter) to remove noise (3304A and 3304B). Then a motion likelihood (3306) is calculated according to some function. The motion likelihood may denote a probability of motion occurring near a Bot or near an Origin. Since the likelihood may also be noisy, e.g., change rapidly when motion occurs, the likelihood values may go through another denoising processor 3308. Finally, a decision of motion localization is made on where the motion happens.

An example function of mapping motion information to likelihood may be defined as follows. Denote $m_i(t)$ as the motion information calculated for Bot i-Origin pair at time t. An example of motion likelihood near Bot i at time t may be calculated as

$$
L_i(t) = \begin{cases} 1, & \text{Bot i detects motion, the other Bot doesn't detect motion;} \\ 0, & \text{Bot i doesn't detect motion, the other Bot detect motion;} \\ \dfrac{m_i^p}{\sum_i m_i^p}, & \text{Both Bots detect motion.} \end{cases}
$$

$L_i(t)$ is undefined when neither of the bots detect motion. Denote $\tilde{L}_i(t)$ as the likelihood after denoising an example of motion localization decision rule can be

$$MLoc(t) = \begin{cases} Near\ Bot\ 1, & when\ \tilde{L}_1(t) > \eta_1 \\ Near\ Bot\ 2, & when\ \tilde{L}_2(t) > \eta_2 \\ Near\ the\ common\ area, & when\ \tilde{L}_1(t) < \eta_1\ and\ \tilde{L}_2(t) < \eta_2 \end{cases}$$

When both $\tilde{L}_1(t)$ and $\tilde{L}_2(t)$ are undefined, then no motion exists in the monitored area.

Different embodiments of the present disclosure will now be described in detail hereinafter. It is noted that the features of the embodiments and examples in the present disclosure may be combined with each other in any manner without conflict.

In one embodiment, a wireless (or radio or RF) monitoring system comprises at least one asynchronous heterogeneous Type1 wireless (or radio or RF) device (or apparatus) and at least one asynchronous heterogeneous Type2 wireless (or radio or RF) device. The wireless (or radio or RF) monitoring system also comprises at least one pair (or pairing, or couple, or duo, or combination, or doublet, or combo, or match, or team) of Type1 and Type2 devices, each pair comprising one of the at least one asynchronous heterogeneous Type1 device, and one of the at least one asynchronous heterogeneous Type2 device. Each of the at least one Type2 device is in at least one respective particular pair of Type1 and Type2 devices. Each Type2 device is associated (or grouped or coupled or paired or conjoined or combined or affiliate or partnered) with at least one respective particular Type1 device through the at least one respective particular pair of Type1 and Type2 devices.

In one embodiment, a disclosed method of the wireless (or radio or RF) monitoring system comprises obtaining (or receiving, or getting, or collecting, or retrieving, or securing, or fetching) asynchronously at least one respective TSCI (or channel data) of a wireless multipath channel impacted (or influenced) by a motion (or movement, or act, or gesture, or change, or drift, or dynamics, or flow, or fluctuation, or flux, or gesticulation, or kinetics, or locomotion, or mobility, or movement, or oscillation, or passing, or progressing, or stirring, or swaying) of an object (or article, or body, or commodity, or gadget, or item, or matter, or substance, or entity, or mass) in a venue (or locale, or place, or site, or setting, or ground) for each Type2 device and for each of the at least one respective particular pair of Type1 and Type2 devices comprising the Type2 device. The at least one respective TSCI (TSCI) associated with the particular pair of Type1 and Type2 devices is extracted (or obtained or derived or gleaned or collected or garnered) asynchronously from a respective asynchronous heterogeneous wireless (or radio or RF) signal. The respective asynchronous heterogeneous wireless (or radio or RF) signal is transmitted asynchronously from a respective one of the at least one respective particular Type1 device using a respective first heterogeneous processor, a respective first heterogeneous memory and a respective first heterogeneous set of instructions of the Type1 device to at least one asynchronous heterogeneous Type2 device through the wireless multipath channel.

The motion of the object in the venue is monitored (or tracked or checked or observed or overseen or observed or surveyed) using a third processor, a third memory and a third set of instructions of a Type 3 device in at least one of the four following ways: (a) the motion of the object in the venue is monitored individually and asynchronously, based on TSCI associated with a pair of Type1 and Type2 devices comprising a particular Type2 device; (b) the motion of the object is monitored jointly and asynchronously based on TSCI associated with any of the at least one pair of Type1 and Type2 devices associated with the particular Type2 device; (c) the motion of the object is monitored jointly and asynchronously based on TSCI associated with any of the at least one pair of Type1 and Type2 devices associated with one of the at least one respective particular Type1 device, and (d) the motion of the object is monitored globally and asynchronously based on TSCI associated with any of the at least one pair of Type1 and Type2 devices.

For example, more than one asynchronous heterogeneous Type1 devices may transmit asynchronously their respective asynchronous heterogeneous wireless (or radio or RF) signals to the same Type2 devices. (Some Type1 device may be transmitting to more than one Type2 devices.) Their transmission may be synchronized momentarily. Their transmission may be asynchronous, but sometimes the wireless (or radio or RF) signals of two of the Type1 devices may overlap in time. Their transmission may be coordinated. The motion of the object may be jointly monitored based on TSCI associated pairs of Type1 and Type2 devices comprising the Type2 device and one of the more than one asynchronous Type1 devices.

In another example, an asynchronous heterogeneous Type1 devices may transmit the asynchronous heterogeneous wireless (or radio or RF) signal to more than one asynchronous heterogeneous Type2 devices. The asynchronous heterogeneous wireless (or radio or RF) signal may be received asynchronously, or near synchronously, or simply synchronously. TSCI may be extracted asynchronously from the received asynchronous heterogeneous wireless (or radio or RF) signal. The motion of the object may be jointly monitored based on the TSCI associated pairs of Type1 and Type2 devices each comprising the asynchronous heterogeneous Type1 device and one of the more than one asynchronous heterogeneous Type2 devices.

Each CI (CI) may comprise at least one of: a channel state information (CSI), a frequency domain CSI, a frequency domain CSI associated with at least one sub-band, a time domain CSI, a CSI in a domain, a channel impulse response (CIR), a channel frequency response (CFR), a channel characteristics, a channel filter response, a CSI of the wireless multipath channel, an information of the wireless multipath channel, a timestamp, an auxiliary information, a data, a meta data, a user data, an account data, an access data, a security data, a session data, a status data, a supervisory data, a household data, an identity (ID), a device data, a network data, a neighborhood data, an environment data, a real-time data, a sensor data, a stored data, an encrypted data, a compressed data, a protected data, and/or another CI.

A CI (CI) may be extracted from a received probe signal (with multi-path) by a Type2 device, an integrated circuit (IC, or chip) of the Type2 device (e.g. a Wi-Fi chip, an LTE chip, a cellular network chip, a wireless network chip, a mesh network chip, a Bluetooth chip, a UWB chip, etc). The probe signal may/may not contain data. More than one CI may be extracted from a received probe signal. One CI may be extracted for each pair of transmitting antenna and receiving antenna. For example, for a Type1 device with 3 transmitting antennas and a Type2 device with 2 receiving antennas, a total of 6 CI may be extracted from each probe signal.

A CI may comprise information of the channel from more than one pairs of transmitting antennas and receiving antennas. A CI may be sent from the Type2 device to another device (e.g. a neighboring device, a companion device, a paired device, an edge device, a cloud device). The another device may be communicatively coupled with the Type2 device. The another device may be another Type2 device, the Type1 device and/or another Type1 device. The CI obtained by different heterogeneous Type2 devices may be different because different heterogeneous Type2 devices may be at different locations within the venue such that they may experience different multipath patterns. In other words, the respective wireless multipath channel experienced by respective heterogeneous Type2 device may be different. The CI may capture information (e.g. motion, periodic motion, shape, size, volume, path, direction, distance, speed, acceleration, change, rate of change, etc.) of the venue and/or any object (people, pet, creature, machine, device with movable parts, door, window, wall, partitioning, furniture, fixture, piping, air conditioning, heating, fan, etc.) in the venue. The CI may have some inherent imperfection such as noise, phase error, timing error, etc. The CI may be preprocessed, processed and/or post-processed.

In the case of at least one Type1 devices interacting with at least one Type2 devices, the processing, preprocessing, post-processing, and/or other processing may be different (heterogeneous) for different devices. The processing/preprocessing/ post-processing/other processing may be based on locations, orientation, direction, roles, user-related characteristics, settings, configurations, available resources, available bandwidth, network connection, hardware, software, processor, co-processor, memory, battery life, available power, antennas, antenna types, directional/unidirectional characteristics of the antenna, power setting, and/or other parameters/characteristics of the devices.

The wireless (or radio or RF) signal transmitted from the heterogeneous Type1 device to the heterogeneous Type2 device may be a series of probe signals. Each probe signal may be an impulse, a number (e.g. 2, 3, 4, or more) of impulses, or another signal waveform. The another signal waveform may be based on the wireless multipath channel. The time separation of the number of impulses may be changed over time. (For example, the time separation between the second and third impulses may be different from the time separation between the first and second impulses). The magnitude of different impulses may be different (e.g. the first impulse may be stronger or weaker than the second impulse). The shape of different impulses may be different. (e.g. the first impulse may have a narrow, triangular waveform while the second impulse may have yet another waveform). The probe signal may be adaptive changed over time. The probe signal(s) may be different for different channels, different space-time-frequency channels, different antenna, etc. The probe signal(s) may be different for different Type1 devices and/or Type2 devices. The probe signal may be changed based on a feedback signal (e.g. from one or more Type2 devices). The probe signal(s) may/may comprise data. Some probe signals may comprise data while some other probe signals may not comprise data.

The series of probe signals may be at regular intervals (e.g. 40 probe signals per second, with an interval of 25ms (1/40 second) between adjacent probe signals), or irregular intervals (e.g. probe signals sent when the wireless channel is less busy or not busy, or probe signals sent in short/longer/long bursts adaptively or on demand). The regular intervals and/or waveform associated with each probe signal may be changed over time. For example, the probe signals may be 40 probe signals per second for a period of time (e.g. 1 day, 1 hour, or 20 seconds), and changed to 3300 probe signals per second for another period of time. The change may be based on a user input, a system command, a task, a change in the task, the venue, a change of the venue, the respective object, a change of the respective object, the respective motion of the respective object, a change of the respective motion of the respective object, and/or the monitoring of the respective object/the respective motion of the respective object.

The wireless multipath channel may be associated with a media access control (MAC) layer, a physical (PHY) layer, Wi-Fi, an IEEE 802.11 standard, 802.11a/b/g/n/ac/ad/af/ag/ah/ai/ajlaq/ax/ay, Bluetooth, Bluetooth 1.0/1.1/1.2/2.0/2.1/3.0/4.0/4.1/ 4.2/5, BLE, mesh network, an IEEE 802.16/1/1a/1b/2/2a/a/b/c/d/e/f/g/h/i/j/k/l/m/n/o/p/, standard, 802.16, Zigbee, WiMax, UWB, mobile channel, 1G/2G/3G/3.5G/4G/LTE/5G/6G/7G, etc.

The venue may comprise an indoor environment, an enclosed environment, an outdoor environment, an open-air environment (e.g. an open-air parking lot), a facility, a warehouse, a factory, a manufacturing facility, an assembly line, a building, a multi-storey building, a house, a home, an office, a store, a supermarket, a casino, a hotel, a room, a box, a stadium, a hall, a station, an airport, a port, a subway, a vehicle, a car, a boat, a ship, a cruise ship, a military vehicle, a submersible, a plane, a drone, a cave, a tunnel, a pipe, a piping system, a crawl space, a maintenance system, a tube, an air conditioning/ventilation system, a fluid, etc. The venue may comprise people, pet, children, animals, plants, partitions, walls, dry walls, concrete walls, brick walls, glass walls, metal walls, doors, windows, glasses, floors, ceilings, attics, garage, fireplace, parking facilities, structure elements, columns, beams, movable objects, non-movable objects, furniture, wardrobe, fixtures, machines, devices, lighting, curtains, blinds, construction features, building features, water pipes, air ducts, crawl space, basement, elevator, stairs, stair wells, hall way, corridor, maintenance space, fan, ventilation system, air-condition system, heat, HVAC, electrical wiring, refrigerator, cooking devices, oven, microwave, stove, television, sound system, smart speaker, lights, carpets, restricted area, limited-access area, forbidden area, etc.

The object may comprise a person, a crowd, a congregation of people, a group of people, a baby, a sick person, an older person, a younger person, an intruder, a suspicious person, a consumer, a supervisor, a professional, an athlete, a waiter, a shopper, a staff, an officer, a customer, a manager, a high-valued person, a targeted person, a doctor, a patient, a guest, a security personnel, a traveler, a pet, an animal, a vehicle, a automated guided vehicle (AGV), a driverless car, a motorcycle, a bicycle, a boat, a ship, a truck, a car, a train, a tram, an electric vehicle, a plane, a drone, an air-borne device, a subway train, a transport, a vehicle on a track/rail, a machine, a fixture, a furniture, a moveable object, a non-moveable object, a lift, a chair, a table, a valuable object, a jewelry, a robot, a robotic arm, a motion capture device, a tool, a body part of a person, a head, a neck, a shoulder, an arm, an elbow, a hand, a finger, a refrigerator, an air conditioner, a vacuum cleaner, a smart device, a smart fabric, a smart material, a smart phone, a phone, an electric device, an electronic device, a television, a media device, a heating device, a microwave oven, an oven, a stove, a light-emitting device, a sound-producing device, a speaker, a light-capturing device, a camera, a infra-red sensor, a sound-capturing device, a microphone, a moveable object, a non-moveable object, a rigid object, a non-rigid object, a fluid object, a deforming object, a fan, a hollow object, a box, a room, a liquid, a fluid, a plasma, a particle, etc.

Two of the respective objects may be the same object. Any of the respective objects may be the same object. The motion may comprise no-motion, inaction, idling, immobility, rest, stagnation, a repose, an impulsive motion, a collision, an impact, an explosion, a stiffening, a relaxation, a locomotion, an induced motion, a reactive motion, an active motion, a sudden motion, a steady motion, a waving motion, a motion sequence, a steady-state motion sequence, a repeatable motion, a changing motion, a timed motion, an oscillation, a periodic motion, a pseudo-periodic motion, a translational motion, a rotational motion, a regular motion, an irregular motion, a transient motion, a resizing, a deformation, a complex motion, a vibration, a shaking, an earthquake, a shock, an event, a chaotic motion, a statistical motion, a stationary motion, a non-stationary motion, a head motion, a neck motion, a shoulder motion, an arm motion, a hand motion, a finger motion, a waist motion, a leg motion, a foot motion, a body motion, an emotion display, an act, a gesture, a gait, a lie-down motion, a get-up motion, a sitting, a standing, a walking, a running, a jogging, a jumping, a dancing, a bow, a curtsy, a signing, an expression, an indication, a talking, a singing, a wind motion, a turbulence, a door movement, a window movement, a machine motion, a movement of a moveable object, an installation of a machine, a movement associated with a motor, a car movement, a braking, a turn, a change of direction, a drift, a sway, a stirring, a fluctuation, a disturbance, a flux, a mixing, a stirring, a right-turn, a left-turn, a wind-induced motion, a heartbeat, a breathing motion, a water motion, a fluid motion, a flow, a fluctuation, a motion of a flexible/non-rigid object, a temperature-related motion, expansion, contraction, a crowd motion, a turmoil, an unrest, etc. The monitoring may comprise monitoring at least one characteristics associated with the respective object and/or the respective motion of the respective object.

A heterogeneous Type1 device and/or a heterogeneous Type2 device may be an antenna, a device with an antenna, a device that has interface to attach/connect to/link an antenna, a device with a wireless transceiver, a device with a wireless transmitter, a device with a wireless receiver, an internet-of-thing (IoT) device, a device with wireless network, a device with both wired networking and wireless networking capability, a device with a wireless integrated circuit (IC), a Wi-Fi device, a device with a Wi-Fi chip (e.g. 802.11a/b/g/n/ac/ax standard compliant, etc), a Wi-Fi access point (AP), a Wi-Fi client, a Wi-Fi router, a Wi-Fi repeater, a Wi-Fi hub, a Wi-Fi mesh network router/hub/AP, a wireless mesh network router, an adhoc network device, a wireless mesh network device, a mobile device (e.g. 2G/2.5G/3G/3.5G/4G/LTE/ 5G/6G/7G, etc), a cellular device, a mobile network base station, a mobile network hub, a mobile network compatible device, an LTE device, a device with an LTE module, a mobile module (e.g. a circuit board with an mobile-enabling chip (IC) such as Wi-Fi chip, LTE chip, BLE chip, etc), a Wi-Fi chip (IC), an LTE chip, a BLE chip, a device with a mobile module, a smart phone, a companion device (e.g. dongle, attachment, plugin) for smart phones, a dedicated device, a plug-in device, an AC-powered device, a battery-powered device, a device with a processor/memory/set of instructions, a smart clock, a smart stationary, a smart pen, a smart user-interface, a smart paper, a smart mat, a smart camera, a smart television (TV), a set-top-box, a smart microphone, a smart speaker, a smart refrigerator, a smart oven, a smart machine, a smart phone, a smart wallet, a smart furniture, a smart door, a smart window, a smart ceiling, a smart floor, a smart wall, a smart table, a smart chair, a smart bed, a smart night-stand, a smart air-conditioner, a smart heater, a smart pipe, a smart duct, a smart cable, a smart carpet, a smart decoration, a smart gadget, a smart USB device, a smart plug, a smart dongle, a smart lamp/light, a smart tile, a smart ornament, a smart bottle, a vehicle, a smart car, a smart AGV, a drone, a smart robot, a laptop, a tablet, a computer, a harddisk, a network card, a smart instrument, a smart racket, a smart ball, a smart shoe, a smart wearable, a smart clothing, a smart glasses, a smart hat, a smart necklace, a smart food, a smart pill, a small device that moves in the body of a creature (e.g. in blood vessels, in lymph fluid, digestive system, etc). The Type1 device and/or Type2 device may be communicatively coupled with: the internet, another device with access to internet (e.g. smart phone), a cloud server, an edge server, a local server, and/or a storage.

There may be more than one heterogeneous Type1 device. The Type1 devices (and/or Type2 devices) may be heterogeneous. For example, a Type1 device (and/or Type2 devices) may be a Wi-Fi access point, another Type1 device (and/or Type2 device) may be a smart TV, and/or yet another Type1 device may be a simple device dedicated to sending the series of probe signals (and/or yet another Type2 device may be a simple device dedicated to receiving the series of probe signals and passing them on). Different Type1 devices (and/or Type2 devices) may have different functionalities, brands (e.g. Sony, Samsung, Philips, Apple, etc), models, size, form factors, shape, color, modules, antennas, circuitry, logic circuits, processors, clock, bus, memory, memory buses, storage, sets of instructions, power (e.g. AC or DC, rechargeable battery, etc), chips (IC), chip sets, firmware, software, network connection, priorities, access rights, security settings, etc.

A device may function as a Type1 device and a Type2 device. The device may function as Type1 device at a time and as Type2 device at a different time. The device may function as Type1 device and Type2 device simultaneously.

A heterogeneous Type1 device and/or a heterogeneous Type2 device may comprise an integrated circuits (IC). The IC may be standard compliant. The IC may support more than one standards/protocols (e.g. 802.11a/b/e/g/n/ac/ax, 2G/2.5G/3G/3.5G/4G/LTE/5G/6G, 802.16, mesh network, adhoc network, beyond 4G, Bluetooth, BLE, another network, UWB, RFID, etc). The Type1 or Type2 device may detect a motion and control another device (e.g. TV, fan, radio, speaker, light, air conditioner, heater, electrical appliance, security system, etc). A heterogeneous Type1 device and/or a Type2 device may comprise more than one antenna. The antennas may be heterogeneous. An antenna may be directional or omni-directional. The antennas may be arranged in a circle/ellipse, in a straight line, in a lattice, or another pattern, or another placing order. A particular heterogeneous Type1 device may/may not establish communication link with a particular Type2 device.

The Type 3 device may be a networked server, a cloud server, a fog server, an edge server, a local server, a local client, a smart device, a smart phone, an internet-of-thing device. The Type 3 processor may be a heterogeneous Type1 device, or a heterogeneous Type2 device.

The first processor, the second processor and/or the third may be a microprocessor, a multi-processor, a multicore processor, a parallel processor, a CISC processor, a RISC processor, a micro-controller, central processing unit (CPU), a graphical processor unit (GPU), a digital signal processor (DSP), an FPGA, an embedded processor (e.g. ARM), a logic circuit, etc. The third processor may be the first processor, the second processor or another processor.

The first memory, the second memory and/or the third memory may be RAM, ROM, PROM, EPROM, EEPROM, harddisk, flash memory, storage network, internal storage, external storage, local storage, edge storage, and/or cloud storage. The first memory, the second memory and/or the third memory may be volatile and/or non-volatile. The first set of instructions, second set of instructions and/or the third set of instructions may be embedded, pre-loaded, loaded upon boot up, loaded on the fly, loaded on demand, pre-installed, installed, and/or downloaded.

For each Type2 device, and for each of the at least one respective particular pair of Type1 and Type2 devices comprising the Type2 device, at least one respective heterogeneous motion information associated with the motion of the object may be computed (or obtained or calculated or estimated or determined or evaluated) asynchronously based on the at least one respective TSCI associated with the respective particular pair of Type1 and Type2 devices.

The motion of the object in the venue may be monitored individually and asynchronously based on asynchronously computed heterogeneous motion information associated with the pair of Type1 and Type2 devices comprising the particular Type2 device. The motion of the object may be monitored jointly and asynchronously based on asynchronously computed heterogeneous motion information associated with any of the at least one pair of Type1 and Type2 devices associated with the particular Type2 device. The motion of the object may be monitored jointly and asynchronously based on asynchronously computed heterogeneous motion information associated with any of the at least one pair of Type1 and Type2 devices associated with the respective particular Type1 device. The motion of the object may be monitored globally and asynchronously based on asynchronously computed heterogeneous motion information associated with any of the at least one pair of Type1 and Type2 devices. For example, at least one combined score may be computed based on all the scores and the motion of the object may be monitored jointly based on the at least one combined score. Computation may be shared among the first processor, the second processor, the third processor, a companion processor, a cloud server, a fog server, and/or a local server.

For each Type2 device, and for each of the at least one respective particular pair of Type1 and Type2 devices comprising the Type2 device, a respective heterogeneous similarity score between a respective current window and a respective past window of the at least one respective TSCI associated with the respective particular pair of wireless devices may be computed asynchronously.

The respective heterogeneous similarity score may be computed based on at least one of: a distance score, an absolute distance (e.g. 1_1 norm), a Euclidean distance, a norm, a metric, a statistical characteristic, a time reversal resonating strength (TRRS), a cross-correlation, an auto-correlation, a covariance, an auto-covariance, and/or an inner product of two vectors. The respective heterogeneous similarity score may also be computed based on at least one of: a preprocessing, a signal conditioning, a denoising, a phase correction, a timing correction, a timing compensation, a phase offset compensation, a transformation, a projection, and/or a filtering. The respective heterogeneous similarity score may also be computed based on at least one of: a feature extraction, a finite state machine, a history of past similarity score, another past window of the at least one TSCI, a component-wise operation, machine learning, a neural network, a deep learning, a training, a discrimination, a weighted averaging, and/or another operation.

The motion of the object in the venue may be monitored individually and asynchronously based on asynchronously computed heterogeneous similarity score associated with the pair of Type1 and Type2 devices comprising the particular Type2 device. The motion of the object may be monitored jointly and asynchronously based on asynchronously computed heterogeneous similarity score associated with any of the at least one pair of Type1 and Type2 devices associated with the particular Type2 device. The motion of the object may be monitored jointly and asynchronously based on asynchronously computed heterogeneous similarity score associated with any of the at least one pair of Type1 and Type2 devices associated with the respective particular Type1 device. The motion of the object may be monitored globally and asynchronously based on asynchronously computed heterogeneous similarity score associated with any of the at least one pair of Type1 and Type2 devices.

Each of the current window and the past window of a (or more than one, or all) TSCI may be represented as or transformed into a vector. The similarity score may be a distance between the two vectors such as absolute distance, Euclidean distance or other distance. The CI (or windows of CI, or sliding window of CI) may be considered a stochastic process. The similarity measure may be a cross-covariance, or auto-covariance. A covariance close to 1 may mean two CIs (or two windows of CI) being very similar (or highly correlated). In the case of CI with zero mean, covariance may become correlation. Inner product may be a way to compute correlation.

The inner product of two vectors: Assuming N components for each CI. A vector associated with a CI may be an N-tuple containing the N components. There may be more than one TSCI, say M time series, with M>=1. A vector associated with the M CI (CI) time series, at a particular time, may be a K-tuple, with K=M*N, containing the N components of the M CI (CI) at the particular time. Consider a window of a TSCI containing P CI (CI), with P>=1. A vector associated with the window may be a K-tuple, with K=P*N, containing the N components of the P CI (CI) of the window. Consider a composite window of M CI (CI) time series containing a window of P CI (CI), with P>=1 of each TSCI. A vector associated with the composite window may be a K-tuple, with K=P*M*N, containing the N components of the P CI (CI) of the window of each of the M time series. Consider a composite window of M CI (CI) time series containing a window of $P\_i$ CI (CI), with $P\_i$>=1, of the $i^{th}$ TSCI, for i=1, ..., M. A vector associated with the composite window may be a K-tuple, with K=($P\_1$+$P\_2$+...+$P\_M$)*N, containing the N components of the $P\_i$ CI (CI) of the window of the $i^{th}$ TSCI, i=1,2, ..., M.

Each CI of the at least one respective TSCI may have at least one respective component. For each Type2 device, and for each of the at least one respective particular pair of Type1 and Type2 devices comprising the Type2 device, and for each of the at least one respective component, the following may be done: (a) a respective current component window of the component of the at least one respective TSCI may be determined asynchronously based on the respective current window; (b) a respective past component window of the component of the at least one respective TSCI may be determined asynchronously based on the respective past window; (c) the respective current component window may be compared component-wise with the respective past component window asynchronously; and (d) the motion of the object may be monitored component-wise based on the component-wise comparing of the respective current component window and the respective past component window asynchronously.

The motion of the object in the venue may be monitored individually and asynchronously based on asynchronous component-wise comparing associated with the pair of Type1 and Type2 devices comprising the particular Type2 device. The motion of the object may be monitored jointly and asynchronously based on asynchronous component-wise comparing associated with any of the at least one pair of Type1 and Type2 devices associated with the particular Type2 device. The motion of the object may be monitored jointly and asynchronously based on asynchronous component-wise comparing associated with any of the at least one pair of Type1 and Type2 devices associated with the respective particular Type1 device. The motion of the object may be monitored globally and asynchronously based on asynchronous component-wise comparing associated with any of the at least one pair of Type1 and Type2 devices.

For example, each CI may comprise a set/record with N elements (components), a collection of N items/features/characteristics/behavioral measures (components), an N-tuple, a matrix with N columns (with each column being a component), a matrix with N rows (with each row being a component), an N-component vector, an N-component channel response, an N-component time signal, an N-component channel, an N-component channel impulse response (CIR), an N-component frequency signal, an N-component channel frequency response (CFR), etc. The N components may be homogeneous or heterogeneous.

Each component may be different. For example, one component may be a complex number and another component may be a logical value. Each component may be associated with at least one of: a frequency, a frequency subcarrier, a frequency band, a time, a time lag, a time window, a time period, and/or a count. The amount of CI in the current window (or current component window) and/or the amount of CI in the past window (or past component window) may be the same, or different. The amount of CI in the current window and/or the past window may be time varying. Complex conjugation may be applied. The comparing may comprise preprocessing, signal conditioning, denoising, phase correction, timing correction, timing compensation, phase offset compensation, transformation, projection, filtering, feature extraction, finite state machine, machine learning, neural network, deep learning, training discrimination, weighted averaging, etc. For example, the past motion information may be past CI, past CI associated with the motion of the object, past comparing, past motion decision, past motion statistics, past scores, past similarity scores, past component similarity scores, past device-wise similarity scores, etc.

For each Type2 device, and for each of the at least one respective particular pair of Type1 and Type2 devices comprising the Type2 device, for each of the at least one component, a component similarity score may be computed asynchronously based on the respective current component window and the respective past component window.

Each component similarity score may be computed based on at least one of: a distance score, a norm, a metric, a statistical characteristics, a time reversal resonating strength (TRRS), a cross-correlation, an auto-correlation, a covariance, an auto-covariance, an inner product of two vectors, a preprocessing, a signal conditioning, a denoising, a phase correction, a timing correction, a timing compensation, a phase offset compensation, a transformation, a projection, a filtering, a feature extraction, a finite state machine, a history of past similarity score, another past window of the at least one TSCI, a component-wise operation, machine learning, a neural network, a deep learning, a training, a discrimination, a weighted averaging, and/or another operation.

A respective heterogeneous similarity score may be computed asynchronously as a heterogeneous function of the at least one component similarity score. The heterogeneous function may comprise at least one of: a representative value, a typical value, a weighted average, a percentile, a maximum, a minimum, a 40% quartile, a 50% quartile, a 60% quartile, a mean, a median, a mode, a sum, a product, a root, an arithmetic mean, a geometric mean, a harmonic mean, a generalized mean, an ordered statistic, a trimmed mean, a statistical function, an expected value, a variance, a selected one, and/or another function.

The motion of the object in the venue may be monitored individually and asynchronously based on at least one of: heterogeneous similarity score, and at least one component similarity score, associated with the pair of Type1 and Type2 devices comprising the particular Type2 device. The motion of the object may be monitored jointly and asynchronously based on at least one of: heterogeneous similarity score, and at least one component similarity score, associated with any of the at least one pair of Type1 and Type2 devices associated with the particular Type2 device. The motion of the object may be monitored jointly and asynchronously based on at least one of: heterogeneous similarity score, and at least one component similarity score, associated with any of the at least one pair of Type1 and Type2 devices associated with the respective particular Type1 device. The motion of the object may be monitored globally and asynchronously based on at least one of: heterogeneous similarity score, and at least one component similarity score, associated with any of the at least one pair of Type1 and Type2 devices.

The function may comprise at least one of: a representative value, a typical value, a weighted average, a percentile, a maximum, a minimum, a 10% quartile, a 20% quartile, a 30% quartile, a 40% quartile, a 50% quartile, a 60% quartile, a 70% quartile, a 80% quartile, a 90% quartile, a mean, a median, a mode, a sum, a product, a root, an arithmetic mean, a geometric mean, a harmonic mean, a generalized mean, an ordered statistics, a trimmed mean, a statistical function, an expected value, a variance, a thresholding, a counting, and/or a selected one.

The feature extraction may comprise at least one of: a sampled feature, a statistical feature, a time domain feature, a frequency domain feature, a decomposition, a singular value decomposition, a principal component analysis, an independent component analysis, a preprocessing, a signal processing, a postprocessing, a transformation, a linear processing, a nonlinear processing, a signal conditioning, a signal processing, a representative value, a typical value, a weighted average, a percentile, a maximum, a minimum, a 10% quartile, a 20% quartile, a 30% quartile, a 40% quartile, a 50% quartile, a 60% quartile, a 70% quartile, a 80% quartile, a 90% quartile, a mean, a median, a mode, a sum, a product, a root, an arithmetic mean, a geometric mean, a harmonic mean, a generalized mean, an ordered statistics, a trimmed mean, a statistical function, an expected value, a variance, a thresholding, a clustering, a training, a machine learning, a neural network, a deep learning, a counting, and/or a robust processing, etc.

For each Type2 device, and for each of the at least one respective particular pair of Type1 and Type2 devices comprising the Type2 device, for each of the at least one component, the motion of the object may be detected component-wise asynchronously when the respective component similarity score is at least one of: greater than, and equal to, a respective component threshold. The motion of the object in the venue may be detected individually and asynchronously when at least one of: a first function of heterogeneous similarity score associated with the pair of Type1 and Type2 devices comprising the particular Type2 device is at least one of: greater than, and equal to, a respective first individual threshold, and/or a first percentage of component-wise motion detection in a first selected group of components associated with the pair of Type1 and Type2 devices comprising the particular Type2 device is at least one of: greater than, and equal to, a respective second individual threshold. The motion of the object may be detected jointly and asynchronously when at least one of: a second function of heterogeneous similarity scores associated with any of the at least one pair of Type1 and Type2 devices comprising the particular Type2 device is at least one of: greater than, and equal to, a respective first joint threshold, and/or a second percentage of component-wise motion detection in a second selected group of components associated with any of the at least one pair of Type1 and Type2 devices comprising the particular Type2 device is at least one of: greater than, and equal to, a respective second joint threshold. The motion of the object may be detected jointly and asynchronously when at least one of: a third function of heterogeneous similarity scores associated with any of the at least one pair of Type1 and Type2 devices associated with the respective particular Type1 device is at least one of: greater than, and equal to, a respective third joint threshold, and/or a third percentage of component-wise motion detection in a third selected group of components associated with any of the at least one pair of Type1 and Type2 devices associated with the respective particular Type1 device is at least one of: greater than, and equal to, a respective fourth joint threshold. The motion of the object may be detected globally and asynchronously when at least one of: a fourth function of heterogeneous similarity scores associated with any of the at least one pair of Type1 and Type2 devices is at least one of: greater than, and equal to, a respective first global threshold, and/or a fourth percentage of component-wise motion detection in a fourth selected group of components associated with any of the at least one pair of Type1 and Type2 devices is at least one of: greater than, and equal to, a respective second global threshold.

The first function, second function, third function and/or fourth function may be a linear function, nonlinear function, an average, a weighted average, an arithmetic mean, a geometric mean, a harmonic mean, a generalized mean, a trimmed mean, a robust mean, a weighted mean, a median, a mode, and/or another function. Two or more of the first function, second function, third function and/or fourth function may be mathematically similar. The individual threshold and/or the joint threshold may be 0.1, 0.2, 0.3, 0.4, 0.5, 0.6, 0.7, 0.8, 0.9 and/or another threshold. It may be time varying. Two or more thresholds may be the same or different for some current time.

The motion of the object in the venue may be monitored asynchronously further based on another TSCI of another wireless multipath channel impacted by the motion of the object in the venue. The another TSCI may be extracted from another asynchronous heterogeneous wireless (or radio or RF) signal transmitted from an asynchronous heterogeneous Type 3 wireless device to an asynchronous heterogeneous Type 4 wireless device through the another wireless multipath channel. For example, Type1 and Type2 devices may be Wi-Fi devices, while Type 3 and Type 4 devices may be LTE, BLE, RFID or a another Wi-Fi devices. For example, Type1 and Type2 may be Wi-Fi at 2.4GHz while Type 3 and Type 4 devices may be Wi-Fi 5GHz.

The selected group of components (e.g. First, second, third, fourth selected group of components) may be a group of significant components, a group of insignificant components, important components, un-important components, improving components, decaying components, good components, bad components, components with certain behavior/-trend, opinion-influencing components, targeted components, components with significant energy, a group of decomposition components with significant energy, a group of components significant/useful/sensitive for the monitoring, a group of components learned in a training stage, a group of components selected in some ways. For example, the selected component may be sensitive/revealing/differentiating/discriminating to some targeted event to be monitored. Two or more of the first, second, third, fourth selected group of components may overlap, and/or be the same. For example, majority vote may be performed with the threshold being 0.5 (50%). The motion may be detected if the percentage is greater than 50%.

For each Type2 device, and for each of the at least one respective particular pair of Type1 and Type2 devices comprising the Type2 device, at least one respective heterogeneous motion information associated with the motion of the object may be computed asynchronously based on the at least one respective TSCI associated with the respective particular pair of Type1 and Type2 device.

A respective current window of the at least one respective TSCI associated with the respective particular pair of wireless devices with a respective past window of the at least one respective TSCI may be compared asynchronously. A respective heterogeneous similarity score between the respective current window and the respective past window of the at least one respective TSCI may be computed asynchronously.

The motion of the object in the venue may be monitored individually and asynchronously based on asynchronously computed heterogeneous motion information and heterogeneous similarity score associated with the pair of Type1 and Type2 devices comprising the particular Type2 device. The motion of the object may be monitored jointly and asynchronously based on asynchronously computed heterogeneous motion information and heterogeneous similarity score associated with any of the at least one pair of Type1 and Type2 devices associated with the particular Type2 device. The motion of the object may be monitored jointly and asynchronously based on asynchronously computed heterogeneous motion information and heterogeneous similarity score associated with any of the at least one pair of Type1 and Type2 devices associated with the respective particular Type1 device. The motion of the object may be monitored globally and asynchronously based on asynchronously computed heterogeneous motion information and heterogeneous similarity score associated with any of the at least one pair of Type1 and Type2 devices.

For example, the Type1 and Type2 devices may be Wi-Fi devices (with the wireless (or radio or RF) signal being a Wi-Fi signal), while the Type 3 and Type 4 devices may be LTE devices (with the another wireless (or radio or RF) signal being an LTE signal). In another example, the Type1 and Type2 devices may be Wi-Fi devices using a Wi-Fi network with a first SSID (e.g. "my home network"), while the Type 3 and Type 4 devices may be Wi-Fi devices using a Wi-Fi network with a second SSID (e.g. "Your Neighborhood"). In another example, the Type1, Type2, Type 3 and Type 4 devices may be in the same Wi-Fi network. The Type1 and Type2 devices may be using 20MHz bandwidth, while the Type 3 and Type4 devices may be using 40MHz bandwidth.

The Type1 device and the Type 3 device may be the same device, or different devices. A Type2 device and a Type 4 device may be the same device. Type1 devices may be Type 3 devices, and vice versa. Type2 devices may be Type 4 devices, and vice versa.

A map of the venue may be partitioned into more than one regions. The motion of the object may be associated asynchronously with at least one of the more than one regions of the map of the venue based on at least one of: (a) asynchronously computed heterogeneous motion information associated with the pair of Type1 and Type2 devices comprising the particular Type2 device, (b) asynchronously computed heterogeneous motion information associated with any of the at least one pair of Type1 and Type2 devices associated with the particular Type2 device, (c) asynchronously computed heterogeneous motion information associated with any of the at least one pair of Type1 and Type2 devices associated with the respective particular Type1 device, and (d) asynchronously computed heterogeneous motion information associated with any of the at least one pair of Type1 and Type2 devices.

The respective similarity score may be one of the at least one respective motion information. The respective current window may have same or different duration (or length) as the respective past window. Current windows associated with different Type2 devices may be different. Past windows associated with different Type2 devices may also be different. The current window and the past window may have same amount of CI, same time duration, same pre-processing, same processing, same post-processing, and/or same settings. The current window and the past window may have different amount of CI, different time duration, different pre-processing, different processing, different post-processing, and/or different settings.

For each respective pair of Type1 and Type2 devices, a respective feature space of the at least one respective heterogeneous motion information may be partitioned associated with the respective pair of Type1 and Type2 devices into more than one respective feature segments. A respective mapping that associates each respective feature segment with at least one of the more than one regions of the map of the venue may be constructed. The motion of the object may be associated asynchronously individually with at least one of the more than one regions of the map of the venue based on the respective mapping. A joint feature space of heterogeneous motion information associated with more than one pairs of Type1 and Type2 devices may be partitioned into more than one joint feature segments. A joint mapping that associates each joint feature segment with at least one of the more than one regions of the map of the venue may be constructed. The motion of the object may be associated with the particular one of the more than one regions of the map of the venue based on the joint mapping.

Two or more of the more than one regions may overlap. A region may be a subset of another region. A region may be a union of more than one other regions. A location of a region may be related to a location of one or more Type2 device. The map and/or the regions may be 1-dimensional, 2-dimensional, 3-dimensional or higher-dimensional. A 2D region may be a rectangular, square, circle, ellipse or other shapes. A region may be concave, or convex. Some regions may be large. Some may be small. The more than one regions may comprise a decomposition (e.g. multi-resolution decomposition) of the venue into disjoint regions or overlapping regions. The more than one regions may be regular or irregular.

For each respective pair of Type1 and Type2 devices, a respective heterogeneous similarity score between a respective current window and a respective past window of the at least one respective TSCI associated with the respective pair of Type1 and Type2 devices may be computed asynchronously.

The motion of the object in the venue may be detected individually and asynchronously when a first function of heterogeneous similarity score associated with the pair of Type1 and Type2 devices comprising the particular Type2 device is at least one of: greater than, and equal to, a respective individual threshold. The motion of the object in the venue may be detected jointly and asynchronously when a second function of heterogeneous similarity scores associated with any of the at least one pair of Type1 and Type2 devices comprising the particular Type2 device is at least one of: greater than, and equal to, a respective joint threshold. The motion of the object in the venue may be detected jointly and asynchronously when a third function of heterogeneous similarity scores associated with any of the at least one pair of Type1 and Type2 devices associated with the respective particular Type1 device is at least one of: greater than, and equal to, another respective joint threshold. The motion of the object in the venue may be detected globally and asynchronously when a fourth function of heterogeneous similarity scores associated with any of the at least one pair of Type1 and Type2 devices is at least one of: greater than, and equal to, a respective global threshold.

A feature may be a motion information, or a feature/characteristics/a function of one or more motion information. The features may be heterogeneous. For example, one feature may be a real number, another feature may be a boolean, another may be a complex number/vector, another may be a set, and yet another may be a collection of things. A feature may be obtained by applying an operation on one or more motion information. A feature space may be a space or a subspace. A feature space may be spanned by one or more of the features.

Some feature segments may be partitioned/bound/defined by hyperplanes and/or manifolds. Some feature segments may be partitioned/defined by scalar quantization and/or vector quantization. The feature space may be decomposed/divided into cells (e.g. "rectangular" cell of unity length in all dimensions). A feature segment may be a union of adjacent cells or non-adjacent cells.

The joint feature space may be a union of the respective feature space. The dimension of the feature space of different Type2 devices may be different. The respective mapping or the joint mapping may be one-to-one, many-to-one, one-to-many, or many-to-many. The mapping may be onto.

An information may be signaled, presented, displayed, played, transmitted, stored asynchronously by a monitoring device. The information may be associated with at least one of: the map of the venue, the more than one partitioned regions of the map, regions associated with any detected motion of the object, heterogeneous motion information associated with at least one pair of Type1 and Type2 devices, component similarity score, heterogeneous similarity score, past motion information, past similarity score, another past information, Type1 devices, Type2 devices, Type 3 devices, and/or Type 4 devices. The signaling may comprise analysis, decomposing, transforming, processing, filtering, ordering, arranging, formating, organizing, presenting, displaying, playing, transmitting, and/or storing.

In another embodiment, a wireless (or radio or RF) monitoring system comprises at least one asynchronous heterogeneous Type1 wireless (or radio) device and at least one asynchronous heterogeneous Type2 wireless (or radio or RF) device. The wireless (or radio or RF) monitoring system comprises at least one pair of Type1 and Type2 devices, each pair comprising one of the at least one Type1 device, and one of the at least one Type2 device.

A particular asynchronous heterogeneous Type2 device is in at least one particular pair of Type1 and Type2 devices of the wireless (or radio or RF) monitoring system. The particular Type2 device is associated with at least one particular Type1 device through the at least one particular pair of Type1 and Type2 devices.

The particular asynchronous heterogeneous Type2 device of the wireless (or radio) monitoring system comprises a wireless circuitry, a second heterogeneous processor, a second heterogeneous memory and a second heterogeneous set of instructions.

The wireless circuitry receives asynchronously at least one asynchronous heterogeneous wireless (or radio or RF) signal. Each asynchronous heterogeneous wireless (or radio or RF) signal is transmitted asynchronously by one of the at least one particular Type1 device using a respective first heterogeneous processor, a respective first heterogeneous memory and a respective first heterogeneous set of instructions of the Type1 device to at least one asynchronous heterogeneous Type2 device through a wireless multipath channel impacted by a motion of an object in a venue.

The second heterogeneous processor is communicatively coupled with the wireless circuitry. The second heterogeneous memory is communicative coupled with the second heterogeneous processor. The second heterogeneous set of instructions is stored in the second heterogeneous memory which, when executed, cause the second heterogeneous processor to, for each of the at least one particular pair of Type1 and Type2 devices, obtain asynchronously at least one respective series of CI (TSCI) of the wireless multipath channel. The at least one respective TSCI associated with the particular pair of Type1 and Type2 devices is being extracted asynchronously from the respective asynchronous heterogeneous wireless (or radio or RF) signal received asynchronously by the wireless circuitry.

One of the following is performed: (a) the motion of the object in the venue is monitored individually and asynchronously, using a third processor, a third memory and a third set of instructions of a Type 3 device, based on TSCI associated with a pair of Type1 and Type2 devices comprising the particular Type2 device; (b) the motion of the object is monitored jointly and asynchronously based on TSCI associated with any pairs of Type1 and Type2 devices comprising the particular Type2 device; (c) the motion of the object is monitored jointly and asynchronously based on TSCI associated with any pairs of Type1 and Type2 devices comprising one of the at least one particular Type1 device; and/or (d) the motion of the object is monitored globally and asynchronously based on TSCI associated with any pairs of Type1 and Type2 devices.

In another embodiment, a wireless (or radio or RF) monitoring system comprises at least one asynchronous heterogeneous Type1 wireless (or radio or RF) device and at least one asynchronous heterogeneous Type2 wireless (or radio or RF) device. The wireless (or radio) monitoring system comprises at least one pair of Type1 and Type2 devices, each pair comprising one of the at least one Type1 device, and one of the at least one Type2 device. The particular asynchronous heterogeneous Type1 device is in at least one particular pair of Type1 and Type2 devices of the wireless (or radio) monitoring system. The particular Type1 device is associated with at least one particular Type2 device through the at least one particular pair of Type1 and Type2 devices. A particular asynchronous heterogeneous Type1 device of a wireless (or radio) monitoring system comprises: a wireless circuitry, a first processor, a first memory and a first set of instructions. The first processor is communicatively coupled with the wireless circuitry. The first memory is communicative coupled with the first processor. The first set of instructions is stored in the first memory which, when executed, cause the first processor to transmit asynchronously using the wireless circuitry an asynchronous heterogeneous wireless (or radio or RF) signal from the particular Type1 device to the at least one particular Type2 device through a wireless multipath channel impacted by a motion of an object in a venue.

For each of the at least one particular Type2 device, at least one series of CI (TSCI) of the wireless multipath channel extracted from the asynchronous heterogeneous wireless (or radio or RF) signal transmitted by the wireless circuitry is obtained by the Type2 device using a second processor, a second memory and a second set of instructions of the Type2 device.

At least one of the following is performed: (a) the motion of the object in the venue is monitored individually and asynchronously, using a third processor, a third memory and a third set of instructions of a Type 3 device, based on TSCI associated with a pair of Type1 and Type2 devices comprising the particular Type1 device, (b) monitoring the motion of the object jointly and asynchronously based on TSCI associated with any pairs of Type1 and Type2 devices comprising the particular Type1 device, (c) monitoring the motion of the object jointly and asynchronously based on TSCI associated with any pairs of Type1 and Type2 devices comprising one of the at least one particular Type2 device, and (d) monitoring the motion of the object globally and asynchronously based on TSCI associated with any pairs of Type1 and Type2 devices.

In another embodiment, a wireless (or radio or RF) monitoring system comprises at least one asynchronous heterogeneous Type1 wireless (or radio or RF) device and at least one asynchronous heterogeneous Type2 wireless (or radio or RF) device. The wireless (or radio) monitoring system comprises at least one pair of Type1 and Type2 devices, each pair comprising one of the at least one asynchronous heterogeneous Type1 device, and one of the at least one asynchronous heterogeneous Type2 device. A Type 3 device of the wireless (or radio) monitoring system comprises a third processor, a third memory and a third set of instructions. The third processor is communicatively coupled with at least one of: at least one asynchronous heterogeneous Type1 device, and at least one asynchronous heterogeneous Type2 device. The third memory is communicative coupled with the third processor. The third set of instructions is stored in the third memory. When executed, the third set of instructions causes the third processor to, for each Type2 device, and for each of the at least one pair of Type1 and Type2 devices comprising the Type2 device, receive asynchronously at least one respective TSCI (TSCI) of a wireless multipath channel impacted by a motion of an object in a venue.

Each TSCI is obtained asynchronously by the Type2 device using a respective second processor, a respective second memory and a respective second set of instructions of the Type2 device. The at least one respective TSCI associated with the respective pair of Type1 and Type2 devices is extracted asynchronously from a respective asynchronous heterogeneous wireless (or radio or RF) signal. The respective asynchronous heterogeneous wireless (or radio or RF) signal is transmitted from a respective Type1 device of the respective pair using a respective first processor, a respective first memory and a respective first set of instructions of the respective Type1 device to at least one asynchronous heterogeneous Type2 device through the wireless multipath channel. When executed, the third set of instructions further causes the third processor to do at least one of the following: (a) monitor the motion of the object in the venue individually and asynchronously, based on TSCI associated with a particular pair of Type1 and Type2 devices comprising a particular Type2 device and a particular Type1 device, (b) monitor the motion of the object jointly and asynchronously based on TSCI associated with any of the at least one pair of Type1 and Type2 devices associated with the particular Type2 device, (c) monitor the motion of the object jointly and asynchronously based on TSCI associated with any of the at least one pair of Type1 and Type2 devices associated with the particular Type1 device, and/or (d) monitor the motion of the object globally and asynchronously based on TSCI associated with any of the at least one pair of Type1 and Type2 devices.

The present teaching discloses systems, apparatus, and methods for detecting and monitoring a periodic or pseudo-periodic object motion in a venue based on a TSCI of a wireless multipath channel that is impacted by the object motion. According to various embodiments, the object may be a life (e.g. a human, a pet, an animal, etc.), a device (e.g. a fan, a machine, etc.), or a land; the periodic or pseudo-periodic object motion may represent: breathing, heartbeat, a periodic hand gesture, a periodic gait, a rotation, a vibration, or an earthquake; and the disclosed system can monitor characteristics of the motion, e.g. a rate or frequency of the motion. In the present teaching, the term "pseudo-periodic motion" refers to a periodic motion with slight perturbation, such as a periodic motion with increasing or decreasing frequency, or a combination/sum of two periodic motions with different frequencies. For simplicity, the following description will focus on methods for detecting and monitoring pseudo-periodic motions, while the same disclosed methods can be applied to periodic motion detection and monitoring as well.

In one embodiment, a robust vital sign monitoring system is disclosed to analyze the tiny temporal variations in the CFRs. On each antenna link, the system transforms CFRs into CIRs and performs spectral analysis on the CIRs via fast Fourier transform (FFT). The spectrum of different antenna links are then fused into one averaged spectrum. Then, persistence-based peak detection is performed on the spectrum which finally leads to the repetition rate (e.g. breathing rate) estimation. Furthermore, FSM is introduced into the disclosed system such that the vital sign monitoring system would adopt different thresholds in different states, which significantly improves the robustness of vital sign monitoring. Experimental results validate that the system could perform well for single-person as well as multi-person breathing tracking.

In one embodiment, the disclosed system uses CSI from regular off-the-shelf WiFi chips which are affordable, widely available, standard compliant, interoperable, FCC approved, with quality control and technical support by reputable brand. These components (WiFi chips, modules, design tools, knowhow) are much more affordable, much more widely available and much richer than the expensive, untested, uncommon, dedicated, limited edition experimental hardware components of an existing system. The disclosed system has a large effective range, due to the large WiFi coverage. The disclosed system can work in both line-of-sight (LOS) and non-line-of-sight (NLOS) conditions. The disclosed system may also use CSI from chips associated with 4G, LTE, LTE-U, 5G and beyond.

The disclosed system includes features that are significantly more than an abstract idea. To measure human breathing remotely without wearables has been a dream for decades. The disclosed system solves the long-time problem using physical WiFi chips as sensors to monitor the environment with WiFi signals and the associated multipath patterns. The disclosed system has hardware components (e.g. Type1 device, Type2 device each with processor, memory, wireless transceivers, cloud server, etc.). The WiFi signals are electromagnetic (EM) waves in the air which are measurable and with deterministic structure and frequency characteristics. The system has matching software components (e.g. embedded software in Type1 device, in Type2 device, in servers, etc.). The software can interface with low-level WiFi chips and firmware to extract CSI. Experimental results show that the disclosed system can detect human breathing rate with very high accuracy.

In addition, sleep monitoring acts as a critical and challenging task that attracts increasing demands and interests. The present teaching discloses the model, design, and implementation of SMARS (Sleep Monitoring via Ambient Radio Signals), which is the first practical Sleep Monitoring system that exploits Ambient Radio Signals to recognize sleep stages and assess sleep quality. This will enable a future smart home that monitors daily sleep in a ubiquitous, non-invasive and contactless manner, without instrumenting the subject's body or the bed. Different from previous RF-based approaches, the present teaching devises a statistical model that accounts for all reflecting and scattering multipaths, allowing highly accurate and instantaneous breathing estimation with ever best performance achieved on commodity devices. On this basis, SMARS then recognizes different sleep stages, including wake, REM, and NREM, which is previously only possible with dedicated hardware. A real-time system is implemented on commercial WiFi chipsets and deployed in 6 homes with 6 participants, resulting in 32 nights of data in total. The results demonstrate that SMARS yields a median error of 0.47 bpm and a 95% error of only 2.92 bpm for breathing estimation, and detects breathing robustly even when a person is 10m away from the link, or behind a wall. SMARS achieves sleep staging accuracy of 85%, outperforming advanced solutions using contact sensor or radar. Furthermore, SMARS is evaluated upon a recently released dataset that measures 20 patients' overnight sleep, which confirms the performance. By achieving promising results with merely a single commodity RF link, SMARS will set the stage for a practical in-home sleep monitoring solution.

Sleep plays a vital role in an individual's health and well-being, both mentally and physically. It is well recognized that sleep quantity and quality is fundamentally related to health risks like cardiovascular decease, stroke, kidney failure, diabetes, and adverse mental conditions, etc. Unfortunately, in modern society, a number of people suffer from sleep disorders. As recently reported, 10% of the population suffers from chronic insomnia (which is even higher among elders), and 1/3 of Americans do not get sufficient sleep. Monitoring sleep emerges as an essential demand to help, manage, diagnose, and treat the growing group of sleep disorders as well as to keep regular tabs on personal health.

Sleep monitoring, however, is a challenging task that has drawn tremendous efforts for decades. Generally, it measures sleep time, recognizes different sleep stages, e.g., wake, REM (Rapid Eye Movement) and NREM (Non-REM), and accordingly assesses an individual's sleep quality. Various solutions have been proposed. The medical gold standard relies on Polysomnography (PSG), which monitors various physiological parameters such as brain activities, respirations, and body movements by a number of wired sensors attached to the patient. Albeit accurate and comprehensive, PSG is usually expensive and cumbersome with the invasive sensors that may cause sleep difficulties, limiting itself to clinical usage for confirmed patients. Other approaches including photoplethysmography (PPG) and actigraphy (ACT) require users to wear dedicated sensors during sleep. Ballistocardiogram (BCG) needs to instrument the mattress with an array of EMFi sensors to measure ballistic force. Despite of the costs, these approaches provide suitable solutions for those who need special cares but are less-than-ideal for the public. Recent efforts in mobile computing envision in-home sleep monitoring using smartphones and wearables. These methods, however, only provide coarse-grained, less accurate measurements and fail to monitor vital signs like respiratory rate. In addition, mobiles and wearables are undesirable for especially elders and those with dementia.

Different from prevailing solutions, the disclosed solution looks forward to a future smart home that monitors daily sleep in a ubiquitous, non-invasive, contactless, and accurate manner, without instrumenting the body or the bed. One can observe an opportunity towards such a system by two perspectives: 1) Clinical study has shown that physiological activity varies among different sleep stages. For example, breathing rate becomes irregular and fast since brain oxygen consumption increases during REM sleep, and is more stable and slower during NREM sleep, rendering the feasibility of sleep staging based on breathing monitoring. 2) Recent advances in wireless technology have demonstrated non-contact sensing of body motions in the environments. Chest and abdomen motions caused by breathing can alter radio signal propagations and thus modulate the received signals, from which it is then possible to decipher breathing. One can explore a synergy between the two perspectives, resulting in a system to leverage ambient radio signals (e.g., WiFi) to capture a person's breathing and motion during sleep and further monitor the sleep behaviors.

While early works have investigated the feasibility of RF-based breathing estimation and sleep monitoring, they either rely on specialized hardware like FMCW radar, or only work in controlled environments. Solutions based on dedicated radios are usually expensive and not ubiquitously applicable. Others using commodity devices typically require the user to lay still on a bed with radios exceedingly close to his/her chest and fail in presence of extraneous motions or in Non-Line-Of-Sight (NLOS) scenarios. In addition, none of them can identify different sleep stages due to their limited accuracy in breathing estimation. Such limitations prevent them from application for practical in-home sleep monitoring.

The present teaching disclose the model, design, and implementation of SMARS, the first practical Sleep Monitoring system that exploits commodity Ambient Radio Signals to recognize sleep stages and assess the otherwise elusive sleep quality. SMARS works in a non-obtrusive way without any body contact. All that a user needs to do is to set up one single link between two commodity radios by, e.g., simply placing a receiver if a wireless router is already installed inside the home. SMARS advances the literature by a novel statistical model that allows highly accurate and instantaneous breathing estimation. On this basis, SMARS is able to distinguish different sleep stages, which is previously only obtainable by expensive dedicated hardware. Specifically, SMARS excels in three unique aspects to deliver practical sleep monitoring. First, one can devise a statistical model on motion in Channel State Information (CSI) that leverages all reflection and scattering multipaths indoors. Existing works usually assume a geometrical model with a few multipaths and one major path reflected off the human body (e.g., using a 2-ray model developed for outdoor space). Under real-world indoor environments, however, there could be as many as hundreds of multipaths, and signals not only reflect but also scatter off a person's body and other objects in the space. As a consequence, previous approaches fail in NLOS environments and for minute motions due to the lack of a dominant reflection path. In contrast, the disclosed model investigates the statistical characteristics of motion in CSI without making such unrealistic assumptions, underlying robust detection of arbitrary motions including breathing.

Second, SMARS achieves accurate respiratory rate estimation instantaneously and robustly. Most of previous breathing estimation schemes assume constant breathing rate during a relatively large time window to gain sufficient frequency resolution, losing fine-grained breathing variations during sleep. In addition, minute breathing motions can be easily buried in CSI measurement noises, rendering existing philosophies effective only in extraordinary close proximity (typically within 2~3m) without any extraneous motions. To improve time resolution, SMARS exploits the time-domain Auto-Correlation Function (ACF) to estimate breathing rate, which can report real-time breathing rates as frequent as every one second and make it possible to capture instantaneous breathing rate changes. By using ACF, SMARS also circumvents the use of noisy phase and the usually handcrafted CSI denoising procedure. More importantly, by eliminating the frequency offsets and thus synchronizing breathing signal over different subcarriers, ACF allows us to perform Maximal Ratio Combining (MRC) to combine multiple subcarriers to combat measurement noises and maximize breathing signals in an optimal way. By doing so, one can push the limit of the breathing signal to noise ratio (SNR) and thus significantly increase the sensing sensitivity for larger coverage as well as weaker breathing. Specifically, SMARS can reliably detect breathing when a person is 10m away from the link, or behind a wall, which is even better than specialized low-power radars.

Finally, based on the extracted breathing rates and motion statistics during sleep, one can recognize different sleep stages (including wake, REM and NREM) and comprehensively assess the overall sleep quantity and quality. Based on in-depth understanding of the relationship between breathing rates and sleep stages, one can extract distinctive breathing features for classification for sleep staging. None of existing works using off-the-shelf devices can achieve the same goal of staging sleep.

A real-time system has been implemented on different commercial WiFi chipsets and its performance is evaluated through extensive experiments. The evaluation includes two parts: 1) One can deploy SMARS in 6 homes with 6 healthy subjects and collect 32 nights of data, 5 out of which have PSG data recorded by commercial devices. The results show that SMARS achieves great performance, with a median breathing estimation error of 0.47 breath per minute (bpm) and a 95%tile error of 2.92 bmp. Regarding sleep staging, SMARS produces a remarkable accuracy of 85.2%, while commercial solutions, e.g., EMFIT based on contact sensors and ResMed using radar, have accuracies of only 69.8% and 83.7% respectively. 2) One can further validate SMARS on a recently released dataset on RF-based respiration monitoring. The dataset collected 20 patients' overnight sleep for comparative evaluation of four state-of-the-art breathing monitoring systems, using clinically labeled PSG data as ground truths. All the four systems (based on ZigBee, Sub-RSS radio, UWB radar, and WiFi CSI, respectively) produce significant median errors of about 2~3 bpm and 95%tile errors of around or above 10 bpm. As comparison, SMARS achieves significant improvements by decreasing the median error to 0.66 bpm and the 95%tile error to 3.79 bpm. By achieving promising performance, SMARS can deliver clinically meaningful sleep monitoring for daily and regular use in practice and takes an important step towards a future smart home that monitors personal health everyday life.

In a nutshell, the core contribution here is SMARS, the first system that enables a smart home to stage an inhabitant's sleep using commodity off-the-shelf WiFi devices, by achieving highly accurate and instantaneous breathing estimation in the wild. SMARS also contributes the first statistical model for understanding and capturing motions in CSI, which will renovate various applications in wireless sensing.

FIG. 35 illustrates an exemplary network environment 3500 for vital sign detection and monitoring in a venue, according to one embodiment of the present teaching. As shown in FIG. 35, the exemplary network environment 3500 includes a transmitter 3510, an antenna 3512, a wireless channel 3530, an antenna 3522, and a receiver 3520. The antenna 3512 is electrically coupled to the transmitter 3510; the antenna 3522 is electrically coupled to the receiver 3520.

In one embodiment, the transmitter 3510 is located at a first position in a venue; while the receiver 3520 is located at a second position in the venue. The transmitter 3510 is configured for transmitting a wireless signal through the wireless channel 3530. The wireless channel 3530 in this example is a wireless multipath channel that is impacted by a pseudo-periodic motion of an object in the venue. According to various embodiments, the object may be a human (e.g. a baby 3542, or a patient 3546) or a pet (e.g. a puppy 3544). The receiver 3520 in this example receives the wireless signal through the wireless multipath channel 130 and obtains at least one TSCI of the wireless multipath channel based on the wireless signal. Because the motion of the object impacts the wireless multipath channel through which the wireless signal is transmitted, the CI 3525 extracted from the wireless signal includes information related to the object motion.

In various embodiments, the transmitter 3510 may be part of a Bot or a Typel device placed in a venue, while the receiver 3520 may be part of an Origin or a Type2 device placed in the venue. In various embodiments, the Bot and/or the Origin may include (not shown) multiple transmitters, multiple receivers, and/or multiple transceivers. In one embodiment, the antenna 3512 and/or the antenna 3522 is replaced with a multi-antenna array that can form a plurality of beams each of which points in a distinct direction. The transmitter 3510 can be configured to wirelessly transmit signals having different types or functions. Similarly, the receiver 3520 is configured to receive wireless signals having different types or functions. In one embodiment, the transmitter 3510 has at least one antenna; and the receiver 3520 has at least one antenna. Each of the at least one TSCI is associated with one of the at least one antenna of the transmitter 3510 and one of the at least one antenna of the receiver 3520.

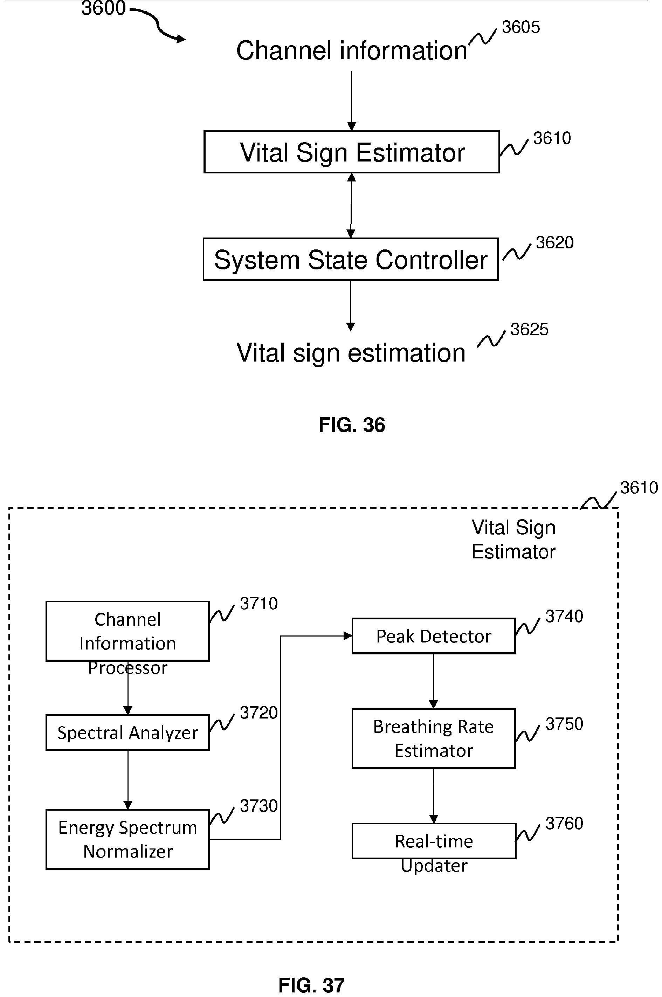

FIG. 36 illustrates an exemplary block diagram of a system 3600 for vital sign detection and monitoring, according to one embodiment of the present teaching. As shown in FIG. 36, the exemplary vital sign monitoring system 3600 comprises a vital sign estimator 3610 and a system state controller 3620. The vital sign estimator 3610 receives an input of a TSCI (TSCI) 3605, which may be the CI 3525 generated by the receiver 3520. According to various embodiments, the system can detect and monitor various periodic and pseudo-periodic motions, whether they represent vital signs or not.

In one embodiment, the system 3600 may comprise the transmitter 3510, the receiver 3520 and their respective antennas as well. The vital sign estimator may be coupled to at least one of: the transmitter 3510, the receiver 3520, an additional transmitter, an additional receiver, a cloud server, a fog server, a local server, and an edge server.

In one embodiment, the vital sign estimator 3610 is configured for determining that at least one portion of the TSCI 3605 in a current sliding time window is associated with a pseudo-periodic motion of the object (e.g. breathing of a person) in the venue, and computing a current characteristics (e.g. a repetition rate, a breathing rate) related to the pseudo-periodic motion of the object in the current sliding time window based on at least one of: the at least one portion of the TSCI 3605 in the current sliding time window, at least one portion of the at least one TSCI 3605 in a past sliding time window, and a past characteristics related to the pseudo-periodic motion of the object in the past sliding time window. Here, a "portion" of a TSCI may be those CI in the corresponding time window such as current time window, or past time window. The computation may be based on a comparison between a current sliding time window of the at least one portion of the at least one TSCI 3605 with a past sliding time window of the at least one portion of the at least one TSCI 3605, and maybe based on the past characteristics. A repetition rate (e.g. breathing rate) can be represented by the repetition frequency (e.g. breathing frequency), e.g. 20 bpm. It can also be a period corresponding to the breathing frequency, e.g. 3 seconds for 20 bpm (60/20=3).

In one embodiment, the vital sign estimator 3610 is further configured for making the current characteristics related to the pseudo-periodic motion of the object available in real time; and moving the current sliding time window by a shift-size as time progresses.

In one embodiment, the system also includes an additional transmitter located at a third position in the venue and configured for transmitting an additional wireless signal through the wireless multipath channel 130 impacted by the pseudo-periodic motion of the object in the venue; and an additional receiver located at a fourth position in the venue and configured for: receiving the additional wireless signal through the wireless multipath channel, and obtaining an additional TSCI of the wireless multipath channel 130 based on the additional wireless signal. In this embodiment, the vital sign estimator 3610 is further configured for: determining that at least one portion of the additional TSCI in the current sliding time window is associated with the pseudo-periodic motion of the object in the venue, and computing the current characteristics related to the pseudo-periodic motion of the object in the current sliding time window based on at least one of: the at least one portion of the additional TSCI in the current sliding time window, at least one portion of the additional TSCI in an additional past sliding time window, and a past characteristics related to the pseudo-periodic motion of the object in the additional past sliding time window. To implement this embodiment, a system may include more than one pair of Bot and Origin, each pair giving rise to independent CI. Repetition rate (e.g. breathing rate) may be computed individually based on the individual pair or jointly based on both pairs. Results of two different Bot-Origin pairs may be combined. Each pair may use different method to obtain the repetition rate (e.g. breathing rate). Thresholds may be different. The two pairs may have a common Bot, or a common Origin.

In another embodiment, two or more pseudo-periodic object motions are monitored at the same time. For example, the same current characteristics (e.g. repetition rate, breathing rate) of the two people are computed based on the same current sliding window of CI. In this case, the vital sign estimator 3610 is further configured for: determining that at least one portion of the at least one TSCI in the current sliding time window is associated with an additional pseudo-periodic motion of an additional object in the venue, wherein the wireless multipath channel is further impacted in the current sliding time window by the additional pseudo-periodic motion of the additional object; and computing a current characteristics related to the additional pseudo-periodic motion of the additional object in the current sliding time window based on at least one of: the at least one portion of the at least one TSCI in the current sliding time window, the at least one portion of the at least one TSCI in the past sliding time window, and a past characteristics related to the additional pseudo-periodic motion of the additional object in the past sliding time window. In one example, while the pseudo-periodic motion represents breathing, the additional pseudo-periodic motion represents heartbeat.

In another embodiment, the number of objects having pseudo-periodic motions is estimated. In this case, the vital sign estimator 3610 is further configured for: determining that the wireless multipath channel is impacted in the current sliding time window by pseudo-periodic motions of a plurality of objects; computing current characteristics related to pseudo-periodic motions of the plurality of objects in the current sliding time window based on at least one of: the at least one portion of the at least one TSCI in the current sliding time window, the at least one portion of the at least one TSCI in the past sliding time window, and a past characteristics related to the pseudo-periodic motions of the plurality of objects in the past sliding time window; and estimating a quantity of the plurality of objects based on the current characteristics.

The system state controller 3620 in this example may comprise a finite state machine (FSM) configured for tuning parameters (e.g. peak detection thresholds under different states) of the system 3600, for the system to output an accurate vital sign estimation 3625, which may include an estimate of the vital sign characteristics (e.g. the breathing rate) and/or the quantity of objects (e.g. persons that are breathing). In one embodiment, the system state controller 3620 is configured for computing adaptively at least one of: decision thresholds, threshold T1, threshold T2, lower bound associated with frequency of the selected significant local peaks, upper bound associated with frequency of the selected significant local peaks, search range, and a parameter associated with a selection of the selected significant local peaks, based on a finite state machine (FSM). The FSM may comprise at least one of: an Initiation (INIT) state, a Verification state, a PeakFound state, and a Motion state.

In one embodiment, the system state controller 3620 is further configured for: entering the INIT state of the FSM in at least one predetermined way; computing the thresholds adaptively in a first way in the INIT state; detecting an event based on the thresholds; transitioning from the INIT state to a different state of the FSM based on at least one transition criterion; and computing the thresholds adaptively in a second way in the different state. In another embodiment, the system state controller 3620 is further configured for: entering the INIT state of the FSM; computing the thresholds adaptively in a first way; detecting an event based on the thresholds and at least one of: the set of selected significant local peaks and related characteristics; detecting excessive background interfering motion based on the thresholds and the remaining spectral energy; staying in the INIT state when the event is concluded to be "not detected" and the excessive background interfering motion is concluded to be "not detected;" transitioning from the INIT state to the Verification state when the event is concluded to be "detected" preliminarily and the detected event needs to be verified; and transitioning from the INIT state to the Motion state when the excessive background interfering motion is concluded to be "detected."

In another embodiment, the system state controller 3620 is further configured for: in the Verification state: computing the thresholds adaptively in a second way; accumulating and computing at least one statistics based on sets of selected significant local peaks and related characteristics in at least one adjacent sliding time window for verification of the detected event that is detected preliminarily; staying in the Verification state while the at least one statistics is being accumulated until sufficient statistics is collected for the verification; verifying the preliminarily detected event based on the thresholds and the at least one statistics; transitioning from the Verification state to the PeakFound state when the preliminarily detected event is concluded as "verified;" transitioning from the Verification state to the INIT state when verification is concluded to be "not verified;" and staying in the Verification state when verification is not concluded.

In a different embodiment, the system state controller 3620 is further configured for: in the PeakFound state: computing the thresholds adaptively in a third way; detecting the verified event based on the thresholds and the at least one of: the set of selected significant local peaks and related characteristics; detecting the excessive background interfering motion based on the thresholds and the remaining spectral energy; staying in the PeakFound state when the verified event is concluded as "detected;" transitioning from the PeakFound state to the INIT state when the verified event is concluded as "not detected" for a number of time instances; and transitioning from the PeakFound state to the Motion state when the excessive background interfering motion is concluded as "detected" for a number of time instances.

In a different embodiment, the system state controller 3620 is further configured for: in the Motion state: computing the thresholds adaptively in a fourth way; detecting the excessive background interfering motion based on the thresholds and the remaining spectral energy; staying in the Motion state when the excessive background interfering motion is concluded as "detected;" and transitioning from the Motion state to the INIT state when the excessive background interfering motion is concluded as "not detected" for a number of time instances.

FIG. 37 illustrates an exemplary block diagram of a vital sign estimator, e.g. the vital sign estimator 3610 in FIG. 36, for vital sign detection and monitoring, according to one embodiment of the present teaching. As shown in FIG. 37, the vital sign estimator 3610 in this example includes a CI processor 3710, a spectral analyzer 3720, an energy spectrum normalizer 3730, a peak detector 3740, a breathing rate estimator 3750, and a real-time updater 3760.

In one embodiment, the CI processor 3710, the spectral analyzer 3720, and the energy spectrum normalizer 3730 are configured for processing the at least one TSCI in the current sliding time window on both time domain and frequency domain, while the peak detector 3740 is configured for detecting at least one peak in the frequency domain, such that the current characteristics related to the pseudo-periodic motion of the object is computed based on the at least one peak in the frequency domain.

In one embodiment, for each CI of a particular portion of the at least one TSCI in the current sliding time window, the CI processor 3710 in this example is configured for: obtaining N1 (an integer greater than one) frequency domain components of the CI; determining a timestamp associated with the CI; and preprocessing the N1 frequency domain components. The preprocessing may comprise: cleaning phases of the N1 frequency domain components, and normalizing the N1 frequency domain components such that the N1 frequency domain components have a unity total power. In this case, the current characteristics related to the pseudo-periodic motion of the object is computed based on the preprocessed N1 frequency domain components of the CI of the particular portion of the at least one TSCI in the current sliding time window.

In another embodiment, for each CI of a particular portion of the at least one TSCI in the current sliding time window, the spectral analyzer 3720 in this example is configured for: converting N1 frequency domain components of the CI using an inverse frequency transform to N2 time domain coefficients of the CI, and retaining first C of the N2 time domain coefficients, wherein C is not larger than N2. Each CI is associated with a timestamp. N2 is not smaller than N1. Each of the N2 coefficients is associated with a time delay. The inverse frequency transform comprises at least one of: inverse Fourier transform, inverse Laplace transform, inverse Hadamard transform, inverse Hilbert transform, inverse sine transform, inverse cosine transform, inverse triangular transform, inverse wavelet transform, inverse integer transform, inverse power-of-2 transform, combined zero padding and transform, and inverse Fourier transform with zero padding. The spectral analyzer 3720 in this example may be configured for correcting timestamps of all CI of the particular portion of the at least one TSCI in the current sliding time window so that the corrected timestamps of time-corrected CI are uniformly spaced in time. The correcting the timestamps may comprise: identifying a particular CI with a particular timestamp to be replaced by a time-corrected CI with a corrected timestamp, and computing the time-corrected CI by computing the C retained time domain coefficients of the time-corrected CI at the corrected timestamp using an interpolation filter associated with the corrected timestamp. In this case, the current characteristics related to the pseudo-periodic motion of the object is computed based on the time-corrected CI of the particular portion of the at least one TSCI in the current sliding time window.

The spectral analyzer 3720 may be further configured for: for each of the C retained time domain coefficients: identifying a corresponding retained time domain coefficient of each CI of the particular portion of the at least one TSCI in the current sliding time window, applying bandpass filtering to all the corresponding identified retained time domain coefficients, and applying a frequency transform to all the corresponding identified retained time domain coefficients. A length of the frequency transform is not smaller than a length of the portion. In this case, the current characteristics related to the pseudo-periodic motion of the object is computed based on outputs of the frequency transform applied to each corresponding retained time domain coefficient of the CI of the particular portion of the at least one TSCI in the current sliding time window.

The spectral analyzer 3720 may be further configured for: processing the outputs of the frequency transform applied to each corresponding retained time domain coefficient of the CI of the particular portion of the at least one TSCI in the current sliding time window. In one embodiment, the spectral analyzer 3720 in this example is configured for: determining a timestamp associated with each CI of the at least one TSCI; correcting timestamps of all CI of a particular portion of the at least one TSCI in the current sliding time window so that the corrected timestamps of time-corrected CI are uniformly spaced in time; and performing an operation on the time-corrected CI with respect to the corrected timestamps. In this case, the current characteristics related to the pseudo-periodic motion of the object is computed based on an output of the operation. The energy spectrum normalizer 3730 in this example may be configured for: processing the outputs of the frequency transform applied to each corresponding retained time domain coefficient of the CI of the particular portion of the at least one TSCI in the current sliding time window, wherein the processing comprises at least one of: averaging, weighted averaging, averaging over selected frequency, averaging over selected time domain coefficients, and averaging over antenna links. The averaging over antenna links may be weighted averaging over the at least one TSCI. The averaging over selected time domain coefficients may be weighted averaging over the C retained time domain coefficients.

The peak detector 3740 in this example is configured for: identifying at least one local maximum and at least one local minimum in the frequency domain; computing at least one local signal-to-noise-ratio-like (SNR-like) parameter for each pair of a local maximum and a local minimum adjacent to each other; and identifying significant local peaks each being at least one of: a local maximum with SNR-like parameter greater than a first threshold T1 and a local maximum with an amplitude greater than a threshold T2. The at least one local maximum and the at least one local minimum may be identified in the frequency domain using a persistence-based approach.

The breathing rate estimator 3750 in this example is configured for: selecting a set of selected significant local peaks from the set of identified significant local peaks based on a selection criterion. In this case, the current characteristics related to the pseudo-periodic motion of the object is computed based on the set of selected significant local peaks and frequency values associated with the set of selected significant local peaks. The breathing rate estimator 3750 may be further configured for: computing information associated with a remaining spectrum with the set of selected significant local peaks removed; and detecting an event associated with the current characteristics related to the pseudo-periodic motion of the object based on at least one of: a remaining spectral energy of the remaining spectrum, an adaptive threshold, and a finite state machine, where the event comprises at least one of: a presence, an absence, an appearance, a disappearance, a steady behavior and a non-steady behavior of at least one of: non-periodic motion, transient motion, strong motion, weak motion, strong background interference and another periodic motion. The real-time updater 3760 in this example is configured for updating the breathing rate estimations generated by the breathing rate estimator 3750 regularly.

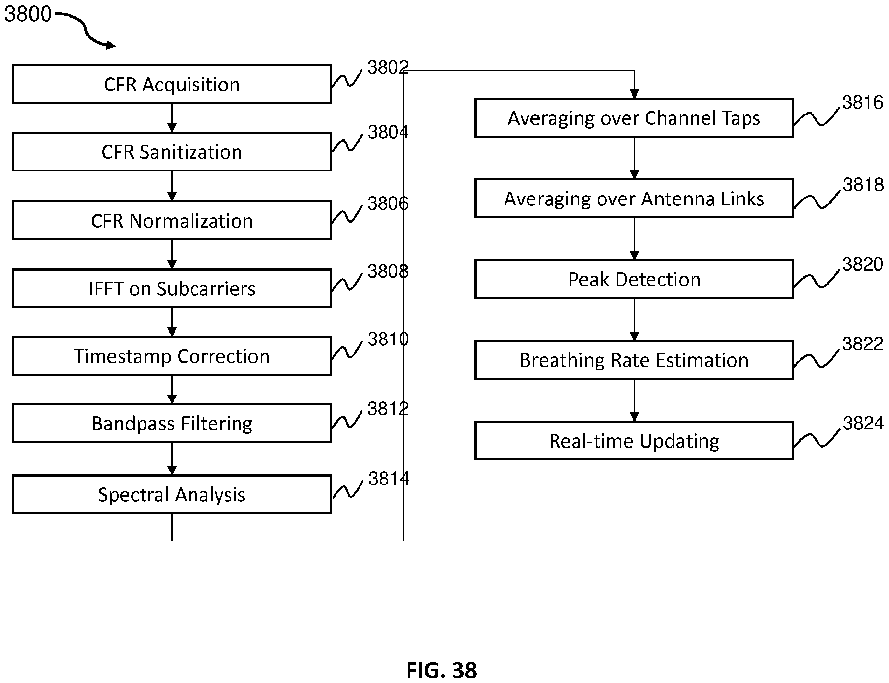

FIG. 38 illustrates a flowchart of an exemplary method 3800 for vital sign detection and monitoring, e.g. for breathing detection and breathing rate estimation, according to one embodiment of the present teaching. At operation 3802, a channel frequency response (CFR) acquisition is performed, e.g. by the CI processor 3710 in FIG. 37. In one embodiment, the CI processor 3710 acquires CFRs from off-the-shelf WiFi chips. For example, one can obtain 114 subcarriers in a CFR frame on a 5GHz WiFi channel with a maximum of 20 dBm transmission power. At operation 3804, CFR sanitization is performed, e.g. by the CI processor 3710 in FIG. 37. In one embodiment, due to the inevitable phase misalignment between the WiFi transmitter and receiver, there exist severe phase distortions in the CFRs. To remedy this issue, one can clean the CFR phase by the conventional linear regression method. One can write the cleaned CFR on subcarrier $k$ as $G[k]$. At operation 3806, a CFR normalization is performed, e.g. by the CI processor 3710 in FIG. 37. In one embodiment, the receiving power of the CFRs is time-varying. Unfortunately, in the absence of automatic-gain-control (AGC) values on the CFR chips adopted, the receiving power is unmeasurable. Therefore, one can normalize the CFRs across all subcarriers such that each CFR has unit power. Although this step incurs information loss, it still leads to much-improved performance. Here, one can write the CFRs captured on different subcarriers and different time instances as a CFR matrix G with dimension $K \times N$ where $N$ is the number of CFR frames collected. The matrix is given as

$$\mathbf{G} = \begin{bmatrix} G_{s_0}[t_0] & G_{s_0}[t_1] & \cdots & G_{s_0}[t_{N-1}] \\ G_{s_1}[t_0] & G_{s_1}[t_1] & \cdots & G_{s_1}[t_{N-1}] \\ \vdots & \vdots & \vdots & \vdots \\ G_{s_{K-1}}[t_0] & G_{s_{K-1}}[t_1] & \cdots & G_{s_{K-1}}[t_{N-1}] \end{bmatrix} \tag{13}$$

where $t_i$ stands for the time instance where the i-th CFR is captured and $s_j$ stands for the subcarrier index of the j-th subcarrier.

At operation 3808, an inverse fast Fourier transform (IFFT) is performed, e.g. by the spectral analyzer 3720 in FIG. 37. In one embodiment, one can convert the filtered CFRs into time-domain CIRs for de-noising using IFFT. The reason why IFFT could improve the performance lies in that: the useful signal contained in multipath components (MPC)s with a large time delay is less significant than the MPCs with a smaller time delay mainly due to the significant attenuations relevant to the propagation distance of EM waves. By using IFFT, one could focus on the first few channel taps and ignore channel taps with a large time delay. The CIR of time $t_i$ takes the form

$$\underline{G}_n[t_i] = \sum_{k=s_0}^{s_{K-1}-1} G_{s_k}[t_i] e^{j2\pi\frac{nk}{K'}}, n = 0,1,2,\cdots,K'-1 \tag{14}$$

where K' is the size of IFFT given as $2^{\text{ceil}[\log_2(K)]}$, where ceil() means ceiling operation.

At operation 3810, a timestamp correction is performed, e.g. by the spectral analyzer 3720 in FIG. 37. In one embodiment, the time instances $t_i$ can be written as $(i - 1)T_S$ where $T_S$ is the CFR sampling interval. Nevertheless, in practice, other wireless systems might co-exist with the breathing tracking system. Due to the Carrier-sense multiple access with collision avoidance (CSMA/CA) mechanism, the transmission of WiFi packets are not uniformly scheduled over the air. More specifically, the WiFi devices would probe the medium before transmission. In case that some other WiFi devices are transmitting packets, the other WiFi networks in the same area must remain silent, back-off for a period of time, and probe the medium again to check if there exists on-going WiFi traffic. Therefore, one can no longer assume that $t_i = (i - 1)T_S$.

To resolve this problem, one can turn to the interpolation technique. One can define the exact time instances to be interpolated as $[t_0, t_{N-1}]$ with a unit step size of $T_S$. Then, one can perform a linear interpolation onto the exact time instances based on the actual timestamps $t_0, t_1, t_2, \cdots, t_{N-1}$. This leads to the temporal corrected CIRs which can be written as

$$\overline{\mathbf{G}} = \begin{bmatrix} \overline{G}_0[t_0] & \overline{G}_0[t_1] & \cdots & \overline{G}_0[t_{N-1}] \\ \overline{G}_1[t_0] & \overline{G}_1[t_1] & \cdots & \overline{G}_1[t_{N-1}] \\ \vdots & \vdots & \vdots & \vdots \\ \overline{G}_{K\prime-1}[t_0] & \overline{G}_{K\prime-1}[t_1] & \cdots & \overline{G}_{K\prime-1}[t_{N-1}] \end{bmatrix} \tag{15}$$

At operation 3812, a bandpass filtering is performed, e.g. by the spectral analyzer 3720 in FIG. 37. In one embodiment, the CFRs can be analyzed using standard spectral analysis tools. The breathing rate of an average adult at rest ranges from 12 to 18 BPM. On the other hand, the breathing rate of an infant can be as high as 40 BPM, while the breathing rate of elderly people ranges from 10 to 30 BPM. Therefore, one can use bandpass filtering to eliminate the high-frequency and low-frequency noise and makes the breathing signal much more stable over time. This lays the foundation of the spectral analysis via FFT as the next step. For example, one can set the passband of the bandpass filter as 8 BPM to 42 BPM, which translates into 0.133 to 0.7 Hz. One can adopt a 81 taps finite-impulse-response (FIR) filter, with coefficients generated by MATLAB filter toolbox. The attenuation is -32 dB for first stopband ([0,8] BPM) and -40 dB for the right stopband ([42,300] BPM) (With a sounding rate of 10 Hz, the maximum resolvable frequency is 5 Hz which translates into 300 BPM). Denoting the filter coefficients as $h_0, h_1, \cdots, h_{H-1}$, the filtered CFR matrix can be written as

$$\tilde{\mathbf{G}} = \begin{bmatrix} \tilde{G}_0[t'_0] & \tilde{G}_0[t'_1] & \cdots & \tilde{G}_0[t'_{N\prime-1}] \\ \tilde{G}_1[t'_0] & \tilde{G}_1[t'_1] & \cdots & \tilde{G}_1[t'_{N\prime-1}] \\ \vdots & \vdots & \vdots & \vdots \\ \tilde{G}_{K\prime-1}[t'_0] & \tilde{G}_{K\prime-1}[t'_1] & \cdots & \tilde{G}_{K\prime-1}[t'_{N\prime-1}] \end{bmatrix} \tag{16}$$

where $t'_i = i \times T_S$, $N'$ is the length of one CFR frame on a given subcarrier expressed as $N - H + 1$, and $\tilde{G}_k[t'_j]$ represents the filtered CIR on the k-th tap, given as

$$\tilde{G}_k[t'_j] = \sum_{n=0}^{H-1} h[n]\tilde{G}_k[t_{j-n}], j \geq H - 1 \tag{17}$$

At operation 3814, a spectral analysis via fast Fourier Transform (FFT) is performed, e.g. by the spectral analyzer 3720 in FIG. 37. In one embodiment, one can perform FFT on the CIRs for each channel tap, which leads to $K'$ spectrum estimations. The energy spectrum for the k-th tap on the u-th frequency bin is given as

$$F_k[u] = \left| \sum_{i=0}^{N\prime\prime-1} \tilde{G}_n[t'_i] e^{-j2\pi \frac{iu}{N\prime\prime}} \right|^2, u = -N''/2, -N''/2 + 1, \cdots, N''/2 - 1. \tag{18}$$

where $N''$ is the size of FFT given as $2^{\text{ceil}[\log_2(N')]}$, and the spectral resolution is thus $\Delta f = \frac{1}{N\prime\prime \times T_s}$. Given that the spectrum range-of-interest is $[f_1, f_2]$ (human breathing rates are confined in a small range), the range of frequency bins of interest can be written as $[u_1 = \text{ceil}[f_1 \times N'' \times T_S], u_2 = \text{floor}[f_2 \times N'' \times T_S]]$. Further taking the harmonics of breathing signal into consideration, one can extend the frequency bin range into $[-u_2, -u_1] \cup [u_1, u_2]$. Then, one can perform a spectrum folding which leads to the folded energy spectrum given as

$$\overline{F}_k[u] = \frac{1}{2}[F_k[u] + F_k[-u]], u \in \{u_1, u_1 + \Delta f, \cdots, u_2 - \Delta f, u_2\}. \tag{19}$$

Assembling the spectrum on all subcarriers together leads to the matrix F:

$$\mathbf{F} = \begin{bmatrix} \overline{F}_{-K\prime/2}[u_1] & \overline{F}_{-K\prime/2}[u_1 + \Delta f] & \cdots & \overline{F}_{-K\prime/2}[u_2] \\ \overline{F}_{-K\prime/2+1}[u_1] & \overline{F}_{-K\prime/2+1}[u_1 + \Delta f] & \cdots & \overline{F}_{-K\prime/2+1}[u_2] \\ \cdots & \cdots & \cdots & \cdots \\ \overline{F}_{K\prime/2-1}[u_1] & \overline{F}_{K\prime/2-1}[u_1 + \Delta f] & \cdots & \overline{F}_{K\prime/2-1}[u_2] \\ \overline{F}_{K\prime/2}[u_1] & \overline{F}_{K\prime/2}[u_1 + \Delta f] & \cdots & \overline{F}_{K\prime/2}[u_2] \end{bmatrix} \tag{20}$$

At operation 3816, an averaging over channel taps is performed, e.g. by the energy spectrum normalizer 3730 in FIG. 37. In one embodiment, since the energy of the breathing signal concentrates in the first few CIR taps, one can take an average over channel taps with index falling in the range of $[0, S - 1]$, where S is termed as the stripe width. The averaged energy spectrum is then given as

$$\underline{F}[u] = \frac{1}{S}\sum_{k=0}^{S} \overline{F}_k[u], u \in \{u_1, u_1 + \Delta f, \cdots, u_2 - \Delta f, u_2\}. \tag{21}$$

At operation 3818, an averaging over antenna links is performed, e.g. by the energy spectrum normalizer 3730 in FIG. 37. In one embodiment, the disclosed system is a MIMO (multiple input multiple output) system. As such, there always exist multiple antennas on either end or both ends of the transmitter and receiver which gives rise to diversity in wireless communication. Here, one may also incorporate multiple antenna links in MIMO systems to improve the overall performance of breathing tracking. For each antenna link $m$ out of a total of M links, one can calculate the folded energy spectrum as shown in (19) denoted as $\underline{F}_m[u]$, followed by computing the averaged energy spectrum over the available antenna links given as

$$\dot{F}[u] = \frac{1}{M}\sum_{m=1}^{M} \underline{F}_m[u], u \in \{u_1, u_2\}. \tag{22}$$

For the convenience of further processing, one can transform the linear scale energy spectrum into its dB scale counterpart as

$$\dot{F}_{dB}[u] = 10\log_{10}\dot{F}[u] \tag{23}$$

At operation 3820, a peak detection is performed, e.g. by the peak detector 3740 in FIG. 37. In one embodiment, the locations of the peaks in the energy spectrum $\dot{F}_{dB}[u]$ indicate the estimated breathing rates. To extract these peaks, one may leverage the persistence-based approach to obtain multiple pairs of local maximals and local minimals. For the i-th pair of local maximum $p_{max}[i]$ and minimum $p_{min}[i]$, one may evaluate their difference as $v_i = p_{max}[i] - p_{min}[i]$, which can be regarded as the signal-to-noise ratio (SNR) for the i-th peak. Here, assuming a total of U detected peaks in the energy spectrum, one may use both the peak amplitude $\{p_{max}[i]\}_{i=1,2,\cdots,U}$ and the local peak SNR $\{v[i]\}_{i=1,2,\cdots,U}$ as features for further peak filtering. In particular, one may keep those peaks with amplitudes and SNRs larger than two thresholds, namely, the amplitude threshold $p_{max}^0$ and SNR threshold $v^0$, respectively.

At operation 3822, a breathing rate estimation is performed, e.g. by the breathing rate estimator 3750 in FIG. 37. In one embodiment, assuming a total of U' peaks remaining after the previous step and P breathing rates to be detected, one can pick the top $P$ out of $U'$ peaks and use the corresponding peak locations $\{\hat{f}_i\}_{i=1,2,\cdots,U}$, measured in Hz as the breathing rate estimations for $P$ people (When $P \geq U'$, then one can suffer from a miss detection rate of $\frac{P-U'}{P}$. On the other hand, when $P < U'$, one can suffer from a false alarm rate of $\frac{U'-P}{U'}$). Meanwhile, one can record the spectrum energy without the peaks, denoted as $\overline{F}$ given as

$$\overline{F}_{dB} = 10\log_{10}\left(\sum_{u=u_1}^{u_2} 10^{\dot{F}_{dB}[u]} - \sum_{i=1}^{P} 10^{p_{max}[i]}\right). \tag{24}$$

The metric $\overline{F}_{dB}$ is utilized to detect breathing in the finite state machine presented in the next. The breathing rate estimations with BPM as the unit are then given as $\{\hat{b}_i\}_{i=1,2,\cdots,U}$, with $\hat{b}_i = 60\hat{f}_i$.

At operation 3824, a real-time updating of the breathing rate estimation is performed, e.g. by the real-time updater 3760 in FIG. 37. In one embodiment, the disclosed breathing monitoring system keeps updating the breathing rate estimations regularly. Here, one can define another two parameters: window size $C_{window}$ and shift size $C_{shift}$, both measured in the number of samples. The first set of breathing rate estimations are generated by performing spectral analysis on CFRs with sample index [0, $C_{window}$ - 1], and the second set of breathing rate estimations are generated by the same procedure on CFRs with sample index [$C_{shift}$, $C_{window}$ + $C_{shift}$ - 1]; the overlap between the two adjacent windows is then given as $C_{window}$ - $C_{shift}$. A smaller $C_{shift}$ leads to a prompter real-time updating rate.

In a realistic environmental setting, there always exist motions from other people and/or objects, which introduces motion interference to the breathing tracking system. At the same time, motions of the subject under monitoring also inject interference. Both types of interferences would significantly deteriorate the performance.

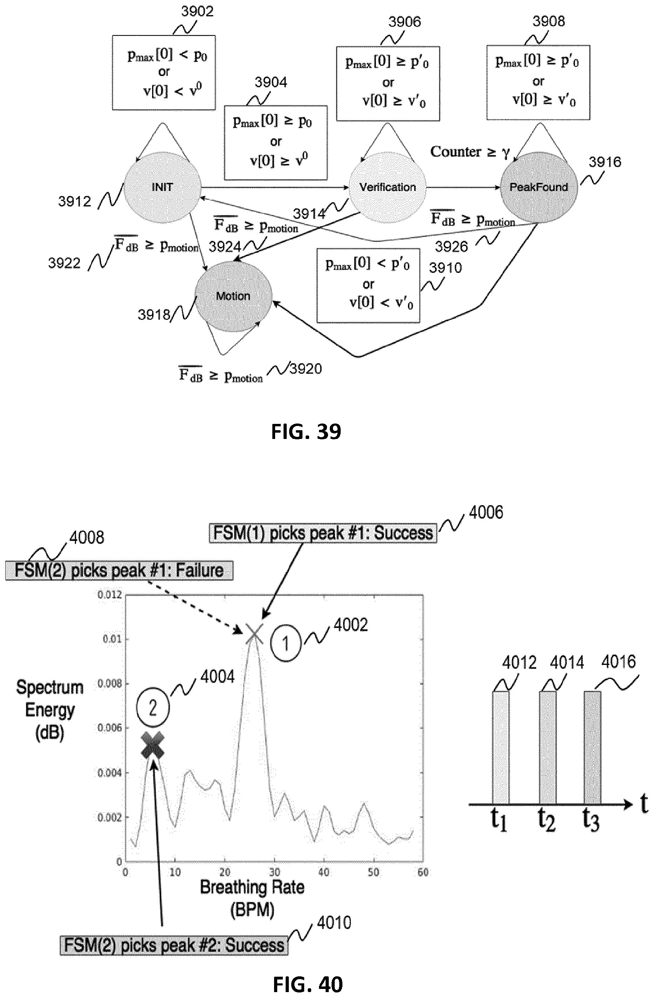

In light of this issue, one can supplement the system with a finite-state-machine (FSM), e.g. in the system state controller 3620, composed of several states into the breathing monitoring system so that the system could automatically tune its parameters such as peak detection thresholds under different states. In one embodiment, there may be four different states in the FSM which are illustrated below and as shown in FIG. 39. For convenience, the transitions are shown under the single-person breathing monitoring case. One can denote the peak search range as [$u_1$, $u_2$].

First, the FSM may have an INIT state 3912. This is the default state when the system is powered on. In this state, the system uses the default peak detection thresholds ( $p_{\max}^0$ , $v^0$). If for a specific CFR time window w, it detects that $p_{\max}$ [0] $\geq p^0$, $v$[0] $\geq v^0$, the system would tune the peak detection thresholds as ( $p'^0 = p_{\max}^0 - \Delta p^0, v'^0 = v^0 - \Delta v^0$ ). One can introduce $\Delta p^0$ and $\Delta v^0$ to leave margins for ensuing peak detections. Then, the FSM switches to state Verification. On the other hand, if $\overline{F}_{dB} \geq p_{motion}$ where $p_{motion}$ is the threshold for motion detection, the FSM switches to the state Motion to indicate that the current time window suffers from the major motion interferences. Otherwise, it stays in the current state. The output of the INIT state is a $P \times 1$ all-zero vector implying that no breathing rate estimations can be formulated. Also, in this state, the system restores the peak search range back to [$u_1$, $u_2$].

Second, the FSM may have a Verification state 3914. To avoid detection of fake peaks, one can use an internal counter to record how many times in consecutive that a peak can be detected. If a peak can be detected for a consecutive of y time windows, then the system switches its state into state PeakFound. In case that the peak becomes weaker, the FSM switches to state INIT. When $\overline{F}_{dB}$ exceeds $p_{motion}$, the FSM switches to the motion state. Otherwise, the system stays in the current state. The output of this state is the $P \times 1$ breathing rate estimations. In this state, the system would zoom into a neighboring region of the estimated breathing rate. For instance, given an estimation of $\hat{b}$ with respect to a peak location at $\hat{f}$ = $\hat{b}$/60, the system would select [$u'_1$,$u'_2$] where $u'_1 = \max[u_1, \hat{f} - \frac{\Delta S}{2} \times N''T_s]$ and $u'_2 = \min[u_2, \hat{f} + \frac{\Delta S}{2} \times N''T_s]$ are the starting and ending index of the updated search range. Here, $\Delta S$ stands for the size of the search range.

Third, the FSM may have a PeakFound state 3916. Now, the system determines that an authentic peak is detected and can be used as the breathing rate estimation. To prevent potential error propagation, the system no longer updates the two thresholds as shown in the Verification state. In case that the peak becomes weaker, the system switches back to the INIT state. Likewise, the system would switch to the motion state for sufficiently large $\overline{F}_{dB}$. The output of this state is the $P \times 1$ breathing rate estimations. Also, the search range is updated based on the breathing rate estimations.

Fourth, the FSM may have a Motion state 3918. In this state, the system decides that no credible breathing rate estimation can be produced due to the presence of strong motions. It can switch to the INIT state when $\overline{F}_{dB}$ falls below $p_{motion}$. The output of this state is the $P \times 1$ breathing rate estimations formulated in the latest Verification or PeakFound state. The idea of using FSM to overcome the motion interference issue under the single-person case can be extended to the multi-person case by running multiple FSMs in parallel, with each FSM captures the status of one specific person. The FSMs associated with different people interact with each other, i.e., they are not independent. To make the FSMs more predictable, one can define the following rules, as shown in FIG. 40.

Rule 1: FSMs operating first would always select the strongest peak in its frequency range. For example, the i-th FSM, denoted as *FSM(i)*, selects the strongest peak in its search range [$u'_{1,i}$,$u'_{2,i}$].

Rule 2: To avoid duplication of breathing rate estimations, no two FSMs can pick the same peak in each time window. For example, if *FSM(i)* selects peak at location $\hat{f}_i$, then *FSM(j)*, *j > i* would look for other peaks not located at $\hat{f}_i$. This rule may not handle the following issue: two persons breathing with different breathing rates. The breathing rate of one person increases and the other decreases. Sooner or later, these two breathing rates would meet each other and the disclosed FSM cannot get the breathing rate of the second person (the rendezvous problem).

Rule 3: To circumvent the situation of selecting peaks encapsulating the harmonics of breathing, *FSM(i)* would select the peak in its search range first denoted as $\hat{f}_i$. Then, *FSM(i)* checks the peaks selected by *FSM(j)*, *j* = 1,2, $\cdots$ , *i* - 1 to see if there exists a selected peak $\hat{f}_j$ such that |2$\hat{f}_i$ - $\hat{f}_j$| < $\varepsilon_{harmonic}$ or |$\hat{f}_i$ - 2$\hat{f}_j$| < $\varepsilon_{harmonic}$ where $\varepsilon_{harmonic}$ is a predetermined threshold. If at least one such peak can be found, then *FSM(i)* continues to select the next significant peak in its search range. Otherwise, *FSM(i)* uses $\hat{f}_i$ as the detected peak, and the same procedure is repeated for the other FSMs sequentially.

FIG. 40 visualizes an example of two FSMs selecting two peaks sequentially. One can assume that both peaks satisfy the criterion of thresholds. At time $t_1$ 4002, FSM 1 searches the energy spectrum and picks the strongest peak denoted as peak 14002, 4006. Then, *FSM(2)* comes in and attempts to select peak 1 4002, 4008. However, since the first peak is already taken by *FSM(1), FSM(2)* gives up its selection and moves on to peak 2 4004, 4010.

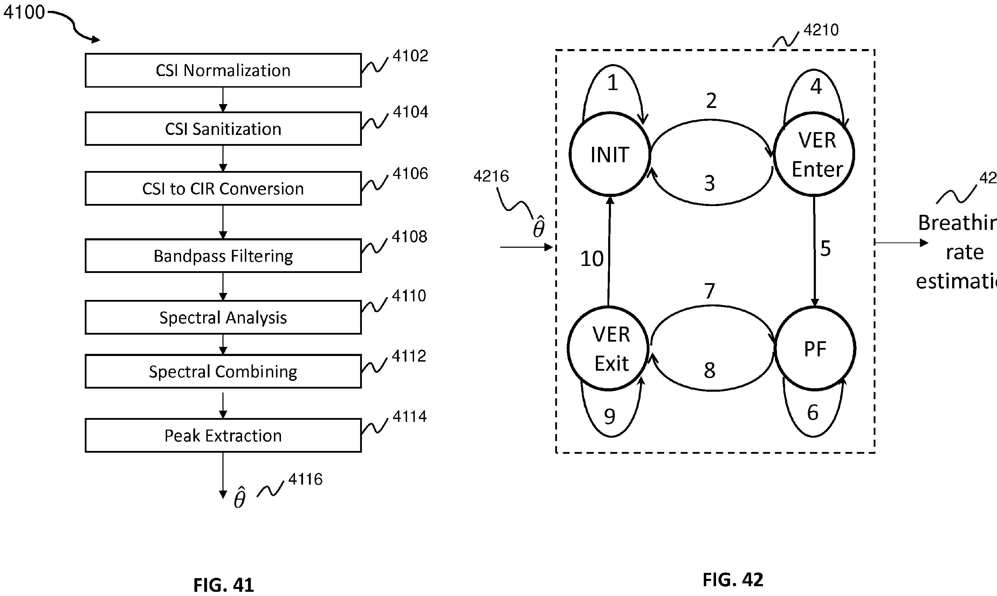

In addition to the embodiments of the peak detection and FSM disclosed above, FIG. 41 illustrates a flowchart of an exemplary method 4100 for vital sign estimation, according to another embodiment of the present teaching. As shown in FIG. 41, there may be no threshold when selecting the peaks. As discussed above, the vital sign estimator 3610 is a core part of the breathing monitoring system. The vital sign estimator 3610 may perform channel state information (CSI) normalization 4102, CSI sanitization 4104, frequency-domain CSI to time-domain channel impulse response (CIR) conversion 4106, bandpass filtering 4108, spectrum analysis 4110, spectrum combining (averaging over tap and/or antenna links) 4112, and peak extraction 4114. Each time, one may feed N CSIs collected at N sample epochs in the vital sign estimator 3610, with N being the CSI block size. Denoting $G'^i_{comb}$ as the combined spectrum after averaging or weighted averaging for CSI block I, any proper peak detector may be applied on $G'^i_{comb}$ to find several local maxima 4116 denoted as $\hat{\theta}_\tau$ = {$\hat{b}_\tau$}$_{i=0,1,...,P-1}$ where $b_\tau$ stands for the breathing rate with respect to the peaks and P is the number of peaks. The process may be repeated for each CSI block i.

FIG. 42 illustrates an example of FSM for single-person monitoring, according to one embodiment of the present teaching. As shown in FIG. 42, the detected peak $\theta_{\hat{\tau}}$ 4116 is forwarded to the control engine sequentially for $i = 0, 1, 2, \cdots, I$ -1, to output the breathing rate estimation 4212. The control engine is empowered by an FSM 4210 which contains four states as initialization (INIT), verification entrance (VER-Enter), peak found (PF), and verification exit (VER-End). INIT is the default state where the peak detector captures the local maxima. VER-Enter is used to evaluate the temporal consistency of the detected peaks. PF is the state after successful verification of temporally consistent peaks producing the breathing rate estimations. VER-Exit handles the case when the spectrum peaks disappear due to motions either from the subject under monitoring or other people nearby.

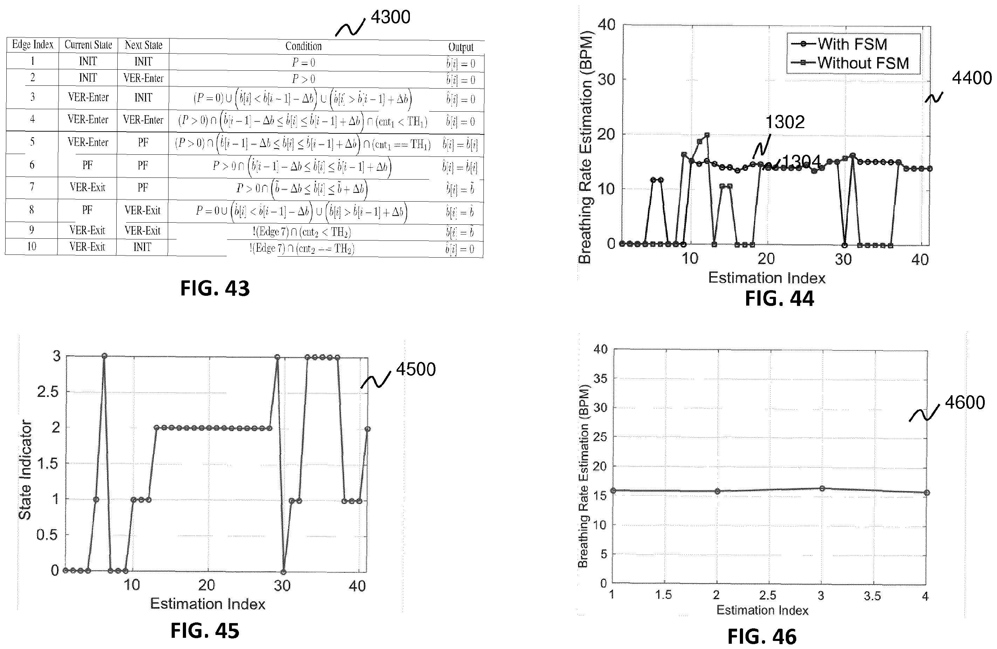

FIG. 43 shows a Table 4300 about the state transition details of the FSM 4210 shown in FIG. 42. The edge index for each column of Table 4300 is marked in FIG. 42. $\hat{b}[i]$ is the output breathing rate for block i, $b[i]$ is the internal breathing rate used by FSM, and $\Delta b$ is a variable capturing the uncertainty bound for estimation. $cnt_1$ and $cnt_2$ are counters that represent the duration of the FSM staying in the VER-Enter and VER-Exit states while $TH_1$ and $TH_2$ are the two thresholds representing how long the FSM is allowed to stay in the two states. $cnt_1$ increments by 1 when the current state is VER-Enter while $cnt_2$ increments by 1 when the current state is VER-Exit. $\cap$ and $\cup$ represent the AND condition and OR condition. b is the internal variable representing the last breathing rate estimation when the current state is PF and the next state is another state. !(Edge i) is the negate of the condition on edge i. The proposed FSM does not use any peak statistic such as the peak amplitudes for detection since the peak statistics could be very similar under cases with and without breathing which is verified experimentally.

**FSM for multi-person monitoring:** In case of M people to be monitored simultaneously, one can run M FSMs followed by the estimation engine. Each FSM works independently except that the estimated breathing rate from FSM m for CSI block i denoted by $\hat{b}_i^m$ should be different from all the other breathing rates and their harmonics denoted as $\{v\hat{b}_i^{m'}\}_{m'=1,2,...,M,m'\neq m}^{v=1,2,...,V}$ where V is the maximum harmonic order. One example is V=2.

**Experimental results:** Single-Person NLOS with an Operating Fan: In this experiment, one can turn on an electronic fan inside the same room with the subject. Since the fan is an electronic device with metal cover, it would reflect some EM waves and thus introduce interference to the CFRs and degrade the breathing monitoring performance. FIG. 44 shows a comparison 4400 of the performances with and without FSM. A breathing rate estimation of 0 indicates that the system is unable to obtain a reliable breathing rate. Although using FSM does not fully recover the ground-truth breathing rate (15 BPM), the utilization of FSM still enhances the overall performance. The accuracy with FSM is 94.48%.

FIG. 45 demonstrates the state transition 4500 of the FSM-based scheme in this experiment. Different states shown in FIG. 39 are encoded as follows: Init $\rightarrow$ 0, Verification $\rightarrow$ 1, PeakFound $\rightarrow$ 2, Motion $\rightarrow$ 3. FIG. 45 shows that the FSM correctly reacts to the fan moving by switching to the Motion state from time to time.

FIG. 46 demonstrates the performance 4600 of the disclosed breathing monitoring scheme when the subject stands up for five seconds and sits down during the measurement. Clearly, the breathing monitoring scheme overcomes the impact of such large motions and produces stable breathing rate estimations over time.

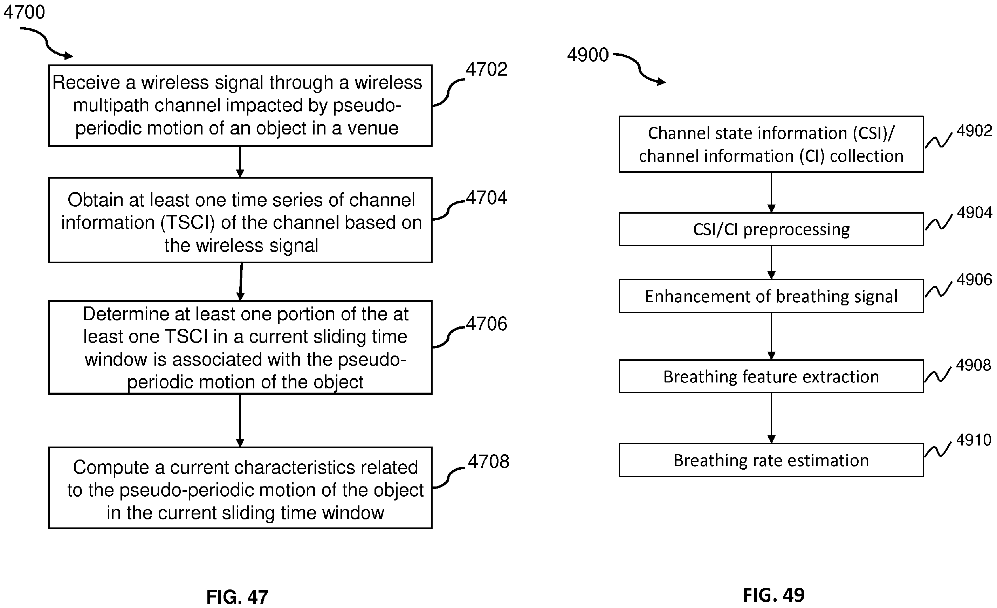

FIG. 47 illustrates an exemplary method 4700 for vital sign detection and monitoring, according to one embodiment of the present teaching. At operation 4702, a wireless signal is received through a wireless multipath channel impacted by pseudo-periodic motion of an object in a venue. At operation 4704, at least one TSCI (TSCI) of the channel is obtained based on the wireless signal. At operation 4706, at least one portion of the at least one TSCI in a current sliding time window is determined to be associated with the pseudo-periodic motion of the object. At operation 4708, a current characteristics related to the pseudo-periodic motion of the object is computed in the current sliding time window.

In one embodiment, the disclosed system can estimate breathing rate of more than one people simultaneously, and can count of the amount of people. The system may include a Type1 device which is a Bot including a transmitter, a Type2 device which is an Origin including a receiver. The receiver can extract a time series of CSI from a wireless signal and collect CSI for a slide window (about 10-15 sec). A vital sign estimator can estimate the breathing rate based on the collected CSI. If the transmitter has M antennas and the receiver has N antennas, there are MN links. One CSI time series is obtained for each link. All MN links are used by the system to compute the breathing rate. The vital sign estimator can be located in the cloud, or in the Origin or the Type2 device for local computing. If there are K Bots, then there can be K instances of the vital sign estimator, each serving one Bot. The breathing results of each Bot may be displayed separately on a client app of a user smart device. Results of some or all Bots may be combined to give combined results. For 1 Bot with one antenna and 1 Origin with one antenna (K=1) scenario, there is only 1 link (M=N=1). Each CSI has N1 (e.g. about 128) frequency subcarriers (called components). Each CSI is actually channel frequency response (CFR). The vital sign estimator starts by cleaning the CSI (including phase noise) and normalizing each CSI so that it has unity power. Then it does zero padding to get a vector of length N2 (e.g. 128, a power of 2). Then it performs N2-point IFFT to convert the N2-point CFR to N2-point time domain channel impulse response (CIR). The vital sign estimator then considers the sliding window of CSI (i.e. portion of the TSCI). Suppose each window has L CSI (e.g. for a 60 seconds window with 30 samples/sec, L=60x30=1800). The vital sign estimator performs time base correction to ensure that all CSI over the sliding window are sampled at uniform sampling time. The time base correction involves resampling the CSI with some interpolation filter. Among the N2 CIR coefficients, a number (C) of significant CIR components are identified (e.g. C=5 identified among N2=128 CIR components). Then the vital sign estimator considers each CIR component of each of the L CSI and treats it as an L-point time series. As such, there are C L-point component time series formed using the C identified CIR components. Then the vital sign estimator applies bandpass filtering to each of the C component time series; and applies L2-point FFT (e.g. L2=20148 for L=1800). Then the vital sign estimator identifies local maximum (i.e. peak) in the L2-point FFT domain. The frequency corresponding to each peak is the breathing rate of a person. If it is known that K people are present, K peaks will be identified. If the amount of people is unknown, each significant peak is considered one person. By counting the significant peaks, the amount of people can be obtained.

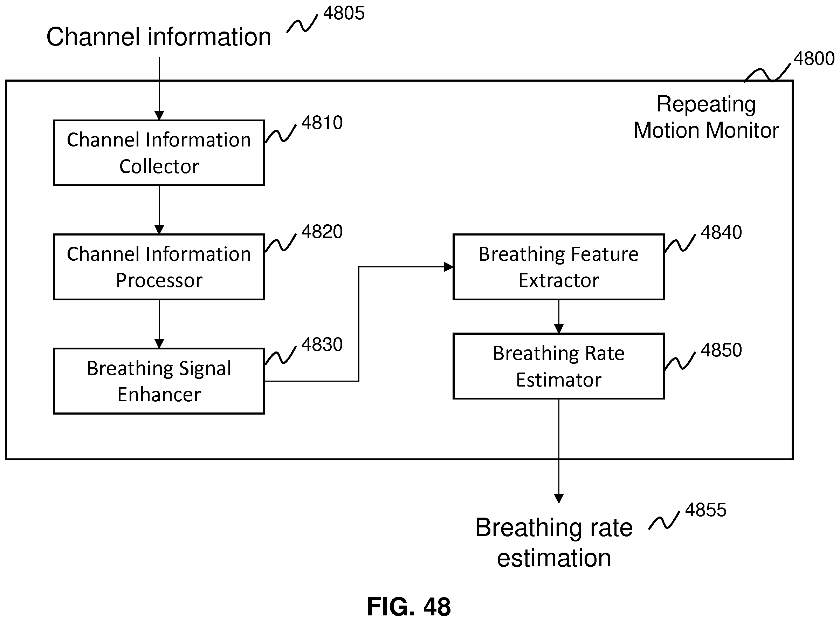

FIG. 48 illustrates an exemplary block diagram of a repeating motion monitor 4800, according to one embodiment of the present teaching. In one embodiment, the repeating motion monitor 4800 is in a system for monitoring a repeating motion of an object in a venue. According to various embodiments, the object may be a life (e.g. a human, a pet, an animal, etc.), a device (e.g. a fan, a machine, etc.), or a land; the repeating motion may represent: breathing, heartbeat, a periodic hand gesture, a periodic gait, a rotation, a vibration, or an earthquake; and the system can monitor periodic characteristics of the motion, e.g. a rate or frequency of the repeating motion. In one embodiment, the system comprises a transmitter (e.g. the transmitter 3510), a receiver (e.g. the receiver 3520) and the repeating motion monitor 4800 that is coupled to at least one of: the transmitter 3510, the receiver 3520, an additional transmitter, an additional receiver, a cloud server, a fog server, a local server, and an edge server.

In one embodiment, the transmitter is located at a first position in the venue and configured for transmitting a wireless signal through a wireless multipath channel impacted by a repeating motion of an object in the venue. The receiver is located at a second position in the venue and configured for: receiving the wireless signal through the wireless multipath channel impacted by the repeating motion of the object in the venue, and obtaining a TSCI of the wireless multipath channel based on the wireless signal. The wireless signal may be a probe signal or an acknowledge (ACK) signal in response to a probe signal.

The repeating motion monitor 4800 is configured for monitoring a periodic characteristics of the repeating motion of the object based on the TSCI. Each CI comprises N1 components, wherein N1 is an integer greater than 1. The repeating motion monitor may be further configured for: decomposing the TSCI into N1 component time series, each respective component time series comprising a respective one of the N1 components of each CI, computing N1 component-feature time series based on the N1 component time series, each respective component-feature time series comprises a feature of the respective component of each CI, associating a current periodic characteristic of the repeating motion of the object with a current sliding time window, compute N1 component sliding functions, each respective component sliding function being a respective transform of a respective component-feature time series in the current sliding time window, and monitoring the current periodic characteristics of the repeating motion of the object based on at least one of: the N1 component sliding functions, and a combined sliding function computed based on the N1 component sliding functions.

In one embodiment, the sliding time window for the repeating motion monitor 4800 to monitor the periodic characteristics can be very short, e.g. 10 to 15 seconds. The sliding window length is related to the initial setup and/or waiting time for the repeating motion monitor 4800. A shorter sliding window means a shorter initial setup time, which means a better user experience, since the user does not need to maintain a same breathing rate in a long sliding time window for the repeating motion monitor 4800 to capture the breathing.

In one embodiment, as shown in FIG. 48, the repeating motion monitor 4800 includes a CI collector 4810, a CI processor 4820, a breathing signal enhancer 4830, a breathing feature extractor 4840, and a breathing rate estimator 4850. The CI collector 4810 in this example collects TSCI 4805, e.g. channel state information or channel frequency response, which may be the CI 3525 generated by the receiver 3520. According to various embodiments, the repeating motion monitor 4800 can not only monitor and estimate breathing rate, but also characteristics of any other repeating motions. In that case, the breathing signal enhancer 4830 is a repeating signal enhancer; the breathing feature extractor 4840 is a repetition feature extractor; the breathing rate estimator 4850 is a repetition rate estimator.

The CI processor 4820 in this example processes the TSCI by e.g. decomposing the TSCI into N1 component time series, each respective component time series comprising a respective one of the N1 components of each CI, computing N1 component-feature time series based on the N1 component time series, each respective component-feature time series comprises a feature of the respective component of each CI, associating a current periodic characteristic of the repeating motion of the object with a current sliding time window, and computing N1 component sliding functions, each respective component sliding function being a respective transform of a respective component-feature time series in the current sliding time window. The CI processor 4820 may further identify and remove any unallowed feature from the at least one feature. According to various embodiments, a feature outside an allowable range is identified as an unallowed feature, a feature in the allowable range is identified as an unallowed feature when it is greater than an adaptive threshold that is computed adaptively based on an adaptive background noise floor, and the adaptive background noise floor is computed based on at least one feature outside the allowable range.

In one embodiment, the breathing signal enhancer 4830 is configured for computing at least one of: N1 component frequency functions based on a frequency transform of each of the N1 respective component sliding functions, and a combined frequency function based on the frequency transform of the combined sliding function. The breathing feature extractor 4840 is configured for computing at least one feature of at least one of: the N1 component frequency functions, and the combined frequency function. The breathing rate estimator 4850 is configured for monitoring at least one of: the current periodic characteristics of the repeating motion of the object, and a current periodic characteristics of an additional repeating motion of an additional object in the venue, based on the at least one feature.

In one embodiment, the breathing feature extractor 4840 is configured for computing at least one feature of at least one of: the combined sliding function, and each of the N1 component sliding functions, wherein the at least one feature comprises at least one of: an extremum, a local maximum, a local minimum, a zero-crossing, a local maximum derivative, a local maximum high-order derivative, a local minimum derivative, a local minimum high-order derivative, a local zero derivative, a positive quantity, a negative quantity, a significant quantity, a positive maximum, a positive minimum, a negative maximum, a negative minimum, a first maximum, a first minimum, a second maximum, a second minimum, an N-th maximum, an N-th minimum, a significant maximum, a significant minimum, a significant derivative, and a significant high-order derivative. The breathing rate estimator 4850 is configured for monitoring the current periodic motion of the object based on at least one of: the at least one feature of at least one of: the combined sliding function and each of the N1 component sliding functions, and at least one domain value associated with the at least one feature of at least one of: the combined sliding function and each of the N1 component sliding functions.

In another embodiment, the breathing feature extractor 4840 is configured for computing at least one dominant periodic feature of each of the N1 component-feature time series in the current sliding time window, wherein the at least one dominant periodic feature comprises at least one of: a spectral extremum, a local spectral maximum, a local spectral minimum, a spectral zero-crossing, a local maximum spectral derivative, a local maximum spectral high-order derivative, a local minimum spectral derivative, a local minimum spectral high-order derivative, a local zero spectral derivative, a positive spectral quantity, a negative spectral quantity, a significant spectral quantity, a positive spectral maximum, a positive spectral minimum, a negative spectral maximum, a negative spectral minimum, a first spectral maximum, a first spectral minimum, a second spectral maximum, a second spectral minimum, an N-th spectral maximum, an N-th spectral minimum, a significant spectral maximum, a significant spectral minimum, a significant spectral derivative, and a significant spectral high-order derivative. The breathing rate estimator 4850 is configured for monitoring the current periodic characteristics of the repeating motion of the object based on at least one of: the dominant periodic features of the N1 component-feature time series in the current sliding time window, and at least one combined dominant periodic feature based on the dominant periodic features of the N1 component-feature time series in the current sliding time window.

In yet another embodiment, the breathing feature extractor 4840 is configured for computing at least one dominant periodic feature of each of the N1 component time series in the current sliding time window, wherein the at least one dominant periodic feature comprises at least one of: a spectral extremum, a local spectral maximum, a local spectral minimum, a spectral zero-crossing, a local maximum spectral derivative, a local maximum spectral high-order derivative, a local minimum spectral derivative, a local minimum spectral high-order derivative, a local zero spectral derivative, a positive spectral quantity, a negative spectral quantity, a significant spectral quantity, a positive spectral maximum, a positive spectral minimum, a negative spectral maximum, a negative spectral minimum, a first spectral maximum, a first spectral minimum, a second spectral maximum, a second spectral minimum, an N-th spectral maximum, an N-th spectral minimum, a significant spectral maximum, a significant spectral minimum, a significant spectral derivative, a significant spectral high-order derivative, and another periodic feature. The breathing rate estimator 4850 is configured for monitoring the periodic characteristics of the repeating motion of the object based on at least one of: the dominant periodic features of the N1 component time series in the current sliding time window, and at least one combined dominant periodic feature based on the dominant periodic features of the N1 component time series in the current sliding time window.

The breathing rate estimator 4850 generates an estimation of a periodic characteristics (e.g. a breathing rate estimation 4855) of the repeating motion of the object. In one embodiment, the breathing rate estimator 4850 may further compute at least one analytics based on the periodic characteristics of the repeating motion of the object, and transmit information of the periodic characteristics of the repeating motion of the object to a user interface. The user interface may be configured for presenting a summary of the periodic characteristics of the repeating motion of the object based on the transmitted information.

In one embodiment, the breathing rate estimator 4850 is configured for: processing the periodic characteristics of the repeating motion of the object; computing a time function based on the periodic characteristics of the repeating motion of the object; analyzing at least one of: the periodic characteristics of the repeating motion of the object, and the time function; and computing at least one analytics based on at least one of: the periodic characteristics of the repeating motion of the object, and the time function. In another embodiment, the breathing rate estimator 4850 is configured for: estimating a missing periodic characteristics of the repeating motion of the object based on at least one of: available periodic characteristics of the repeating motion of the object computed based on the TSCI, a different characteristics computed based on the TSCI, and a different characteristics computed based on a different TSCI of the wireless multipath channel. In yet another embodiment, the breathing rate estimator 4850 is configured for: computing a trend function by lowpass filtering a time function of the periodic characteristics of the repeated motion of the object; analyzing at least one of: the time function, the trend function, and a detrended function computed by subtracting the trend function from the time function of the periodic characteristics; and monitoring the repeating motion of the object based on at least one of: the time function, the trend function, and the detrended function.

The object may be a life (e.g. a human, a baby). The breathing rate estimator 4850 may be configured to monitor sleeping of the human. Various stages of sleeping including rapid-eye-movement (REM), non-REM, awake, asleep, interruption may be identified. At least one analytics associated with the sleeping stages may be computed. Analytics associated with sleeping quality may be computed. The object may be a device. The breathing rate estimator 4850 may be configured to monitor a repeated motion such as vibration, rotation, back-and-forth action, and/or repeated machine action. Analytics associated with normal and/or abnormal operation of the device may be computed. Analytics associated with location of machine parts in the repeated cycle may be computed. The object may be land. The breathing rate estimator 4850 may be configured to monitor signs of earthquakes, aftershocks, shaking, and/or vibration.

In one embodiment, the system can measure more than one breathing rate simultaneously. In this case, the repeating motion monitor 4800 is further configured for: monitoring an additional periodic characteristics of an additional repeating motion of an additional object contemporaneously based on the TSCI, wherein the wireless multipath channel is further impacted by the additional repeating motion of the additional object in the venue. The at least one feature comprises: a first feature associated with the periodic characteristics of the repeating motion of the object, and a second feature associated with the periodic characteristics of the additional repeating motion of the additional object. The periodic characteristics of the repeating motion of the object and the periodic characteristics of the additional repeating motion of the additional object are monitored contemporaneously based on the first feature and the second feature.

In one embodiment, the breathing rate is monitored in a series of overlapping time window. In this case, the repeating motion of the object is locally repeating such that, in each of a series of overlapping time windows, the locally repeating motion resembles a periodic motion; and the periodic characteristics of the locally repeating motion of the object is monitored in each of the series of overlapping time window. In another embodiment, the system can perform not only the breathing rate detection and estimation, but also other detections and estimations for, e.g. fall-down detection, tracking monitoring, etc. In this case, the repeating motion monitor 4800 is further configured for: performing a joint analysis on the periodic characteristics of the repeating motion of the object and at least one of: a different characteristics computed based on the TSCI, and a different characteristics computed based on a different TSCI of the wireless multipath channel; and computing at least one joint analytics based on the joint analysis.

FIG. 49 illustrates a flowchart of an exemplary method 4900 for estimating and monitoring a vital sign (e.g. breathing rate, heart rate), according to one embodiment of the present teaching. First, channel state information (CSI) or CI (CI, e.g. CSI) may be obtained at operatoin 4902 by channel estimation after a radio receiver (e.g. wireless, RF, WiFi, LTE, UWB, etc) receives a wireless signal (e.g. a channel probing radio signal, a reply signal, an acknowledgement signal, a data signal, or a control signal) from a radio transmitter. The CSI/CI is preprocessed at operatoin 4904, e.g., to remove noise and/or to calculate some feature function (e.g. autocorrelation function (ACF)) from the breathing signal. Since breathing signal may be very weak, the received signal may be enhanced at operatoin 4906 to boost the signal-to-noise ratio (SNR) through some operation, e.g., maximal ratio combining (MRC). The ACF may exhibit a periodic behavior since breathing is a periodic process, and features about breathing can be extracted at operation 4908 from the ACF, e.g., detecting a (first) local maximum from the ACF. The time lag of the first local maximum correspond to the period T of breathing, and then the breathing rate estimation can be obtained at operation 4910, e.g. by 60/T breath per minute.

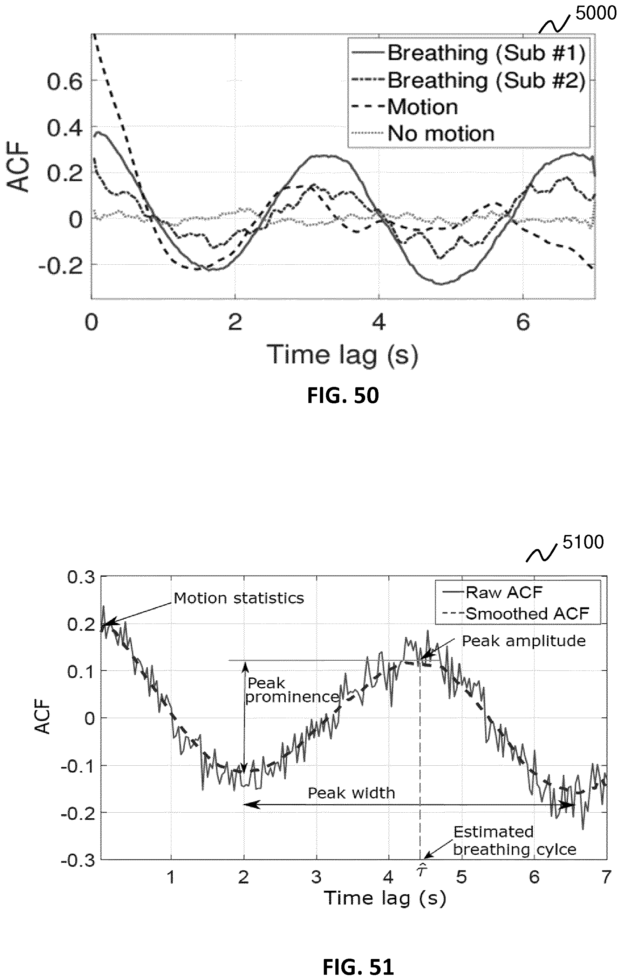

FIG. 50 illustrates exemplary autocorrelation functions 5000 of the received signals under different scenarios, according to one embodiment of the present teaching. As shown in FIG. 50, when there is a breathing signal, the ACF will exhibit a definite peak at a certain delay (although the peak value may differ over different subcarriers), contributed by the periodic breathing motions. On the contrary, no certain peaks can be observed on any subcarrier when there is no breathing (i.e., no periodic motions). In principle a time delay slightly longer than one breathing cycle (e.g., 5 to 7 seconds) is sufficient to pick up the first breathing rate and later on instantaneous estimates can be produced every one second.

Motion Statistics: In prior to breathing estimation, a key step is to examine whether there exists detectable breathing signal (i.e., whether breathing exists without extra big motions). As mentioned previously, breathing will easily be buried in other large body motions, and should not be estimated if there is none. One can also investigate the property of the ACF for this purpose since, in addition to breathing, the calculated ACF actually offers indicators for detection of arbitrary motions.

**Breathing Detection and Estimation:** Based on the calculated ACF and its inherent characteristics, the system first detects the presence and absence of breathing and, if presented, then estimates the breathing rate, accurately and instantaneously. FIG. 51 illustrates exemplary features 5100 extracted from the derived autocorrelation functions for breathing detection and estimation, according to one embodiment of the present teaching. As shown in FIG. 51, for a subcarrier with frequency $f$, one can extract several features from $\rho_G(\tau, f)$ for breathing detection, in addition to the motion statistics. The first feature is Peak Prominence, which is the vertical distance between the peak value and the largest height of the adjacent valleys, which measures the likelihood of the existence of the peak. The second feature is Peak Width, which is the horizontal distance between the two adjacent valleys, which also measures the likelihood of the existence of the peak. The third feature is Peak Amplitude, which is the height of the peak, which measures the amplitude of the ACF of the breathing signal and will be comparable to the value of motion statistic in presence of only breathing motion. The fourth feature is Motion Interference Ratio, which is the ratio between the motion statistics and peak amplitude, which measures degree of the interference of the non-breathing motion, such as body movements, walking, standing up, typing keyboard, etc., in the environment. The fifth feature is Peak Location, which is the horizontal distance between the origin and the peak (i.e., time lags), which measures the breathing cycle. Other features may be computed.

In general, the larger the motion statistics, peak prominence, peak width, peak amplitude and the smaller the motion interference ratio, the more likely for the presence of the breathing signal. In one embodiment, the above features are jointly fused to claim the existence of breathing signal and the corresponding breathing rate. Once there is a breathing signal, the breathing rate can be estimated as BR = $60/\hat{\tau}$ breath per minute, where $\hat{\tau}$ is the location (i.e., time lags) of the first dominant peak of $\hat{\rho}_G(\tau, f)$.

In practice, the SNR of the breathing signal on each subcarrier modulated by minute breathing motions is very low, especially when the person being monitored is far away from the link, covered by quilts, or behind the wall, resulting in limited coverage and vulnerable estimation that prevents the applications of the existing RF-based approaches. Previous approaches attempt to select a best subcarrier among others, or to average over all to improve breathing signal do not produce reliable, not to mention optimal results.

To boost the breathing SNR, the system may combine the breathing signals measured on different subcarriers in the optimal way. In one embodiment, the disclosed design is based on Maximal Ratio Combining (MRC), a classical diversity combining method in telecommunications that maximize signal SNR by optimally combining received signals on multiple antennas.

**MRC on Breathing Signal.** In the context of breathing estimation with CSI, the breathing signal b(t) denotes a periodic stationary breathing signal with zero mean, which is related to the movement of the chest and abdomen, and is measured by multiple subcarriers. Let $g(f)$ and $\Delta t_f$ stand for the gain and the random initial phase of the breathing signal measured at the frequency $f$, respectively. The SNR of the breathing signal, denoted as $y(f)$, measured on subcarrier with frequency $f$ at time $t$ is defined as

$$\gamma(f) = \frac{\mathbb{E}[(g(f)b(t-\Delta t_f))^2]}{\mathbb{E}[\varepsilon^2(t,f)]} = \frac{g^2(f)\mathbb{E}(b^2(t-\Delta t_f))}{\sigma^2(f)}, \qquad (25)$$

where $\mathbb{E}[\cdot]$ stands for the expectation operator and $\varepsilon(t,f)$ is defined as the noise term. For convenience, the average power of the breathing signal $b(t)$ is normalized to unit power by definition, that is, $\mathbb{E}[b^2(t)] = 1$, and thus one can have $\gamma(f) = g^2(f)/\sigma^2(f)$.

Defining $k(f) \triangleq \frac{g^2(f)}{g^2(f)+\sigma^2(f)}$ as the normalized channel gain, it can reflect the strength of total motion existing in the monitored area. The SNR of the ACF of each subcarrier can be estimated as $N\hat{k}^2(f)$, where $N$ is the number of samples used in the estimation of the ACF. Then the weight can be set to $\rho_G(\tau = 1/F_S, f)$, the ACF of $G(t, f)$, where $G(t, f)$ is the power response (i.e., the square of the magnitude of the channel frequency response $H(t, f)$ at time t and frequency/subcarrier f).

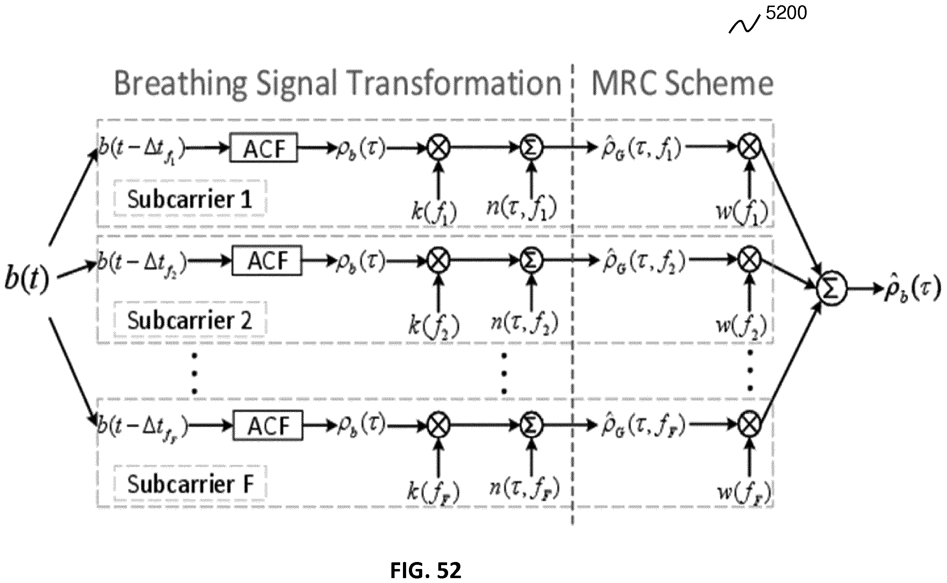

FIG. 52 illustrates an exemplary scheme 5200 for breathing signal extraction and maximization, according to one embodiment of the present teaching. The left part of the FIG. 52 shows the decomposition of the measured ACF of the channel power response when a person breathes normally in the monitored area, while the right part shows the MRC scheme for boosting the SNR of the ACF of the breathing signal.

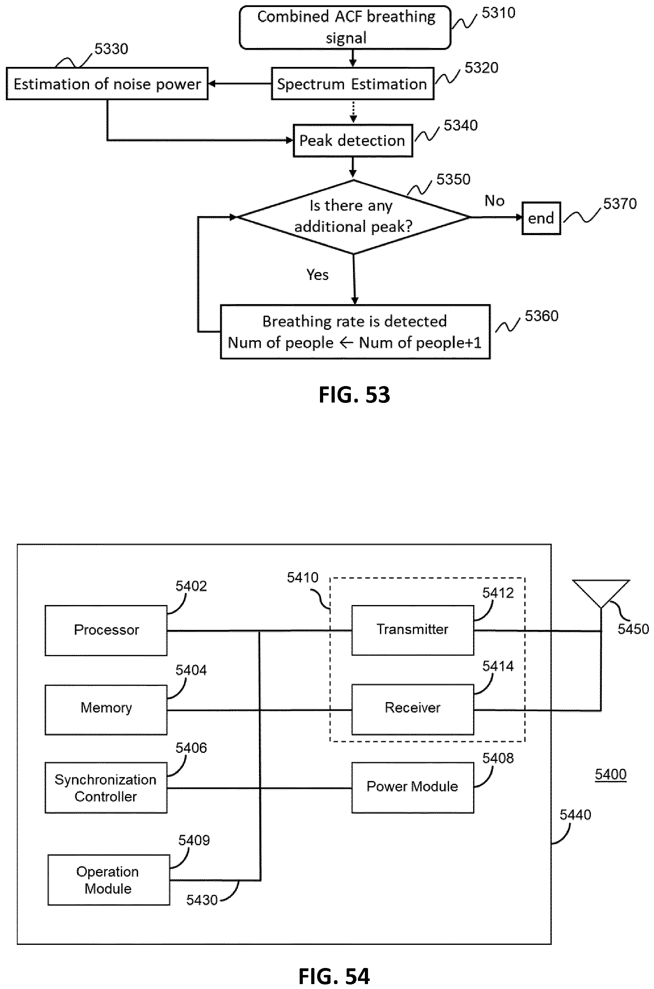

FIG. 53 illustrates an exemplary procedure of multi-person breathing rate estimation, according to one embodiment of the present teaching. The input signal 5310 $\rho(\tau)$, where $0 \le \tau \le T$, denotes a combined ACF breathing signal, which is the ACF of the breathing signal after the MRC.

Spectrum estimation is performed at operation 5320 by calculating the power spectral density S(f), where $0 \le f \le F$, of the breathing signal $\rho(\tau)$. The spectrum estimation can be based on non-parametric methods, e.g., periodogram algorithm, or parametric spectrum estimators, e.g., MUSIC algorithm.

Estimation of noise power is performed at operation 5330. Let fl and fh denote the lower and upper bounds of the breathing rate of interests. The noise power n is estimated by calculating the average of the energy of the power spectral density outside the breathing range, i.e., $n = \frac{1}{T+f_l-f_h}\sum_{f \le f_l}S(f) + \sum_{f \ge f_h}S(f)$.

Peak detection is performed at operation 5340 by detecting all the peaks of S(f) within the range fl $\le$ f $\le$ fh, where a peak is defined as the point whose value is large than its neighbors. Let fi denote the i-th peak. In one embodiment, after the operation 5320, the process can go directly to the operation 5340 without perform the operation 5330.

At operation 5350, it is determined whether there is any additional peak. Each peak, e.g. the i-th peak, is examined, e.g. by comparing its value S(fi) with the estimated noise power n. If the difference is larger than a preset threshold η, i.e., S(fi)≥ η, then the peak is a valid peak and the corresponding breathing rate estimate is 60* fi. The process then goes to operation 5360 to update the number of people by adding 1 and the process goes back to operation 5350 to detect any additional peak. Otherwise, if the difference is not larger than a preset threshold, the peak is discarded. The number of people detected stays the same and the process stays at operation 5350 to detect any additional peak. When it is determined at operation 5350 that there is no additional peak, the process ends at operation 5370.

In one embodiment, the disclosed system may include a Type1 device which is a Bot including a transmitter, a Type2 device which is an Origin including a receiver, and the repeating motion monitor 4800. The receiver can extract a time series of CSI from a wireless signal and collect CSI for a slide window (about 10-15 sec). The repeating motion monitor 4800 can estimate the breathing rate based on the collected CSI. If the transmitter has M antennas and the receiver has N antennas, there are MN links. One CSI time series is obtained for each link. All MN links are used by the system to compute the breathing rate. The repeating motion monitor 4800 can be located in the cloud, or in the Origin or the Type2 device for local computing. If there are K Bots, then there can be K instances of the repeating motion monitor 4800, each serving one Bot. The breathing results of each Bot may be displayed separately on a client app of a user smart device. Results of some or all Bots may be combined to give combined results. For 1 Bot with one antenna and 1 Origin with one antenna (K=1) scenario, there is only 1 link (M=N=1). Each CSI has N1 (e.g. about 128) frequency subcarriers (called components). As the phase of each subcarrier tends to be noisy, the repeating motion monitor 4800 can extract a noise-robust feature, e.g. magnitude square (i.e. square of magnitude, no phase) for each subcarrier. As each subcarrier is associated with a unique frequency, the feature is also associated with the same frequency. For a time series of CSI, the repeating motion monitor 4800 treats a respective feature associated with a respective frequency as a feature time series (called component-feature time series). Thus there are N1 component-feature time series. It computes autocorrelation function (ACF) for each component-feature time series (within the sliding window of about 10-15 sec). This is repeated for all frequency. The repeating motion monitor 4800 then computes an overall ACF as a weighted average of ACF of all frequency components. The weights are determined adaptively using an existing method called maximal ratio combining (MRC). Finally, the repeating motion monitor 4800 identifies the first positive significant local maximum. The time lag (T) associated with the identified local max gives the breathing rate (=1/T). When there are multiple persons, each person should have a corresponding peak in the overall ACF. But these peaks may be close to each other, which makes it hard to tell whether there is one peak or two or three, etc.

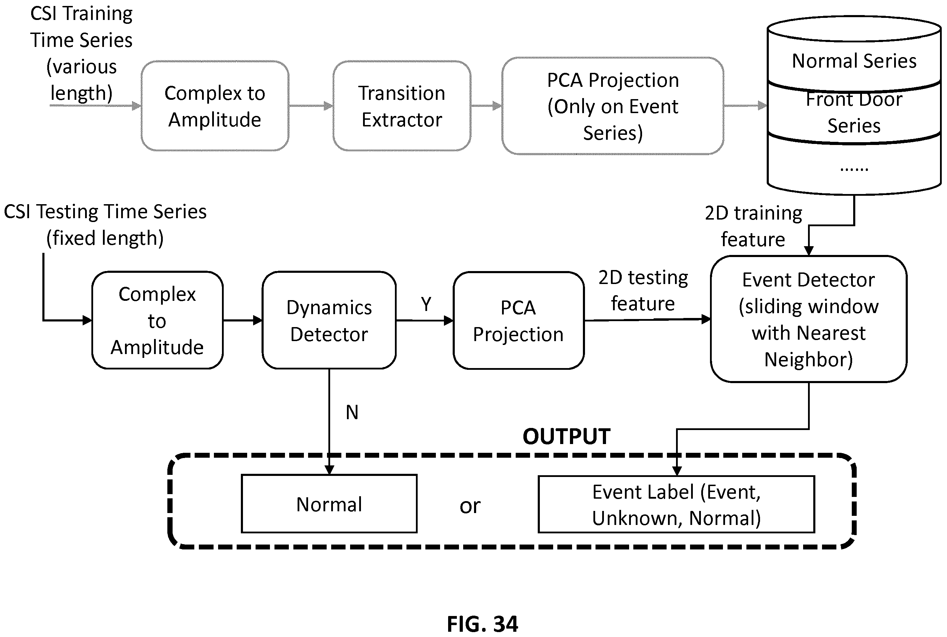

FIG. 54 illustrates an exemplary diagram of a device 5400 in a motion monitoring system, according to one embodiment of the present teaching. The device 5400 is an example of a device that can be configured to implement the various methods described herein. According to various embodiments, the device may be: a Type1 device which is a Bot including a transmitter, a Type2 device which is an Origin including a receiver, the vital sign estimator 3610, the system state controller 3620, the repeating motion monitor 4800, and/or other components in FIGs. 1-53 and 55-60. As shown in FIG. 54, the device 5400 includes a housing 5440 containing a processor 5402, a memory 5404, a transceiver 5410 comprising a transmitter 5412 and a receiver 5414, a synchronization controller 5406, a power module 5408, and an operation module 5409.

In this embodiment, the processor 5402 controls the general operation of the device 5400 and can include one or more processing circuits or modules such as a central processing unit (CPU) and/or any combination of general-purpose microprocessors, microcontrollers, digital signal processors (DSPs), field programmable gate array (FPGAs), programmable logic devices (PLDs), controllers, state machines, gated logic, discrete hardware components, dedicated hardware finite state machines, or any other suitable circuits, devices and/or structures that can perform calculations or other manipulations of data.

The memory 5404, which can include both read-only memory (ROM) and random access memory (RAM), can provide instructions and data to the processor 5402. A portion of the memory 5404 can also include non-volatile random access memory (NVRAM). The processor 5402 typically performs logical and arithmetic operations based on program instructions stored within the memory 5404. The instructions (a.k.a., software) stored in the memory 5404 can be executed by the processor 5402 to perform the methods described herein. The processor 5402 and the memory 5404 together form a processing system that stores and executes software. As used herein, "software" means any type of instructions, whether referred to as software, firmware, middleware, microcode, etc. which can configure a machine or device to perform one or more desired functions or processes. Instructions can include code (e.g., in source code format, binary code format, executable code format, or any other suitable format of code). The instructions, when executed by the one or more processors, cause the processing system to perform the various functions described herein.

The transceiver 5410, which includes the transmitter 5412 and receiver 5414, allows the device 5400 to transmit and receive data to and from a remote device (e.g., an Origin or a Bot). An antenna 5450 is typically attached to the housing 5440 and electrically coupled to the transceiver 5410. In various embodiments, the device 5400 includes (not shown) multiple transmitters, multiple receivers, and multiple transceivers. In one embodiment, the antenna 5450 is replaced with a multi-antenna array 5450 that can form a plurality of beams each of which points in a distinct direction. The transmitter 5412 can be configured to wirelessly transmit signals having different types or functions, such signals being generated by the processor 5402. Similarly, the receiver 5414 is configured to receive wireless signals having different types or functions, and the processor 5402 is configured to process signals of a plurality of different types.

In one embodiment, the device 5400 may be a Bot or an Origin of a motion monitoring system. The motion monitoring system may comprise at least one Bot and at least one Origin. The synchronization controller 5406 in this example may be configured to control the operations of the device 5400 to be synchronized or un-synchronized with another device, e.g. another Origin or another Bot. In one embodiment, each of the device 5400 and other Bots or Origins in the system may transmit or receive the wireless signals individually and asynchronously.

The operation module 5409 in this example may perform one or more operations for vital sign detection and monitoring. The operation module 5409 may comprise one or more sub-modules to implement different methods disclosed herein. In one embodiment, the device 5400 may be the vital sign estimator 3610 of a motion monitoring system, where the operation module 5409 includes one or more of the components shown in FIG. 37. In another embodiment, the device 5400 may be the repeating motion monitor 4800 of a motion monitoring system, where the operation module 5409 includes one or more of the components shown in FIG. 48.

The power module 5408 can include a power source such as one or more batteries, and a power regulator, to provide regulated power to each of the above-described modules in FIG. 54. In some embodiments, if the device 5400 is coupled to a dedicated external power source (e.g., a wall electrical outlet), the power module 5408 can include a transformer and a power regulator.

The various modules discussed above are coupled together by a bus system 5430. The bus system 5430 can include a data bus and, for example, a power bus, a control signal bus, and/or a status signal bus in addition to the data bus. It is understood that the modules of the device 5400 can be operatively coupled to one another using any suitable techniques and mediums.

Although a number of separate modules or components are illustrated in FIG. 54, persons of ordinary skill in the art will understand that one or more of the modules can be combined or commonly implemented. For example, the processor 5402 can implement not only the functionality described above with respect to the processor 5402, but also implement the functionality described above with respect to the operation module 5409. Conversely, each of the modules illustrated in FIG. 54 can be implemented using a plurality of separate components or elements.

In one embodiment, the present teaching discloses a method, apparatus, and a system for sleep monitoring. The disclosed method comprises: obtaining a TSCI of a wireless multipath channel using a processor, a memory communicatively coupled with the processor and a set of instructions stored in the memory, and monitoring the sleep-related motion of the user based on the TSCI.

The TSCI is extracted from a wireless signal transmitted between a Typel heterogeneous wireless device and a Type2 heterogeneous wireless device in a venue through the wireless multipath channel. The wireless multipath channel is impacted by a sleep-related motion of a user in the venue.

Monitoring the sleep-related motion comprises monitoring at least one of the following of the user: sleep timings, sleep durations, sleep stages, sleep quality, sleep apnea, sleep problems, sleep disorders, breathing problems, gasping, choking, teeth-grinding, pause of sleep, absence of sleep, insomnia, restlessness during sleep, hypersomnia, parasomnia, day-time sleepiness, sleep locations, sleep-while-driving, sleep disruptions, nightmares, night terrors, sleep walking, REM sleep behavior disorder, Circadian rhythm disorder, non-24-hour sleep-wake disorder, periodic limb movement disorder, shift-work sleep disorder, narcolepsy, confusional arousals, sleep paralysis, another sleep-related condition, and/or another sleep-related behavior.

Sleep timings comprises timings of at least one of: go-to-bed, sleep-onset, wake-up, REM-onset, NREM-onset, onset of sleep stage transitions, sleep disorders, sleep problems, breathing problems, insomnia, hypersomnia, parasomnia, sleep hypnogram-related events, sleep disruptions, sleep apnea, snoring during sleep, sleeping-not-on-a-bed, day-time sleep, sleep-walking, sleep-related events, sleep-related condition, and/or, sleep-related behavior, etc.

Sleep stages comprises at least one of: wake-up, rapid-eye-movement (REM) and/or non-REM (NREM). At least one of: a time function of breathing rate, and a time function of motion statistics, of the user may be computed based on the series of CI. If breathing is not detected at time t, the breathing rate at time t may be computed as zero. The sleep-related motion of the user may be monitored based on at least one of: the time function of breathing rate, and/or the time function of motion statistics, of the user. At least one of: a time function of breathing ratio, and a time function of motion ratio, of the user may be computed based on the series of CI. The breathing ratio at time t may be computed as percentage of time when the time function of breathing rate is non-zero in a first time window comprising the time t. The motion ratio at time t may be computed as percentage of time when the time function of motion statistics is larger than a first threshold within a second time window comprising the time t. The sleep-related motion of the user may be monitored based on at least one of: the time function of breathing ratio, and/or the time function of motion ratio, of the user.

A sleep stage may be classified as "awake" if at least one of: the motion ratio is greater than a second threshold, and/or the breathing ratio is less than a third threshold. The sleep stage may be classified as "asleep" if at least one of: the motion ratio is less than the second threshold, and/or the breathing ratio is greater than the third threshold. The "asleep" stage may comprise at least one of: rapid-eye-movement (REM) stage, and/or non-REM (NREM) stage.

A breathing rate trend function may be computed by low-pass filtering the time function of breathing rate. A detrended breathing rate function may be computed by subtracting the breathing rate trend function from the time function of breathing rate. A time function of breathing rate variance may be computed by computing variance of the detrended breathing rate function within a sliding time window. The sleep-related motion of the user may be monitored based on the time function of breathing rate variance.

An average NREM breathing rate may be computed by identifying a peak of a histogram of the time function of breathing rate in "asleep" stage in an overnight period. (e.g. the whole night, or the whole night subtracting any "awake" periods). A time function of breathing rate deviation may be computed by computing a distance between the average NREM and a percentile of the breathing rate within a sliding time window. The sleep stage may be classified as at least one of: REM stage and/or NREM stage, based on the time function of breathing rate deviation. A time function of breathing rate variance may be computed by computing variance of a detrended breathing rate function within a first sliding time window. A time function of breathing rate deviation may be computed by computing a distance between an average NREM and a percentile of the breathing rate within a second sliding time window. The sleep stage may be classified as at least one of: REM stage, and/or NREM stage, based on the time function of breathing rate variance and the time function of breathing rate deviation.

A classifier may be trained based on at least one of: breathing rate variance, and breathing rate deviation, using machine learning. The machine learning may comprise at least one of: supervised learning, unsupervised learning, semi-supervised learning, active learning, reinforcement learning, support vector machine, deep learning, feature learning, clustering, regression, and/or dimensionality reduction. The sleep stage may be classified as at least one of: REM stage, and/or NREM stage, based on the classifier. A quantity related to the sleep-related motion of the user may be computed based on the TSCI. The sleep-related motion of the user may be monitored based on the quantity.

The quantity may comprise at least one of: the time the user goes to bed, the time the user gets out of bed, the sleep onset time, total time it takes the user to fall asleep, the wake up time, sleep disruption time, number of sleep disruption period, mean disruption duration, variance of disruption duration, total time in bed, total time the user is asleep, time periods of REM, time periods of NREM, time periods of awake, total time of REM, total time of NREM, number of REM periods, number of NREM periods, time of toss and turn in bed, duration of tossing and turning, hypnogram, periods of apnea, periods of snore, total duration of apnea, number of apnea periods, average duration of apnea period, periods of breathing problems, sleep quality score, daytime sleep, time periods of daytime sleep, total duration of daytime sleep, number of period of daytime sleep, average duration of period of daytime sleep, and another quantity.

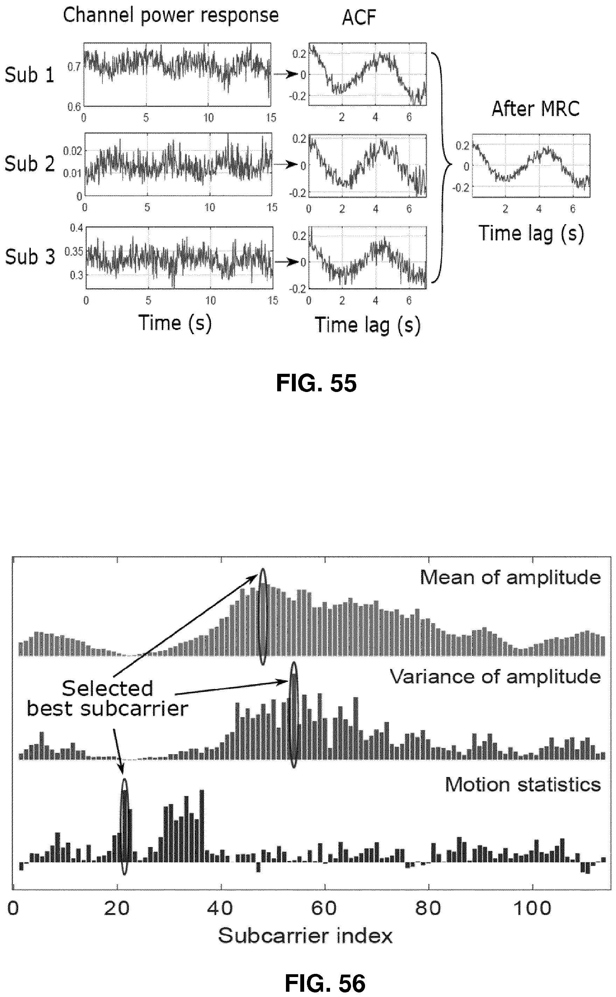

As discussed above, FIG. 52 summarizes a proposed scheme for breathing signal extraction and maximization. The left part of FIG. 52 shows the decomposition of the measured ACF of the channel power response when a person breathes normally in the monitored area, and the right part shows the MRC scheme for boosting the SNR of the ACF of the breathing signal. FIG. 55 depicts an illustrative example based on real-world measurements, where the SNR of the breathing signal is amplified by 2.5 dB compared to the best subcarrier indicated by largest variance and by 3.7 dB compared to directly averaging all subcarriers. FIG. 56 further demonstrates the gains of the disclosed ACF-based MRC scheme and confirms the observations herein that amplitudes and their variances are not effective metrics for subcarrier selection. As seen, the subcarrier that is the most sensitive to motion (i.e., holding the largest motion statistic) could experience very small amplitude and low variance.

Sleep Stage Recognition

SMARS divides the continuous motion and breathing estimates of overnight sleep into 300-second epochs. For each epoch, SMARS recognizes three different sleep stages, i.e., wake, REM sleep and NREM sleep. The staging is performed in two steps: First, SMARS differentiates wake from sleep mainly by body motions; Second, REM and NREM stages are further identified during sleep period.

**Sleep/Wake Detection.** SMARS first implements a sleep-wake detector to identify the sleep and wake states. The key insight is that, more frequent body movements will be observed when a subject is awake, while mainly breathing motion presents when he/she is asleep. Since bodily movements are significantly stronger than breathing motions, and both of them can be easily captured and quantified by the motion statistic defined herein, SMARS utilizes it to distinguish between sleep and wake states.

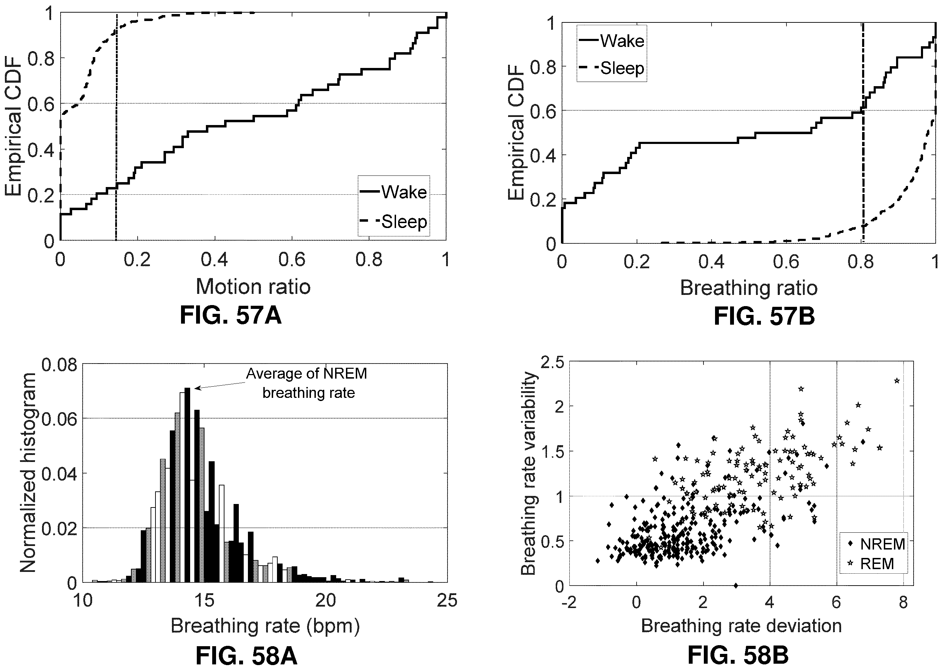

Specifically, one can define motion ratio as the percentage of time when the motion statistic, $\hat{\rho}_b(1/F_S)$, is larger than a preset threshold. Thus for the wake state, a higher motion ratio is expected, as shown in FIG. 57A. Similarly, one can also define breathing ratio as the percentage of time when the breathing signal is detected. Since bodily movements destroy the periodicity of the environmental dynamics, the breathing ratio will be lower when a subject is awake, as shown in FIG. 57B.

Combining the above two features, SMARS identifies an epoch as sleep only when the motion ratio is smaller than the predefined threshold and the breathing ratio is larger than the other threshold. Both thresholds are empirically determined as in FIG. 57A and FIG. 57B. Since the disclosed model statistically considers all multipaths indoors, the values of both thresholds generalize to different environments and subjects.

**REM/NREM Recognition.** SMARS exploits the following clinical facts and accordingly extracts two distinctive features from breathing rate estimates for REM/NREM stages classification: Breathing rate is usually faster and presents higher variability and irregular patterns for REM stage, while more stable and slower for NREM stage.

Since NREM stage constitutes the majority (about 75% to 80%) of total sleep for typical healthy adults, the average breathing rate during NREM stage can be estimated by localizing the peak of the histogram of overnight breathing rate estimates, as shown in FIG. 58A. On this basis, one can define breathing rate deviation, the distance between the estimated average NREM breathing rate and the 90%tile of the breathing rate for each epoch, to quantify the deviation of the breathing rate during REM stage from that during NREM stage.

To extract the variability of the breathing rate for each epoch, one can first estimate the trend of breathing rate by applying a low pass filter to the breathing estimates of the whole night, and obtain the detrended breathing rate estimates by subtracting the trend from the original breathing rate estimates. Then, the breathing rate variability is defined and calculated for each epoch as the variance of the detrended estimates normalized by the length of epoch.

FIG. 58B visualizes the distribution of the proposed two features under NREM and REM sleep, respectively. As one can see from FIG. 58B, the majority of the breathing rate variability and breathing rate deviation of NREM sleep are much smaller than those of REM sleep. Based on these two features, one can train a support vector machine (SVM), a widely used binary classifier, to differentiate between REM and NREM sleep.

Sleep Quality Assessment

When one obtains the estimates of wake, REM, and NREM stages of a whole sleep, one can assess the elusive sleep quality for a user by following standard approach used in clinical practice. In particular, one can calculate the sleep score for each night based on the recognized sleep stages as follows. Let $T_N$, $T_R$ and $T_W$ denote the durations (measured in hours) of NREM sleep, REM sleep and wake, respectively. Since there is no standard formula for sleep score calculation, a simple formula for the sleep score is applied in SMARS:

$$S = 10 * T_N + 20 * T_R - 10 * T_W, \tag{26}$$

which means that the more you sleep, the more you have REM sleep, the less you keep awake in the bed, the better your sleep score is. According to recent research, REM sleep is crucial for mental recovery, and thus a higher weight has been assigned to REM sleep.

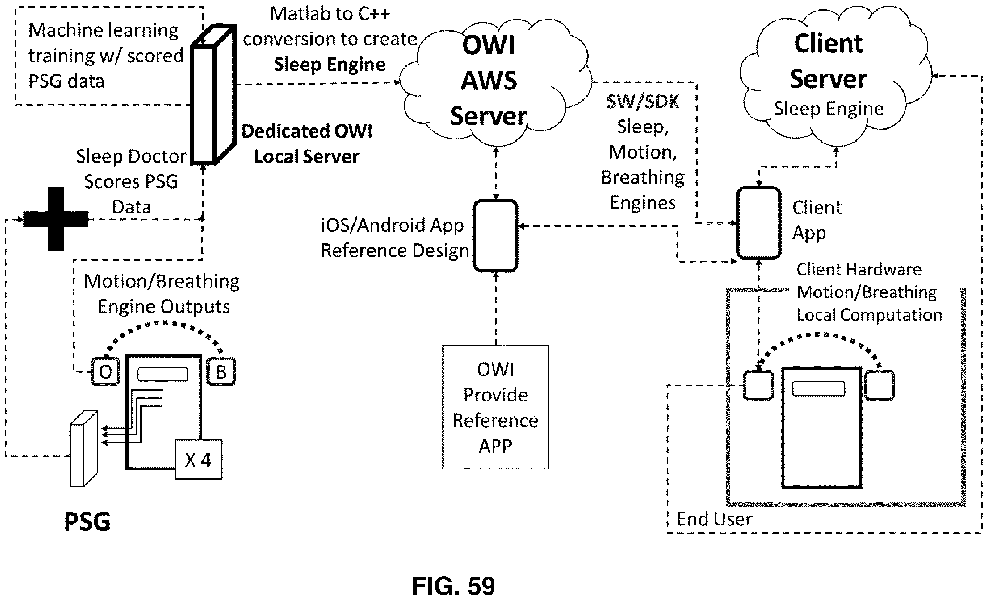

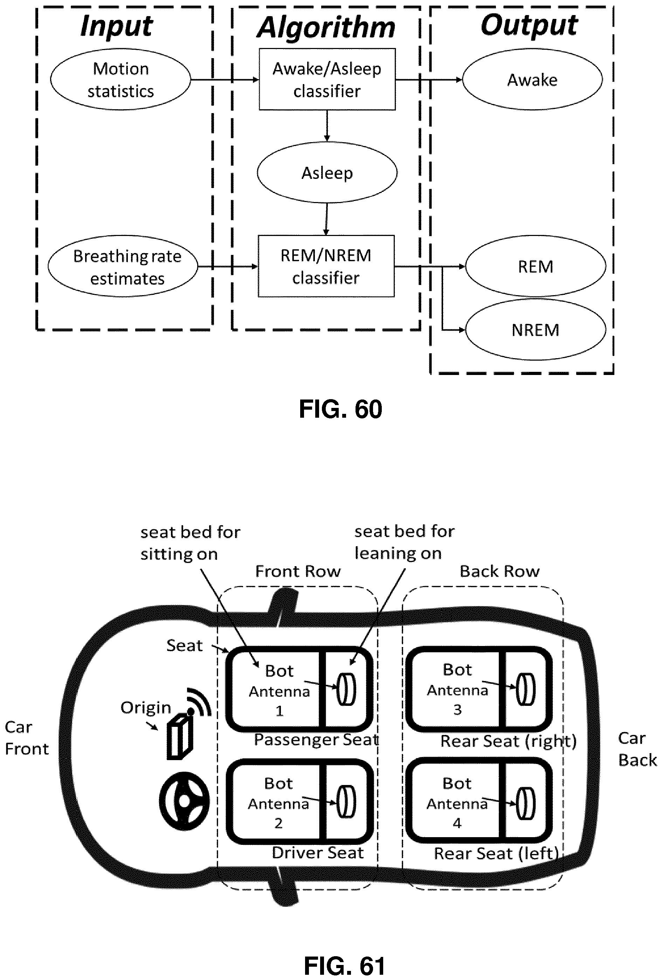

SMARS envisions a practical sleep monitoring for daily in-home use. Although it does not make much sense to compare the sleep score among different users, the trend or history of the sleep score for a particular user would reflect the changes of his/her sleep quality. Such results provide clinically meaningful evidences to help diagnose sleep disorders and manage personal health, in an attractive way. FIG. 59 illustrates an exemplary network environment for sleep monitoring, according to one embodiment of the present teaching. FIG. 60 illustrates an exemplary algorithm design for sleep monitoring, according to one embodiment of the present teaching.

FIG. 61 illustrates an exemplary interior bird-view of a car for seat occupancy detection and people counting, according to one embodiment of the present teaching. In an example, one or more Type1 devices and multiple Type2 devices (e.g. N=4) may be placed in a confined area (e.g. closed area such as a car, a conference room, a bus, an airplane, or a cinema, semi-open area such as a bus with windows open, or a balcony with 8 outdoor chairs around an outdoor table) with multiple "seats" at fixed locations (may be a space for standing, sitting, kneeling, lying down, etc.,) that may hold a person. A presence of a person at one or more "seats" may be detected based on TSCI extracted from wireless signals sent from the Type1 devices to the Type2 devices.

FIG. 61 shows a particular example of a car with 4 seats, two in front row and two in back row. Note that each seat has a "seat bed" for a person to sit on and a "seat back" for a person to lean back on. The Type1 device may be placed in the front, on the dash board. Four Type2 devices may be deployed, one at each of the 4 seats (e.g. in/on/under the seat bed, or in/on/under the seat bed). When a seat A (e.g. driver seat, or right seat on front row, or back row left seat, etc.) is occupied by the driver or a passenger or a baby in a car-seat, the CI (CI) associated with the occupied seat A may behave differently (e.g. become smaller or bigger) from the CI associated with an empty seat A. Thus one can detect the seat occupancy of each seat by examining the CI. By performing such test for all the seats, one can count the amount of people in the car. If a person sits at non-standard locations (e.g. between two seats, in the center of back row, in the center of front row, or a baby in a car seat), CI associated multiple Type2 devices can be analyzed jointly to determine if there is a person there. As signature of a baby in car-seat may be different from an adult or a children, adult/children/baby classification may be performed based on the CI.

A task may be performed based on the seat occupancy described above. For example, the task may be to arm an air-bag if a seat is occupied, but disarm the airbag if the seat is not occupied. If a small size person (e.g. a child) instead of a regular-size adult is detected, an air-bag designed for adults may not be armed. The heating/air-condition setting may be adjusted. The task may be to control windows, lighting, audio system, entertainment system (e.g. video), noise cancelling, shock-absorbing system, stabilizing size, car avoidance system, safety features, tire pressure, any other car subsystems, etc. For example, if a passenger is detected at the front right seat, the temperature at that region (front, right) may be controlled to a preset level. If the seat is empty, the temperature may be adjusted differently.

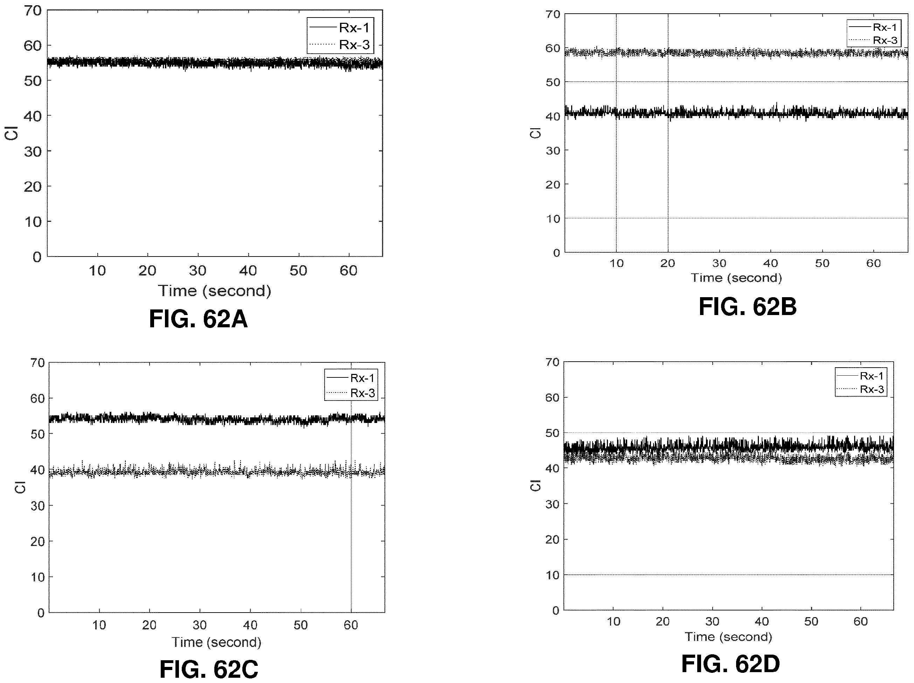

FIGs. 62A-62D illustrate changes of CI (CI) according to various seat occupancy situations in a car, according to one embodiment of the present teaching. FIG. 62A shows CI when no seat is occupied. FIG. 62B shows CI when seat 1 (e.g. seat with Bot antenna 1 in FIG. 61) is occupied. FIG. 62C shows CI when seat 3 (e.g. seat with Bot antenna 3 in FIG. 61) is occupied. FIG. 62D shows CI when both seat 1 and seat 3 are occupied.

The features described above may be implemented advantageously in one or more computer programs that are executable on a programmable system including at least one programmable processor coupled to receive data and instructions from, and to transmit data and instructions to, a data storage system, at least one input device, and at least one output device. A computer program is a set of instructions that may be used, directly or indirectly, in a computer to perform a certain activity or bring about a certain result. A computer program may be written in any form of programming language (e.g., C, Java), including compiled or interpreted languages, and it may be deployed in any form, including as a stand-alone program or as a module, component, subroutine, a browser-based web application, or other unit suitable for use in a computing environment.

Suitable processors for the execution of a program of instructions include, e.g., both general and special purpose microprocessors, digital signal processors, and the sole processor or one of multiple processors or cores, of any kind of computer. Generally, a processor will receive instructions and data from a read-only memory or a random access memory or both. The essential elements of a computer are a processor for executing instructions and one or more memories for storing instructions and data. Generally, a computer will also include, or be operatively coupled to communicate with, one or more mass storage devices for storing data files; such devices include magnetic disks, such as internal hard disks and removable disks; magneto-optical disks; and optical disks. Storage devices suitable for tangibly embodying computer program instructions and data include all forms of non-volatile memory, including by way of example semiconductor memory devices, such as EPROM, EEPROM, and flash memory devices; magnetic disks such as internal hard disks and removable disks; magneto-optical disks; and CD-ROM and DVD-ROM disks. The processor and the memory may be supplemented by, or incorporated in, ASICs (application-specific integrated circuits).

While the present teaching contains many specific implementation details, these should not be construed as limitations on the scope of the present teaching but rather as descriptions of features specific to particular embodiments of the present teaching. Certain features that are described in this specification in the context of separate embodiments may also be implemented in combination in a single embodiment. Conversely, various features that are described in the context of a single embodiment may also be implemented in multiple embodiments separately or in any suitable subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems may generally be integrated together in a single software product or packaged into multiple software products.

Particular embodiments of the subject matter have been described. In some cases, the actions recited in the claims may be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

**1.** A system for monitoring object motion in a venue, comprising:
a receiver (2614) located at a second position in the venue and configured for:

receiving a second wireless signal through a wireless multipath channel impacted by a pseudo-periodic motion of an object in the venue,
wherein a transmitter (2612) located at a first position in the venue transmits a first wireless signal through the wireless multipath channel,
wherein the second wireless signal is determined based on both the wireless multipath channel impacted by the pseudo-periodic motion of the object in the venue and the first wireless signal, and
extracting at least one time series of channel information (CI) of the wireless multipath channel from the second wireless signal,
wherein each CI comprises at least one of: a channel state information (CSI), a frequency domain CSI, a frequency domain CSI associated with at least one sub-band, a time domain CSI, a channel impulse response (CIR), a channel frequency response (CFR), a channel characteristics, and a channel filter response, of the wireless multipath channel; and
a vital sign estimator (3610) configured for:

determining that at least one portion of the at least one time series of CI (TSCI) in a current sliding time window is associated with the pseudo-periodic motion of the object in the venue, and
computing a current characteristics related to the pseudo-periodic motion of the object in the current sliding time window based on at least one of:

the at least one portion of the at least one TSCI in the current sliding time window, at least one portion of the at least one TSCI in a past sliding time window, and a past characteristics related to the pseudo-periodic motion of the object in the past sliding time window,
wherein at least one of the current characteristics and the past characteristics comprises information related to at least one of: a frequency of pseudo-periodic motion, a frequency characteristics, a frequency spectrum, a time period of pseudo periodic motion, a temporal characteristics, a temporal profile, a timing of pseudo-periodic motion; a starting time, an ending time, a duration, a history of motion, a motion type, a motion classification, a location of the object, a speed; a displacement, an acceleration, a rotational speed, a rotational characteristics, a gait cycle of the object, a transient behavior of the object, a transient motion, a change in pseudo-periodic motion, a change in frequency of pseudo-periodic motion, a change in gait cycle, an event associated with pseudo-periodic motion, an event associated with transient motion, a sudden-motion event, and a fall-down event;
wherein the vital sign estimator (3610) comprises a channel information processor (3710) configured for, for each CI of a particular portion of the at least one TSCI in the current sliding time window:

obtaining N1 frequency domain components of the CI;
determining a timestamp associated with the CI; and
preprocessing the N1 frequency domain components, wherein the preprocessing comprises:

cleaning phases of the N1 frequency domain components, and
normalizing the N1 frequency domain components such that the N1 frequency domain components have a unity total power,

wherein the current characteristics related to the pseudo-periodic motion of the object are computed based on the preprocessed N1 frequency domain components of the CI of the particular portion of the at least one TSCI in the current sliding time window.

2. The system according to any previous claim, wherein the vital sign estimator (3610) comprises a spectral analyzer configured for:
for each CI of a particular portion of the at least one TSCI in the current sliding time window:

converting N1 frequency domain components of the channel information using an inverse frequency transform to N2 time domain coefficients of the channel information, wherein: each channel information is associated with a timestamp, N2 is not smaller than N1, each of the N2 coefficients is associated with a time delay, and the inverse frequency transform comprises at least one of: inverse Fourier transform, inverse Laplace transform, inverse Hadamard transform, inverse Hilbert transform, inverse sine transform, inverse cosine transform, inverse triangular transform, inverse wavelet transform, inverse integer transform, inverse power-of-2 transform, combined zero padding and transform, and inverse Fourier transform with zero padding, and
retaining first C of the N2 time domain coefficients, wherein C is not larger than N2; and
correcting timestamps of all channel information of the particular portion of the at least one TSCI in the current sliding time window so that the corrected timestamps of time-corrected channel information are uniformly spaced in time, wherein correcting the timestamps comprises:

identifying a particular CI with a particular timestamp to be replaced by a time-corrected CI with a corrected timestamp, and
computing the time-corrected CI by computing the C retained time domain coefficients of the time-corrected CI at the corrected timestamp using an interpolation filter associated with the corrected timestamp,
wherein the current characteristics related to the pseudo-periodic motion of the object is computed based on the C retained time domain coefficients of the CI of the particular portion of the at least one TSCI in the current sliding time window.

3. The system of claim 2, wherein the spectral analyzer is further configured for:
for each of the C retained time domain coefficients:

identifying a corresponding retained time domain coefficient of each CI of the particular portion of the at least one TSCI in the current sliding time window,
applying bandpass filtering to all the corresponding identified retained time domain coefficients, and
applying a frequency transform to all the corresponding identified retained time domain coefficients, wherein a length of the frequency transform is not smaller than a length of the portion,
wherein the current characteristics related to the pseudo-periodic motion of the object is computed based on outputs of the frequency transform applied to each corresponding retained time domain coefficient of the CI of the particular portion of the at least one TSCI in the current sliding time window.

4. The system according to any previous claim, wherein the vital sign estimator (3610) further comprises an energy spectrum normalizer, and wherein a spectral analyzer and the energy spectrum normalizer are configured for:

processing the outputs of a frequency transform applied to each corresponding retained time domain coefficient of the CI of the particular portion of the at least one TSCI in the current sliding time window,
wherein the frequency transform comprises at least one of: Fourier transform, Laplace transform, Hadamard transform, Hilbert transform, sine transform, cosine transform, triangular transform, wavelet transform, integer transform, power-of-2 transform, combined zero padding and transform, and Fourier transform with zero padding,
wherein the processing comprises at least one of: preprocessing, processing, post-processing, filtering, linear filtering, nonlinear filtering, folding, grouping, energy computation, low-pass filtering, bandpass filtering, high-pass filtering, matched filtering, enhancement, restoration, de-noising, spectral analysis, inverse linear transform, nonlinear transform, feature extraction, machine learning, recognition, labeling, training, clustering, sorting, thresholding, comparison with time-corrected channel information of another portion of another time series of channel information in another sliding time window, similarity score computation, vector quantization, compression, encryption, coding, storing, transmitting, representing, merging, combining, splitting; restricting to selected frequency band, spectrum folding, averaging, weighted averaging, averaging over selected frequency, averaging over selected time domain coefficients, averaging over antenna links, weighted averaging over the at least one TSCI, and weighted averaging over the C retained time domain coefficients,
wherein the averaging over antenna links is weighted averaging over the at least one TSCI; and
wherein the averaging over selected time domain coefficients is weighted averaging over the C retained time domain coefficients.

5. The system of according to any previous claim, wherein the vital sign estimator (3610) further comprises a peak detector configured for:

identifying at least one local maximum and at least one local minimum in frequency domain;
computing at least one local signal-to-noise-ratio-like (SNR-like) parameter for each pair of a local maximum and a local minimum adjacent to each other;
identifying significant local peaks each being at least one of: a local maximum with SNR-like parameter greater than a first threshold T1 and a local maximum with an amplitude greater than a threshold T2.

6. The system according to any previous claim, wherein the vital sign estimator (3610) further comprises a periodic motion rate estimator configured for:

selecting a set of selected significant local peaks from a set of identified significant local peaks based on a selection criterion,
wherein the current characteristics related to the pseudo-periodic motion of the object is computed based on the set of selected significant local peaks and frequency values associated with the set of selected significant local peaks.

7. The system according to any previous claim, wherein the vital sign estimator (3610) further comprises a periodic motion rate estimator configured for:

computing information associated with a remaining spectrum with a set of selected significant local peaks removed; and

detecting an event associated with the current characteristics related to the pseudo-periodic motion of the object based on at least one of: a remaining spectral energy of the remaining spectrum, an adaptive threshold, and a finite state machine, where the event comprises at least one of: a presence, an absence, an appearance, a disappearance, a steady behavior and a non-steady behavior of at least one of: non-periodic motion, transient motion, strong motion, weak motion; strong background interference and another periodic motion.

8. The system according to any previous claim, wherein a system state controller (3620) is configured for:

entering an initiation (INIT) state of a finite state machine (FSM) of the system;
in the initiation state of the FSM:

computing a number of thresholds adaptively in a first way,
detecting the event based on the number of thresholds and at least one of: a set of selected significant local peaks and related characteristics,
detecting an excessive background interfering motion based on the number of thresholds and a remaining spectral energy,
staying in the INIT state when the event is concluded to be "not detected" and the excessive background interfering motion is concluded to be "not detected",
transitioning from the INIT state to a Verification state of the FSM when the event is concluded to be "detected" preliminarily and the detected event needs to be verified, and
transitioning from the INIT state to a Motion state of the FSM when the excessive background interfering motion is concluded to be "detected";
in the Motion state of the FSM:

computing the number of thresholds adaptively in a fourth way,
detecting the excessive background interfering motion based on the number of thresholds and a remaining spectral energy,
staying in the Motion state when the excessive background interfering motion is concluded as "detected", and
transitioning from the Motion state to the INIT state when the excessive background interfering motion is concluded as "not detected" for a number of time instances.

9. The system according to any previous claim, wherein a system state controller (3620) is further configured for:
in the Verification state of the FSM:

computing a number of thresholds adaptively in a second way,
accumulating and computing at least one statistics based on sets of selected significant local peaks and related characteristics in at least one adjacent sliding time window for verification of the detected event that is detected preliminarily,
staying in the Verification state while the at least one statistics is being accumulated until sufficient statistics is collected for the verification,
verifying the preliminarily detected event based on the number of thresholds and the at least one statistics,
transitioning from the Verification state to a PeakFound state of the FSM when the preliminarily detected event is concluded as "verified",
transitioning from the Verification state to the INIT state when verification is concluded to be "not verified", and
staying in the Verification state when verification is not concluded;
in the PeakFound state of the FSM:

computing the number of thresholds adaptively in a third way,
detecting the verified event based on the number of thresholds and the at least one of: the sets of selected significant local peaks and related characteristics,
detecting an excessive background interfering motion based on the number of thresholds and a remaining spectral energy,
staying in the PeakFound state when the verified event is concluded as "detected",
transitioning from the PeakFound state to the INIT state when the verified event is concluded as "not detected" for a number of time instances, and

transitioning from the PeakFound state to the Motion state when the excessive background interfering motion is concluded as "detected" for a number of time instances.

10. The system according to any previous claim, further comprising:

an additional transmitter located at a third position in the venue and configured for transmitting a third wireless signal through the wireless multipath channel impacted by the pseudo-periodic motion of the object in the venue; an additional receiver located at a fourth position in the venue and configured for:

receiving a fourth wireless signal through the wireless multipath channel, wherein the fourth wireless signal is determined based on both the wireless multipath channel and the third wireless signal, and obtaining an additional TSCI of the wireless multipath channel based on the fourth wireless signal, wherein the vital sign estimator (3610) is further configured for:

determining that at least one portion of the additional TSCI in the current sliding time window is associated with the pseudo-periodic motion of the object in the venue, and computing the current characteristics related to the pseudo-periodic motion of the object in the current sliding time window based on at least one of:

the at least one portion of the additional TSCI in the current sliding time window, at least one portion of the additional TSCI in an additional past sliding time window, and a past characteristics related to the pseudo-periodic motion of the object in the additional past sliding time window.

11. The system according to any previous claim, wherein the vital sign estimator (3610) is further configured for:

determining that at least one portion of the at least one TSCI in the current sliding time window is associated with an additional pseudo-periodic motion of an additional object in the venue, wherein the wireless multipath channel is further impacted in the current sliding time window by the additional pseudo-periodic motion of the additional object; and computing a current characteristics related to the additional pseudo-periodic motion of the additional object in the current sliding time window based on at least one of: the at least one portion of the at least one TSCI in the current sliding time window, the at least one portion of the at least one TSCI in the past sliding time window, and a past characteristics related to the additional pseudo-periodic motion of the additional object in the past sliding time window.

12. The system according to any previous claim, wherein the vital sign estimator (3610) is further configured for:

determining that the wireless multipath channel is impacted in the current sliding time window by pseudo-periodic motions of a plurality of objects; computing current characteristics related to pseudo-periodic motions of the plurality of objects in the current sliding time window based on at least one of: the at least one portion of the at least one TSCI in the current sliding time window, the at least one portion of the at least one TSCI in the past sliding time window, and a past characteristics related to the pseudo-periodic motions of the plurality of objects in the past sliding time window; and estimating a quantity of the plurality of objects based on the current characteristics.

13. A method performed by a system according to any previous claim.

14. A receiver (2614) of a system according to any previous claim, comprising the vital sign estimator (3610) of the system.

**Patentansprüche**

1. System zum Überwachen einer Objektbewegung in einer Lokalität, umfassend:
einen Empfänger (2614), welcher an einer zweiten Position in der Lokalität angeordnet und eingerichtet ist zum:

Empfangen eines zweiten drahtlosen Signals durch einen drahtlosen Mehrfachpfad-Kanal, welcher durch eine pseudo-periodische Bewegung eines Objekts in der Lokalität beeinflusst ist,

wobei eine Übertragungseinheit (2612), welche an einer ersten Position in der Lokalität angeordnet ist, ein erstes drahtloses Signal durch den drahtlosen Mehrfachpfad-Kanal überträgt,

wobei das zweite drahtlose Signal auf Grundlage von sowohl dem drahtlosen Mehrfachpfad-Kanal, welcher durch die pseudo-periodische Bewegung des Objekts in der Lokalität beeinflusst wird, als auch dem ersten drahtlosen Signal bestimmt wird, und

Extrahieren von wenigstens einer Zeitserie von Kanalinformationen (CI) des drahtlosen Mehrfachpfad-Kanals aus dem zweiten drahtlosen Signal,

wobei jede CI wenigstens eines umfasst aus: einer Kanal-Zustandsinformation (CSI), einer Frequenzdomänen-CSI, einer Frequenzdomänen-CSI, welche wenigstens einem Unter-Band zugeordnet ist, einer Zeitdomänen-CSI, einer Kanal-Impulsantwort (CIR), einer Kanal-Frequenzantwort (CFR), einer Kanalcharakteristik und einer Kanal-Filterantwort des drahtlosen Mehrfachpfad-Kanals; und

eine Lebenszeichen-Schätzeinheit (3610), welche dazu eingerichtet ist:

zu bestimmen, dass wenigstens ein Abschnitt der wenigstens einen Zeitserie von CI (TSCI) in einem momentanen gleitenden Zeitfenster der pseudo-periodischen Bewegung des Objekts in der Lokalität zugeordnet ist, und

eine momentane Charakteristik im Zusammenhang mit der pseudo-periodischen Bewegung des Objekts in dem momentanen gleitenden Zeitfenster auf Grundlage von wenigstens einem zu berechnen aus:

dem wenigstens einen Abschnitt der wenigstens einen TSCI in dem momentanen gleitenden Zeitfenster, wenigstens einem Abschnitt der wenigstens einen TSCI in einem vergangenen gleitenden Zeitfenster und einer vergangenen Charakteristik im Zusammenhang mit der pseudo-periodischen Bewegung des Objekts in dem vergangenen gleitenden Zeitfenster,

wobei wenigstens eine aus der momentanen Charakteristik und der vergangenen Charakteristik Informationen im Zusammenhang mit wenigstens einem umfasst aus: einer Frequenz der pseudo-periodischen Bewegung, einer Frequenzcharakteristik, einem Frequenzspektrum, einer Zeitperiode einer pseudo-periodischen Bewegung, einer zeitlichen Charakteristik, einem zeitlichen Profil, einer Zeitgabe einer pseudo-periodischen Bewegung; einer Startzeit, einer Endzeit, einer Dauer, einer Historie einer Bewegung, einem Bewegungstyp, einer Bewegungsklassifikation, einer Position des Objekts, einer Geschwindigkeit; einer Verlagerung, einer Beschleunigung, einer Rotationsgeschwindigkeit, einer Rotationscharakteristik, einem Gangzyklus des Objekts, einem vorübergehenden Verhalten des Objekts, einer vorübergehenden Bewegung, einer Änderung in einer pseudo-periodischen Bewegung, einer Änderung in einer Frequenz einer pseudo-periodischen Bewegung, einer Änderung in einem Gangzyklus, einem Ereignis, welches einer pseudo-periodischen Bewegung zugeordnet ist, einem Ereignis, welches einer vorübergehenden Bewegung zugeordnet ist, einem Ereignis einer plötzlichen Bewegung und einem Herabfall-Ereignis;

wobei die Lebenszeichen-Schätzeinheit (3610) einen Kanalinformation-Prozessor (3710) umfasst, welcher für jede CI eines bestimmten Abschnitts der wenigstens einen TSCI in dem momentanen gleitenden Zeitfenster eingerichtet ist zum:

Erhalten von N1 Frequenzdomänen-Komponenten der CI;
Bestimmen eines Zeitstempels, welcher der CI zugeordnet ist; und
Vorverarbeiten der N1 Frequenzdomänen-Komponenten, wobei die Vorverarbeitung umfasst:

Säubern von Phasen der N1 Frequenzdomänen-Komponenten, und
Normalisieren der N1 Frequenzdomänen-Komponenten derart, dass die N1 Frequenzdomänen-Komponenten eine einheitliche Gesamtleistung aufweisen, wobei die momentanen Charakteristiken im Zusammenhang mit der pseudo-periodischen Bewegung des Objekts auf Grundlage der vorverarbeiteten N1 Frequenzdomänen-Komponenten der CI des bestimmten Abschnitts der wenigstens einen TSCI in dem momentanen gleitenden Zeitfenster berechnet werden.

2. System nach einem vorhergehenden Anspruch, wobei die Lebenszeichen-Schätzeinheit (3610) eine Spektralanalyse-Einheit umfasst, welche dazu eingerichtet ist:

für jede CI eines bestimmten Abschnitts der wenigstens einen TSCI in dem momentanen gleitenden Zeitfenster:

N1 Frequenzdomänen-Komponenten der Kanalinformationen unter Verwendung einer inversen Frequenztransformation zu N2 Zeitdomänen-Koeffizienten der Kanalinformationen zu konvertieren, wobei: jede Kanalinformation einem Zeitstempel zugeordnet ist, N2 nicht kleiner als N1 ist, jeder der N2 Koeffizienten einer Zeitverzögerung zugeordnet ist und die inverse Frequenztransformation wenigstens eines umfasst aus: inverser Fourier-Transformation, inverser Laplace-Transformation, inverser Hadamard-Transformation, inverser Hilbert-Transformation, inverser Sinus-Transformation, inverser Cosinus-Transformation, inverser Triangular-Transformation, inverser Wavelet-Transformation, inverser Integer-Transformation, inverser Hoch-2-Transformation, kombiniertem Null-Auffüllen und Transformieren, und inverser Fourier-Transformation mit Null-Auffüllen, und Beibehalten erster C der N2 Zeitdomänen-Koeffizienten, wobei C nicht größer als N2 ist; und

Korrigieren von Zeitstempeln aller Kanalinformationen des bestimmten Abschnitts der wenigstens einen TSCI in dem momentanen gleitenden Zeitfenster, so dass die korrigierten Zeitstempel der zeitkorrigierten Kanalinformationen gleichförmig in der Zeit beabstandet sind, wobei das Korrigieren der Zeitstempel umfasst:

Identifizieren einer bestimmten CI mit einem bestimmten Zeitstempel, um durch eine zeitkorrigierte CI mit einem korrigierten Zeitstempel ersetzt zu werden, und

Berechnen der zeitkorrigierten CI durch Berechnen der C beibehaltenen Zeitdomänen-Koeffizienten der zeitkorrigierten CI bei dem korrigierten Zeitstempel unter Verwendung eines Interpolationsfilters, welcher dem korrigierten Zeitstempel zugeordnet ist,

wobei die momentanen Charakteristiken im Zusammenhang mit der pseudo-periodischen Bewegung des Objekts auf Grundlage der C beibehaltenen Zeitdomänen-Koeffizienten der CI des bestimmten Abschnitts der wenigstens einen TSCI in dem momentanen gleitenden Zeitfenster berechnet werden.

3. System nach Anspruch 2, wobei die Spektralanalyse-Einheit ferner eingerichtet ist zum:
für jeden der C beibehaltenen Zeitdomänen-Koeffizienten:

Identifizieren eines entsprechenden beibehaltenen Zeitdomänen-Koeffizienten von jeder CI des bestimmten Abschnitts der wenigstens einen TSCI in dem momentanen gleitenden Zeitfenster,

Anwenden von Bandpass-Filterung auf alle der entsprechenden identifizierten beibehaltenen Zeitdomänen-Koeffizienten, und

Anwenden einer Frequenztransformation auf alle der entsprechenden identifizierten beibehaltenen Zeitdomänen-Koeffizienten, wobei eine Länge der Frequenztransformierten nicht kleiner als eine Länge des Abschnitts ist,

wobei die momentanen Charakteristiken im Zusammenhang mit der pseudo-periodischen Bewegung des Objekts auf Grundlage von Ausgaben der Frequenztransformation berechnet werden, welche auf jeden entsprechenden beibehaltenen Zeitdomänen-Koeffizienten der CI des bestimmten Abschnitts der wenigstens einen TSCI in dem momentanen gleitenden Zeitfenster angewandt werden.

4. System nach einem vorhergehenden Anspruch, wobei die Lebenszeichen-Schätzeinheit (3610) ferner eine Energiespektrum-Normalisierungseinheit umfasst, und wobei eine Spektralanalyse-Einheit und die Energiespektrum-Normalisierungseinheit eingerichtet sind zum:

Verarbeiten der Ausgaben einer Frequenztransformation, welche auf jeden entsprechenden beibehaltenen Zeitdomänen-Koeffizienten der CI des bestimmten Abschnitts der wenigstens einen TSCI in dem momentanen gleitenden Zeitfenster angewandt wird,

wobei die Frequenztransformation wenigstens eines umfasst aus: Fourier-Transformation, Laplace-Transformation, Hadamard-Transformation, Hilbert-Transformation, Sinus-Transformation, Cosinus-Transformation, Triangular-Transformation, Wavelet-Transformation, Integer-Transformation, Hoch-2-Transformation, kombiniertem Null-Auffüllen und Transformieren und Fourier-Transformation mit Null-Auffüllen,

wobei das Verarbeiten wenigstens eines umfasst aus: Vorverarbeitung, Verarbeitung, Nachverarbeitung, Filtern, lineares Filtern, nichtlineares Filtern, Falten, Gruppieren, Energie-Berechnen, Tiefpass-Filtern, Bandpass-Filtern, Hochpass-Filtern, angepasstes Filtern, Erhöhen, Wiederherstellen, Entrauschen, Spektralanalyse, inverse lineare Transformation, nichtlineare Transformation, Merkmalsextraktion, Maschinenlernen, Erkennen, Bezeichnen, Trainieren, Clustern, Sortieren, Schwellenwert-Verarbeitung, Vergleichen mit zeitkorrigierten Kanalinformationen eines anderen Abschnitts einer anderen Zeitserie von Kanalinformationen in einem anderen gleitenden Zeitfenster, Ähnlichkeitswert-Berechnen, Vektor-Quantisieren, Komprimieren, Verschlüsseln, Kodieren, Speichern, Übertragen, Repräsentieren, Zusammenfügen, Kombinieren, Aufteilen;

Beschränken auf ein ausgewähltes Frequenzband, Spektrum-Falten, Mitteln, gewichtetes Mitteln, Mitteln über eine ausgewählte Frequenz, Mitteln über ausgewählte Zeitdomänen-Koeffizienten, Mitteln über Antennenverbindungen, gewichtetes Mitteln über die wenigstens eine TSCI und gewichtetes Mitteln über die C beibehaltenen Zeitdomänen-Koeffizienten,

wobei das Mitteln über Antennenverbindungen ein gewichtetes Mitteln über die wenigstens eine TSCI ist; und

wobei das Mitteln über ausgewählte Zeitdomänen-Koeffizienten ein gewichtetes Mitteln über die C beibehaltenen Zeitdomänen-Koeffizienten ist.

5. System nach einem vorhergehenden Anspruch, wobei die Lebenszeichen-Schätzeinheit (3610) ferner einen Spitzenwert-Detektor umfasst, welcher eingerichtet ist zum:

Identifizieren von wenigstens einem lokalen Maximum und wenigstens einem lokalen Minimum in der Frequenzdomäne;
Berechnen von wenigstens einem lokalen Signal-Rauschverhältnis-artigen (SNR-artigen) Parameter für jedes Paar aus einem lokalen Maximum und einem lokalen Minimum benachbart zueinander;
Identifizieren von signifikanten lokalen Spitzenwerten, welche jeweils wenigstens eines sind aus: einem lokalen Maximum mit einem SNR-artigen Parameter, welcher größer als ein erster Schwellenwert T1 ist, und einem lokalen Maximum mit einer Amplitude, welche größer als ein Schwellenwert T2 ist.

6. System nach einem vorhergehenden Anspruch, wobei die Lebenszeichen-Schätzeinheit (3610) ferner eine Raten-Schätzeinheit für eine periodische Bewegung umfasst, welche eingerichtet ist zum:

Auswählen eines Satzes von ausgewählten signifikanten lokalen Spitzenwerten aus einem Satz von identifizierten signifikanten lokalen Spitzenwerten auf Grundlage eines Auswahlkriteriums,
wobei die momentanen Charakteristiken im Zusammenhang mit der pseudo-periodischen Bewegung des Objekts auf Grundlage des Satzes von ausgewählten signifikanten lokalen Spitzenwerten und Frequenzwerten berechnet werden,
welche dem Satz von ausgewählten signifikanten lokalen Spitzenwerten zugeordnet sind.

7. System nach einem vorhergehenden Anspruch, wobei die Lebenszeichen-Schätzeinheit (3610) ferner eine Raten-Schätzeinheit für eine periodische Bewegung umfasst, welche eingerichtet ist zum:

Berechnen von Informationen im Zusammenhang mit einem verbleibenden Spektrum mit einem Satz von ausgewählten signifikanten lokalen Spitzenwerten entfernt; und
Detektieren eines Ereignisses, welches den momentanen Charakteristiken im Zusammenhang mit der pseudo-periodischen Bewegung des Objekts zugeordnet ist, auf Grundlage von wenigstens einem aus: einer verbleibenden spektralen Energie des verbleibenden Spektrums, einem adaptiven Schwellenwert und einer finiten Zustandsmaschine, wobei das Ereignis wenigstens eines umfasst aus: einer Anwesenheit, einer Abwesenheit, einem Erscheinen, einem Verschwinden, einem beständigen Verhalten und einem unbeständigen Verhalten von wenigstens einem aus: einer nicht-periodischen Bewegung, einer Übergangsbewegung, einer starken Bewegung, einer schwachen Bewegung; starker Hintergrund-Interferenz und einer anderen periodischen Bewegung.

8. System nach einem vorhergehenden Anspruch, wobei eine Systemzustand-Steuereinheit (3620) eingerichtet ist zum:

Eintreten in einen Initialisierungs- (INIT) Zustand einer finiten Zustandsmaschine (FSM) des Systems;
In dem Initialisierungszustand der FSM:

adaptives Berechnen einer Anzahl von Schwellenwerten auf eine erste Weise, Detektieren des Ereignisses auf Grundlage der Anzahl der Schwellenwerte und von wenigstens einem aus: einem Satz von ausgewählten signifikanten lokalen Spitzenwerten und verwandten Charakteristiken,
Detektieren einer übermäßigen Hintergrund-Interferenzbewegung auf Grundlage der Anzahl der Schwellenwerte und einer verbleibenden spektralen Energie, Verbleiben in dem INIT-Zustand, wenn für das Ereignis geschlossen wird, dass es "nicht detektiert" ist, und für die übermäßige Hintergrund-Interferenzbewegung geschlossen wird, dass sie "nicht detektiert" ist,
Übergehen von dem INIT-Zustand zu einem Verifikationszustand der FSM, wenn für das Ereignis geschlossen wird, dass es vorläufig "detektiert" ist und das detektierte Ereignis verifiziert werden muss, und
Übergehen von dem INIT-Zustand zu einem Bewegungszustand der FSM, wenn für die übermäßige Hintergrund-Interferenzbewegung geschlossen wird, dass sie "detektiert" ist;
in dem Bewegungszustand der FSM:

adaptives Berechnen der Anzahl von Schwellenwerten auf eine vierte Weise, Detektieren der exzessiven Hintergrund-Interferenzbewegung auf Grundlage der Anzahl von Schwellenwerten und einer verbleibenden spektralen Energie, Verbleiben in dem Bewegungszustand, wenn für die übermäßige Hintergrund-Interferenzbewegung geschlossen wird, dass sie "detektiert" ist, und

Übergehen von dem Bewegungszustand zu dem INIT-Zustand, wenn für die übermäßige Hintergrund-Interferenzbewegung geschlossen wird, dass sie für eine Anzahl von Zeitinstanzen "nicht detektiert" ist.

**9.** System nach einem vorhergehenden Anspruch, wobei eine Systemzustand-Steuereinheit (3620) ferner eingerichtet ist zum:

in dem Verifikationszustand der FSM:

adaptives Berechnen einer Anzahl von Schwellenwerten auf eine zweite Weise, Akkumulieren und Berechnen von wenigstens einer Statistik auf Grundlage von Sätzen von ausgewählten signifikanten lokalen Spitzenwerten und verwandten Charakteristiken in wenigstens einem benachbarten gleitenden Zeitfenster für eine Verifikation des detektierten Ereignisses, welches vorläufig detektiert ist,

Verbleiben in dem Verifikationszustand, während die wenigstens eine Statistik akkumuliert wird, bis ausreichende Statistiken für die Verifikation gesammelt sind, Verifizieren des vorläufig detektierten Ereignisses auf Grundlage der Anzahl von Schwellenwerten und der wenigstens einen Statistik,

Übergehen von dem Verifikationszustand zu einem SchwellenwertGefunden-Zustand der FSM, wenn für das vorläufig detektierte Ereignis geschlossen wird, dass es "verifiziert" ist,

Übergehen von dem Verifikationszustand zu dem INIT-Zustand, wenn für die Verifikation geschlossen wird, dass sie "nicht verifiziert" ist, und

Verbleiben in dem Verifikationszustand, wenn die Verifikation nicht geschlossen wird;

In dem SchwellenwertGefunden-Zustand der FSM:

adaptives Berechnen der Anzahl von Schwellenwerten auf eine dritte Weise, Detektieren des verifizierten Ereignisses auf Grundlage der Anzahl von Schwellenwerten und dem wenigstens einem aus: den Sätzen von ausgewählten signifikanten lokalen Spitzenwerten und verwandten Charakteristiken,

Detektieren einer übermäßigen Hintergrund-Interferenzbewegung auf Grundlage der Anzahl von Schwellenwerten und einer verbleibenden spektralen Energie,

Verbleiben in dem SchwellenwertGefunden-Zustand, wenn für das verifizierte Ereignis geschlossen wird, dass es "detektiert" ist,

Übergehen von dem SchwellenwertGefunden-Zustand zu dem INIT-Zustand, wenn für das verifizierte Ereignis geschlossen wird, dass es für eine Anzahl von Zeitinstanzen "nicht detektiert" ist, und

Übergehen von dem SchwellenwertGefunden-Zustand zu dem Bewegungszustand, wenn für die übermäßige Hintergrund-Interferenzbewegung geschlossen wird, dass sie für eine Anzahl von Zeitinstanzen "detektiert" ist.

**10.** System nach einem vorhergehenden Anspruch, ferner umfassend:

ein zusätzliches Übertragungselement, welches an einer dritten Position in der Lokalität angeordnet und dazu eingerichtet ist, ein drittes drahtloses Signal durch den drahtlosen Mehrfachpfad-Kanal zu übertragen, welcher durch die pseudo-periodische Bewegung des Objekts in der Lokalität beeinflusst wird;

einen zusätzlichen Empfänger, welcher an einer vierten Position in der Lokalität angeordnet und eingerichtet ist zum:

Empfangen eines vierten drahtlosen Signals durch den drahtlosen Mehrfachpfad-Kanal, wobei das vierte drahtlose Signal auf Grundlage von sowohl dem drahtlosen Mehrfachpfad-Kanal als auch dem dritten drahtlosen Signal bestimmt wird, und

Erhalten der zusätzlichen TSCI des drahtlosen Mehrfachpfad-Kanals auf Grundlage des vierten drahtlosen Signals,

wobei die Lebenszeichen-Schätzeinheit (3610) ferner eingerichtet ist zum:

Bestimmen, dass wenigstens ein Abschnitt der zusätzlichen TSCI in dem momentanen gleitenden Zeitfenster der pseudo-periodischen Bewegung des Objekts in der Lokalität zugeordnet ist, und

Berechnen der momentanen Charakteristiken im Zusammenhang mit der pseudo-periodischen Bewegung des Objekts in dem momentanen gleitenden Zeitfenster auf Grundlage von wenigstens einem aus: dem wenigstens einen Abschnitt der zusätzlichen TSCI in dem momentanen gleitenden Zeitfenster, wenigstens einem Abschnitt der zusätzlichen TSCI in einem zusätzlichen vergangenen gleitenden Zeitfenster und einer vergangenen Charakteristik im Zusammenhang mit der pseudo-periodischen Bewegung des Objekts in dem zusätzlichen vergangenen gleitenden Zeitfenster.

11. System nach einem vorhergehenden Anspruch, wobei die Lebenszeichen-Schätzeinheit (3610) ferner eingerichtet ist zum:

Bestimmen, dass wenigstens ein Abschnitt der wenigstens einen TSCI in dem momentanen gleitenden Zeitfenster einer zusätzlichen pseudo-periodischen Bewegung eines zusätzlichen Objekts in der Lokalität zugeordnet ist, wobei der drahtlose Mehrfachpfad-Kanal ferner in dem momentanen gleitenden Zeitfenster durch die zusätzliche pseudo-periodische Bewegung des zusätzlichen Objekts beeinflusst ist; und
Berechnen einer momentanen Charakteristik im Zusammenhang mit der zusätzlichen pseudo-periodischen Bewegung des zusätzlichen Objekts in dem momentanen gleitenden Zeitfenster auf Grundlage von wenigstens einem aus: dem wenigstens einen Abschnitt der wenigstens einen TSCI in dem momentanen gleitenden Zeitfenster, dem wenigstens einen Abschnitt der wenigstens einen TSCI in dem vergangenen gleitenden Zeitfenster und einer vergangenen Charakteristik im Zusammenhang mit der zusätzlichen pseudo-periodischen Bewegung des zusätzlichen Objekts in dem vergangenen gleitenden Zeitfenster.

12. System nach einem vorhergehenden Anspruch, wobei die Lebenszeichen-Schätzeinheit (3610) ferner eingerichtet ist zum:

Bestimmen, dass der drahtlose Mehrfachpfad-Kanal in dem momentanen gleitenden Zeitfenster durch pseudo-periodische Bewegungen einer Mehrzahl von Objekten beeinträchtigt ist;
Berechnen von momentanen Charakteristiken im Zusammenhang mit pseudo-periodischen Bewegungen der Mehrzahl von Objekten in dem momentanen gleitenden Zeitfenster auf Grundlage von wenigstens einem aus: dem wenigstens einen Abschnitt der wenigstens einen TSCI in dem momentanen gleitenden Zeitfenster, dem wenigstens einen Abschnitt der wenigstens einen TSCI in dem vergangenen gleitenden Zeitfenster und einer vergangenen Charakteristik im Zusammenhang mit den pseudo-periodischen Bewegungen der Mehrzahl von Objekten in dem vergangenen gleitenden Zeitfenster; und
Schätzen einer Anzahl der Mehrzahl von Objekten auf Grundlage der momentanen Charakteristiken.

13. Verfahren, welches von einem System nach einem vorhergehenden Anspruch durchgeführt wird.

14. Empfänger (2614) eines Systems nach einem vorhergehenden Anspruch, umfassend die Lebenszeichen-Schätzeinheit (3610) des Systems.

**Revendications**

1. Système de surveillance du mouvement d'un objet dans un lieu, comprenant :
un récepteur (2614) situé à une deuxième position dans le lieu et configuré de manière à :

recevoir un deuxième signal sans fil par le biais d'un canal à trajets multiples sans fil affecté par un mouvement pseudo-périodique d'un objet dans le lieu ;
dans lequel un émetteur (2612) situé à une première position dans le lieu transmet un premier signal sans fil par le biais du canal à trajets multiples sans fil ;
dans lequel le deuxième signal sans fil est déterminé sur la base, à la fois, du canal à trajets multiples sans fil affecté par le mouvement pseudo-périodique de l'objet dans le lieu, et du premier signal sans fil ; et
extraire au moins une série temporelle d'informations de canal (CI) du canal à trajets multiples sans fil à partir du deuxième signal sans fil ;
dans lequel chaque information CI comprend au moins l'un des éléments parmi : une information d'état de canal (CSI), une information CSI de domaine fréquentiel, une information CSI de domaine fréquentiel associée à au moins une sous-bande, une information CSI de domaine temporel, une réponse impulsionnelle de canal (CIR), une réponse en fréquence de canal (CFR), une caractéristique de canal, et une réponse de filtre de canal, du canal à trajets multiples sans fil ; et
un estimateur de signes vitaux (3610) configuré de manière à :

déterminer qu'au moins une partie de ladite au moins une série temporelle d'informations CI (TSCI) dans une fenêtre temporelle glissante actuelle est associée au mouvement pseudo-périodique de l'objet dans le lieu ; et

calculer une caractéristique actuelle connexe au mouvement pseudo-périodique de l'objet dans la fenêtre temporelle glissante actuelle sur la base d'au moins l'un des éléments parmi : ladite au moins une partie de ladite au moins une série temporelle TSCI dans la fenêtre temporelle glissante actuelle, au moins une partie de ladite au moins une série temporelle TSCI dans une fenêtre temporelle glissante passée, et une caractéristique passée connexe au mouvement pseudo-périodique de l'objet dans la fenêtre temporelle glissante passée ;

dans lequel au moins l'une de la caractéristique actuelle et de la caractéristique passée comprend des informations connexes à au moins l'un des éléments parmi : une fréquence de mouvement pseudo-périodique, une caractéristique de fréquence, un spectre de fréquence, une période de temps de mouvement pseudo-périodique, une caractéristique temporelle, un profil temporel, une temporisation de mouvement pseudo-périodique ; une heure de début, une heure de fin, une durée, un historique de mouvement, un type de mouvement, une classification de mouvement, un emplacement de l'objet, une vitesse ; un déplacement, une accélération, une vitesse de rotation, une caractéristique de rotation, un cycle de marche de l'objet, un comportement transitoire de l'objet, un mouvement transitoire, un changement dans le mouvement pseudo-périodique, un changement dans la fréquence de mouvement pseudo-périodique, un changement dans le cycle de marche, un événement associé au mouvement pseudo-périodique, un événement associé au mouvement transitoire, un événement de mouvement soudain, et un événement de chute ;

dans lequel l'estimateur de signes vitaux (3610) comprend un processeur d'informations de canal (3710) configuré de manière à, pour chaque information CI d'une partie particulière de ladite au moins une série temporelle TSCI dans la fenêtre temporelle glissante actuelle :

obtenir N1 composantes de domaine fréquentiel des informations CI ;
déterminer un horodatage associé aux informations CI ; et
prétraiter les N1 composantes de domaine fréquentiel, dans lequel le prétraitement comprend le fait de :

nettoyer des phases des N1 composantes de domaine fréquentiel ; et
normaliser les N1 composantes de domaine fréquentiel de sorte que les N1 composantes de domaine fréquentiel présentent une puissance totale unitaire ;

dans lequel la caractéristique actuelle connexe au mouvement pseudo-périodique de l'objet est calculée sur la base des N1 composantes de domaine fréquentiel prétraitées de l'information CI de la partie particulière de ladite au moins une série temporelle TSCI dans la fenêtre temporelle glissante actuelle.

2. Système selon l'une quelconque des revendications précédentes, dans lequel l'estimateur de signes vitaux (3610) comprend un analyseur spectral configuré de manière à :
pour chaque information CI d'une partie particulière de ladite au moins une série temporelle TSCI dans la fenêtre temporelle glissante actuelle :

convertir N1 composantes de domaine fréquentiel des informations de canal, en faisant appel à une transformée en fréquence inverse, en N2 coefficients de domaine temporel des informations de canal, dans lequel : chaque information de canal est associée à un horodatage, N2 n'est pas inférieur à N1, chacun des N2 coefficients est associé à un retard temporel, et la transformée en fréquence inverse comprend au moins l'un des éléments parmi : une transformée de Fourier inverse, une transformée de Laplace inverse, une transformée de Hadamard inverse, une transformée de Hilbert inverse, une transformée en sinus inverse, une transformée en cosinus inverse, une transformée triangulaire inverse, une transformée en ondelettes inverse, une transformée en nombres entiers inverse, une transformée en puissance de 2 inverse, un bourrage de zéros et une transformée combinés, et une transformée de Fourier inverse avec bourrage de zéros ; et
conserver un premier nombre C des N2 coefficients de domaine temporel, dans lequel C n'est pas supérieur à N2 ; et
corriger les horodatages de toutes les informations de canal de la partie particulière de ladite au moins une série temporelle TSCI dans la fenêtre temporelle glissante actuelle de sorte que les horodatages corrigés des informations de canal à temps corrigé sont uniformément espacés dans le temps, dans lequel la correction des horodatages comprend le fait de :

identifier une information CI particulière avec un horodatage particulier à remplacer par une information CI à temps corrigé avec un horodatage corrigé ; et

calculer l'information CI à temps corrigé en calculant les C coefficients de domaine temporel conservés de l'information CI à temps corrigé à l'horodatage corrigé, en faisant appel à un filtre d'interpolation associé à l'horodatage corrigé ;

dans lequel la caractéristique actuelle connexe au mouvement pseudo-périodique de l'objet est calculée sur la base des C coefficients de domaine temporel conservés de l'information CI de la partie particulière de ladite au moins une série temporelle TSCI dans la fenêtre temporelle glissante actuelle.

3. Système selon la revendication 2, dans lequel l'analyseur spectral est en outre configuré de manière à : pour chacun des C coefficients de domaine temporel conservés :

identifier un coefficient de domaine temporel conservé correspondant de chaque information CI de la partie particulière de ladite au moins une série temporelle TSCI dans la fenêtre temporelle glissante actuelle ;

appliquer un filtrage passe-bande à tous les coefficients de domaine temporel conservés identifiés correspondants ; et

appliquer une transformée en fréquence à tous les coefficients de domaine temporel conservés identifiés correspondants, dans lequel une longueur de la transformée en fréquence n'est pas inférieure à une longueur de la partie ;

dans lequel la caractéristique actuelle connexe au mouvement pseudo-périodique de l'objet est calculée sur la base de sorties de la transformée en fréquence appliquée à chaque coefficient de domaine temporel conservé correspondant de l'information CI de la partie particulière de ladite au moins une série temporelle TSCI dans la fenêtre temporelle glissante actuelle.

4. Système selon l'une quelconque des revendications précédentes, dans lequel l'estimateur de signes vitaux (3610) comprend en outre un normalisateur de spectre d'énergie, et dans lequel un analyseur spectral et le normalisateur de spectre d'énergie sont configurés de manière à :

traiter les sorties d'une transformée en fréquence appliquée à chaque coefficient de domaine temporel conservé correspondant de l'information CI de la partie particulière de ladite au moins une série temporelle TSCI dans la fenêtre temporelle glissante actuelle ;

dans lequel la transformée en fréquence comprend au moins l'un des éléments parmi : une transformée de Fourier, une transformée de Laplace, une transformée de Hadamard, une transformée de Hilbert, une transformée en sinus, une transformée en cosinus, une transformée triangulaire, une transformée en ondelettes, une transformée en nombres entiers, une transformée en puissance de 2, un bourrage de zéros et une transformée combinés, et une transformée de Fourier avec bourrage de zéros ;

dans lequel le traitement comprend au moins l'un des éléments parmi : un prétraitement, un traitement, un post-traitement, un filtrage, un filtrage linéaire, un filtrage non linéaire, un repliement, un groupement, un calcul d'énergie, un filtrage passe-bas, un filtrage passe-bande, un filtrage passe-haut, un filtrage adapté, une amélioration, une restauration, un débruitage, une analyse spectrale, une transformée linéaire inverse, une transformée non linéaire, une extraction de caractéristiques, un apprentissage automatique, une reconnaissance, un étiquetage, un apprentissage, un regroupement, un tri, un seuillage, une comparaison à des informations de canal à temps corrigé d'une autre partie d'une autre série temporelle d'informations de canal dans une autre fenêtre temporelle glissante, un calcul de score de similarité, une quantification vectorielle, une compression, un chiffrement, un codage, un stockage, une transmission, une représentation, une fusion, une combinaison, une division, une restriction à une bande de fréquences sélectionnée, un repliement de spectre, un calcul de moyenne, un calcul de moyenne pondéré, un calcul de moyenne sur une fréquence sélectionnée, un calcul de moyenne sur des coefficients de domaine temporel sélectionnés, un calcul de moyenne sur des liaisons d'antenne, un calcul de moyenne pondéré sur ladite au moins une série temporelle TSCI, et un calcul de moyenne pondéré sur les C coefficients de domaine temporel conservés ;

dans lequel le calcul de moyenne sur des liaisons d'antenne est un calcul de moyenne pondéré sur ladite au moins une série temporelle TSCI ; et

dans lequel le calcul de moyenne sur des coefficients de domaine temporel sélectionnés est un calcul de moyenne pondéré sur les C coefficients de domaine temporel conservés.

5. Système selon l'une quelconque des revendications précédentes, dans lequel l'estimateur de signes vitaux (3610) comprend en outre un détecteur de crête configuré de manière à :

identifier au moins un maximum local et au moins un minimum local dans le domaine fréquentiel ;

calculer au moins un paramètre de type « rapport signal sur bruit » (type SNR) local pour chaque paire d'un maximum local et d'un minimum local mutuellement adjacents ;

identifier des crêtes locales significatives, représentant chacune au moins l'un parmi : un maximum local avec un paramètre de type SNR supérieur à un premier seuil T1 et un maximum local avec une amplitude supérieure à un seuil T2.

**6.** Système selon l'une quelconque des revendications précédentes, dans lequel l'estimateur de signes vitaux (3610) comprend en outre un estimateur de taux de mouvement périodique configuré de manière à :

sélectionner un ensemble de crêtes locales significatives sélectionnées, à partir d'un ensemble de crêtes locales significatives identifiées sur la base d'un critère de sélection ;

dans lequel la caractéristique actuelle connexe au mouvement pseudo-périodique de l'objet est calculée sur la base de l'ensemble de crêtes locales significatives sélectionnées et des valeurs de fréquence associées à l'ensemble de crêtes locales significatives sélectionnées.

**7.** Système selon l'une quelconque des revendications précédentes, dans lequel l'estimateur de signes vitaux (3610) comprend en outre un estimateur de taux de mouvement périodique configuré de manière à :

calculer des informations associées à un spectre restant avec un ensemble de crêtes locales significatives sélectionnées supprimées ; et

détecter un événement associé à la caractéristique actuelle connexe au mouvement pseudo-périodique de l'objet, sur la base d'au moins l'un des éléments parmi : une énergie spectrale restante du spectre restant, un seuil adaptatif et une machine à états finis, dans lequel l'événement comprend au moins l'un des éléments parmi : une présence, une absence, une apparition, une disparition, un comportement stable et un comportement non stable d'au moins l'un des éléments parmi : un mouvement non périodique, un mouvement transitoire, un mouvement fort, un mouvement faible ; un brouillage d'arrière-plan fort et un autre mouvement périodique.

**8.** Système selon l'une quelconque des revendications précédentes, dans lequel un contrôleur d'état système (3620) est configuré de manière à :

entrer dans un état d'initialisation (INIT) d'une machine à états finis (FSM) du système ;

dans l'état d'initialisation de la machine FSM :

calculer un nombre de seuils de manière adaptative d'une première façon ;

détecter l'événement sur la base du nombre de seuils et d'au moins l'un des éléments parmi : un ensemble de crêtes locales significatives sélectionnées et des caractéristiques associées ;

détecter un mouvement parasite d'arrière-plan excessif sur la base du nombre de seuils et d'une énergie spectrale restante ;

rester dans l'état « INIT » lorsque l'événement est conclu comme étant « non détecté » et que le mouvement parasite d'arrière-plan excessif est conclu comme étant « non détecté » ;

passer de l'état « INIT » à un état « Vérification » de la machine FSM lorsque l'événement est conclu comme étant « détecté » de manière préliminaire, et que l'événement détecté doit être vérifié ; et

passer de l'état « INIT » à un état « Mouvement » de la machine FSM lorsque le mouvement parasite d'arrière-plan excessif est conclu comme étant « détecté » ;

dans l'état « Mouvement » de la machine FSM :

calculer le nombre de seuils de manière adaptative d'une quatrième façon ;

détecter le mouvement parasite d'arrière-plan excessif sur la base du nombre de seuils et d'une énergie spectrale restante ;

rester dans l'état « Mouvement » lorsque le mouvement parasite d'arrière-plan excessif est conclu comme étant « détecté » ; et

passer de l'état « Mouvement » à l'état « INIT » lorsque le mouvement parasite d'arrière-plan excessif est conclu comme étant « non détecté » pour un nombre d'instances temporelles.

**9.** Système selon l'une quelconque des revendications précédentes, dans lequel un contrôleur d'état système (3620) est en outre configuré de manière à :

dans l'état « Vérification » de la machine FSM :

calculer un nombre de seuils de manière adaptative d'une deuxième façon ;

accumuler et calculer au moins une statistique sur la base d'ensembles de crêtes locales significatives sélectionnées et de caractéristiques connexes dans au moins une fenêtre temporelle glissante adjacente pour la vérification de l'événement détecté qui est détecté de manière préliminaire ;

rester dans l'état « Vérification » tandis que ladite au moins une statistique est accumulée jusqu'à ce que suffisamment de statistiques soient collectées pour la vérification ;

vérifier l'événement détecté de manière préliminaire sur la base du nombre de seuils et de ladite au moins un statistique ;

passer de l'état « Vérification » à un état « PeakFound » (crête trouvée) de la machine FSM lorsque l'événement détecté de manière préliminaire est conclu comme étant « vérifié » ;

passer de l'état « Vérification » à l'état « INIT » lorsque la vérification est conclue comme étant « non vérifiée » ; et

rester dans l'état « Vérification » lorsque la vérification n'est pas conclue ;

dans l'état « PeakFound » de la machine FSM :

calculer le nombre de seuils de manière adaptative d'une troisième façon ;

détecter l'événement vérifié sur la base du nombre de seuils et d'au moins l'un des éléments parmi : les ensembles de crêtes locales significatives sélectionnées et les caractéristiques connexes ;

détecter un mouvement parasite d'arrière-plan excessif sur la base du nombre de seuils et d'une énergie spectrale restante ;

rester dans l'état « PeakFound » lorsque l'événement vérifié est conclu comme étant « détecté » ;

passer de l'état « PeakFound » à l'état « INIT » lorsque l'événement vérifié est conclu comme étant « non détecté » pour un nombre d'instances temporelles ; et

passer de l'état « PeakFound » à l'état « Mouvement » lorsque le mouvement parasite d'arrière-plan excessif est conclu comme étant « détecté » pour un nombre d'instances temporelles.

10. Système selon l'une quelconque des revendications précédentes, comprenant en outre :

un émetteur supplémentaire situé à une troisième position dans le lieu et configuré de manière à transmettre un troisième signal sans fil à travers le canal à trajets multiples sans fil affecté par le mouvement pseudo-périodique de l'objet dans le lieu ;

un récepteur supplémentaire situé à une quatrième position dans le lieu et configuré de manière à :

recevoir un quatrième signal sans fil à travers le canal à trajets multiples sans fil, dans lequel le quatrième signal sans fil est déterminé sur la base, à la fois, du canal à trajets multiples sans fil et du troisième signal sans fil ; et

obtenir une série temporelle TSCI supplémentaire du canal à trajets multiples sans fil sur la base du quatrième signal sans fil ;

dans lequel l'estimateur de signes vitaux (3610) est en outre configuré de manière à :

déterminer qu'au moins une partie de la série temporelle TSCI supplémentaire dans la fenêtre temporelle glissante actuelle est associée au mouvement pseudo-périodique de l'objet dans le lieu ; et

calculer la caractéristique actuelle connexe au mouvement pseudo-périodique de l'objet dans la fenêtre temporelle glissante actuelle sur la base d'au moins l'un des éléments parmi :

ladite au moins une partie de la série temporelle TSCI supplémentaire dans la fenêtre temporelle glissante actuelle, au moins une partie de la série temporelle TSCI supplémentaire dans une fenêtre temporelle glissante passée supplémentaire, et une caractéristique passée connexe au mouvement pseudo-périodique de l'objet dans la fenêtre temporelle glissante passée supplémentaire.

11. Système selon l'une quelconque des revendications précédentes, dans lequel l'estimateur de signes vitaux (3610) est en outre configuré de manière à :

déterminer qu'au moins une partie de ladite au moins une série temporelle TSCI dans la fenêtre temporelle glissante actuelle est associée à un mouvement pseudo-périodique supplémentaire d'un objet supplémentaire dans le lieu, dans lequel le canal à trajets multiples sans fil est en outre affecté, dans la fenêtre temporelle glissante actuelle, par le mouvement pseudo-périodique supplémentaire de l'objet supplémentaire ; et

calculer une caractéristique actuelle connexe au mouvement pseudo-périodique supplémentaire de l'objet supplémentaire dans la fenêtre temporelle glissante actuelle sur la base d'au moins l'un des éléments parmi :

ladite au moins une partie de ladite au moins une série temporelle TSCI dans la fenêtre temporelle glissante actuelle, ladite au moins une partie de ladite au moins une série temporelle TSCI dans la fenêtre temporelle glissante passée, et une caractéristique passée connexe au mouvement pseudo-périodique supplémentaire de l'objet supplémentaire dans la fenêtre temporelle glissante passée.

**12.** Système selon l'une quelconque des revendications précédentes, dans lequel l'estimateur de signes vitaux (3610) est en outre configuré de manière à :

déterminer que le canal à trajets multiples sans fil est affecté, dans la fenêtre temporelle glissante actuelle, par des mouvements pseudo-périodiques d'une pluralité d'objets ;

calculer des caractéristiques actuelles connexes à des mouvements pseudo-périodiques de la pluralité d'objets dans la fenêtre temporelle glissante actuelle sur la base d'au moins l'un des éléments parmi : ladite au moins une partie de ladite au moins une série temporelle TSCI dans la fenêtre temporelle glissante actuelle, ladite au moins une partie de ladite au moins une série temporelle TSCI dans la fenêtre temporelle glissante passée, et une caractéristique passée connexe aux mouvements pseudo-périodiques de la pluralité d'objets dans la fenêtre temporelle glissante passée ; et

estimer une quantité de la pluralité d'objets sur la base de la caractéristique actuelle.

**13.** Procédé mis en œuvre par un système selon l'une quelconque des revendications précédentes.

**14.** Récepteur (2614) d'un système selon l'une quelconque des revendications précédentes, comprenant l'estimateur de signes vitaux (3610) du système.

FIG. 1

**FIG. 2**

**FIG. 3**

Channel state information (CSI)/
channel information (CI) collection

↓

CSI/CI preprocessing

↓

Enhancement of breathing signal

↓

Breathing feature extraction

↓

Breathing rate estimation

↓

Breathing rate output

**FIG. 4**

EP 4 233 709 B1

```
┌─────────────────────────┐
│          Start          │
└─────────────────────────┘
             │
             ▼
┌─────────────────────────┐
│     CFR Acquisition     │╲╱ 502 ──────────────────┐
└─────────────────────────┘                         │
             │                                       ▼
             ▼                          ┌─────────────────────────────┐
┌─────────────────────────┐            │  Averaging over Channel Taps │╲╱ 516
│     CFR Sanitization    │╲╱ 504       └─────────────────────────────┘
└─────────────────────────┘                         │
             │                                       ▼
             ▼                          ┌─────────────────────────────┐
┌─────────────────────────┐            │  Averaging over Antenna Links│╲╱ 518
│    CFR Normalization    │╲╱ 506       └─────────────────────────────┘
└─────────────────────────┘                         │
             │                                       ▼
             ▼                          ┌─────────────────────────────┐
┌─────────────────────────┐            │   Breathing Rate Estimation │╲╱ 520
│    IFFT on Subcarriers  │╲╱ 508       └─────────────────────────────┘
└─────────────────────────┘                         │
             │                                       ▼
             ▼                          ┌─────────────────────────────┐
┌─────────────────────────┐            │       Peak Detection        │╲╱ 522
│   Timestamp Correction  │╲╱ 510       └─────────────────────────────┘
└─────────────────────────┘                         │
             │                                       ▼
             ▼                          ┌─────────────────────────────┐
┌─────────────────────────┐            │     Real-time Updating      │╲╱ 524
│    Bandpass Filtering   │╲╱ 512       └─────────────────────────────┘
└─────────────────────────┘                         │
             │                                       ▼
             ▼                          ┌─────────────────────────────┐
┌─────────────────────────┐            │             End             │
│    Spectral Analysis    │╲╱ 514       └─────────────────────────────┘
└─────────────────────────┘
             │
             └───────────────────────────────────────┘
```

**FIG. 5**

FIG. 6

FIG. 8

FIG. 7A

FIG. 7B

EP 4 233 709 B1

**FIG. 9**

**FIG. 10**

FIG. 11

**FIG. 12**

(a)

(b)

**FIG. 13**

**FIG. 14**

**FIG. 15**

FIG. 16

FIG. 17

Obtain at least one time series of channel information of a wireless multipath channel impacted by a current movement of an object in a venue using a processor, a memory and a set of instructions

1802

Determine a spatial-temporal information of the object based on at least one of: the at least one time series of channel information extracted from a wireless signal transmitted between a Type 1 heterogeneous wireless device at a first position in the venue and a Type 2 heterogeneous wireless device at a second position in the venue, a time parameter associated with the current movement, and a past spatial-temporal information of the object

1804

Perform a task based on the spatial-temporal information

1806

FIG. 18

EP 4 233 709 B1

FIG. 19

EP 4 233 709 B1

Spatial-temporal information
1902

Location of
the object

Navigation for
the object

Object
tracking

Obstacle avoidance
for the object

Detecting motion
of the object

Tracking activity
of the object

Tracking behavior
of the object

Object
identification

User
identification

Detecting a vital
sign of the object

Detecting a periodic motion
associated with the object

Detecting breathing
of the object

Detecting an event
associated with the object

Detecting a fall-down
event of the object

Presenting the spatial-
temporal information

Graphical display of spatial-
temporal information

Task 1904

**FIG. 20**

```
┌─────────────────────────┐
│   Time series of channel │
│  information 111A/111B   │
└─────────────────────────┘
             │
             ▼
   ┌──────────────────┐
   │ Determine distance│
   │       2102        │
   └──────────────────┘
             │
             ▼
┌──────────────────┐      ┌──────────────────┐
│ Distance of current│     │ Estimated direction of│
│  movement of the  │     │ current movement of │
│   object 2104     │     │   the object 2106   │
└──────────────────┘      └──────────────────┘
             │                       │
             ▼                       ▼
┌────────────────────────────────────────────┐
│ Determine spatial-temporal information 1804 │
└────────────────────────────────────────────┘
                    │
                    ▼
┌────────────────────────────────────────────┐
│     Spatial-temporal information 1902       │
└────────────────────────────────────────────┘
```

**FIG. 21**

**FIG. 22**

**FIG. 23**

EP 4 233 709 B1

**FIG. 24**

CRF concatenation of Antenna 2 and 3 at time $t_1$, $t_1 + \Delta t, ..., t_1 + (N-1)\Delta t$: $H_{lead}(t_1)$ — 2402

↓

CRF concatenation of Antenna 1 and 2 at time $t_2$, $t_2 + \Delta t, ..., t_2 + (N-1)\Delta t$: $H_{follow}(t_2)$ — 2404

↓

Compute the TRRS between $H_{lead}(t_1)$ and $H_{follow}(t_2)$: $\gamma(t_1, t_2)$ — 2406

↓

Find $t_2 = \arg \max \gamma(t_1, t_2)$, and obtain $\tau = t_2 - t_1$ — 2408

↓

Speed is estimated as $v = d/\tau$ — 2410

**FIG. 24**

---

2500

Receive a wireless signal through a wireless multipath channel impacted by a motion of an object — 2502

↓

Obtain a time series of channel information (CI) that has at least one component of the channel based on the wireless signal — 2504

↓

Determine asynchronously a respective current component window of the component based on a current time window — 2506

↓

Determine asynchronously a respective past component window of the component based on a past time window — 2508

↓

Compare component-wise the respective current component window with the respective past component window asynchronously to generate a comparison result — 2510

↓

Detect motion based on the asynchronous component-wise comparing — 2512

↓

Monitor component-wise the motion of the object based on the comparison results — 2514

**FIG. 25**

**FIG. 26**

**FIG. 27**

**FIG. 28**

Origin
2800

| | |
|---|---|
| Processor (2802) | Transmitter (2812) |
| Memory (2804) | Receiver (2814) |
| Synchronization Controller (2806) | Power Module (2808) |
| Channel Information Extractor (2820) | Motion Detector (2822) |

2810, 2850, 2830, 2840

---

2900

Receive a wireless signal through a wireless multipath channel impacted by a motion of an object — 2902

Obtain a time series of channel information (CI) of the channel based on the wireless signal — 2904

Determine at least one time window and/or at least one component of the time series of CI — 2906

Compute motion information related to the motion of the object based on the time series of CI — 2908

Detect the motion of the object based on the computed motion information — 2910

Estimate a location associated with the motion of the object — 2912

**FIG. 29**

Motion Detector 3000

Motion Information Determiner 3002

Motion Determiner 3006

Time Window Determiner 3004

3010

Motion Location Estimator 3008

FIG. 30

Obtain at least one time series of channel information of a wireless multipath channel impacted by a current movement of an object in a venue using a processor, a memory and a set of instructions 3202

Determine a spatial-temporal information of the object based on at least one of: the at least one time series of channel information extracted from a wireless signal transmitted between a Type 1 heterogeneous wireless device at a first position in the venue and a Type 2 heterogeneous wireless device at a second position in the venue, a time parameter associated with the current movement, and a past spatial-temporal information of the object 3204

Perform a task based on the spatial-temporal information 3206

FIG. 32

Algorithm: Motion Detection

Initialization: The most recent $T$ amplitudes for each subcarrier $f$:

$[G(f;1),\ldots, G(f;T)]$;

Output: The human motion status inside a venue;

1: for $f = 1:F$ do

2:     Motion statistic computation for each subcarrier $f$:

3:     $\bar{G}_{(1)}(f) \leftarrow \frac{1}{T-1}\sum_{t=1}^{T-1} G(f;t);\quad \bar{G}_{(2)}(f) \leftarrow \frac{1}{T-1}\sum_{t=2}^{T} G(f;t);$

4:     $\psi(f) \leftarrow \dfrac{\sum_{t=2}^{T}\big(G(f;t)-\bar{G}_{(1)}(f)\big)\big(G(f;t-1)-\bar{G}_{(2)}(f)\big)}{\big(\sum_{t=2}^{T}(G(f;t)-\bar{G}_{(1)}(f))^2\big)^{\frac{1}{2}}\big(\sum_{t=2}^{T}(G(f;t-1)-\bar{G}_{(2)}(f))^2\big)^{\frac{1}{2}}};$

5:     Motion detection for each subcarrier f:

6:     if $\psi(f) > \dfrac{-1+\gamma\sqrt{T-2}}{T-1}$ then

7:     $D(f) \leftarrow 1$;

8:     else

9:     $D(f) \leftarrow 0$;

10:     end if

11: end for

12: Final motion detection:

13: if $\frac{1}{F}\sum_{f=1}^{F} D(f) > 0.5$ then

14:     Motion is detected!

15: else

16:     No Motion is detected.

17: end if

## FIG. 31

FIG. 33

FIG. 35

**FIG. 34**

3600

Channel information ~ 3605

|
v

| Vital Sign Estimator | ~ 3610

↕

| System State Controller | ~ 3620

|
v

Vital sign estimation ~ 3625

**FIG. 36**

~ 3610

Vital Sign
Estimator

| Channel Information Processor | ~ 3710

|
v

| Spectral Analyzer | ~ 3720

|
v

| Energy Spectrum Normalizer | ~ 3730

| Peak Detector | ~ 3740

|
v

| Breathing Rate Estimator | ~ 3750

|
v

| Real-time Updater | ~ 3760

**FIG. 37**

3800

CFR Acquisition — 3802

↓

CFR Sanitization — 3804

↓

CFR Normalization — 3806

↓

IFFT on Subcarriers — 3808

↓

Timestamp Correction — 3810

↓

Bandpass Filtering — 3812

↓

Spectral Analysis — 3814

Averaging over Channel Taps — 3816

↓

Averaging over Antenna Links — 3818

↓

Peak Detection — 3820

↓

Breathing Rate Estimation — 3822

↓

Real-time Updating — 3824

**FIG. 38**

EP 4 233 709 B1

**FIG. 39**

**FIG. 40**

FIG. 42

FIG. 41

| Edge Index | Current State | Next State | Condition | Output |
|---|---|---|---|---|
| 1 | INIT | INIT | $P = 0$ | $\hat{b}[i] = 0$ |
| 2 | INIT | VER-Enter | $P > 0$ | $\hat{b}[i] = 0$ |
| 3 | VER-Enter | INIT | $(P = 0) \cup \left(\hat{b}[i] < \hat{b}[i-1] - \Delta b\right) \cup \left(\hat{b}[i] > \hat{b}[i-1] + \Delta b\right)$ | $\hat{b}[i] = 0$ |
| 4 | VER-Enter | VER-Enter | $(P > 0) \cap \left(\hat{b}[i-1] - \Delta b \le \hat{b}[i] \le \hat{b}[i-1] + \Delta b\right) \cap (cnt_1 < TH_1)$ | $\hat{b}[i] = 0$ |
| 5 | VER-Enter | PF | $(P > 0) \cap \left(\hat{b}[i-1] - \Delta b \le \hat{b}[i] \le \hat{b}[i-1] + \Delta b\right) \cap (cnt_1 == TH_1)$ | $\hat{b}[i] = \hat{b}[i]$ |
| 6 | PF | PF | $P > 0 \cap \left(\hat{b}[i-1] - \Delta b \le \hat{b}[i] \le \hat{b}[i-1] + \Delta b\right)$ | $\hat{b}[i] = \hat{b}[i]$ |
| 7 | VER-Exit | PF | $P > 0 \cap \left(\tilde{b} - \Delta b \le \hat{b}[i] \le \tilde{b} + \Delta b\right)$ | $\hat{b}[i] = \tilde{b}$ |
| 8 | PF | VER-Exit | $P = 0 \cup \left(\hat{b}[i] < \hat{b}[i-1] - \Delta b\right) \cup \left(\hat{b}[i] > \hat{b}[i-1] + \Delta b\right)$ | $\hat{b}[i] = \tilde{b}$ |
| 9 | VER-Exit | VER-Exit | $!(Edge\ 7) \cap (cnt_2 < TH_2)$ | $\hat{b}[i] = \tilde{b}$ |
| 10 | VER-Exit | INIT | $!(Edge\ 7) \cap (cnt_2 == TH_2)$ | $\hat{b}[i] = 0$ |

4300

**FIG. 43**

4400

1302
1304

**FIG. 44**

4500

**FIG. 45**

4600

**FIG. 46**

EP 4 233 709 B1

4700

Receive a wireless signal through a wireless multipath channel impacted by pseudo-periodic motion of an object in a venue ⟶ 4702

Obtain at least one time series of channel information (TSCI) of the channel based on the wireless signal ⟶ 4704

Determine at least one portion of the at least one TSCI in a current sliding time window is associated with the pseudo-periodic motion of the object ⟶ 4706

Compute a current characteristics related to the pseudo-periodic motion of the object in the current sliding time window ⟶ 4708

**FIG. 47**

4900

Channel state information (CSI)/ channel information (CI) collection ⟶ 4902

CSI/CI preprocessing ⟶ 4904

Enhancement of breathing signal ⟶ 4906

Breathing feature extraction ⟶ 4908

Breathing rate estimation ⟶ 4910

**FIG. 49**

EP 4 233 709 B1

Channel information ∿ 4805

∿ 4800

Repeating
Motion Monitor

Channel Information
Collector ∿ 4810

Channel Information
Processor ∿ 4820

Breathing Feature
Extractor ∿ 4840

Breathing Signal
Enhancer ∿ 4830

Breathing Rate
Estimator ∿ 4850

Breathing rate ∿ 4855
estimation

**FIG. 48**

EP 4 233 709 B1

FIG. 50

FIG. 51

**FIG. 52**

Combined ACF breathing signal — 5310

Estimation of noise power — 5330

Spectrum Estimation — 5320

Peak detection — 5340

Is there any additional peak? — 5350

No → end — 5370

Yes

Breathing rate is detected
Num of people ← Num of people+1 — 5360

**FIG. 53**

Processor — 5402

Memory — 5404

Synchronization Controller — 5406

Operation Module — 5409

5410

Transmitter — 5412

Receiver — 5414

Power Module — 5408

5450

5400

5440

5430

**FIG. 54**

**FIG. 55**

**FIG. 56**

FIG. 57B

FIG. 57A

FIG. 58B

FIG. 58A

**FIG. 59**

FIG. 60

FIG. 61

**FIG. 62A**

**FIG. 62B**

**FIG. 62C**

**FIG. 62D**

**EP 4 233 709 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017180698 A